# EUROPEAN PATENT SPECIFICATION

(11) **EP 1 147 095 B1**
(45) Date of publication and mention of the grant of the patent: **11.08.2004**
(21) Application number: 00909990.4
(22) Date of filing: 27.01.2000
(51) Int. Cl.: C07D 243/14, C07D 401/06, C07D 405/06, C07D 409/06, C07D 521/00, A61K 31/5513, A61P 35/00, A61P 19/02

(54) **2, 3, 4, 5-TETRAHYDRO-1H-[1, 4] BENZODIAZEPINE-3-HYDROXAMIC ACIDS AS MATRIX METALLOPROTEINASE INHIBITORS**
2,3,4,5-TETRAHYDRO-1H-[1,4]BENZODIAZEPIN-3-HYDROXAMSÄURE ALS MATRIX METALLOPROTEINASEINHIBITOREN
ACIDES 2,3,4,5-TETRAHYDRO-1H- 1,4]BENZODIAZEPINE-3-HYDROXAMIQUES UTILISES COMME INHIBITEURS DES METALLOPROTEINASES MATRICIELLES

(30) Priority: 27.01.1999 US 239080
(43) Date of publication of application: 24.10.2001
(73) Proprietor: Wyeth Holdings Corporation, Madison, NJ 07940 (US)
(72) Inventor: ALBRIGHT, Jay, Donald, Nanuet, NY 10954 (US); DELOS SANTOS, Efren, Guillermo, Nanuet, NY 10954 (US); LEVIN, Jeremy, Ian, New City, NY 10956 (US); CHEN, James, Ming, Bedminster, NJ 07921 (US)
(74) Representative: Wileman, David Francis Dr.
(86) International application number: PCT/US2000/001991
(87) International publication number: WO 2000/044730

(56) References cited:
- WO-A-96/33172
- WO-A-97/18194
- WO-A-97/20824
- WO-A-98/08822
- WO-A-98/08823
- WO-A-98/08825
- WO-A-98/08827
- WO-A-99/37625
- US-A- 5 455 258
- US-A- 5 753 653

## Description

### FIELD OF INVENTION

This invention relates to 4-(4-substituted-benzenesulfonyl)-2,3,4,5-tetrahydro-1H-[1,4]benzodiazepine-3-hydroxamic acids which act as matrix metalloproteinase inhibitors and as inhibitors of TNF-α converting enzyme(TACE). The compounds of the present invention are useful in disease conditions mediated by matrix metalloproteinases and TACE, such as tumor growth, osteoarthritis, rheumatoid arthritis and degenerative cartilage loss.

### BACKGROUND OF THE INVENTION

Matrix metalloproteinases (MMPs) are a group of enzymes that have been implicated in the pathological destruction of connective tissue and basement membranes. These zinc-containing endopeptidases consist of several subsets of enzymes, including collagenases, stromelysins and gelatinases. Of these, the gelatinases have been shown to be the MMPs most intimately involved with the growth and spread of tumors.

For example, it is known that the level of expression of gelatinase is elevated in malignancies, and that gelatinase can degrade the basement membrane which leads to tumor metastasis. Angiogenesis, required for the growth of solid tumors, has also recently been shown to have a gelatinase component to its pathology as reported in "Matrix Metalloproteinases, Novel Targets for Directed Cancer Therapy", Drugs and Aging, 11:229-244 (1997).

Other conditions mediated by MMPs include restenosis, MMP-mediated osteopenias, inflammatory diseases of the central nervous system, skin aging, osteoarthritis, rheumatoid arthritis, septic arthritis, corneal ulceration, abnormal wound healing, bone disease, proteinuria, aneurysmal aortic disease, degenerative cartilage loss following traumatic joint injury, demyelinating diseases of the nervous system, cirrhosis of the liver, glomerular disease of the kidney, premature rupture of fetal membranes, inflammatory bowel disease, periodontal disease, age-related macular degeneration, diabetic retinopathy, proliferative vitreoretinopathy, retinopathy of prematurity, ocular inflammation, keratoconus, Sjogren's syndrome, myopia, ocular tumors, ocular angiogenesis/ neo-vascularization and corneal graft rejection. Studies relating to these conditions are set forth, e.g., in "Recent Advances in Matrix Metalloproteinase Inhibitor Research", R. P. Beckett et al., Research Focus, 1:16-26, (1996); Curr. Opin. Ther. Patents, 4(1): 7-16, (1994); Curr. Medicinal Chem., 2: 743-762, (1995); Exp. Opin. Ther. Patents, 5(2): 1087-110, (1995); Exp. Opin. Ther. Patents, 5(12): 1287-1196, (1995); "Inhibition of Matrix Metalloproteinases: Structure Based Design", Current Pharmaceutical Design, 2:524-661, (1996). "Matrix Metalloproteinase Inhibitor Drugs", Emerging Drugs, 2:205-230 (1997).

TNF-α converting enzyme (TACE) catalyzes the formation of TNF-α from membrane bound TNF-α precursor protein. TNF-α is a pro-inflammatory cytokine that is believed to have a role in rheumatoid arthritis, septic shock, graft rejection, cachexia, anorexia, inflammation, congestive heart failure, inflammatory disease of the central nervous system, inflammatory bowel disease, insulin resistance and HIV infection, in addition to its well-documented antitumor properties. Research with anti-TNF-α antibodies in transgenic animals has demonstrated that blocking the formation of TNF-α inhibits the progression of arthritis. This observation has recently been extended to humans as described in "TNF-α in Human Diseases", Current Pharmaceutical Design, 2:662-667 (1996).

It is expected that small molecule inhibitors of MMPs and TACE would have the potential for treating a variety of disease states. Although a variety of MMP and TACE inhibitors are known, many of these molecules are peptidic and peptide-like which demonstrate bioavailability and pharmacokinetic problems. Long acting, orally bioavailable non-peptide inhibitors of MMPs and/or TACE would thus be highly desirable for the treatment of the disease states discussed above.

U.S. Patent No, 5,455,258 discloses 2-substituted-2-(arylsulfonylamino) hydroxyamic acids and their use as MMP inhibitors. WO 97/18194, discloses N-(arylsulfonyl)tetrahydroisoquinolone-hydroxamic acids and related bicyclic derivatives thereof and their use as MMP inhibitors. WO 97/20824 and U.S. Patent 5,753,653 disclose 1-(arylsulfonyl)-4-(substituted)piperazine-2-hydroxamic acids, 4-(arylsulfonyl)morpholine-3-hydroxamic acids, 4-(arylsulfonyl)-tetrahydro-2H,1,4-thiazine-3-hydroxamic acids, 3-(substituted-1-(arylsulfonyl)hexahydro-2-hydroxamic acids and related compounds as useful MMP inhibitors.

WO 98/08822, WO 98/08823 and WO 98/08825, disclose 6-membered 1-(arylsnlfonyl)hexahydropyrimidine-2-hydroxamic acids, 1-substituted-3-[(4-methoxybenzenesulfonyl)]hexahydropyrimidine-4-hydroxamic acids, 4-(arylsulfonyl)-tetrahydro-1,2-thiazine-3-hydroxamic acids and (arylsulfonyl)-4-substitutedpiperazine-2-hydroxamic acids. WO 98/08827 discloses 4-(arylsulfonyl)-hexahydrothiazepine-3-hydroxamic acids and 4-(arylsulfonyl)-hexadydro[1,4]-diazepine-3-hydroxamic acids.

### SUMMARY OF THE INVENTION

This invention relates to novel derivatives of substituted 2,3,4,5-tetrahydro-1H-[1,4]benzodiazepine-3-carboxylic acid, hydroxamide which exhibit inhibitory activity against MMPs. The compounds of the present invention are represented by the following formula 1 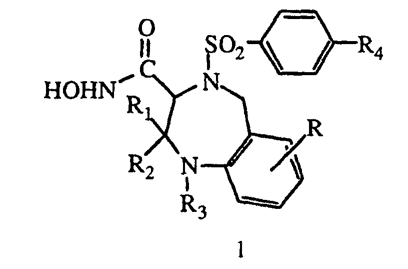 wherein
R is selected from hydrogen, (C₁-C₃)alkyl,-CN,-OR',-SR',-CF₃,-OCF₃, Cl, F, NH₂, NH(C₁-C₃)alkyl,-N(R')CO(C₁-C₃)alkyl,-N(R')(R'), NO₂,-CONH₂, -SO₂NH₂,-SO₂N(R')(R'), or-N(R')COCH₂O-(C₁-C₃)alkyl, wherein R' is (C₁-C₃) alkyl or hydrogen;
R₄ is (C₁-C₆) alkyl-O- containing one triple bond, eg C₂-C₆; 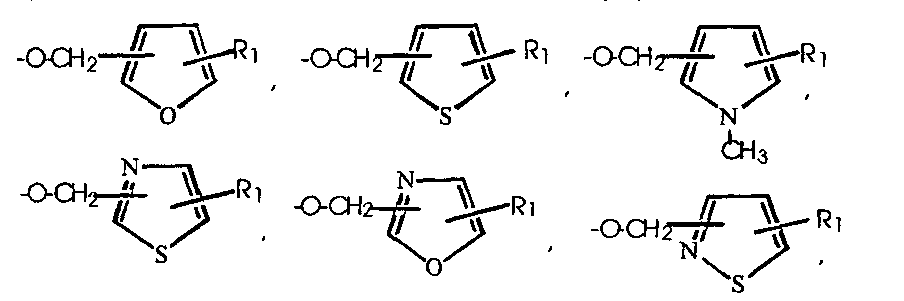 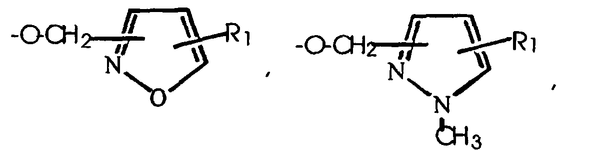 - O-CH₂- C ≡ C-R"
wherein R" is hydrogen, -CH₂OH, (C₁-C₆)alkyl, (C₁-C₆)alkyl-O-CH₂-, (C₁C₆)alkyl-S-CH₂-, (C₁-C₆)alkyl-NH-CH₂-, [(C₁-C₃)alkyl]₂-NCH₂-, (C₁-C₆)cycloalkyl-O-CH₂-, [(C₁-C₃)alkyl]₂-N-(CH₂)₂₋₄NHCH₂-, [(C₁-C₃)akyl]₂-N-(CH₂)₂₋₄N(CH₃)CH₂-, 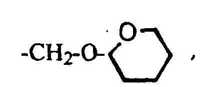 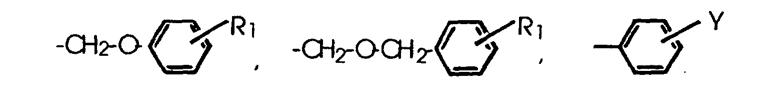 R₁ and R₂ are each, independently, hydrogen or CH₃;
R₃ is (C₁- C₈)alkyl, NH₂CH₂CO-, (C₁- C₆)alkylNHCH₂CO-, HO(CH₂)ₘCO-, HCO-, Aryl(CH₂)ₙCO-, Heteroaryl(CH₂)ₙCO-, (C₁- C₃)alkyl-O-(CH₂)ₙCO-, (C₁- C₃)alkylCO-, (C₁- C₃)alkylCO-NHCH₂CO-, (C₃- C₇)cycloalkylCO-, (C₁- C₃)alkylSO₂-, Aryl(CH₂)ₙSO₂-, Heteroaryl(CH₂)ₙSO₂-, (C₁- C₃)alkyl-O-(CH₂)ₘ-SO₂-, (C₁- C₃)alkyl-O-(CH₂)ₘ-, (C₁- C₃)alkyl-O-(C₁- C₃)alkyl-O-(C₁- C₃)alkyl, HO-(C₁- C₃)alkyl-O-(C₁- C₃)alkyl, Aryl-O-CH₂CO-, Heteroaryl-O-CH₂CO-, AryICH=CHCO-, HeteroarylCH=CHCO-, (C₁- C₃)alkylCH=CHCO-, 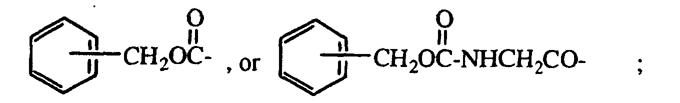 Aryl(C₁- C₃)alkyl, Heteroaryl(C₁- C₃)alkyl, ArylCH=CHCH₂-,
HeteroarylCH=CHCH₂-, (C₁- C₆)alkylCH=CHCH₂-, 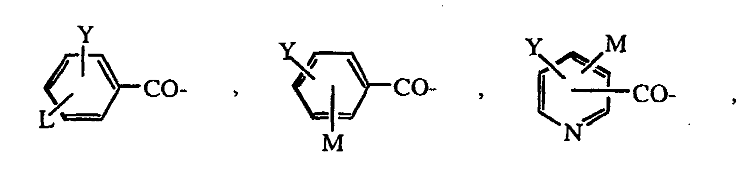 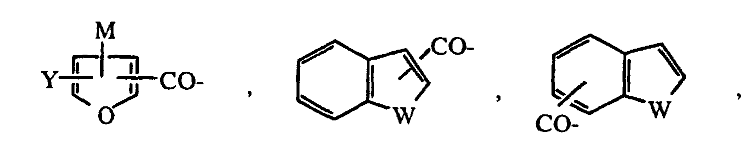 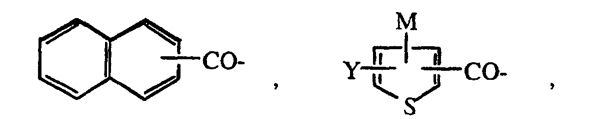 R'OCH₂ CH(OR')CO-, (R'OCH₂)₂C(R')CO-, 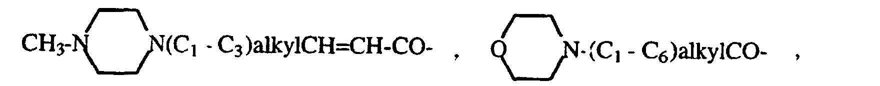 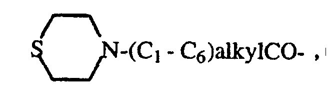 (C₁- C₃)alkylCONHCO-, 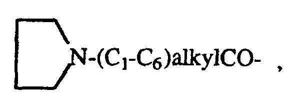 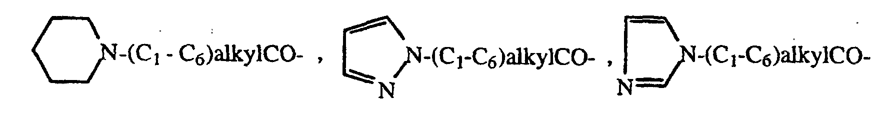 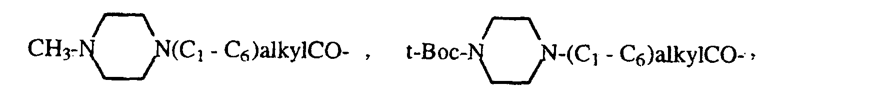 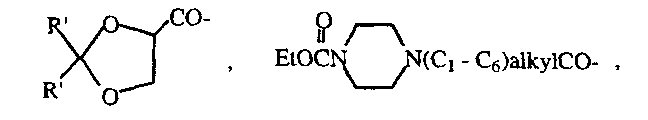 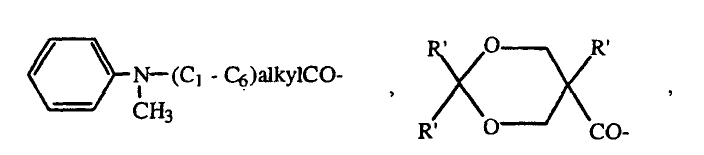 [(C₁- C₆)alkyl]₂-N-(C₁- C₆)alkyl CO-, or (C₁- C₆)alkyl-NH-(C₁- C₆)alkylCO-;
wherein
m = 1 to 3; n = 0 to 3;
Aryl is 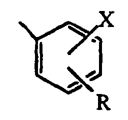 and
Heteroaryl is 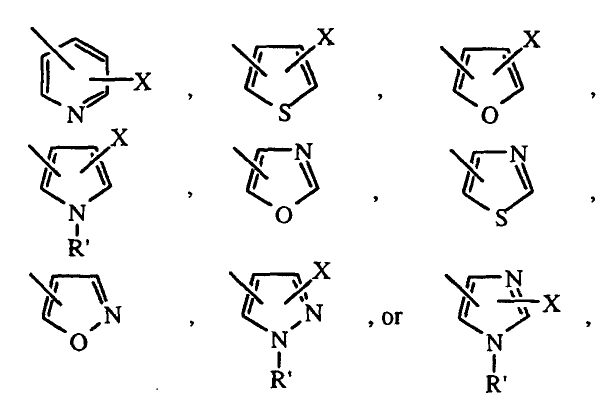 wherein X is hydrogen, halogen, (C₁- C₃) alkyl or-OCH₃ and R and R' are as defined above;
L is hydrogen, (C₁- C₃)alkyl,-CN,-OR',-SR',-CF₃,-OCF₃, Cl, F, NH₂,-NH-(C₁-C₃)alkyl,-N(R')CO(C₁- C₃)alkyl, N(R')(R'),-NO₂,-CONH₂,-SO₂NH₂,-SO₂N(R')(R'),-N(R')COCH₂O-(C₁- C₃)alkyl, 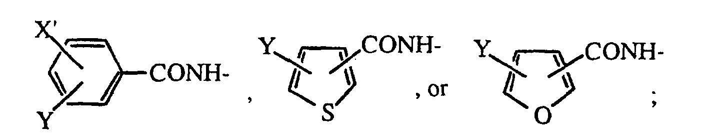 M is 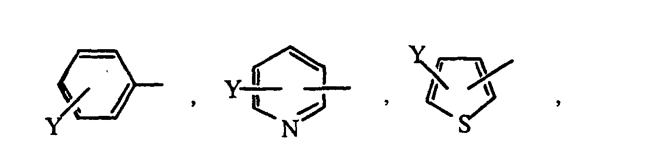 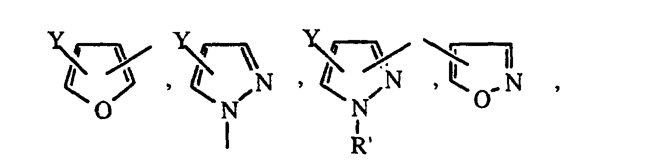 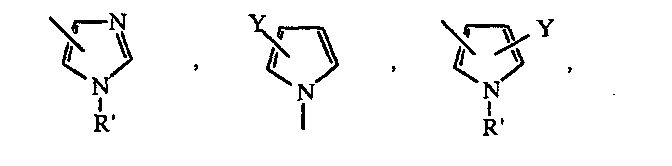 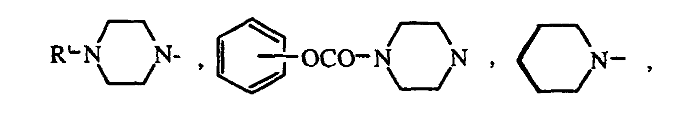 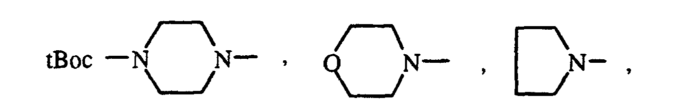 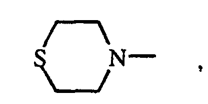 or N(R')(R') where R' is as defined above;
W is O, S, NH or N(C₁- C₃)alkyl;
Y is hydrogen, F, Cl, CF₃ or OCH₃; and X' is halogen, hydrogen, (C₁- C₃)alkyl, O-(C₁- C₃)alkyl, or-CH₂OH;
and pharmaceutically acceptable salts thereof.

### DETAILED DESCRIPTION OF THE INVENTION

Preferably, the compounds of the present invention are those of formula I
wherein R is hydrogen, (C₁- C₃) alkyl,-CN,-OR',-SR',-CF₃, -OCF₃, Cl, F, NH₂, NH(C₁- C₃)alkyl,-N(R')CO(C₁- C₃)alkyl,-N(R')(R'), NO₂, - CONH₂,-SO₂NH₂,-SO₂N(R')(R'), or-N(R')COCH₂O-(C₁- C₃)alkyl, wherein R' is (C₁- C₃) alkyl or hydrogen;
R₄ is (C₁- C₆) alkyl-O- containing one triple bond, 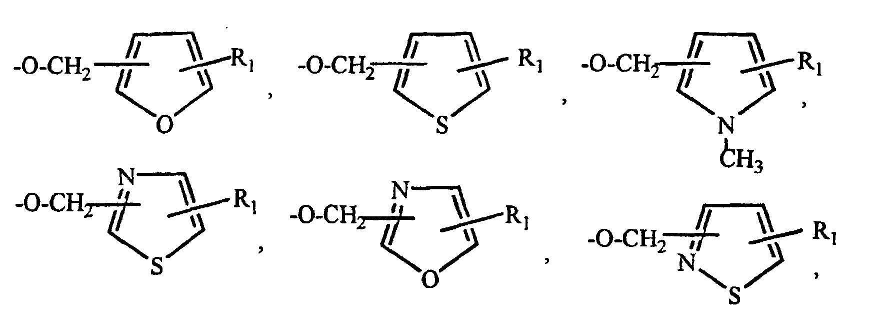 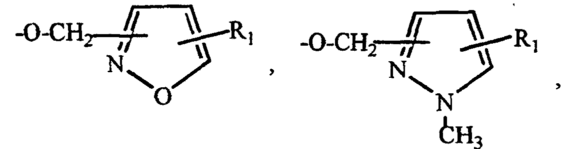 - O-CH₂- C ≡ C-R"
wherein R" is hydrogen, -CH₂OH, (C₁-C₆)alkyl, (C₁-C₆)alkyl-O-CH₂-, (C₁-C₆)alkyl-S-CH₂-, (C₁-C₆)alkyl-NH-CH₂-, [(C₁-C₃)alkyl]₂-NCH₂-, (C₁-C₆)cycloalkyl-O-CH₂-, [(C₁-C₃)alkyl]₂-N-(CH₂)₂₋₄NHCH₂-, [(C₁-C₃) alkyl]₂-N-(CH₂)₂₋₄N(CH₃)CH₂-, 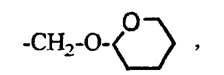 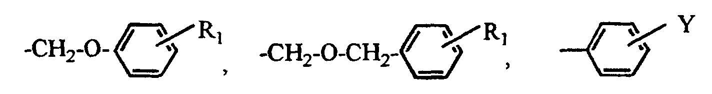 R₁ and R₂ are each, independently, hydrogen or CH₃;
R₃ is (C₁-C₈)alkyl, NH₂CH₂CO-, (C₁-C₆)alkylNHCH₂CO- HO(CH₂)ₘCO-, HCO-, Aryl(CH₂)ₙCO-, Heteroaryl(CH₂)ₙCO-, (C₁-C₃)alkyl-O-(CH₂)ₙCO-, (C₁-C₃)alkylCO-, (C₁- C₃)alkylCO-NHCH₂CO-, (C₃-C₇)cycloalkylCO-, Aryl-O-CH₂CO-, HeteroarylOCH₂CO-, 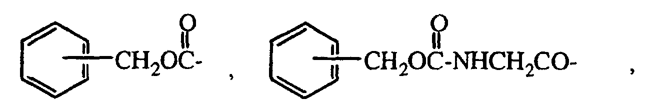 wherein
m = 1 to 3; n = 0 to 3;
Aryl is 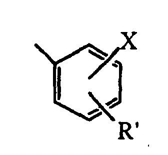 and
Heteroaryl is 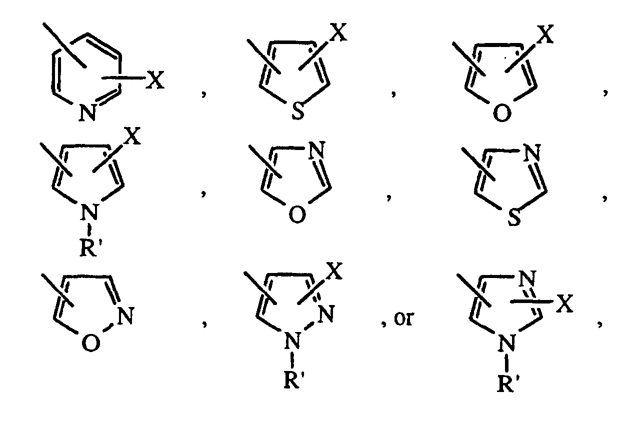 wherein X is hydrogen, halogen, (C₁- C₃) alkyl or-OCH₃ wherein R and R' are as defined above; and pharmaceutically acceptable salts thereof.

It is more preferred that the compounds of the present invention include those of formula 1 wherein R is hydrogen, (C₁- C₃) alkyl,-CN,-OR', -SR', -CF₃, -OCF₃, Cl, F, NH₂, NH(C₁ - C₃)alkyl, -N(R')CO(C₁ - C₃)alkyl, -N(R')(R'), NO₂, -CONH₂, - SO₂NH₂, -SO₂N(R')(R'), or -N(R')COCH₂O-(C₁ - C₃)alkyl, wherein R' is (C₁ - C₃) alkyl or hydrogen;
R₄ is (C₁ - C₆) alkyl-O- containing one triple bond, 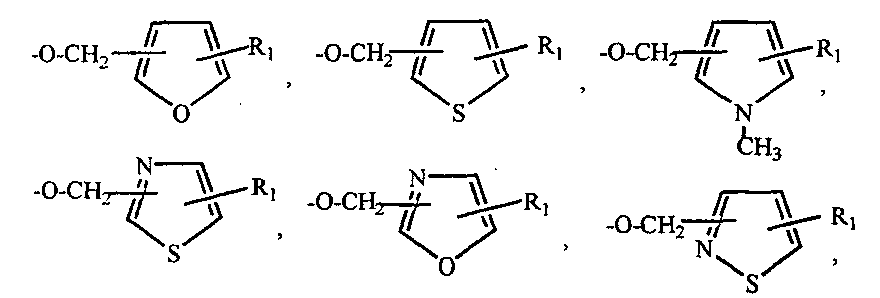 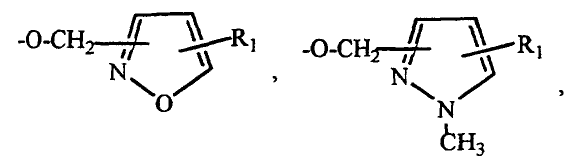 -O-CH₂-C≡C-R"
wherein R" is hydrogen, -CH₂OH, (C₁-C₆)alkyl, (C₁-C₆)alkyl-O-CH₂-, (C₁-C₆)alkyl-S-CH₂-, (C₁-C₆)alkyl-NH-CH₂-, [(C₁-C₃)alkyl]₂-NCH₂-, (C₁-C₆)cycloalkyl-O-CH₂-, [(C₁-C₃)alkyl]₂-N-(CH₂)₂₋₄NHCH₂-, [(C₁-C₃)alkyl]₂-N-(CH₂)₂₋₄N(CH₃)CH₂-, 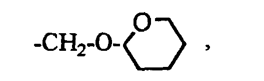 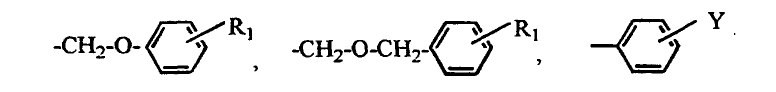 R₁ and R₂ are each, independently hydrogen or CH₃;
R₃ is (C₁ - C₃)alkylCO-, (C₁ - C₃)alkyl-O-(CH₂)ₘCO- ArylCO-
wherein
m = 1 to 3; n = 0 to 3;
Aryl is 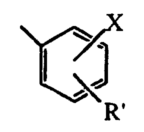 and
Heteroaryl is 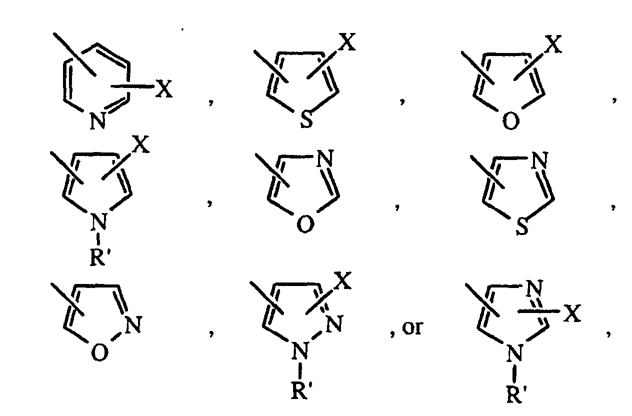 wherein X is hydrogen, halogen, (C₁- C₃) alkyl or -OCH₃ and R and R' are as defined above; and pharmaceutically acceptable salts thereof.

It is more preferred that the compounds of the present invention include those of formula 1 wherein R is hydrogen, (C₁-C₃) alkyl, -CN, -OR,-SR,-CF₃, OCF₃,Cl, F, NH₂, NH(C₁-C₃)alkyl,-N(R')CO(C₁-C₃)alkyl,-N(R')(R'), NO₂,-CONH₂, -SO₂NH₂,-SO₂ NH₂, SO₂N(R')(R') or-N(R') COCH₂O-(C₁- C₃) alkyl, wherein R' is (C₁- C₃)alkyl or hydrogen; R₄ is (C₁-C₆)alkyl-O- containing one triple bond 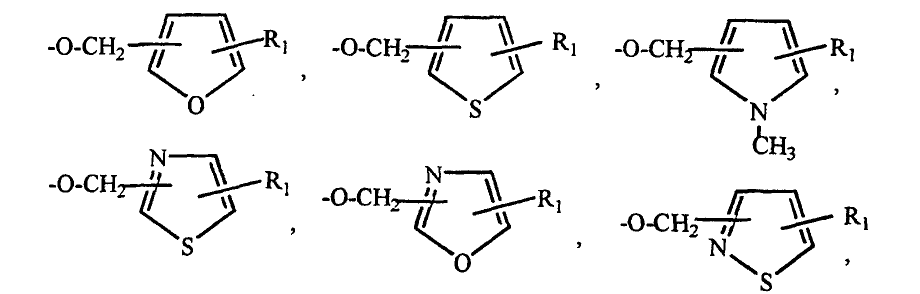 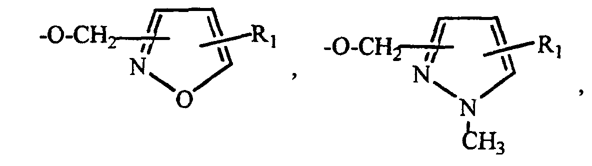 -O-CH₂-C≡C-R"
wherein R" is hydrogen, -CH₂OH, (C₁-C₆)alkyl, (C₁-C₆)alkyl-O-CH₂-, (C₁-C₆)alkyl-S-CH₂-, (C₁-C₆)alkyl-NH-CH₂-, [(C₁-C₃)alkyl]₂-NCH₂-, (C₁-C₆)cycloalkyl-O-CH₂-, [(C₁-C₃)alkyl]₂-N-(CH₂)₂₋₄NHCH₂-, [(C₁-C₃)alkyl]₂-N-(CH₂)₂₋₄N(CH₃)CH₂-, 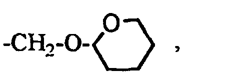 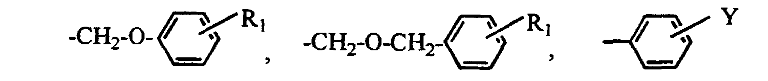 R₁ and R₂ are each, independently hydrogen or CH₃
R₃ is (C₁- C₃) alkyl SO₂- Aryl (CH₂)ₙSO₂-, Heteroary(CH₂)ₙSO₂-, or (C₁- C₃) alkyl-O-(CH₂)ₙSO₂-,
wherein
m=1 to 3; n=0 to 3;
Aryl is

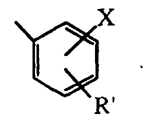

A further, more preferred embodiment of the present invention includes compounds represented by formula 1 wherein
R is selected from hydrogen, (C₁- C₃) alkyl,-CN,-OR',-SR',-CF₃, -OCF₃, Cl, F, NH₂, NH(C₁- C₃)alkyl,-N(R')CO(C₁- C₃)alkyl,-N(R')(R'), NO₂, -CONH₂,-SO₂NH₂,-SO₂N(R')(R'), or-N(R')COCH₂O-(C₁- C₃)alkyl, wherein R' is (C₁- C₃) alkyl or hydrogen;
R₄ is (C₁-C₆)alkyl-O- containing one triple bond, 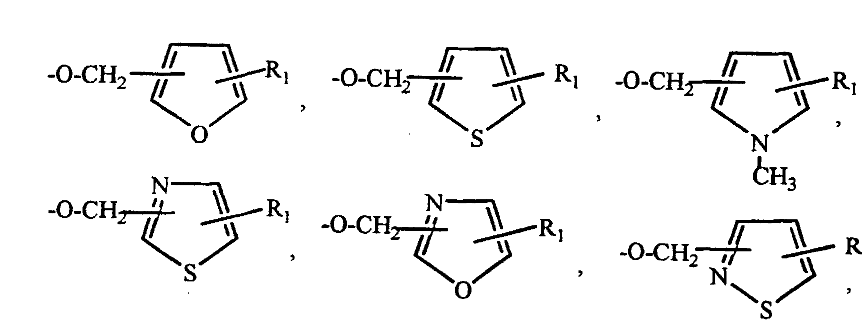 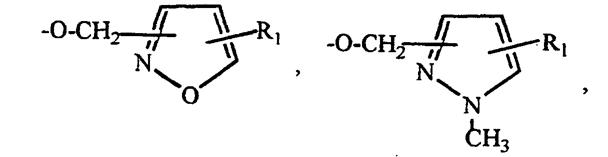 - O-CH₂-C≡C-R"
wherein R" is hydrogen, -CH₂OH, (C₁-C₆)alkyl, (C₁-C₆)alkyl-O-CH₂-, (C₁-C₆)alkyl-S-CH₂-, (C₁-C₆)alkyl-NH-CH₂-, [(C₁-C₃)alkyl]₂-NCH₂-, (C₁-C₆)cycloalkyl-O-CH₂-, [(C₁-C₃)alkyl]₂-N-(CH₂)₂₋₄NHCH₂-, [(C₁-C₃) alkyl]₂-N-(CH₂)₂₋₄N(CH₃)CH₂-, 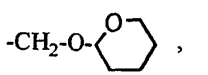 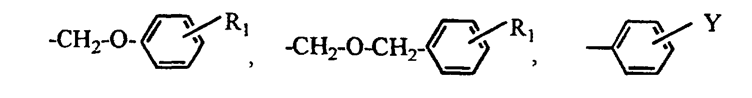 R₁ and R₂ are each, independently hydrogen or CH₃,
R₃ is (C₁- C₃) alkylCO-, (C₁- C₃)alkyl-CO-, (C₁-C₇)cycloalkyCO-, (C₁-C₃)alkyl -O-(CH₂)ₘ-CO-, Ar (CH₂)ₙCO-, HO-(CH₂)ₘCO-, Heteroaryl(CH₂)ₘ-CO-
wherein
Aryl is 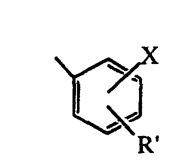 and
Heteroaryl is 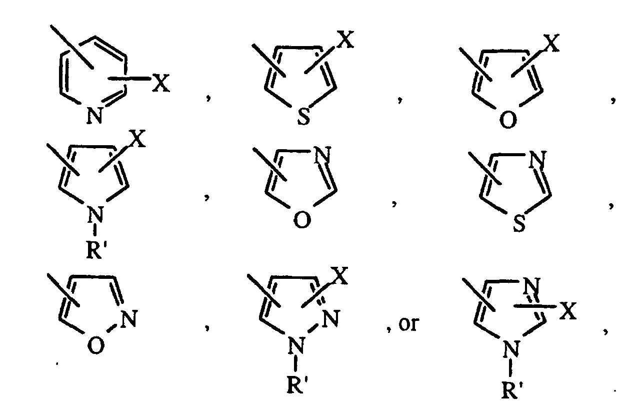 wherein X is hydrogen, halogen, (C₁- C₃) alkyl or-OCH₃ and R and R' are as defined above; and pharmaceutically acceptable salts thereof.

Additionally highly preferred compounds of the present invention include those of formula 1 wherein R is hydrogen, (C₁- C₃) alkyl,-CN,-OR',-SR',-CF₃, -OCF₃, Cl, F, NH₂, NH(C₁- C₃)alkyl,-N(R')CO(C₁- C₃)alkyl,-N(R')(R'), NO₂, -CONH₂,-SO₂NH₂,-SO₂N(R')(R'), or-N(R')COCH₂O-C₁- C₃)alkyl, wherein R' is (C₁- C₃) alkyl or hydrogen;
R₄ is-O-CH₂-C≡C-R";
wherein R" is hydrogen, -CH₂OH, (C₁-C₆)alkyl, (C₁-C₆)alkyl-O-CH₂-, (C₁-C₆)alkyl-S-CH₂-, (C₁-C₆)alkyl-NH-CH₂, [(C₁-C₃)alkyl]₂-NCH₂-, (C₁-C₆)cycloalkyl-O-CH₂-, [(C₁-C₃)alkyl]₂-N-(CH₂)₂₋₄NHCH₂-, [(C₁-C₃) alkyl]₂-N-(CH₂)₂₋₄N(CH₃)CH₂-, 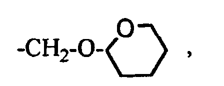 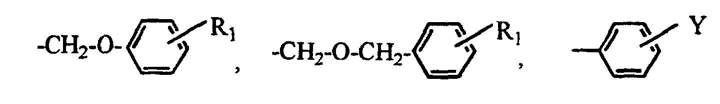 R₁ and R₂ are each, independently hydrogen or CH₃;
R₃ is 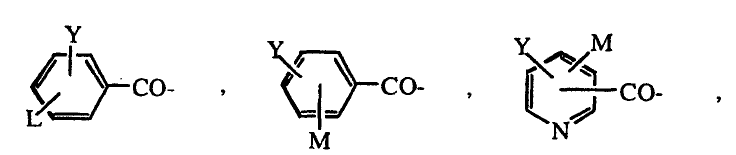 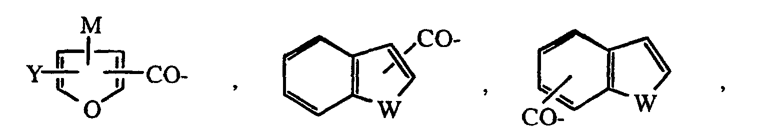 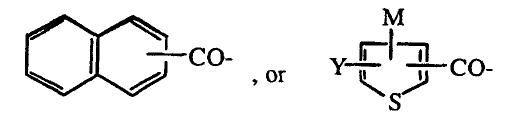 wherein
m=1 to 3;n=0 to 3;
L is hydrogen, (C₁- C₃)alkyl,-CN,-OR',-SR',-CF₃,-OCF₃, Cl, F, NH₂, -NH-(C₁- C₃)alkyl,-N(R')CO(C₁- C₃)alkyl, N(R')(R'),-NO₂,-CONH₂,-SO₂NH₂, - SO₂N(R')(R'),-N(R')COCH₂O-(C₁- C₃)alkyl, 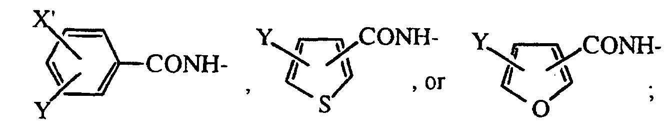 M is 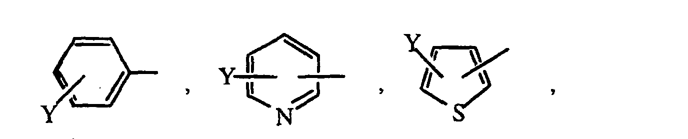 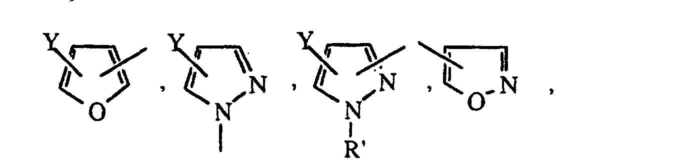 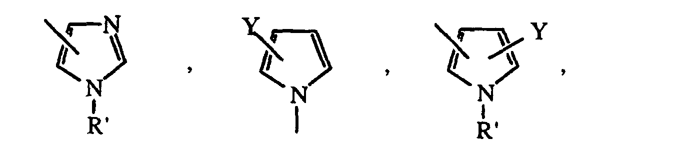 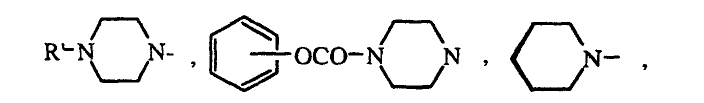 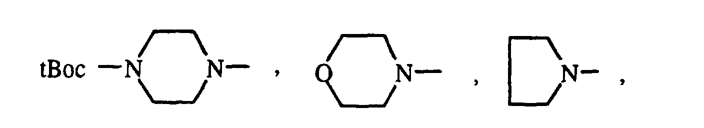 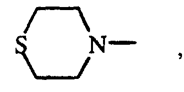 or N(R')(R') where R' is as defined above;
W is O, S, NH or N(C₁- C₃)alkyl;
Y is hydrogen, F, Cl, CF₃ or OCH₃; and X' is halogen, hydrogen, (C₁- C₃)alkyl, O-(C₁- C₃)alkyl, or-CH₂OH; and pharmaceutically acceptable salts thereof.

Accordingly this invention provides a process for preparing compounds of formula 1, as defined above, which comprises one of the following:
a) reacting a compound of formula II: 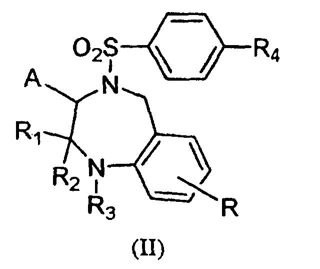 wherein R, R₁, R₂, R₃, and R₄ are defined above, and A is COOH or a reactive derivative thereof, with a compound of formula III

   NH₂OH (III)

   to give a corresponding compound of formula I;
b) resolving a mixture (e.g. racemate) of optically active isomers of a compound of formula I to isolate one enantiomer or diastereomer substantially free of the other enantiomer or diastereomers;
c) acidifying a basic compound of formula I with a pharmaceutically acceptable acid to give a pharmaceutically acceptable salt.

With regards to process a) the reaction can be carried out by processes known in the art e.g. by reaction with the acid chloride to form a reactive derivative before reaction with the hydroxylamine.

With regard to process b) standard separation techniques may be used to isolate particular enantiomeric or diastereomeric forms. For example a racemic mixture may be converted to a mixture of optically active diastereoisomers by reaction with a single enantiomer of a 'resolving agent' (for example by diastereomeric salt formation or formation of a covalent bond). The resulting mixture of optically active diastereoisomers may be separated by standard techniques (e.g. crystallisation or chromatography) and individual optically active diastereoisomers then treated to remove the 'resolving agent' thereby releasing the single enantiomer of the compound of the invention. Chiral chromatography (using a chiral support, eluent or ion pairing agent) may also be used to separate enantiomeric mixtures directly.

The compounds of formula I may be isolated in the form of a salt of a pharmaceutically acceptable acid e.g. an organic or inorganic acid by treatment with an acid such as described above.

Some of the intermediate compounds for the preparation of derivatives of formula 1 are those wherein R₄ is OCH₃ and they may be advantageously prepared according to the illustrated Reaction Schemes. Ester derivatives of formulae 8, 14, 15, 20, 22, 23, 24, 25, 29 and 30 wherein R₄ is OCH₃ are used to prepare derivatives wherem R₄ is OH (via cleavage of O-CH₃ group). As illustrated in Scheme 8 derivatives 40 with a phenolic OH group are reacted under standard organic synthetic conditions to convert the OH moiety into the R₄ substituents (as previously defined). Variations in these schemes may be made to improve productivity without negatively impacting the amount and nature of the product, by means that will be recognized by those skilled in the art. For example, reactive groups may be blocked with suitable blocking moieties which may then be deblocked under standard conditions (for instance, hydroxy groups may be protected with trimethylsilyl or t-butyl-dimethylsilyl moieties which are then removed in a later reaction step). In addition, those skilled in the art will recognize that catalylic hydrogenation conditions are inappropiate for preparing intermediates with an R₄ moiety containing a triple bond; the R₄ moiety is then introduced into intermediates not requiring a reduction step.

In general, the compounds of Formula 1 are synthesized from an alkyl ester (such as methyl, ethyl, t-butyl and the like) of serine, threonine, or 3,3-dimethyl-3-hydroxypropionic acids. One reaction pathway is shown in Reaction Scheme 1. It is noted that methyl esters are shown in all of the Reaction Schemes, however, it is to be understood that the use of methyl esters is for purposes of illustration only, and other suitable alkyl esters. benzyl esters and the like may similarly be used.

In Reaction Scheme 1, serine, threonine, beta-hydroxyvaline and related derivatives are converted to the corresponding N-(4-substituted-benzenesulfonyl) derivatives 3 and alkylated with suitable substituted or unsubstituted 2-nitrobenzyl bromides or 2-nitrobenzyl chlorides to provide the corresponding nitro derivatives 5. Reduction under conventional reducing conditions, such as catalytic hydrogeneration (with Pd/C) or chemical reduction (e.g., with SnCl₂ or FeCl₃) results in amino derivatives 6. Reaction of the N-(2-aminobenzyl) derivatives 6 with alkanoyl chlorides, alkylsulfonyl chlorides, aroyl chlorides, heteroaroyl chlorides, aryl sulfonyl chlorides, heteroarylsulfonyl chlorides and the like, in the presence of trialkylamines or pyridene, provides the dehydroalanine derivatives 7. Ring closure to the [1,4]benzodiazepine compounds 9 is carried out by reaction with a mild base such as sodium or potassium bicarbonate in an alcohol solvent such as methanol or ethanol. Standard conditions which involve hydrolysis of the ester (NaOH), acid chloride formation and reaction of the acid chloride with hydroxylamine are then used to convert the ester derivatives 8 to the hydroxamic acids 9. Ester derivatives 8 (where the ester function is a t-butyl ester) are converted to the acid with trifluoroacetic acid under standard conditions.

As illustrated in Reaction Scheme 2, derivatives 10, which contain a blocked hydroxyl group, are alkylated with 2-nitro or 2-amino benzyl alcohol derivatives 11 by application of the Mitsunobu reaction to give intermediates 12. Reduction of the 2-nitro group and removal of the hydroxy blocking group with derivatives 12, where the R₄ group is a protected amino moiety with simultaneous deblocking of the amino and hydroxyl functions, gives intermediate compounds 13. The intermediates 13 may then be reacted with benzyloxycarbonyl chloride to give the closed ring [1,4] benzodiazepines 14. Reaction of the derivatives 14 with acyl chlorides, aroyl chlorides, heteroaroyl chlorides, alkysulfonyl chlorides, arylsulfonyl chlorides and heteroarylsulfonyl chlorides and the like affords the intermediates 15. 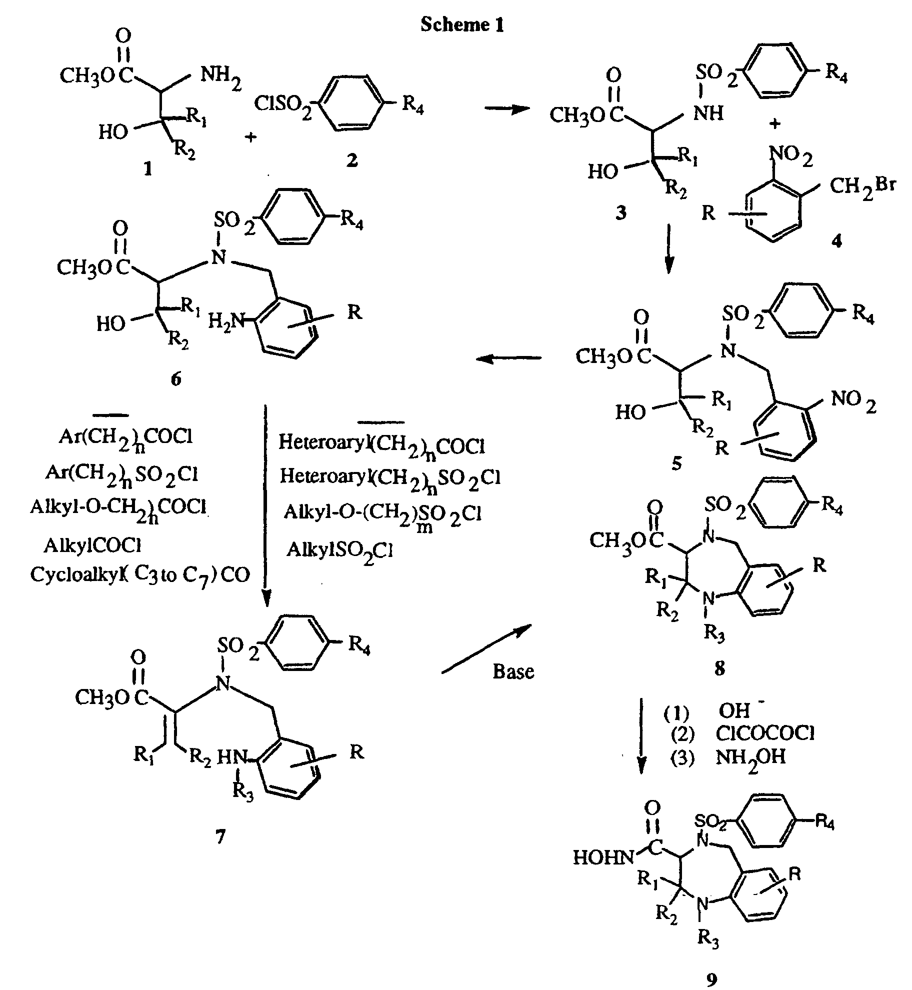 wherein
n = 0 to 3;
m = 1 to 3;
R₁ = R₂ = (C₁- C₃)alkyl; R = Hydrogen; halogen; OCH₃; NO₂; NH₂; CF₃; NHCOCH₃; NHCOCH₂OCH₃; CONH₂;-N(R')(R'),-N(R')CO(C₁- C₃)alkyl; (C₁- C₃)alkyl;
R₃ = Ar(CH₂)ₙCO-; Heteroaryl(CH₂)ₙCO-, Ar(CH₂)ₙSO₂-; Heteroaryl(CH₂)ₙSO₂-; Alkyl-O-CH₂)ₙCO-; Alkyl-O-(CH₂)ₘSO₂-; AlkylCO-; AlkylSO₂-; AlkylCO-NHCH₂CO-; and cycloalkyl(C₃ - C₇)CO-; and
R₄ is as defined herein. 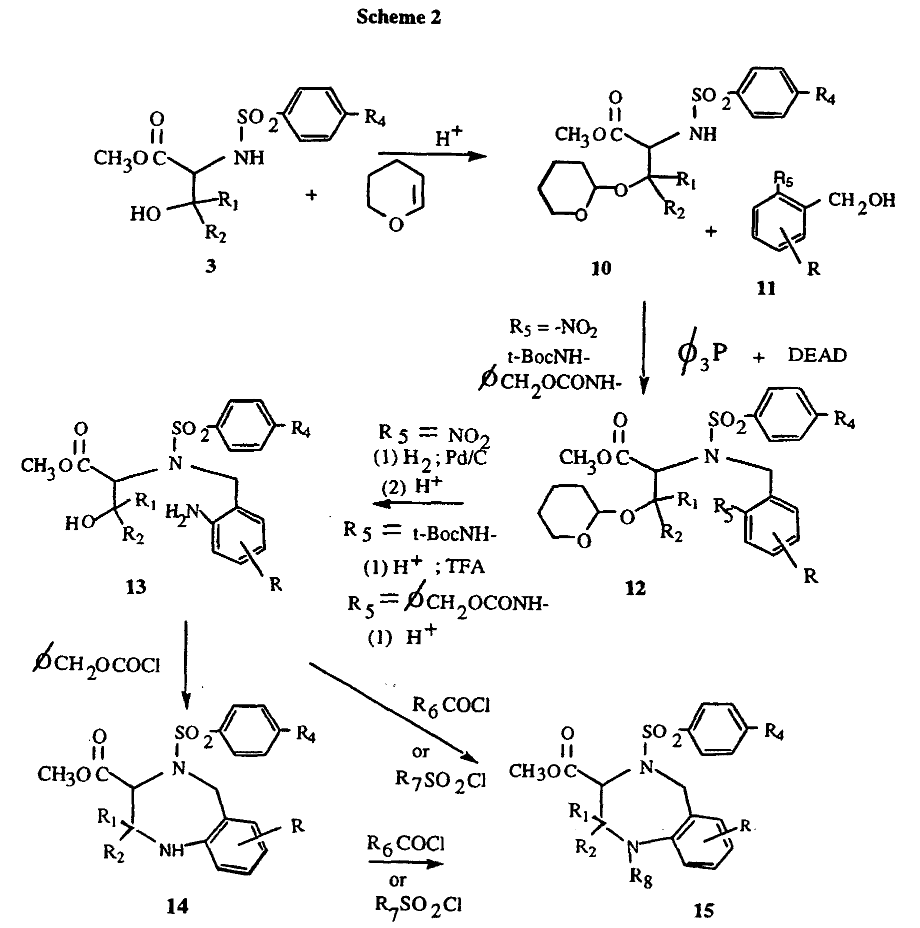 wherein
n = 0 to 3;
m = 1 to 3;
Ø = phenyl;
DEAD = diethylazodicarboxylate;
R₆ = Ar(CH₂)ₙ-; Alkyl-; Heteroaryl(CH₂)ₙ-; Alkyl-O-(CH₂)ₙ-; Cycloalkyl(C₃ - C₇);
R₇ = Ar(CH₂)ₙ-; Alkyl-; Heteroaryl(CH₂)ₙ-; Alkyl-O-(CH₂)ₘ-;
R₈ = Ar(CH₂)ₙCO-; Ar(CH₂)ₙSO₂-; AlkylCO-; AlkylSO₂-; Heteroaryl(CH₂)ₙCO-; Heteroaryl(CH₂)ₙSO₂-; Alkyl-O-(CH₂)ₙCO-; Alkyl-O-(CH₂)ₘSO₂-.

1-substituted arylmethyl-2,3,4,5-tetrahydro-1H [1,4]-benzodiazepines may be prepared in the manner illustrated in Reaction Schemes 3 and 4. In Reaction Scheme 3, the methyl 3-hydroxy-2-[4-methoxybenzenesulfonyl)-(2-amino-benzyl)-amino]-propionates 6 are subjected to reductive alkylation with arylcarboxaldehydes and heteroarylcarboxaldehydes to provide intermediates 17. Standard reaction conditions such as reactions with triphenylphosphine and diethyl azodicarboxylate (DEAD) or triplenylphosphine with either carbon tetrachloride or carbon tetrabromide, results in the "dehydroalanine" derivatives 18 which are then ring closed to the [1,4]benzodiazepines 20.

In an alternative route to the 3-hydroxamic acid derivatives 21 (Scheme 4), N-aroyl derivatives 22 are reduced with reducing agents such as borane or lithium aluminum hydride to reduce both the ester and amide functions. The 3-(hydroxymethyl)-1-(arylmethyl)-2,3,4,5-tetrahydro-1H-[1,4]benzodiazepines 23 are oxidized with stardard reagents known to convert a hydroxymethyl group to a carboxylic acid:reagents such as NaIO₄ with catalyst RuO₂ (e.g., see J. Org. Chem., 46:3936 (1981); Synlett, p. 143, (1996)). Coupling the acids (via the acid chlorides) to hydroxylamine then gives products 21. Certain intermediates as exemplified by formula 25 may be reduced with borane under mild conditions to give derivatives 25a in which the amide carbonyl is selectively reduced. These intermediates 25a are then converted to hydroxamic acid derivatives via hydrolysis of the ester to the acid and coupling the acid chloride with hydroxylamine. 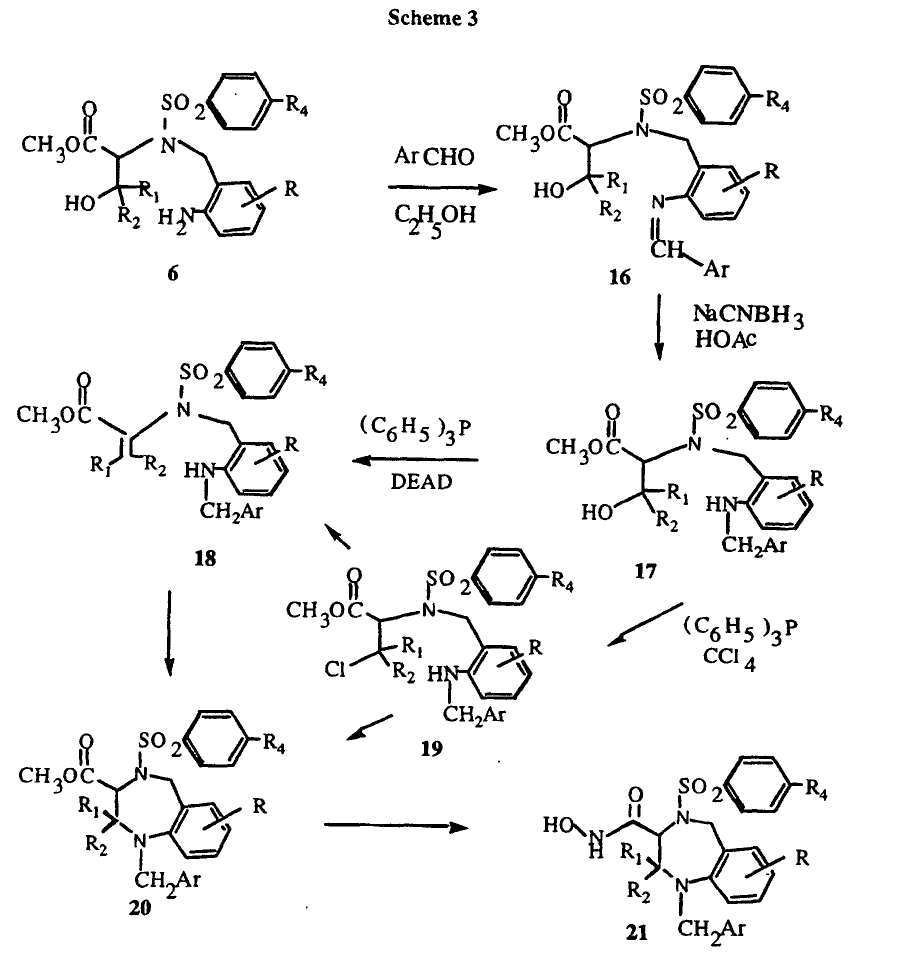 wherein 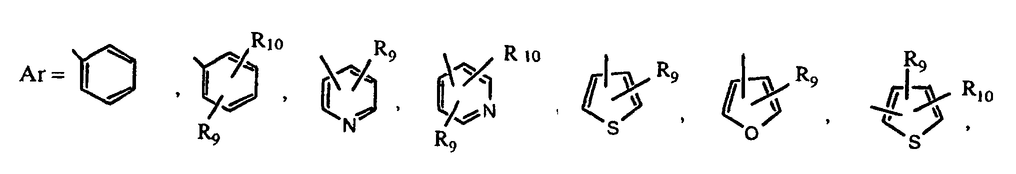 or 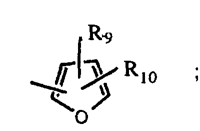 and R₉ and R₁₀ are: Cl, Br, F, OCH₃, OEt, SCH₃, 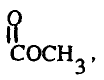 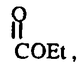 CF₃, OCF₃, 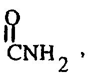 -NHCOCH₃, or Me₂N-. 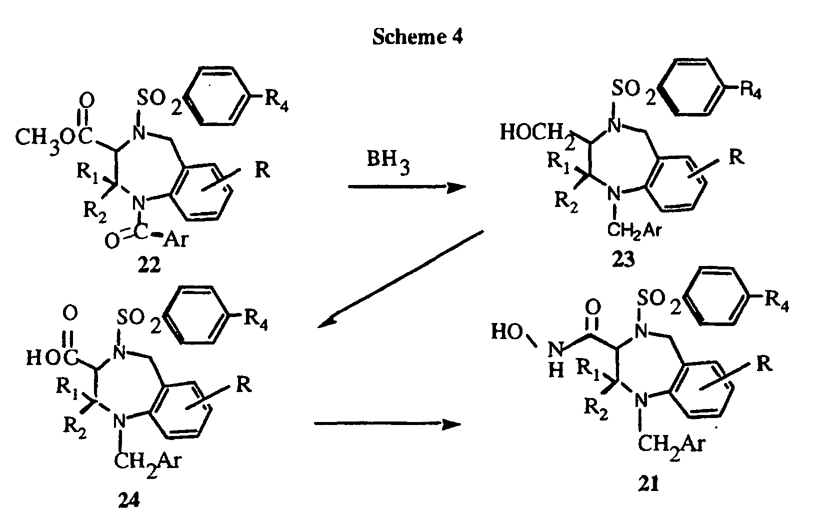 wherein 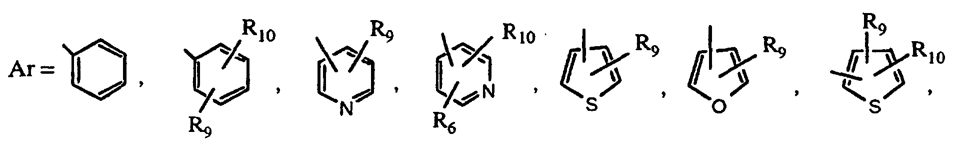 or 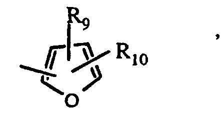 and R₉ and R₁₀ are hydrogen, Cl, Br, F, OCH₃, OEt, SCH₃, 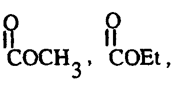 CF₃, OCF₃, or Me₂N-. 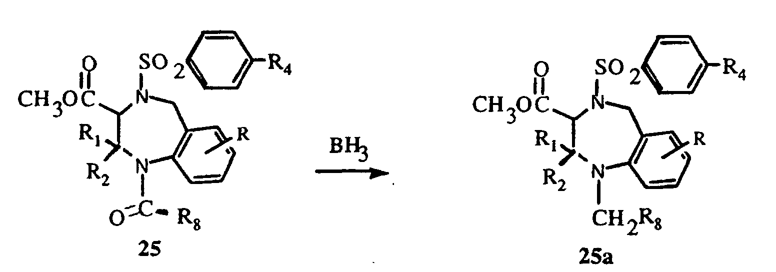 wherein R₈ = alkyl, arylalkyl, aryloxyalkyl, helerocyclicalkyl, or alkyloxyalkyloxyalkyl.

Other, preferred compounds of the present invention are those with basic moieties in the 1-(substituted carbonyl) group which may be prepared in the manner shown in Reaction Scheme 5. Reaction of the 2,3,4,5-terahydro-1H-[1,4]-benzo-diazepines 14 (without a substituent at the 1-position) with carbonyl chloride derivatives in the manner depicted in Reaction Scheme 5, results in intermediates 25 which are then converted to acid 26 and hydroxamic acids 27. The intermediates 25 may also be synthesized by reaction of methyl 2-[(2-aminobenzyl)-(4-methoxy-benzenesulfonyl) amino]-3-hydroxypropionates 6 with acid chlorides to give "dehydroalanine" derivatives 28. As previously described, mild bases such as NaHCO₃ can be reacted with these derivatives to cause ring closure via a 1,4-addition to the double bond in intermediate 28 to provide the 7-membered 2,3,4,5-tetrahydro-1H-[1,4] diazepines 25.

As illustrated in Reaction Scheme 6, aryl-arylcarbonyl, heteroaryl-arylcarbonyl, aryl-heteroarylcarbonyl, heteroaryl-heteroarylcarbonyl derivatives 30 may be synthesized by standard palladium catalysed coupling of bromoaroyl or bromheteroaroyl derivatives 29 with appropriate arylstannanes, heteroarylstannanes, arylboronic acids, heteroarylboronic acids, aryl triflates, heteroaryl triflates and the like, under known conditions. For example, see Synthesis, 563-566 (1997); J. Org. Chem., 62:3405-3406, (1997); Tetrahedron Lett., 36:5247-5250, (1995); Heterocycles, 45:467, (1997); Tetrahedron Lett., 38:1118-1182, (1997); Heterocycles, 42:189-194, (1996); Tetrahedron Lett., 5005-5006, (1993); Synthesis, 843, (1987); Heterocycles, 2711-2716, (1987); and Tetrahedron Lett., 4407-4410, (1986).

By coupling with such palladium catalysts, aryl-aryl, heteroaryl-aryl, aryl-heteroaryl and heteroaryl-heteroaryl carboxylic ester derivatives can be prepared and these derivatives converted to carboxylic acid intermediates. The acids are then converted to acid chlorides which are reacted with esters of 2-[(2-aminobenzyl)-(4-substituted-bcnzenesulfonyl)amino]-3-hydroxypropionate as illustrated for conversion of derivatives 6 to intermediates 31.

The following references describe procedures for the synthesis of methyl 3-arylpyrrole-4-carboxylates as in J. Org. Chem., 62:2649-2651, (1997); methyl (2-methylphenyl) benzoates as in J. Org. Chem., 62:3405-3406, (1997); and methyl benzoates substitued with heterocyclic moieties such as furanyl, thienyl or pyridinyl groups as in Tetrahedron Lett., 27:4407-4410, (1986). 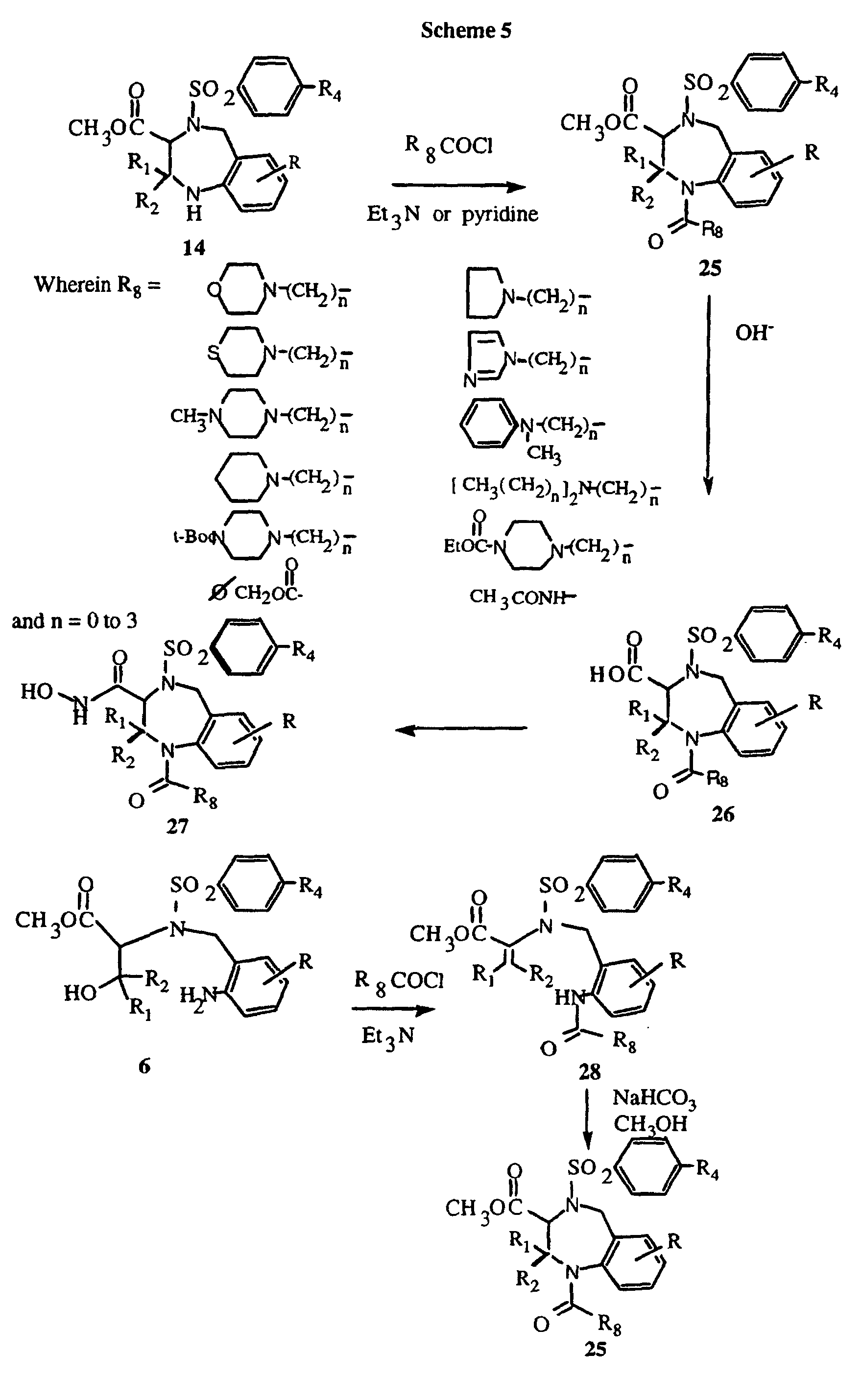 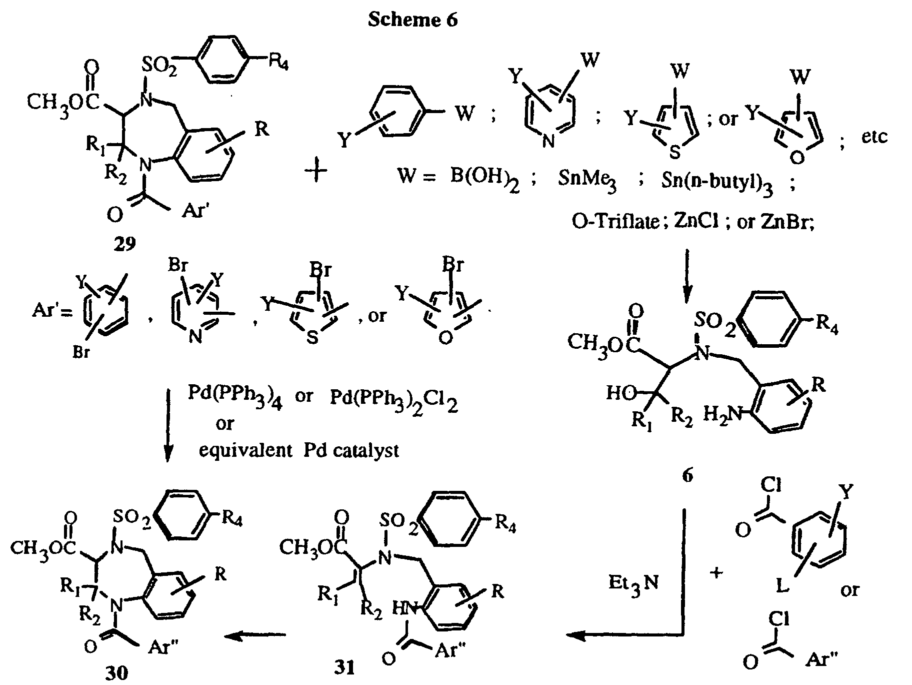 where 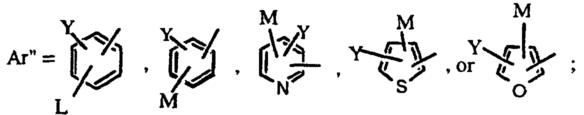 L is 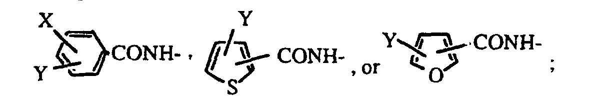 M is 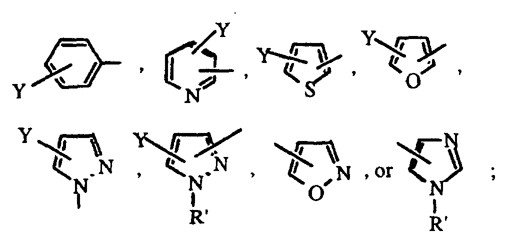 Y is H, F, Cl, CF₃, CH₃, or OCH₃;
X is halogen, hydrogen, or (C₁-C₃)alkyl;
R and R' are as defined herein;
R₁ and R₂ are as defined herein; and
R₄ is as defined herein.

The intermediates 2,4,5,6-tetrahydro-1H-[1,4]benzodiazepines 39 and 38 may be prepared from glycine esters in the manner exemplified in Reaction Scheme 7. In this synthetic route, N-(4-substituted-benzenesulfonyl) derivatives of glycine ethyl ester, glycine t-butyl ester or glycine methyl ester 33 are alkylated with a substituted (R) or unsubstituted (R=H) 2-nitrobenzyl bromide in N,N-dimethylformamide or 1-methyl-2-pyrrolidinone in the presence of potassium carbonate to give intermediates 34. Alternatively, the esters of N-(4-substituted-benzenesulfonyl) glycines, such as the methyl ester 33, are first reacted with sodium hydride in N, N-dimethylformamide or 1-methyl-2-pyrrolidinone and the resulting anion reacted with substituted or unsubstituted 2-nitrobenzylbromides to provide compounds 34. Reaction of derivates 34 with N,N-dimethyl(methylene)ammonium chloride or the iodide salts under standard reaction conditions (e.g., as set forth in Fieser and Fieser, 10:160-161; 8:194 affords the dimethylaminomethyl (Mannich type) compounds as intermediates for elimination to the "dehydroalanine" derivatives 37 or direct ring closure of 36 to 39 via an elimination-addition reaction. Ring closure of compounds 37 provides intermediates 38 for conversion to hydroxamic acids. Variations of the reactions conditions for conversion of 36 to 39 involve heating in the presence of Lewis acids, such as BF₃, or heating an acid salt of 36 to effect the elimination-addition reaction.

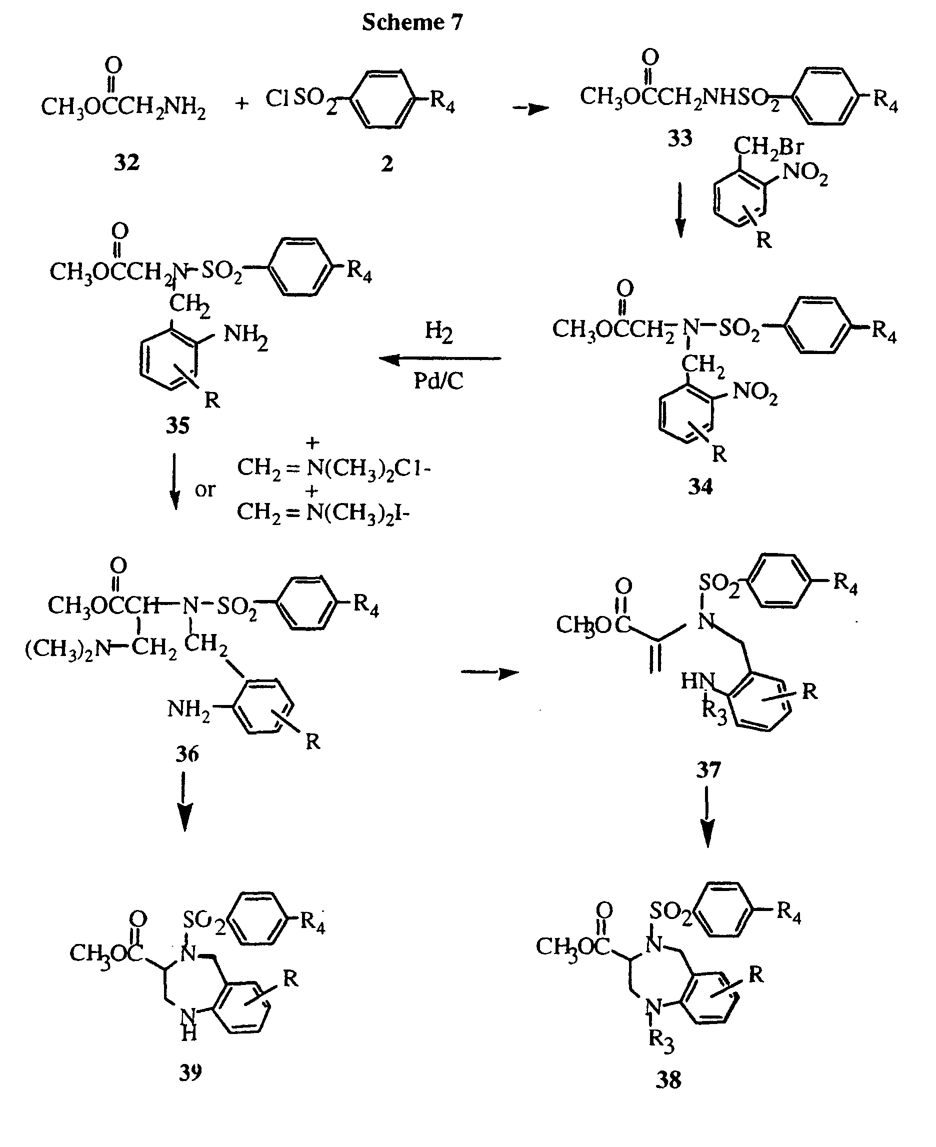

The intermediate carboxylic acids for conversion to the tetrahydro[1,4]-benzodiazepine-3-carboxylic acid, hydroxyamides may be synthesized via different routes as shown in **Schemes 1-8.** For the synthesis of some of the desired products of Formula 1, alternate routes are preferred as shown in **Scheme 8.** These routes may be preferred when the R₄ group contains a triple bond or when it is preferred to introduce the R₄ group toward the end of the synthetic sequence. Under these conditions, intermediate carboxylate esters of formula **41** or acids of formula **44** wherein the R₄ substituent is an OH group are prepared. Intermediates with R₄ an OH group any be prepared from derivatives wherein the OH group is protected by a group which can be selectively removed. Deriviatives **40** wherein R₄ is an OCH₃ moiety are suitable precurssors to the desired phenolic compounds **41** and **44** through cleavage of the oxygen methyl bond. As shown in **Scheme 8** the anion of the phenolic OH group may be prepared in situ and then alkylated. Suitable bases are alkaline metal carbonates, hydrides, alkoxides and organic bases. Reaction with an alkylating moiety represented by the formula R₁₅CH₂X wherein X is a reactive leaving group such as a chloride,bromide, iodide, O-mesylate or an O-tosylate gives the derivatives **42** and **45**.

The alkylation reaction may be carried out with carboxylate esters such as **41** or with the carboxylic acids represented by formula **44.** Alternatively, the phenolic compounds **41** and **44** may be reacted under Mitsunobe Reaction conditions to afford the O-alkylated derivatives **42** and **45.** Standard Mitsunobe Reaction conditions, which are described in the following literature references, may be used in the coupling reactions.
(a) J. Heterocyclic Chem. **34**, 349 (1997);(b) Tetrahedron Lett **37**, 6439 (1996);(c) J. Org Chem, 56, 7173 (1991);(d) Tetrahedron Lett 5709 (1989;(e) Synthesis 1-28 (1981).

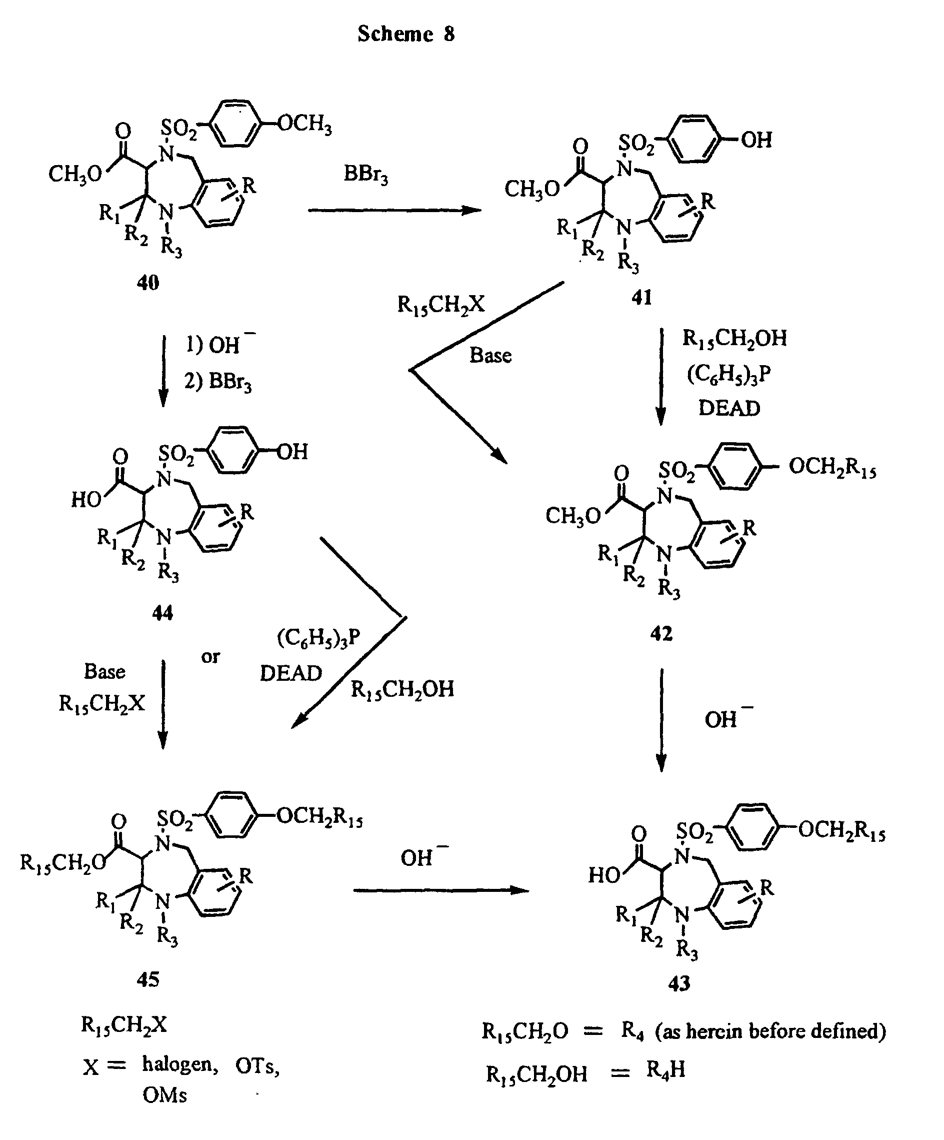

The compounds of the present invention which have a basic moiety may be used in the form of salts derived from pharmaceutically or physiologically acceptable. acids. These salts include, but are not hmited to, salts with inorganic acids (such as hydrochloric acid, sulfuric acid, nitric acid, phosphoric acid) or organic acids (such as acetic acid, oxalic acid, succinic acid, and maleic acid). Other salts of compounds with an acidic moiety include those with alkali metals or alkaline earth metals (such as sodium, potassium, calcium, and magnesium) or organic bases.

When the present compounds are utilized in pharmaceutical compositions, they may be combined with one or more pharmaceutically acceptable carriers, e.g., solvents, diluents and the like. Such compositions containing the present compounds may be administered orally, in the form of tablets, capsules, dispersible powders, granules, suspensions, syrups or elixirs; parentally, in the form of a sterile injectable solution or suspension; or topically, in the form of creams, lotions, ointments, etc. Such pharmaceutical compositions may contain from about 1 to about 100 mg of active ingredient in combination with the carrier.

The effective dosage of the present compounds utilized to treat a specific condition will vary depending upon the particular compound employed, the mode of administration and the type and severity of the condition being treated. However, in general, satisfactory results are obtained when the present compounds are administered at a dosage of about 0.001 to 1000 mg/kg of body weight.

As noted above, the compounds of the present invention may be administered orally, as well as by intravenous, intramuscular, subcutaneous or topical routes. Solid carriers useful for preparing tablets, capsules, etc., include starch, lactose, dicalcium phosphate, microcrystalline cellulose, sucrose and kaolin. Liquid carriers useful for preparing compositions of the present compounds include sterile water, polyethylene, glycols, non-ionic surfactants, and edible oils such as corn, sesame, and peanut oils. Adjuvants conventionally used in the preparation of pharmaceutical compositions may also be included, such as flavoring agents, coloring agents, preservatives and antioxidants.

The compounds of the present invention were tested for biological activity according to the following procedures.

### In Vitro Gelatinase Assay

The assay is based on the cleavage of the thiopeptide substrate ((Ac-Pro-Leu-Gly(2-mercapto-4-methyl-pentanoyl)-Leu-Gly-OEt), available from Bachem Bioscience) by the enzyme gelatinase, releasing the substrate product which reacts colorimetrically with DTNB ((5,5'-dithio-bis(2-nitro-benzoic acid)). This assay is disclosed in Weingarten et al., "Spectrophotometric Assay for Vertebrate Collegenase", Anal. Biochem., 147:437-440, (1985). The enzyme activity is measured by the rate of the color increase.

The thiopeptide substrate was made up fresh as a 20 mM stock in 100% DMSO and the DTNB was dissolved in 100% DMSO as a 100 mM stock and stored in the dark at room temperature. The substrate and the DTNB were diluted together to 1 mM with substrate buffer (50 mM HEPES, pH 7.5, 5 mM CaCl₂) before use. The stock of human neutrophil gelatinase B was diluted with assay buffer (50 mM HEPES, pH 7.5, 5 mM CaCl₂, 0.02% Brij) to a final concentration of 0.15 nM.

The assay buffer, enzyme, DTNB/substrate (500 µM final concentration) and vehicle or inhibitor were added to a 96 well plate (total reaction volume of 200µl) and the increase in color was monitored spectrophotometrically for 5 minutes at 405 nm on a plate reader.

The increase in OD₄₀₅ was plotted and the slope of the line was calculated. The slope represents the reaction rate. The linearity of the reaction rate was confirmed (r² >0.85) and the mean (x ± sem) of the control rate was calculated and compared for statistical significance (p <0.05) with drug-treated rates using Dunnett's multiple comparison test. Dose-response relationships were generated using multiple doses of drug and IC₅₀ values with 95% CI were estimated using linear regression (IPRED, HTB).

### In Vitro Collagenase Assay

This assay was based on the cleavage of a peptide substrate ((Dnp-Pro-Cha-Gly-Cys(Me)-His-Ala-Lys(NMa)-NH₂), available from Peptide International, Inc.) by collagenase releasing the fluorescent NMa group which was quantitated on the fluorometer as disclosed in Bickett et al., "A High Throughput Fluorogenic Substrate for Interstitial Collagenase (MMP-1) and Gelatinase (MMP-9)", Anal. Biochem., 212:58-64, (1993). Dnp quenches the NMa fluorescence in the intact substrate.

The assay was run in HCBC assay buffer (50 mM HEPES, pH 7.0, 5 mM Ca⁺², 0.02% Brij, 0.5% Cysteine), with human recombinant fibroblast collagenase (truncated, mw=18,828, from Wyeth-Ayerst Research, Radnor, PA). The substrate was dissolved in methanol and stored frozen in 1 mM aliquots. Collagenase was stored frozen in buffer in 25 µM aliquots. In conducting the assay, the substrate was dissolved in HCBC buffer to a final concentration of 10 µM and collagenase to a final concentration of 5 nM. The compounds being examined were dissolved in methanol, DMSO, or HCBC. The methanol and DMSO were diluted in HCBC to < 1.0%. The compounds were added to a 96 well plate containing enzyme and the reaction was started by the addition of substrate.

The reaction was read (excitation 340 nm, emission 444 nm) for 10 min. and the increase in fluorescence over time was plotted as a linear line. The slope of the line was calculated representing the reaction rate. The linearity of the reaction rate was confirmed (r² >0.85). The mean (x ± sem) of the control rate was calculated and compared for statistical significance (p <0.05) with drug-treated rates using Dunnett's multiple comparison test. Dose-response relationships were generated using multiple doses of drug and IC₅₀ values with 95% CI were estimated using linear regression.

### Procedure for Measuring TACE Inhibition

In a 96-well black microtiter plate, each well received a solution composed of 10 µL TACE (available from Immunex) at a final concentration of 1µg/mL, 70µL Tris buffer, have a pH of 7.4 and containing 10% glycerol (final concentration 10 mM), and 10 µL of test compound solution in DMSO (final concentration 1µM, DMSO concentration <1%). The plates were incubated for 10 minutes at room temperature. The reaction was initiated by addition of a fluorescent peptidyl substrate (final concentration 100 µM) to each well with shaking on a shaker for 5 sec.

The reaction was read (excitation 340 nm, emission 420 nm) for 10 min. and the increase in fluorescence over time was plotted as a linear line. The slope of the line was calculated and this represents the reaction rate. The linearity of the reaction rate was confirmed (r² >0.85). The mean (x±sem) of the control rate was calculated and compared for statistical significance (p<0.05) with drug-treated rates using Dunnett's multiple comparison test. Dose-response relationships were generated using multiple doses of drug and IC₅₀ values with 95% CI were estimated using linear regression.

Some results obtained following these standard experimental test procedures are presented in Table 1.

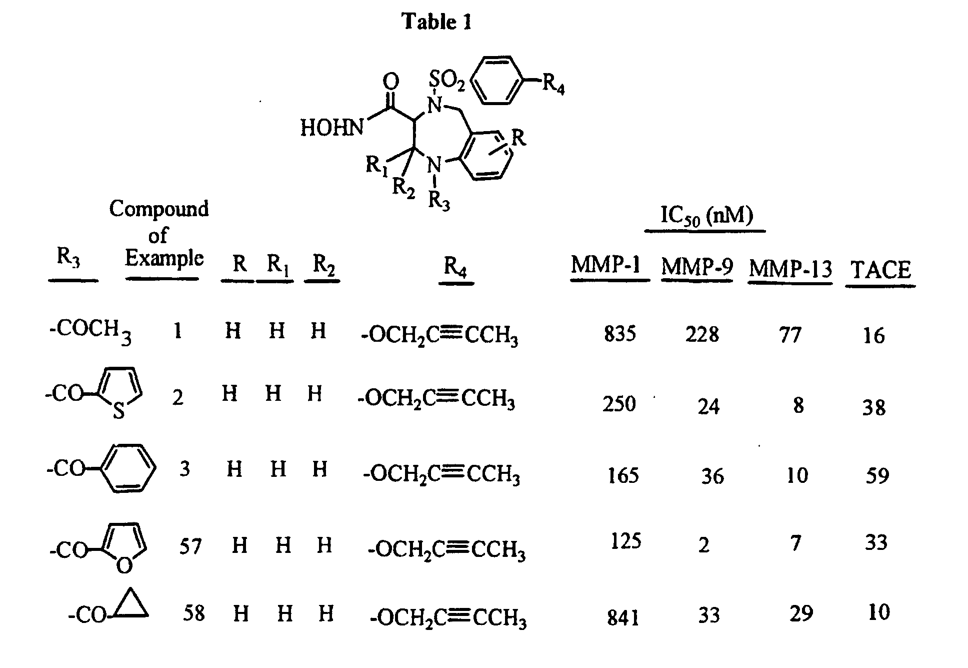

Some intermediate compounds for the synthesis of derivatives of formula 1 are presented in Table 2 (Reference Examples 10, 11, 41, 43-90, 92-97, 99, 100, 162, 166, 176, 181, 182, 186, 188, 190)

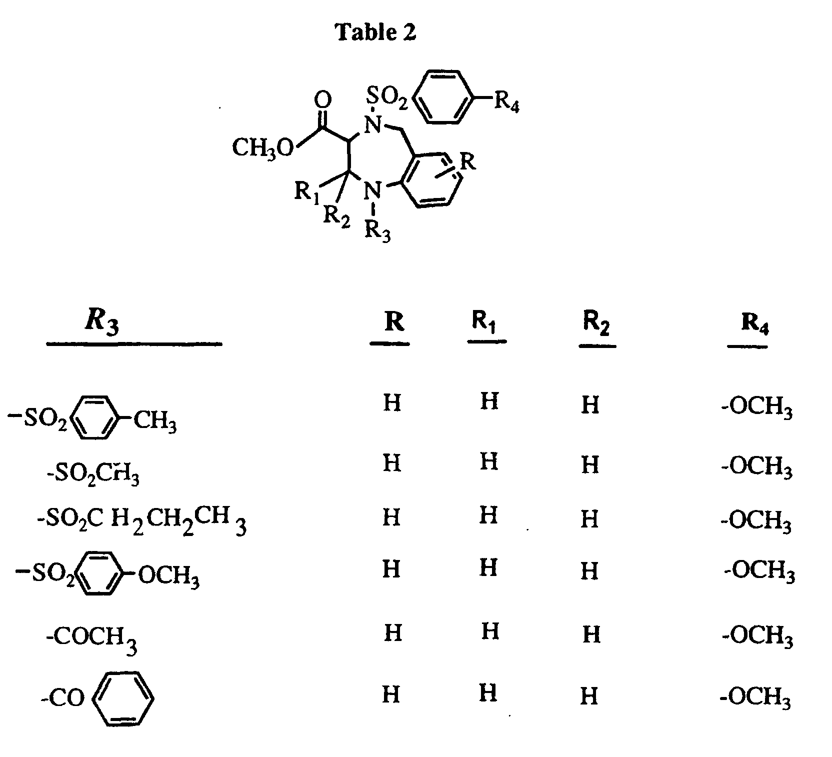

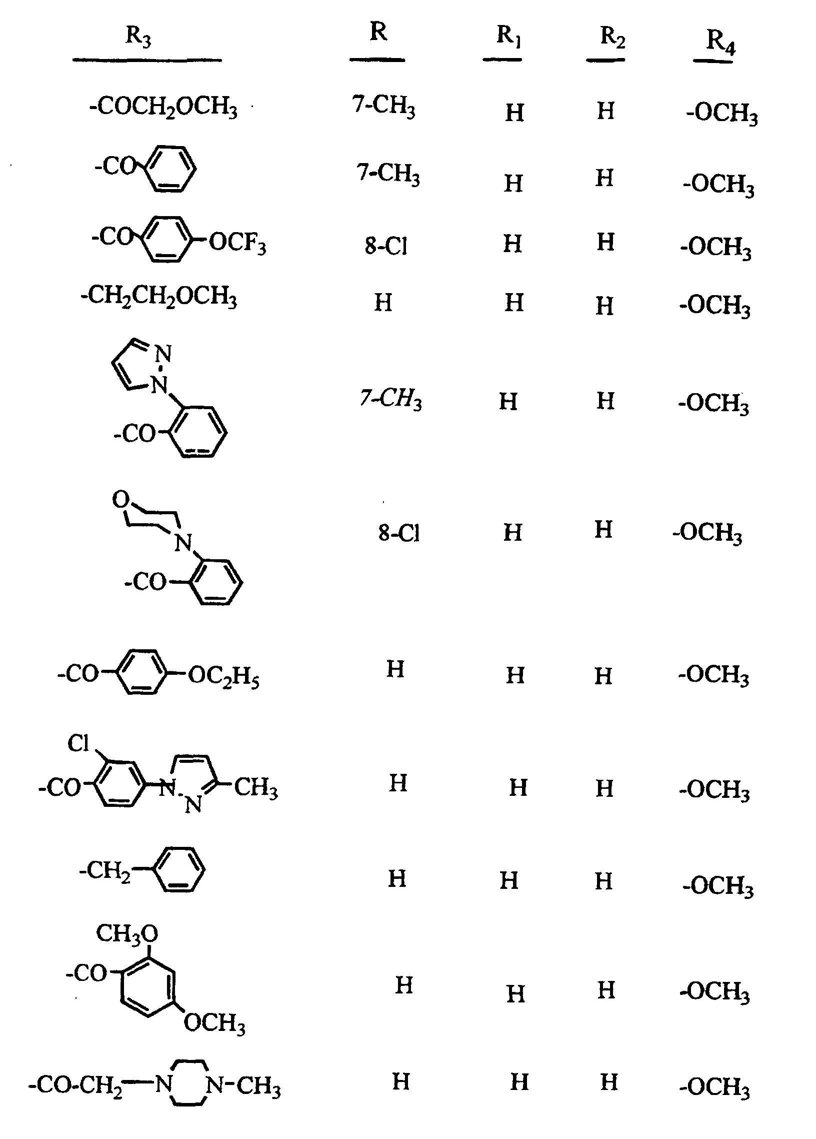

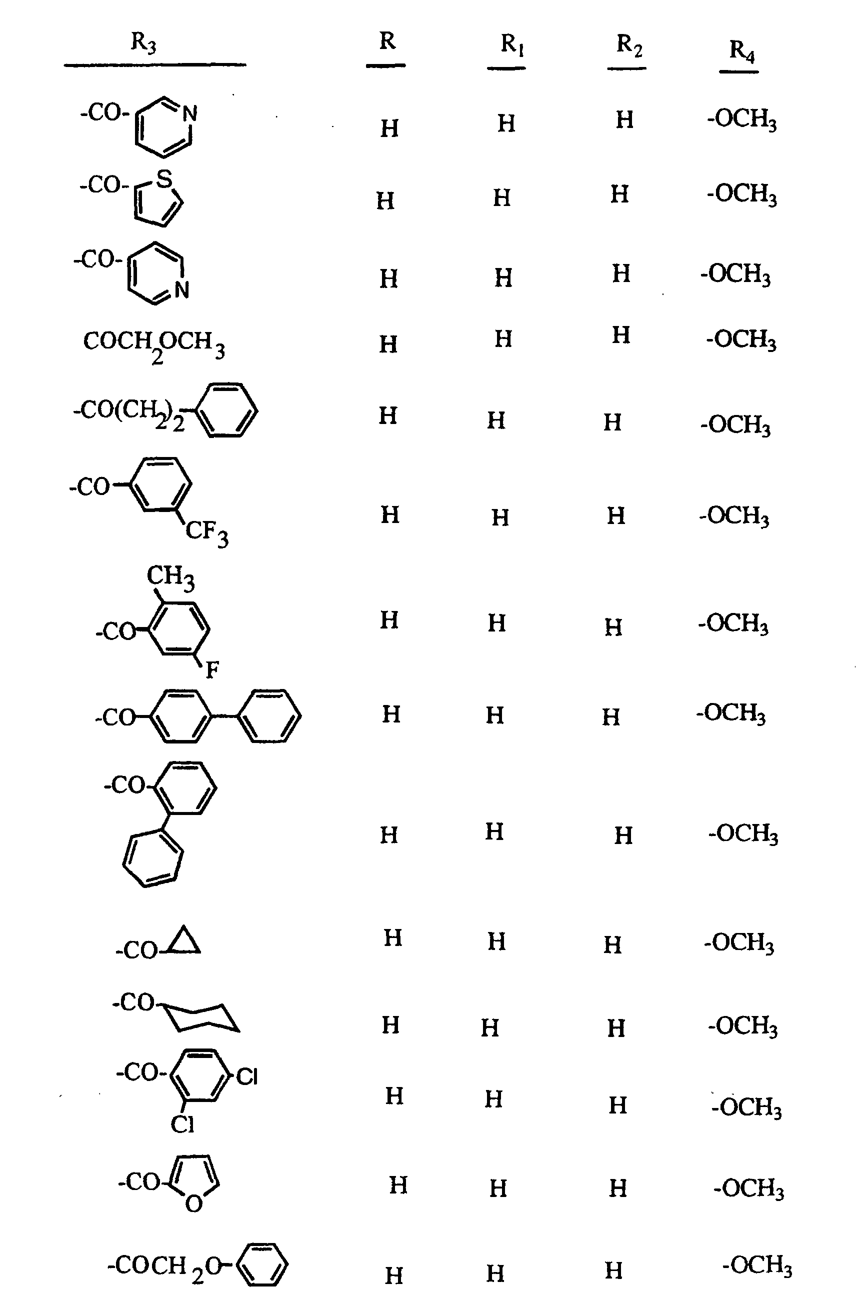

The present invention will now be illustrated with reference to the following, nonlimiting examples.

### Reference Example 1

### (L) N-(Benzyloxycarbonyl)-O-benzylserine, t-butyl ester

Into a solution of 25 g (0.076 mol) of N-(benzyloxycarbonyl)-O-benzylserine in 600 ml of CH₂Cl₂ cooled to -6°C in an ice-salt bath was bubbled isobutylene, while 4.1 ml of concentrated sulfuric acid was added dropwise thereto. The mixture was stirred for 4 hours and worked up as described in Synthetic Commun., 26:2723 (1996) to give 29.24 g of product as a yellow oil.

### Reference Example 2

### L-Serine, t-butyl ester

A mixture of 29.24 g (0.076 mol) of (L) N-(benzyloxycarbonyl)-O-benzylserine, t-butyl ester from Reference Example 1, 24.1 g (0.38 mol) of ammonium formate and 38.3 g of 10% palladium on carbon in 600 ml of methanol was heated at 65°C for 20 hours and stirred at room temperature overnight. The mixture was filtered through diatomaceous earth and the filter pad was washed with methanol. The filtrate was concentrated to give 12.18 g (99.6%) of product as described in Synthetic Commun., 26:2723 (1996).

### Reference Example 3

### N-(4-Methoxybenzenesulfonyl)-L-serine, t-butyl ester (3-hydroxy-2-(4-methoxybenzenesulfonylamino)propionic acid, tert-butyl ester)

To a solution of 12.18 g (0.0756 mol) of L-serine, t-butyl ester, 26.52 ml of triethylamine in 160 ml of CH₂Cl₂ (cooled in an ice bath) was added, in small portions, 16.1 g (0.0771 mol) of 4-methoxybenzene-sulfonyl chloride. The mixture was stirred at 0°C for 0.5 hours and at room temperature overnight. The mixture was washed with H₂O, 2N citric acid, brine and dried with Na₂SO₄. The solvent was removed under vacuum to give 25.34 g of solid which was triturated with hexane. The solid was recrystallized from 120 ml of toluene to give 12.18 g (48.7%) of product as a white solid. The filtrate was concentrated and the residue chromatographed on silica gel with hexane-ethyl acetate (7:3) as eluent to give 5.71 g (22.8%) of white solid. m.p. 70-75°C. Anal. for C₁₄H₂₁NO₆S:

| | | | |
|---|---|---|---|
| Calc'd | C, 50.7; | H,6.4; | N,4.2; |
| Found | C, 50.4; | H,6.3; | N,4.4. |

### Reference Example 4

### 3-Hydroxy-2-[(4-methoxybenzenesulfonyl)-(2-nitrobenzyl)amino] propionic acid, tert-butyl ester

To 6.16 g (18.6 mmol) of 3-hydroxy-2-(4-methoxybenzenesulfonylamino)-propionic acid tert-butyl ester in 50 ml of N,N-dimethylformamide, cooled in an ice bath, was added 0.781 g (19.5 mmol) of sodium hydride. After gas evolution ceased, a solution of 4.02 g (18.6 mmol) of 2-nitrobenzylbromide in 18 ml of N,N-dimethylformamide was added dropwise. The mixture was stirred under nitrogen at room temperature for 4 hours and 1.0 g of 2-nitrobenzyl bromide was added. The mixture was stirred at room temperature overnight and the solvent removed under vacuum. The residue was diluted with water and extracted with CH₂Cl₂. The organic extract was washed with H₂O, brine and dried with Na₂SO₄. The solvent was removed to give 11.2 g of solid which was chromatographed on silica gel with hexane-ethyl acetate (1:1) as eluent followed by hexane-ethyl acetate (35:65) as eluent. The fractions containing product were combined and the solvent was then removed to gave 7.7 g (89%) of solid. A sample from a 3 mmol run gave a gum. Anal. for C₂₁H₂₆N₂O₈S:

| | | | |
|---|---|---|---|
| Calc'd | C,54.1; | H,5.6; | N,6.0; |
| Found | C,54.0; | H,5.7; | N,6.0. |

### Reference Example 5

### 2-[(2-Aminobenzyl)-(4-methoxybenzenesulfonyl)amino]-3-hydroxypropionic acid, tert-butyl ester

A mixture of 0.60 g (1.28 mmol) of 3-hydroxy-2-[(4-methoxybenzenesulfonyl)-(2-nitrobenzyl)amino] propionic acid, tert-butyl ester and 1.45 g (6.45 mmol) of SnCl₂·2H₂O in 20 ml of methanol was heated in an oil bath at 90°C for 2 hours. The solvent was removed under vacuum and ethyl acetate added to the residue. The mixture was neutralized with saturated sodium bicarbonate solution and filtered through diatomaceous earth. The ethyl acetate layer was separated and washed with H₂O, brine and dried with Na₂SO₄. The solvent was removed under vacuum to give 0.30 g (53%) of a gum. Anal. for C₂₁H₂₈N₂O₆S:

| | | | |
|---|---|---|---|
| Calc'd | C, 57.8; | H,6.5; | N,6.4; |
| Found | C, 57.8; | H,7.0; | N,6.2. |

### Reference Example 6

### 2-[(2-Aminobenzyl)-(4-methoxybenzenesulfonyl)amino]-3-hydroxypropionic acid

A solution of 0.75 g (1.72 mmol) of 2-[(2-aminobenzyl)-(4-methoxybenzenesulfonyl)amino]-3-hydroxypropionic acid, tert-butyl ester and 6 ml of trifluoroacetic acid in 6 ml of CH₂Cl₂ was stirred at room temperature for 3 hours and then concentrated to dryness under vacuum. To the residue was added H₂O, CH₂Cl₂ and 1N NaOH until the aqueous layer reached pH 8. The aqueous layer was then separated, acidified with 2 N citric acid and extracted with ethyl acetate. The extract was washed with H₂O, brine and dried Na₂SO₄. The solvent was removed under vacuum to give 0.35 g (54%) of a solid. Anal. for C₁₇H₂₀N₂O₆S:

| | | | |
|---|---|---|---|
| Calc'd | C, 53.7; | H,5.3; | N,7.4; |
| Found | C, 53.0; | H,5.3; | N,6.9. |

### Reference Example 7

### 2-{(2-[3-(Trifluoromethylbenzoyl)aminobenzyl]-(4-methoxybenzenesulfonyl)amino}acrylic acid, tert-butyl ester

A mixture of 0.431 g (1 mmol) of 2-[(2-amino-benzyl)-(4-methoxybenzenesulfonyl)amino]-3-hydroxy-propionic acid, tert-butyl ester, 0.474 g (2.2 mmol) of 3-(trifluoromethyl)benzoyl chloride and 1 ml of pyridine in 2 ml of CH₂Cl₂ was stirred at room temperature for 3.5 hours. The mixture was poured into H₂O and extracted with CH₂Cl₂. The extract was washed with H₂O, 2 N citric acid, H₂O, 1 N NaHCO₃, brine and dried with Na₂SO₄. The solvent was removed to give 0.72 g of solid. The solid was dissolved in 2 ml of tetrahydrofuran and 1.5 ml of triethylamine was added thereto. The solution was heated at 65°C overnight and concentrated to dryness under vacuum. The residue was extracted with CH₂Cl₂ and the extract washed with H₂O and dried with Na₂SO₄. The solvent was removed under vacuum to give 0.55 g of product as a solid. From a similar run the product was chromatographed on silica gel with hexane-ethyl acetate to give a solid, m.p. 65-72°C. Anal. for C₂₉H₂₉F₃N₂O₆S:

| | | | |
|---|---|---|---|
| Calc'd | C, 59.0; | H,5.0; | N,4.7; |
| Found | C, 59.2; | H,5.2; | N,4.4. |

### Reference Example 8

### 4-(4-Methoxybenzenesulfonyl)-1-(3-trifluoromethylbenzoyl)-2,3,4,5-tetrahydro-1H-[1,4]benzodiazepine-3-carboxylic acid, tert-butyl ester

A mixture of 0.55 g (0.932 mmol) of 2-{(2-[3-(trifluoromethyl)benzoyl]-aminobenzoyl]-(4-methoxybenzenesulfonyl)amino}acrylic acid, tert-butyl ester and 0.102 g (1.21 mmol) of NaHCO₃ in 4 ml of methanol was stirred at room temperature overnight and the solvent removed. The residue was extracted with CH₂Cl₂ and the extract washed with H₂O, brine and dried with Na₂SO₄. The solvent was removed to give 0.57 g of solid. The solid was chromatographed on thick layer silica gel plates with hexane-ethyl acetate (1:1) as solvent to give 0.30 g of a light yellow solid, m.p. 57-60°C. Anal. for C₂₉H₂₉F₃N₂O₆S:

| | | | |
|---|---|---|---|
| Calc'd | C,59.0; | H,5.0; | N,4.7; |
| Found | C,58.8; | N,5.0; | N,4.6. |

### Reference Example 9

### 4-(4-Methoxybenzenesulfonyl)-1-(3-trifluoromethylbenzoyl)-2,3,4,5-tetrahydro-1H-[1,4]benzodiazepine-3-carboxylic acid

A mixture of 0.36 g (0.61 mmol) of 4-(4-methoxybenzenesulfonyl)-1-(3-trifluoromethylbenzoyl)-2,3,4,5-tetrahydro-1H-[1,4]benzodiazepine-3-carboxylic acid, tert-butyl ester and 3 ml of trifluoroacetic acid in 3 ml of CH₂Cl₂ was stirred at room temperature for 3 hours. The mixture was concentrated to dryness under vacuum and the residue extracted with CH₂Cl₂. The CH₂Cl₂ was washed with 1 N NaHCO₃ and the aqueous layer (pH 8) was acidified with 2 N citric acid and extracted with ethyl acetate. The extract was dried (Na₂SO₄). The original CH₂Cl₂ extract was washed with 2 N citric acid, H₂O, brine and dried with Na₂SO₄. The CH₂Cl₂ extract and the ethyl acetate extract were combined and the solvent removed under vacuum to give 0.31 g of solid, m.p. 105-110°C. Anal. for C₂₅H₂₁F₃N₂O₆S:

| | | | |
|---|---|---|---|
| Calc'd | C,56.2; | H,4.0; | N,5.2; |
| Found | C,55.1; | H,3.7; | N,5.0. |

### Reference Example 10

### Methyl 1-([1,1'-Biphenyl]-2-carbonyl)-4-(4-methoxybenzenesulfonyl)-2,3,4,5-tetrahydro-1H-[1,4]benzodiazepine-3-carboxylate

To a mixture of 1.5 g (3.8 mmol) of methyl 2-[(2-aminobenzyl)-(4-methoxybenzenesulfonyl)amino]-3-hydroxypropionate and 2.65 ml of triethylamine in 12 ml of CH₂Cl₂ chilled at 0°C was added a solution of [1,1'-biphenyl]-2-carbonyl chloride in 6 ml of CH₂Cl₂. The mixture was stirred at room temperature overnight and diluted with CH₂Cl₂ and H₂O. The organic layer was separated and washed with 2 N citric acid, brine and dried with Na₂SO₄. The solvent was removed under vacuum to give 2.2 g of a white foam. Anal. for C₃₁H₂₈N₂O₆S:
Calc'd: C,66.9; H,5.1; N,5.0; Found: C,67.3; H,5.2;N,4.7.

### Reference Example 11

### Methyl 4-(4-Methoxybenzenesulfonyl)-1-(2-methyl-5-fluorobenzoyl)-2,3,4,5-tetrahydro-1H-[1,4]benzodiazepine-3-carboxylate

To a mixture of 1.5 g (3.80 mmol) of methyl 2-[(2-aminobenzyl)-(4-methoxybenzenesulfonyl)amino]-3-hydroxypropionate and 2.64 ml (18.97 mmol) of triethylamine in 15 ml of CH₂Cl₂, chilled to 0°C, was added 1.36 g (11.4 mmol) of 2-methyl-5-fluorobenzoyl chloride. The mixture was stirred at room temperature overnight. The solution was then diluted with CH₂Cl₂ and water and the organic layer separated. The organic layer was washed with 2 N citric acid, brine and dried with Na₂SO₄. The solvent was removed under vacuum to give 2.2 g of a white foam. Anal. for C₂₆H₂₅FN₂O₆S:
Calc'd: C,60.9; H,4.9; N,5.5; Found: C,60.9; H,5.0; N,5.0;
Mass spectrum (ES) 513.4 (M+H).

### Reference Example 12

### Methyl 4-(4-Methoxybenzenesulfonyl)-2,3,4,5-tetrahydro-1H-[1,4] benzodiazepine-3-carboxylate

To a mixture of 5.0 g (12.68 mmol) of methyl 2-[(2-aminobenzyl)-(4-methoxybenzenesulfonyl)amino]-3-hydroxypropionate and 17.7 ml (26.8 mmol) of triethylamine in 50 ml of CH₂Cl₂ chilled to 0°C was added 9.05 ml (63.4 mmol) of benzyl chloroformate. The mixture was stirred overnight and then cooled to 0°C and .8 ml of triethylamine and 9.05 ml (63.4 mmol) of benzyl chloroformate were added thereto. The mixture was stirred overnight and then washed with H₂O, 2 N citric acid, brine and dried with Na₂SO₄. The solvent was removed under vacuum to give 6.95 g of solid. The solid was chromatographed on silica gel with hexane-ethyl acetate (1:1) to give 2.7 g of product as a viscous yellow oil. From a similar 0.5 g run, there was obtained 0.178g of an oil. Anal. for C₁₈H₂₀N₂O₅S:
Calc'd: C,57.4; H,5.4; N,7.4; S,8.5; Found: C,57.9; H,5.4; N,6.7; S,7.9;
Mass spectrum (ES) 377.2 (M+H).

### Reference Example 13

### Methyl 3-Hydroxy-2-(4-methoxybenzenesulfonylamino)propionate

To a mixture of 5.0 g (32.14 mmol) of D,L-serine, methyl ester and 15.7 ml (0.012 mol) of triethylamine in 100 ml of CH₂Cl₂, cooled to 0°C, was added portionwise 6.64 g (32.14 mmol) of 4-methoxybenzenesulfonyl chloride. The mixture was then stirred under argon at room temperature for 2 days. The mixture was diluted with 100 ml of CH₂Cl₂ and then washed with 60 ml each of H₂O, 2 N citric acid, brine and dried with Na₂SO₄. The solvent was removed under vacuum to give a solid. Crystallization from ethyl acetate gave 5.0 g (54%) of white crystals, m.p. 92-94°C. Anal. for C₁₁H₁₅NO₆S:

| | | | | |
|---|---|---|---|---|
| Calc'd | C,45.7; | H,5.2; | N,4.8; | S,11.1; |
| Found | C,45.6; | H,5.2; | N,4.8; | S,11.1. |

### Reference Example 14

### Methyl 3-Hydroxy-2-[(4-methoxybenzenesulfonyl)-(2-nitrobenzyl)amino]propionate

To a solution of 15.0 g (51.85 mmol) of methyl 3-hydroxy-2-(4-methoxybenzenesulfonylamino)propionate in 125 ml of N,N-dimethylformamide, cooled in an ice bath, was added portionwise 2.29 g (57.03 mmol) of NaH (60% in oil). The mixture was stirred at 0°C for 20 minutes and then a solution of 12.32 g (57.03 mmol) of 2-nitrobenzyl bromide in 25 ml of dry N,N-dimethylformamide was added dropwise. The solution was stirred at room temperature for 48 hours and diluted with 500 ml of ethyl acetate and water. The organic layer was separated and the aqueous layer extracted with 250 ml of ethyl acetate. The combined organic layer and extract was washed with 200 ml each of H₂O, 1 N NaHCO₃, brine and dried with Na₂SO₄. The solvent was removed and the residual solid was triturated with ethyl acetate, cooled and filtered to give 13.5 g (61%) of white crystals, having a m.p. 127-129°C. From a small scale run (3.0 g) there was obtained 2.32 g of white crystals, having a m.p. 127-129°C. Anal. for C₁₈H₂₀N₂O₈S:

| | | | |
|---|---|---|---|
| Calc'd | C,50.9; | H,4.8; | N,6.6; |
| Found | C,50.9; | H,4.8; | N,6.5. |

### Reference Example 15

### Methyl 2-[(2-Aminobenzyl)-(4-methoxybenzenesulfonyl)amino]-3-hydroxypropionate

To a mixture under nitrogen of 1.5 g (3.53 mmol) of methyl 3-hydroxy-2-[(4-methoxybenzenesulfonyl)-(2-nitrobenzyl)amino]propionate in 5 ml of dry ethanol was added 1.12 g (17.69 mmol) of ammonium formate followed by the addition of 0.50 g of 10% palladium on carbon. The mixture was stirred overnight at room temperature and heated at 80°C for 2 hours. The mixture was filtered through diatomaceous earth and the filtrate concentrated to dryness under vacuum to give a semisolid. Trituration with ethyl acetate gave 0.65 g (47%) of white crystals, m.p. 138-140°C; Anal. for C₁₈H₂₂N₂O₆S:

| | | | |
|---|---|---|---|
| Calc'd | C,54.8; | H,5.6; | N,7.1; |
| Found | C,53.0; | H,5.6; | N,6.8. |

### Reference Example 16

### Methyl 3-Hydroxy-2-{(4-methoxybenzenesulfonyl)-[2-(2,2,2-trifluoroacetylamino)benzyl]amino}propionate

To a solution of 0.50 g (1.27 mmol) of methyl 2-[(2-aminobenzyl)-(4-methoxybenzenesulfonyl)amino]-3-hydroxypropionate in 5 ml of CH₂Cl₂ was added 1.8 ml (12.7 mmol) of trifluoroacetic anhydride. The solution was stirred for 1 hour and concentrated to dryness under vacuum. Methanol was added to the residue and the solvent was removed under vacuum. The addition of methanol and concentration to dryness was repeated twice. The residue was chromatographed on silica gel thick layer plates with hexane-ethyl acetate (1:1) to give 0.50 g of a colorless glass. Anal. for C₂₀H₂₁F₃N₂O₇S:

| | | | |
|---|---|---|---|
| Calc'd | C,49.0; | H,4.3; | N,5.7; |
| Found | C,49.0; | H,4.5; | N,5.4. |

### Reference Example 17

### Methyl 2-[(4-Methoxybenzenesulfonyl)-(2-nitrobenzyl)amino]acrylate

To a solution of 1.0 g (2.356 mmol) of methyl 3-hydroxy-2-[(4-methoxybenzenesulfonyl)-(2-nitrobenzyl) amino]propionate in 2 ml of pyridine, cooled to -10°C was added 0.539 g (2.83 mmol) of 4-methylbenzeuesulfonyl chloride. The solution was chilled overnight and 4 ml of pyridine and 0.539 g (2.83 mmol) of 4-methylbenzene-sulfonyl chloride were added. The mixture was stirred and chilled at -10°C for 24 hours and diluted with H₂O. The mixture was extracted with ethyl acetate and the extract washed with H₂O, 2 N citric acid, and brine and then dried (Na₂SO₄). The solvent was removed under vacuum to give 1.2 g of an oil. The oil was dissolved in 6 ml of pyridine and 1.08 g of 4-methylbenzenesulfonyl chloride was added thereto. The mixture was stirred at room temperature overnight and diluted with H₂O. The mixture was extracted with ethyl acetate and the extract was washed with H₂O, 2 N citric acid, and brine and then dried with Na₂SO_{4.} The solvent was removed to give 1.0 g of brown oil. The oil was crystallized from ethanol to give white crystals, m.p. 65-67°C. Anal. for C₁₈H₁₈N₂O₇S:

| | | | |
|---|---|---|---|
| Calc'd | C,53.2; | H,4.5; | N,6.9; |
| Found | C,53.7; | H,4.5; | N,7.2. |

### Reference Example 18

### Methyl 2-{(4-Methoxybenzenesulfonyl)-[2-(4-pyridinylcarbonyl) aminobenzyl]amino}acrylate

To a mixture of 1.5 g (3.80 mmol) of methyl 2-[(2-aminobenzyl)-(4-methoxybenzenesulfonyl)amino]-3-hydroxypropionate and 3.0 ml (21.6 mmol) of triethylamine in 15 ml of CH₂Cl₂, cooled to 0°C was added 1.7 g (9.5 mmol) ml of 4-pyridinecarbonyl chloride (isonicotinoyl chloride). The mixture was stirred at room temperature overnight and diluted with CH₂Cl₂. The mixture was washed with H₂O, 2 N citric acid, and brine and then dried with Na₂SO₄. The solvent was removed to give 1.8 g of a light tan solid; Anal. for C₂₄H₂₃N₃O₆S:

| | | | | |
|---|---|---|---|---|
| Calc'd | C,59.9; | H,4.8; | N,8.7; | S,6.6; |
| Found | C,59.0; | H,4.8; | N,8.5; | S,6.9; |

Mass spectrum (ES) 482.6(M+H).

Utilizing the procedure described in Reference Example 18, the following intermediate compounds can be prepared from the appropriately unsubstituted methyl 2-[(2-aminobenzyl)-(4-methoxybenzenesulfonyl)amino]-3-hydroxypropionate or the appropriately substituted methyl 2-[(substituted-2-aminobenzyl)-(4-methoxybenzenesulfonyl)amino]-3-hydroxypropionate.

### Reference Example 19

### Methyl 2-{(4-Methoxybenzenesulfonyl)-[2-(2,2,2-trifluoroacetylamino)benzyl]amino}acrylate

white crystals, m.p. 120-121°C. Anal. for C₂₀H₁₉F₃N₂O₆S:

| | | | |
|---|---|---|---|
| Calc'd | C,50.9; | H,4.1; | N,5.9; |
| Found | C,50.8; | H,4.2; | N,5.6. |

### Reference Example 20

### Methyl 2-[(2-Benzoylaminobenzyl)-(4-methoxybenzenesulfonyl) amino]acrylate

yellow oil. Anal. for C₂₅H₂₄N₂O₆S:

| | | | |
|---|---|---|---|
| Calc'd | C,62.5; | H,5.0; | N,5.8; |
| Found | C,62.7; | H,5.3; | N,5.0. |

### Reference Example 21

### Methyl 2-[(2-Acetylaminobenzyl)-(4-methoxybenzenesulfonyl) amino]acrylate

### Reference Example 22

### Methyl 2-((4-Methoxybenzenesulfonyl)-{2-[(3-pyridinylcarbonyl)amino]benzyl}amino)acrylate

off-white solid. Anal. for C₂₄H₂₃N₃O₆S:

| | | | | |
|---|---|---|---|---|
| Calc'd | C,59.9; | H,4.8; | N,8.7; | S,6.6; |
| Found | C,58.9; | H,4.8; | N,8.4; | S,6.4; |

Mass spectrum (ES) 482.8(M+H).

### Reference Example 23

### Methyl 2-((4-Methoxybenzenesulfonyl)-{[(2-thienylcarbonyl)amino] benzyl}amino)acrylate

tan solid. Anal. for C₂₃H₂₂N₂O₆S₂:

| | | | |
|---|---|---|---|
| Calc'd | C,56.8; | H,4.6; | N,5.8; |
| Found | C,55.7; | H,4.4; | N,4.9. |

### Reference Example 24

### Methyl 2-{[2-(-Methoxyacetylamino)benzyl]-(4-methoxybenzenesulfonyl)amino}acrylate

yellow oil. Anal. for C₂₁H₂₄N₂O₇S:

| | | | |
|---|---|---|---|
| Calc'd | C,56.2; | H,5.4; | N,6.3; |
| Found | C,55.3; | H,5.6; | N,5.8. |

### Reference Example 25

### Methyl 2-{(4-Methoxybenzenesulfonyl)-[2-(n-propylsulfonylamino)benzyl]amino}acrylate

light brown oil. Anal. for C₂₁H₂₆N₂O₇S₂:

| | | | |
|---|---|---|---|
| Calc'd | C,52.3; | H,5.4; | N,5.8; |
| Found | C,51.9; | H,5.4; | N,5.7. |

### Reference Example 26

### Methyl 2-{[2-(3-Phenylpropionyl)aminobenzyl]-(4-methoxybenzenesulfonyl)amino}acrylate

light brown oil. Anal. for C₂₇H₂₈N₂O₆S:

| | | | |
|---|---|---|---|
| Calc'd | C,63.8; | H,5.6; | N,5.5; |
| Found | C,66.7; | H,5.8; | N,4.1. |

### Reference Example 27

### tert-Butyl 2-{[2-(3-Trifluoromethylbenzoyl)aminobenzyl]-(4-methoxybenzenesulfonyl)amino}acrylate

yellow solid; m.p. 65-72°C.

### Reference Example 28

### Methyl 2-{[2-(4-Biphenylcarbonyl)aminobenzyl]-(4-methoxybenzenesulfonyl)amino}acrylate

white solid. Anal for C₃₁H₂₈N₂O₆S:

| | | | |
|---|---|---|---|
| Calc'd | C,66.9; | H,5.1; | N,5.0; |
| Found | C,66.1; | H,5.0; | N,5.1. |

### Reference Example 29

### Methyl 2-{[2-(Cyclopropylcarbonyl)aminobenzyl]-(4-methoxybenzenesulfonyl)amino}acrylate

yellow oil. Anal. for C₂₂H₂₄N₂O₆S:

| | | | |
|---|---|---|---|
| Calc'd | C,59.5; | H,5.4; | N,6.3; |
| Found | C,60.0; | H,5.7; | N,6.0; |

Mass spectrum (ES) 445.5 (M+H).

### Reference Example 30

### Methyl 2-{[2-(Cyclohexylcarbonyl)aminobenzyl]-(4-methoxybenzenesulfonyl)amino}acrylate

white foam. Anal. for C₂₅H₃₀N₂O₆S:

| | | | |
|---|---|---|---|
| Calc'd | C,61.7; | H,6.2; | N,5.8; |
| Found | C,59.1; | H,6.0; | N,5.4; |

Mass spectrum (ES) 487.5 (M+H).

### Reference Example 31

### Methyl 2-{[2-(3-Fluorobenzoyl)aminobenzyl]-(4-methoxybenzenesulfonyl)amino}acrylate

### Reference Example 32

### Methyl 2-{[2-(3-Chlorobenzoyl)aminobenzyl]-(4-methoxybenzenesulfonyl)amino}acrylate

### Reference Example 33

### Methyl 2-{[2-(2,4-Dichlorobenzoyl)aminobenzyl]-(4-methoxybenzenesulfonyl)amino}acrylate

### Reference Example 34

### Methyl 2-{[2-(2,3-Difluorobenzoyl)aminobenzyl]-(4-methoxybenzenesulfonyl)amino}acrylate

### Reference Example 35

### Methyl 2-{[2-(2-Chloro-4-fluorobenzoyl)aminobenzyl]-(4-methoxybenzenesulfonyl)amino}acrylate

### Reference Example 36

### Methyl 2-{[2-(2-Furanylcarbonyl)aminobenzyl]-(4-methoxybenzenesulfonyl)amino}acrylate

off-white solid. Anal. for C₂₃H₂₂N₂O₇S.

| | | | |
|---|---|---|---|
| Calc'd | C,58.7; | H,4.7; | N,6.0; |
| Found | C,58.0; | H,4.1; | N,3.8; |

Mass Spectrum (ES) 470.9 (M+H).

### Reference Example 37

### Methyl 2-((4-Methoxybenzenesulfonyl)-{2-[(3-thienylcarbonyl)amino]benzyl}amino)acrylate

### Reference Example 38

### Methyl 2-{[2-(2-Acetylaminoacetyl)aminobenzyl]-(4-methoxybenzenesulfonyl)amino}acrylate

### Reference Example 39

### Methyl 2-{[2-(2-Dimethylacetyl)aminobenzyl]-(4-methoxybenzenesulfonyl)amino}acrylate

### Reference Example 40

### Methyl 2-{[2-(Cyclobutylcarbonyl)aminobenzyl]-(4-methoxybenzenesulfonyl)amino}acrylate

### Reference Example 41

### Methyl 1-Methoxyacetyl-4-(4-methoxybenzenesulfonyl)-2,3,4,5-tetrahydro-1H-[1,4]benzodiazepine-3-carboxylate

To a mixture of 0.449g (1 mmol) of methyl 2-[[2-(2-methoxy-acetamido)-benzyl]-(4-methoxybenzene-sulfonyl]amino)acrylate in 5 ml of anhydrous methanol was added 0.109 g (1.3 mmol) of anhydrous sodium bicar-bonate. The mixture was stirred at room temperature overnight and the solvent removed under vacuum. To the residue was added ethyl acetate and water. The organic layer was separated and washed with H₂O and brine and then dried with Na₂SO₄. The solvent was removed to give 0.41 g of solid. The solid was crystallized from ethyl acetate to give 0.28 g of white crystals, m.p. 160-163°C. Anal. for C₂₁H₂₄N₂O₇S:

| | | | | |
|---|---|---|---|---|
| Calc'd | C,56.2; | H,5.4; | N,6.3; | |
| Found | C,56.1; | H,5.3; | N,6.3; | S,6.9; |

Mass spectrum (ES) 449.1 (M+H).

Utilizing the procedure in Reference Example 41, the following intermediate compounds can be prepared from the appropriate methyl 2-{(4-methoxybenzenesulfonyl)-[2-(substituted amino)benzyl]amino}acrylates.

### Reference Example 42

### Methyl 4-(4-Methoxybenzenesulfonyl)-1-(4-methylphenylsulfonyl)-2,3,4,5-tetrahydro-1H-[1,4]benzodiazepine-3-carboxylate

white foam. Anal. for C₂₅H₂₆N₂O₇S₂:

| | | | |
|---|---|---|---|
| Calc'd | C,56.6; | H,4.9; | N,5.3 |
| Found | C,56.2; | H,5.2; | N,5.2. |

### Reference Example 43

### Methyl 1,4-Bis-(4-methoxybenzenesulfonyl)-2,3,4,5-tetrahydro-1H-[1,4]benzodiazepine-3-carboxylate

white solid. Anal. for C₂₅H₂₆N₂O₈S₂:

| | | | |
|---|---|---|---|
| Calc'd | C,54.9; | H,4.8; | N,5.1; |
| Found | C,54.8; | H,4.9; | N,5.1. |

### Reference Example 44

### Methyl 1-Methanesulfonyl-4-(4-methoxybenzeuesulfonyl)-2,3,4,5-tetrahydro-1H-[1,4]benzodiazepine-3-carboxylate

white crystals, m.p. 136-137°C. Anal. for C₁₉H₂₂N₂O₇S₂:

| | | | |
|---|---|---|---|
| Calc'd | C,50.2; | H,4.9; | N,6.2; |
| Found | C,50.1; | H,4.9; | N,6.4. |

### Reference Example 45

### Methyl 1-Benzoyl-4-(4-methoxybenzenesulfonyl)-2,3,4,5-tetrahydro-1H-[1,4]benzodiazepine-3-carboxylate

tan sohd. Anal. for C₂₅H₂₄N₂O₂S:

| | | | |
|---|---|---|---|
| Calc'd | C,62.2; | H,5.4; | N,5.8; |
| Found | C,62.3; | H,5.2; | N,5.6. |

### Reference Example 46

### Methyl 1-Acetyl-4-(4-methoxybenzenesulfonyl)-2,3,4,5-tetrahydro-1H-[ 1,4]benzodiazepine-3-carboxylate

white crystals, m.p. 150-155°C. Anal. for C₂₀H₂₂N₂O₆S:

| | | | |
|---|---|---|---|
| Calc'd | C,57.4; | H,5.3; | N,6.7; |
| Found | C,56.6; | H,5.2; | N,6.5. |

### Reference Example 47

### Methyl 4-(4-Methoxybenzenesulfonyl)-1-(3-pyridinylcarbonyl)-2,3,4,5-tetrahydro-1H-[1,4]benzodiazepine-3-carboxylate

off-white solid; Anal. for C₂₄H₂₃N₃O₆S:

| | | | |
|---|---|---|---|
| Calc'd | C,59.9; | H,4.8; | N,8.7; |
| Found | C,59.2; | H,4.8; | N,8.3; |

Mass spectrum (ES) 482.2 (M+H).

### Reference Example 48

### Methyl 4-(4-Methoxybenzenesulfonyl)-1-(2-thienylcarbonyl)-2,3,4,5-tetrahydro-1H-[1,4]benzodiazepine-3-carboxylate

off-white solid. Anal. for C₂₃H₂₂N₂O₆S₂:

| | | | |
|---|---|---|---|
| Calc'd | C,56.8; | H,4.6; | N,5.8; |
| Found | C,56.0; | H,4.6; | N,5.2. |

### Reference Example 49

### Methyl 4-(4-Methoxybenzenesulfonyl)-1-(4-pyridinylcarbonyl)-2,3,4,5-tetrahydro-1H-[1,4]benzodiazepine-3-carboxylate

off-white crystals, m.p. 162-164°C. Anal. for C₂₄H₂₃N₃O₆S:

| | | | |
|---|---|---|---|
| Calc'd | C,59.9; | H,4.8; | N,8.7; |
| Found | C,59.9; | H,4.8; | N,8.7. |

### Reference Example 50

### Methyl 1-(4-Biphenylcarbonyl)-4-(4-methoxybenzenesulfonyl)-2,3,4,5-tetrahydro-1H-[1,4]benzodiazepine-3-carboxylate

white solid; Anal. for C₃₁H₂₈N₂O₆S:

| | | | |
|---|---|---|---|
| Calc'd | C,66.9; | H,5.1; | N,5.0; |
| Found | C,65.8; | H,5.2; | N,5.0; |

Mass spectrum (ES) 557.6 (M+H).

### Reference Example 51

### Methyl 4-(4-Methoxybenzenesulfonyl)-1-(propane-1-sulfonyl)-2,3,4,5-tetrahydro-1 H-[ 1,4]benzodiazepine-3-carboxylate

yellow oil. Anal. for C₂₁H₂₆N₂O₇S₂:

| | | | |
|---|---|---|---|
| Calc'd | C,52.3; | H,5.4; | N,5.8; |
| Found | C,51.8; | H,5.4; | N,5.6. |

### Reference Example 52

### Methyt 1-([1,1'-Biphenyl]-2-carbonyl)-4-(4-methoxybenzenesulfonyl)-2,3,4,5-tetrahydro-1H-[1,4]benzodiazepine-3-carboxylate

white foam. Anal. for C₃₁H₂₈N₂O₆S:

| | | | |
|---|---|---|---|
| Calc'd | C,66.9; | H,5.1; | N,5.0; |
| Found | C,67.3; | H,5.2; | N,4.7; |

Mass spectrum (ES) 557.6 (M+H).

### Reference Example 53

### Methyl 1-(3-Fluorobenzoyl)-4-(4-methoxybenzenesulfonyl)-2,3,4,5-tetrahydro-1H-[1,4]benzodiazepine-3-carboxylate

### Reference Example 54

### Methyl 4-(4-Methoxybenzenesulfonyl)-1-(2-methyl-5-fluorobenzoyl)-2,3,4,5-tetrahydro-1H-[1,4]benzodiazepine-3-carboxylate

white solid; Anal. for C₂₆H₂₅FN₂O₆S:

| | | | |
|---|---|---|---|
| Calc'd | C,60.9; | H,4.9; | N,5.5; |
| Found | C,60.9; | H,5.0; | N,5.0. |

### Reference Example 55

### Methyl 4-(4-Methoxybenzenesulfonyl)-1-(2-methyl-3-fluorobenzoyl)-2,3,4,5-tetrahydro-1H-[1,4]benzodiazepine-3-carboxylate

### Reference Example 56

### Methyl 4-(4-Methoxybenzenesulfonyl)-1-(3-phenylpropionyl)-2,3,4,5-tetrahydro-1H-[1,4]benzodiazepine-3-carboxylate

white solid; Anal. for C₂₇H₂₈N₂O₆S:
Calc'd: C,63.8; H,5.6; N,5.5; Found: C,64.0; H,5.7; N,5.3; S,6.5.

### Reference Example 57

### Methyl 4-(4-Methoxybenzenesulfonyl)-1-(2-trifluoromethylbenzoyl)-2,3,4,5-tetrahydro-1H-[1,4]benzodiazepine-3-carboxylate

### Reference Example 58

### Methyl 1-(2-Chloro-6-trifluoromethylbenzoyl)-4(4-methoxybenzenesulfonyl)-2,3,4,5-tetrahydro-1H-[1,4]benzodiazepine-3-carboxylate

### Reference Example 59

### Methyl 1-(4-Fluoro-2-trifluorometlhylbenzoyl)-4-(4-methoxybenzenesulfonyl)-2,3,4,5-tetrahydro-1H-[1,4]benzodiazepine-3-carboxylate

### Reference Example 60

### Methyl 1-(2-Fluoro-6-trifluoromethylbenzoyl)-4-(4-methoxybenzenesulfonyl)-2,3,4,5-tetrahydro-1H-[1,4]benzodiazepine-3-carboxylate

### Reference Example 61

### Methyl 4-(4-Methoxybenzenesulfonyl)-1-(2-methylbenzoyl)-2,3,4,5-tetrahydro-1H-[1,4]benzodiazepine-3-carboxylate

### Reference Example 62

### Methyl 4-(4-Methoxybenzenesulfonyl)-1-(2-methyl-6-chlorobenzoyl)-2,3,4,5-tetrahydro-1H-[1,4]benzodiazepine-3-carboxylate

### Reference Example 63

### Methyl 1-(2,4-Dimethylbenzoyl)-4-(4-methoxybenzenesulfonyl)-2,3,4,5-tetrahydro-1H-[1,4]benzodiazepine-3-carboxylate

### Reference Example 64

### Methyl 1-(2,5-Dimethylbenzoyl)-4-(4-methoxybenzenesulfonyl)-2,3,4,5-tetrahydro-1H-[1,4]benzodiazepine-3-carboxylate

### Reference Example 65

### Methyl 1-(2-Chloro-4-fluorobenzoyl)-4-(4-methoxybenzenesulfonyl)-2,3,4,5-tetrahydro-1H-[1,4]benzodiazepine-3-carboxylate

### Reference Example 66

### Methyl 1-(2-Chlorobenzoyl)-4-(4-methoxybenzenesulfonyl)-2,3,4,5-tetrahydro-1H-[1,4]benzodiazepine-3-carboxylate

### Reference Example 67

### Methyl 1-(2-Fluorobenzoyl)-4-(4-methoxybenzenesulfonyl)-2,3,4,5-tetrahydro-1H-[1,4]benzodiazepine-3-carboxylate

### Reference Example 68

### Methyl 1-(2-Chloro-6-fluorobenzoyl)-4-(4-methoxybenzenesulfonyl)-2,3,4,5-tetrahydro-1H-[1,4]benzodiazepine-3-carboxylate

### Reference Example 69

### Methyl 1-(2,3-Difluorobenzoyl)-4-(4-methoxybenzenesulfonyl)-2,3,4,5-tetrahydro-1H-[1,4]benzodiazepine-3-carboxylate

### Reference Example 70

### Methyl 1-(2,4-Dichlorobenzoyl)-4-(4-methoxybenzenesulfonyl)-2,3,4,5-tetrahydro-1H-[1,4]benzodiazepine-3-carboxylate

Prepared according to the procedure set forth in Reference Example 10; white solid. Anal. for C₂₅H₂₂Cl₂N₂O₆S:

| | | | |
|---|---|---|---|
| Calc'd | C,54.7; | H,4.0; | N,5.1; |
| Found | C,54.4; | H,3.8; | N,4.9; |

Mass spectrum (548.9) (M+H); 550.9 (M+H).

### Reference Example 71

### Methyl 1-(2,3-Dichlorobenzoyl)-4-(4-methoxybenzenesulfonyl)-2,3,4,5-tetrahydro-1H-[1,4] benzodiazepine-3-carboxylate

### Reference Example 72

### Methyl 1-(2,5-Dichlorobenzoyl)-4-(4-methoxybenzenesulfonyl)-2,3,4,5-tetrahydro-1H-[1,4]benzodiazepine-3-carboxylate

### Reference Example 73

### Methyl 1-(2-Methoxybenzoyl)-4-(4-methoxybenzenesulfonyl)-2,3,4,5-tetrahydro-1H-[1,4]benzodiazepine-3-carboxylate

### Reference Example 74

### Methyl 1-(4-Chloro-2-methoxybenzoyl)-4-(4-methoxybenzenesulfonyl)-2,3,4,5-tetrahydro-1H-[1,4]benzodiazepine-3-carboxylate

### Reference Example 75

### Methyl 4-(4-Methoxybenzenesulfonyl)-1-(2-methylthiobenzoyl)-2,3,4,5-tetrahydro-1H-[1,4]benzodiazepine-3-carboxylate

### Reference Example 76

### Methyl 4-(4-Methoxybenzenesulfonyl)-1-(3-methyl-2-thienylcarbonyl)-2,3,4,5-tetrahydro-1H-[1.4] benzodiazepine-3-carboxylate

### Reference Example 77

### Methyl 4-(4-Methoxybenzenesulfonyl)-1-(4-methyl-2-thienylcarbonyl)-2,3,4,5-tetrahydro-1H-[1,4] benzodiazepine-3-carboxylate

### Reference Example 78

### Methyl 1-(3-Chloro-2-thienylcarbonyl)-4-(4-methoxybenzenesulfonyl)-2,3,4,5-tetrahydro-1H-[1,4]benzodiazepine-3-carboxylate

### Reference Example 79

### Methyl 1-(2-Furanylcarbonyl)-4-(4-methoxybenzenesulfonyl)-2,3,4,5-tetrahydro-1H-[1,4]benzodiazepine-3-carboxylate

off-white solid, m.p. 165-167°C. Anal. for C₂₃H₂₂N₂O₇S:

| | | | |
|---|---|---|---|
| Calc'd | C,58.7; | H,4.7; | N,6.0; |
| Found | C,58.4; | H,4.6; | N,5.7; |

Mass spectrum (ES) 470.9 (M+H).

### Reference Example 80

### Methyl 4-(4-Methoxybenzenesulfonyl)-1-(3-methyl-2-furanylcarbonyl)-2,3,4,5-tetrahydro-1H-[1,4]benzodiazepine-3-carboxylate

### Reference Example 81

### Methyl 4-(4-Methoxybenzenesulfonyl)-1-(4-methyl-2-furanylcarbonyl)-2,3,4,5-tetrahydro-1H-[1,4]benzodiazepine-3-carboxylate

### Reference Example 82

### Methyl 1-(5-Chloro-2-furanylcarbonyl)-4-(4-methoxybenzenesulfonyl)-2,3,4,5-tetrahydro-1H-[1,4]benzodiazepine-3-carboxylate

### Reference Example 83

### Methyl 1-(5-Chloro-2-thienylcarbonyl)-4-(4-methoxybenzenesulfonyl)-2,3,4,5-tetrahydro-1H-[1,4]benzodiazepine-3-carboxylate

### Reference Example 84

### Methyl 1-Propionyl-4-(4-methoxybenzenesulfonyl)-2,3,4,5-tetrahydro-1H-[1,4]benzodiazepine-3-carboxylate

### Reference Example 85

### Methyl 1-Hexanoyl-4-(4-methoxybenzenesulfonyl)-2,3,4,5-tetrahydro-1H-[1,4]benzodiazepine-3-carboxylate

### Reference Example 86

### Methyl 1-(3-Methoxypropionyl)-4-(4-methoxybenxenesutfonyl)-2,3,4,5-tetrahydro-1H-[1,4]benzodiazepine-3-carboxylate

### Reference Example 87

### Methyl 4-(4-Methoxybenzenesulfonyl)-1-(3-thienylcarbonyl)-2,3,4,5-tetrahydro-1H-[ 1, 4]benzodiazepine-3-carboxylate

### Reference Example 88

### Methyl 1-(3-Furanylcarbonyl)-4-(4-methoxybenzenesulfonyl)-2,3,4,5-tetrahydro-1H-[1,4]benzodiazepine-3-carboxylate

### Reference Example 89

### Methyl 1-(trans-Crotonyl)-4-(4-methoxybenzenesulfonyl)-2,3,4,5-tetrahydro-1H-[1,4]benzodiazepine-3-carboxylate

### Reference Example 90

### Methyl 1-(Methacryloyl)-4-(4-metboxybenzenesulfonyl)-2,3,4,5-tetrahydro-1H-[1,4]benzodiazepine-3-carboxylate

### Reference Example 91

### Methyl 1-(Chloroacetyl)-4-(4-methoxybenzenesulfonyl)-2,3,4,5-tetrahydro-1H-[1,4]benzodiazepine-3-carboxylate

Following the method described for Reference Example 18, 3.0 g (7.61 mmol) of methyl 2-[2-aminobenzyl)-(4-methoxy-benzenesulfonyl)-amino]-3-hydroxypropionate was reacted with 1.82 ml (22.8 mmol) of chloroacetylchloride to give 4.0 g of solid. Chromatography on silica gel with ethyl acetate-hexane (1:1) as a solvent gave 1.5 g of methyl 2-[(2-chloroacetylaminobenzyl)-(4-methoxybenzenesulfonyl)-amino]acrylate. A 1.3 g sample of the preceding compound was reacted with 0.312 g of anhydrous NaHCO₃ in 10 ml of anhydrous methanol at room temperature overnight and the mixture was then heated at 80°C for 5 hours. The solvent was removed and the residue partitioned between H₂O and ethyl acetate. The ethyl acetate extract was washed with brine, dried with Na₂SO₄ and the solvent removed. The residue was triturated with hexane-ethyl acetate, chilled and filtered to give the product; Mass spectrum (ES) 453.1 (M+H).

### Reference Example 92

### Methyl 1-(Acetylaminoacetyl)-4-(4-methoxybenzenesulfonyl)-2,3,4,5-tetrahydro-1H-[1,4]benzodiazepine-3-carboxylate

### Reference Example 93

### Methyl 1-(N,N-Dimethylaminoacetyl)-4-(4-methoxybenzenesulfonyl)-2,3,4,5-tetrahydro-1H-[1,4]benxodiazepine-3-carboxylate

### Reference Example 94

### Methyl 1-(Cyclopropylcarbonyl)-4-(4-methoxybenzenesulfonyl)-2,3,4,5-tetrahydro-1H-[1,4]benzodiazepine-3-carboxylate

white crystals, m.p. 98-100°C. Anal. for C₂₂H₂₄N₂O₆S:

| | | | |
|---|---|---|---|
| Calc'd | C,59.5; | H,5.4; | N,6.3; |
| Found | C,59.3; | H,5.6; | N,6.2; |

Mass spectrum (ES) 445.1 (M+H).

### Reference Example 95

### Methyl 1-(Cyclobutylcarbonyl)-4-(4-methoxybenzenesulfonyl)-2,3,4,5-tetrahydro-1H-[1,4]benzodiazepine-3-carboxylate

### Reference Example 96

### Methyl 4-(4-Methoxybenzenesulfonyl)-1-(trifluoroacetyl)-2,3,4,5-tetrahydro-1H-[1,4]benzodiazepine-3-carboxylate

To a solution of 1.0 g (2.54 mmol) of methyl 3-hydroxy-2-{(4-methoxybenzenesulfonyl)-[2-(2,2,2-trifluoroacetylamino)benzyl]amino}propionate in 10 ml of CH₂Cl₂ was added 1.8 ml (12.7 mmol) of trifluoroacetic anhydride. After 1 hour at room temperature, the solvent was removed. Dichloromethane was added several times and the solvent removed under vacuum after each addition. Methanol was then added 2 times and the solvent removed under vacuum to give methyl 2-{(4-methoxybenzenesulfonyl)-[2-(2,2,2-trifluoroacetylamino)benzyl]-amino} acrylate as a glass. The glass was dissolved in methanol and 0.213 g of anhydrous NaHCO₃ was added. The mixture was stirred at room temperature overnight and concentrated under vacuum to dryness. To the residue was added ethyl acetate and water. The organic layer was separated, washed with H₂O, brine and dried with Na₂SO₄. The solvent was removed and the residue (1.0 g) was chromatographed on silica gel thick layer plates with hexane-ethyl acetate (1:1) as solvent to give 0.365 g of product as a glass. Anal. for C₂₀H₁₉F₃N₂O₆S:

| | | | | | |
|---|---|---|---|---|---|
| Calc'd | C,50.9; | H,4.1; | N,5.9; | F,12.1; | S,6.7; |
| Found | C,50.8; | H,4.4; | N,5.5; | F,11.7; | S,6.7; |

Mass spectrum (ES) 473.1 (M+H).

### Reference Example 97

### Methyl 4-(4-Methoxybenzenesulfonyl)-1-(4-methylphenylsulfonyl)-2,3,4,5-tetrahydro-1H-[1,4]benzodiazepine-3-carboxylate

To 0.50 g (1.26 mmol) of 2-[(2-aminobenzyl)-(4-methoxybenzene-sulfonyl)-amino]-3-hydroxypropionate in 5 ml of pyridine cooled to 0°C was added 0.284 g (2.59 mmol) of tosyl chloride. The mixture was stirred at 0°C for 2 hours and then concentrated to remove the solvent. To the residue was added 8 ml of anhydrous ethanol and the mixture refluxed for 2 days. The mixture was concentrated to dryness and ethyl acetate added. The mixture was washed with H₂O, 2 N citric acid, brine and dried with Na₂SO₄. The filtrate was filtered through a thin pad of hydrous magnesium silicate and the filter pad washed with ethyl acetate. The filtrate was concentrated to dryness to give 0.60 g of a foam. Anal. for C₂₅H₂₆N₂O₇S₂:

| | | | | |
|---|---|---|---|---|
| Calc'd | C,56.6; | H,4.9; | N,5.3; | S,12.1; |
| Found | C,56.2; | H,5.2; | N,5.2; | S,11.4; |

Mass spectrum (ES) 531.6 (M+H).

### Reference Example 98

### Methyl 2-[(4-Methoxybenzenesulfonyl)-(2-methylsulfonylaminobenzyl)amino]acrylate

To a solution of 1.0 g (2.54 mmol) of methyl [(2-aminobenzyl)-(4-methoxybenzenesulfonyl)amino]-3-hydroxypropionate in 10 ml of pyridine cooled to -5°C was added 0.432 ml (5.58 mmol) of methanesulfonyl chloride. The mixture was stirred at 0°C for 48 hours. To the mixture was added ice and H₂O and the mixture was extracted with ethyl acetate. The extract was washed with H₂O, 2 N citric acid, brine and dried with Na₂SO₄. The solvent was removed under vacuum and the residue triturated with ethyl acetate-hexane to give 0.90 g of a solid, 128-142°C. Anal. for C₁₉H₂₂N₂O₇S₂:

| | | | | |
|---|---|---|---|---|
| Calc'd | C,50.2; | H,4.9; | N,6.2; | 5,14.1; |
| Found | C,49.6; | H,5.0; | N,6.9; | S,14.0; |

Mass spectrum (ES) 455.5 (M+H).

### Reference Example 99

### Methyl 1,4-Bis-(4-Methoxybenzenesulfonyl)-2,3,4,5-tetrahydro-1H-[1,4]benzodiazepine-3-carboxylate

To a solution of 1.0 g (2.34 mmol) of methyl 2-[(2-aminobenzyl)-(4-methoxybenzenesulfonyl)amino]-3-hydroxypropionate in 6 ml of pyridine cooled to 0°C to -5°C was added 1.07 (5.18 mmol) of 4-methoxybenzenesulfonyl chloride. After 2 hours, the mixture was concentrated to dryness under vacuum. To the residue was added 12 ml of ethanol and the mixture refluxed overnight. The solvent was removed under vacuum and the residue chromatographed on silica gel thick layer plates with ethyl acetate-hexane (1:1) as solvent to give 0.83 g (60%) of product as a white foam; Anal. calc'd for C₂₅H₂₆N₂O₈S₂: C,54.9; H,4.8; N,5.1; S,11.7. Found: C,54.8; H,4.9; N,5.0; S,11.5; Mass spectrum (ES) 547.1 (M+H); and a second component (0.38 g) methyl 2-{[2-(4-methoxybenzenesulfonyl)aminobenzyl]-(4-methoxy-benzenesulfonyl)amino}-3-hydroxypropionate. Anal. for C₂₅H₂₈N₂O₉S₂:

| | | | | |
|---|---|---|---|---|
| Calc'd | C,53.2; | H,5.0; | N,5.0; | S,11.4; |
| Found | C,51.8; | H,5.1; | N,4.7; | S,11.3; |

Mass spectrum (ES) 565.2 (M+H).

### Reference Example 100

### Methyl 1-Acetyl-4-(4-methoxybenzenesulfonyl)-2,3,4,5-tetrahydro-1H-[1,4]benzodiazepine-3-carboxylate

To a solution of 0.70 g (1.52 mmol) of methyl 2-[(2-diacetylaminobenzyl)-(4-methoxybenzenesulfonyl) amino]acrylate in 5 ml of anhydrous methanol was added 0.332 g (3.95 mmol) of anhydrous sodium bicarbonate. The mixture was stirred at room temperature overnight and the solvent removed under vacuum. To the residue was added ethyl acetate and H₂O. The organic layer was separated, washed with brine and dried with Na₂SO₄. The solvent was removed and the residue dried under vacuum to give 0.59 g of white crystals, m.p. 150-155°C. Anal. for C₂₀H₂₂N₂O₆S:

| | | | | |
|---|---|---|---|---|
| Calc'd | C,57.4; | H,5.3; | N,6.7; | S,7.7; |
| Found | C,56.6; | H,5.2; | N,6.5; | S,7.5; |

Mass spectrum (ES) 419.9 (M+H).

### Reference Example 101

### Methyl 3-Acetoxy-2-[(2-diacetylaminobenzyl)-(4-methoxybenzenesulfonyl)amino]propionate

A mixture of 1.0 g (2.54 mmol) of methyl 2-[(2-aminobenzyl)-(4-mcthoxybenzenesulfonyl)amino]-3-hydroxypropionate and 1.3 ml of acetic anhydride in 8 ml of toluene was heated at 100°C for 2 hours. The mixture was concentrated and 3 ml of acetic anhydride added thereto. The mixture was heated at 100°C overnight and concentrated to dryness under high vacuum to give an oil. The oil was dried at 75°C under vacuum for 48 hours to give 1.2 g of a yellow oil. Anal. for C₂₄H₂₈N₂O₉S:

| | | | | |
|---|---|---|---|---|
| Calc'd | C,54.5; | H,5.2; | N,5.5; | S,6.2; |
| Found | C,54.6; | H,5.1; | N,5.4; | S,6.4; |

Mass spectrum (ES) 520.8 (M+H).

### Reference Example 102

### Methyl 2-[(2-Diacetylaminobenzyl)-(4-methoxybenzenesulfonyl)amino]acrylate

A mixture of 1.0 g (1.97 mmol) of methyl 3-acetoxy-2-[(2-diacetylaminobenzyl)-(4-methoxybenzenesulfonyl)amino]propionate and 0.826 ml (5.92 mmol) of triethylamine in 5 ml of CH₂Cl₂ was stirred at room temperature overnight. The solution was diluted with 30 ml of CH₂Cl₂ and washed with 20 ml each of H₂O, 2 N citric acid, brine and dried with Na₂SO₄. The solvent was removed under vacuum to give a brown oil. Anal. for C₂₂H₂₄N₂O₇S:

| | | | | |
|---|---|---|---|---|
| Calc'd | C,57.4; | H,5.3; | N,6.1; | S,7.0; |
| Found | C,56.2; | H,5.5; | N,5.6; | S,7.2. |

### Reference Example 103

### Methyl 2-{(4-Methoxybenzenesulfonyl)-[2-(2,2,2-trifluoroacetylamino)benzyl]amino}acrylate

To a suspension of 1.0 g (2.54 mmol) of methyl 2-[(2-aminobenzyl)-(4-methoxybenzenesulfonyl)amino]-3-hydroxypropionate in 10 ml of toluene was added 1.8 ml (12.7 mmol) of trifluoroacetic anhydride (solid dissolves). The solution was stirred for 2 hours at room temperature and heated at 100°C overnight. The mixture was then concentrated to dryness under vacuum. To the residue was added 0.9 ml of trifluoroacetic anhydride and the solution stirred at room temperature for 1.5 hours and concentrated to dryness. To the residue was added 10 ml of toluene and the mixture refluxed for 2 hours. The solution was cooled to room temperature and 2.5 ml of triethylamine added and the mixture stirred at room temperature overnight. The solution was concentrated to dryness and the residue dissolved in ethyl acetate. The ethyl acetate was washed with H₂O, brine and dried (Na₂SO₄). The solvent was removed under vacuum to give 1.0 g of colorless oil. Crystallization from ethyl acetate-hexane gave 0.625 g of colorless crystals, m.p. 120-121°C. Anal. for C₂₀H₁₉F₃N₂O₆S:

| | | | | | |
|---|---|---|---|---|---|
| Calc'd | C,50.9; | H,4.1; | N,5.9; | S,6.7; | F,12.1; |
| Found | C,50.8; | H,4.2; | N,5.6; | S,6.8; | F,11.9; |

Mass spectrum (ES) 473.1 (M+H).

### Reference Example 104

### 4-(4-Methoxybenzenesulfonyl)-1-(2-methyl-5-fluorobenzoyl)-2,3,4,5-tetrahydro-1H-[1,4]benzodiazepine-3-carboxytic Acid

To a mixture of 1.9 g (3.71 mmol) of methyl 4-(4-methoxybenzenesulfonyl)-1-(2-methyl-5-fluorobenzoyl)-2,3,4,5-tetrahydro-1H-[1,4]benzodiazepine-3-carboxylate in 10 ml of tetrahydrofuran was added 5 ml (4.82 mmol) of 1 N NaOH. The mixture was stirred at room temperature for 1.5 hours and the solvent removed under vacuum. To the residue was added ethyl acetate and the mixture neutralized with I N HCl. The organic layer was separated, washed with brine and dried with Na₂SO₄. The solvent was removed under vacuum to give 1.41 g of white solid. Anal. for C₂₅H₂₃FN₂O₆S:

| | | | | | |
|---|---|---|---|---|---|
| Calc'd | C,60.2; | H,4.7; | N,5.6; | | |
| Found | C,60.2; | H,4.8; | N,5.4 | S,6.4; | F,3.6; |

Mass spectrum (ES) 497.5 (M-H).

Utilizing the method described in Reference Example 104, the following benzodiazepine-3-carboxylic acids can be prepared.

### Reference Example 105

### 4-(4-Methoxybenzenesulfonyl)-1-(4-methylphenylsuifonyl)-2,3,4,5-tetrahydro-1H-[1,4]benzodiazepine·3-carboxylic acid

white foam. Anal. for C₂₄H₂₄N₂O₇S₂:

| | | | |
|---|---|---|---|
| Calc'd | C,55.8; | H,4.7; | N,5.4; |
| Found | C,53.9; | H,5.1; | N,4.8; |

Mass spectrum (ES) 512.2 (M+H).

### Reference Example 106

### 1,4-Bis-(4-methoxybenzenesulfonyl)-2,3,4,5-tetrahydro-1H-[1,4]benzodiazepine-3-carboxylic add

off-white solid. Anal. for C₂₄H₂₄N₂O₈S₂:

| | | | |
|---|---|---|---|
| Calc'd | C,54.1; | H,4.5; | N,5.3; |
| Found | C,52.4; | H,4.8; | N,4.7; |

Mass spectrum (ES) 533.1 (M+H).

### Reference Example 107

### 1-Methanesulfonyl-4-(4-methoxybenzenesulfonyl)-2,3,4,5-tetrahydro-1H-[1,4]benzodiazepine-3-carboxylic acid

white solid. Anal. for C₁₈H₂₀N₂O₇S₂:

| | | | |
|---|---|---|---|
| Calc'd | C,49.1; | H,4.6; | N,6.3; |
| Found | C,47.5; | H,5.0; | N,5.5; |

Mass spectrum (ES) 441.1 (M+H).

### Reference Example 108

### 1-Benzoyl-4-(4-methoxybenzenesulfonyl)-2,3,4,5-tetrahydro-1H-[1,4]benzodiazepine-3-carboxylic acid

white foam. Anal. for C₂₄H₂₂N₂O₆S:

| | | | |
|---|---|---|---|
| Calc'd | C,61.5; | H,5.2; | N,6.0; |
| Found: | C,60.8; | H,5.2; | N,5.7; |

Mass spectrum (ES) 467.9 (M+H).

### Reference Example 109

### 1-Acetyl-4-(4-methoxybenzenesulfonyl)-2,3,4,5-tetrahydro-1H-[1,4]benzodiazepine-3-carboxylic acid

white solid; Anal. for C₁₉H₂₂N₂O₆S:

| | | | | |
|---|---|---|---|---|
| Calc'd | C,56.4; | H,5.0; | N,6.9; | |
| Found | C,55.2; | H,4.9; | N,6.6; | S,7.8; |

Mass spectrum (ES) 404.9 (M+H).

### Reference Example 110

### 4-(4-Methoxybenzenesulfonyl)-1-(3-pyridinylcarbonyl)-2,3,4,5-tetrahydro-1H-[1,4]benzodiazepine-3-carboxylic acid

white solid; m.p. 250-255. Anal. for C2₃H₂₁N₃O₆S:

| | | | |
|---|---|---|---|
| Calc'd | C,59.1; | H,4.5; | N,9.0; |
| Found | C,58.3; | H,4.7; | N,8.3; |

Mass spectrum (ES); 468.2 (M+H).

### Reference Example 111

### 4-(4-Methoxybenzenesulfonyl)-1-(2-thienylcarbonyl)-2,3,4,5-tetrahydro-1H- [1,4]benzodiazepine-3-carboxylic acid

white solid; Anal. for C₂₂H₂₀N₂O₆S₂:

| | | | |
|---|---|---|---|
| Calc'd | C,55.9; | H,4.3; | N,5.9; |
| Found | C,54.9; | H,4.4; | N,5.4; |

Mass spectrum (ES) 473.1 (M+H).

### Reference Example 112

### 1-Methoxyacetyl-4-(4-methoxybenzenesalfonyl)-2,3,4,5-tetrahydro-1H-[1,4]benzodiazepine-3-carboxylic acid

white crystals, m.p. 193-194°C. Anal. for C₂₀H₂₂N₂O₇S:

| | | | |
|---|---|---|---|
| Calc'd | C,55.3; | H,5.1; | N,6.5; |
| Found | C,55.1; | H,4.9; | N,6.2; |

Mass spectrum (ES) 433.1 (M-H).

### Reference Example 113

### 4-(4-Methoxybenzenesulfonyl)-1-(4-pyridinylcarbonyl)-2,3,4,5-tetrahydro-1H-[1,4]benzodiazepine-3-carboxylic acid

white crystals, m.p. 258-261°C. Anal. for C₂₃H₂₁N₃O₆S:

| | | | |
|---|---|---|---|
| Calc'd | C,59.1; | H,4.5; | N,9.0; |
| Found | C,58.8; | H,4.5; | N,8.8; |

Mass spectrum (ES) 483.3 (M+H).

### Reference Example 114

### 1-(4-Biphenylcarbonyl)-4-(4-methoxybenzenesulfonyl)-2,3,4,5-tetrahydro-1H-[1,4]benzodiazepine-3-carboxylic acid

white foam. Anal. for C₃₀H₂₆N₂O₆S:

| | | | |
|---|---|---|---|
| Calc'd | C,66.4; | H,4.8; | N,5.2; |
| Found | C,64.7; | H,5.2; | N,4.8; |

Mass spectrum (ES) 543.6 (M+H).

### Reference Example 115

### 4-(4-Methoxybenzenesulfonyl)-1-(propane-1-sulfonyl)-2,3,4,5-tetrahydro-1H-[1,4]benzodiazepine-3-carboxylic acid

white foam. Anal. for C₂₀H₂₄N₂O₇S₂:

| | | | |
|---|---|---|---|
| Calc'd | C,51.3; | H,5.2; | N,6.0; |
| Found | C,50.3; | H,5.3; | N,5.7; |

Mass spectrum (ES) 467.3 (M-H).

### Reference Example 116

### 1-([1,1'-Biphenyl]-2-carbonyl)-4-(4-methoxybenzenesulfonyl)-2,3,4,5-tetrahydro-1H-[1,4]benzodiazepine-3-carboxylic acid

white foam; m.p. 106-145°C. Anal. for C₃₀H₂₆N₂O₆S:

| | | | |
|---|---|---|---|
| Calc'd | C,66.4; | H,4.8; | N,5.2; |
| Found | C,65.7; | H,5.0; | N,4.8; |

Mass spectrum (ES) 541.1 (M-H).

### Reference Example 117

### 1-(3-Fluorobenzoyl)-4-(4-methoxybenzenesulfonyl)-2,3,4,5-tetrahydro-1H-[1,4]benzodiazepine-3-carboxylic acid

### Reference Example 118

### 4-(4-Methoxybenzenesulfonyl)-1-(2-methyl-3-fluorobenzoyl)-2,3,4,5-tetrahydro-1H-[1,4]benzodiazepine-3-carboxylic acid

### Reference Example 119

### 4-(4-Methoxybenzenesulfonyl)-1-(3-phenylpropionyl)-2,3,4,5-tetrahydro-1H-[1,4]benzodiazepine-3-carboxylic acid

white solid. Anal. for C₂₆H₂₆N₂O₆S:

| | | | |
|---|---|---|---|
| Calc'd | C,63. 1; | H,5.3; | N,5.7; |
| Found | C,61.5; | H,5.4; | N,5.2; |

Mass spectrum (ES) 493.2 (M-H).

### Reference Example 120

### 4-(4-Methoxybenzenesulfonyl)-1-(2-trifluoromethylbenzoyl)-2,3,4,5-tetrahydro-1H-[1,4]benzodiazepine-3-carboxylic acid

### Reference Example 121

### 1-(2-Chloro-6-trifluoromethylbenzoyl)-4-(4-methoxybenzenesulfonyl)-2,3,4,5-tetrahydro-1H-[1,4]benzodiazepine-3-carboxylic acid

### Reference Example 122

### 1-(4-Fluoro-2-trifluoromethylbenzoyl)-4-(4-methoxybenzenesulfonyl)-2,3,4,5-tetrahydro-1H-[1,4]benzodiazepine-3-carboxylic acid

### Reference Example 123

### 1-(2-Fluoro-6-tritluoromethylbenzoyl)-4-(4-methoxybenzenesulfonyl)-2,3,4,5-tetrahydro-1H-[1,4]benzodiazepine-3-carboxylic acid

### Reference Example 124

### 4-(4-Methoxybenzenesulfonyl)-1-(2-methylbenzoyl)-2,3,4,5-tetrahydro-1H-[1,4]benzodiazepine-3-carboxylic acid

### Reference Example 125

### 4-(4-Methoxybenzenesulfonyl)-1-(2-methyl-6-chlorobenzoyl)-2,3,4,5-tetrahydro-1H-[1,4)benzodiazepine-3-carboxylic acid

### Reference Example 126

### 1-(2,4-Dimethylbenzoyl)-4-(4-methoxybenzenesulfonyl)-2,3,4,5-tetrahydro-1H-[1,4]benzodiazepine-3-carboxylic acid

### Reference Example 127

### 1-(2,5-Dimethylbenzoyl)-4-(4-methoxybenzenesulfonyl)-2,3,4,5-tetrahydro-1H-[1,4]benzodiazepine-3-carboxylic acid

### Reference Example 128

### 1-(2-Chloro-4-fluorobenzoyl)-4-(4-methoxybenzenesulfonyl)-2,3,4,5-tetrahydro-1H-[1,4]benzodiazepine-3-carboxylic acid

### Reference Example 129

### 1-(2-Chlorobenzoyl)-4-(4-methoxybenzenesulfonyl)-2,3,4,5-tetrahydro-1H-[1,4]benzodiazepine-3-carboxylic acid

### Reference Example 130

### 1-(2-Fluorobenzoyl)-4-(4-methoxybenzenesulfonyl)-2,3,4,5-tetrahydro-1H-[1,4]benzodiazepine-3-carboxylic acid

### Reference Example 131

### 1-(2-Chloro-6-fluorobenzoyl)-4-(4-methoxybenzenesulfonyl)-2,3,4,5-tetrahydro-1H-[ 1,4]benzodiazepine-3-carboxylic acid

### Reference Example 132

### 1-(2,3-Difluorobenzoyl)-4-(4-methoxybenzenesulfonyl)-2,3,4,5-tetrahydro-1H-[1,4]benzodiazepine-3-carboxylic acid

### Reference Example 133

### 1-(2,4-Dichlorobenzoyl)-4-(4-methoxybenzenesulfonyl)-2,3,4,5-tetrahydro-1H-[1,4]benzodiazepine-3-carboxylic acid

white solid. Anal. for C₂₄H₂₀Cl₂N₂O₆S:

| | | | |
|---|---|---|---|
| Calc'd | C,53.8; | H,3.8; | N,5.2; |
| Found | C,52.8; | H,3.9; | N,4.9; |

Mass spectrum (ES) 533 (M-H).

### Reference Example 134

### 1-(2,3-Dichlorobenzoyl)-4-(4-methoxybenzenesulfonyl)-2,3,4,5-tetrahydro-1H-[1,4]benzodiazepine-3-carboxylic acid

### Reference Example 135

### 1-(2,5-Dichlorobenzoyl)-4-(4-methoxybenzenesulfonyl)-2,3,4,5-tetrahydro-1H-[1,4]benzodiazepine-3-carboxylic acid

### Reference Example 136

### 1-(2-Methoxybenzoyl)-4-(4-methoxybenzenesulfonyl)-2,3,4,5-tetrahydro-1H-[1,4]benzodiazepine-3-carboxylic acid

### Reference Example 137

### 1-(4-Chloro-2-methoxybenzoyl)-4-(4-methoxybenzenesulfonyl)-2,3,4,5-tetrahydro-1H-[1,4]benzodiazepine-3-carboxylic acid

### Reference Example 138

### 4-(4-Methoxybenzenesulfonyl)-1-(2-methylthiobenzoyl)-2,3,4,5-tetrahydro-1H-[1,4]benzodiazepine-3-carboxylic acid

### Reference Example 139

### 4-(4-Methoxybenzenesulfonyl)-1-(3-methyl-2-thienylcarbonyl)-2,3,4,5-tetrahydro-1H-[1,4]benzodiazepine-3-carboxylic acid

### Reference Example 140

### 4-(4-Methoxybenzenesulfonyl)-1-(4-methyl-2-thienylcarbonyl)-2,3,4,5-tetrahydro-1H-[1,4]benzodiazepine-3-carboxylic acid

### Reference Example 141

### 1-(3-Chloro-2-thienylcarbonyl)-4-(4-methoxybenzenesulfonyl)-2,3,4,5-tetrahydro-1H-[1,4]benzodiazepine-3-carboxylic acid

### Reference Example 142

### 1-(2-Furanylcarbonyl)-4-(4-methoxybenzenesulfonyl)-2,3,4,5-tetrahydro-1H-[1,4]benzodiazepine-3-carboxylic acid

white solid. Anal. for C₂₂H₂₀N₂O₇S:

| | | | |
|---|---|---|---|
| Calc'd | C, 57.9; | H, 4:4; | N, 6.1; |
| Found | C, 56.5; | H, 4.5; | N, 5.7; |

Mass spectrum (ES) 455.1 (M-H).

### Reference Example 143

### 4-(4-Methoxybenzenesulfonyl)-1-(3-methyl-2-furanylcarbonyl)-2,3,4,5-tetrahydro-1H-[1,4]benzodiazepine-3-carboxylic acid

### Reference Example 144

### 4-(4-Methoxybenzenesulfonyl)-1-(4-methyl-2-furanylcarbonyl)-2,3,4,5-tetrahydro-1H-[1,4]benzodiazepine-3-carboxylic acid

### Reference Example 145

### 1-(5-Chloro-2-furanylcarbonyl)-4-(4-methoxybenzenesulfonyl)-2,3,4,5-tetrahydro-1H-[1,4]benzodiazepine-3-carboxylic acid

### Reference Example 146

### 1-(5-Chloro-2-thienylcarbonyl)-4-(4-methoxybenzenesulfonyl)-2,3,4,5-tetrahydro-1H-[1,4]benzodiazepine-3-carboxylic acid

### Reference Example 147

### 1-Propionyl-4-(4-methoxybenzenesulfonyl)-2,3,4,5-tetrahydro-1H-[1,4]benzodiazepine-3-carboxylic acid

### Reference Example 148

### 1-Hexanoyl-4-(4-methoxybenzenesulfonyl)-2,3,4,5-tetrahydro-1H-[1,4]benzodiazepine-3-carboxylic acid

### Reference Example 149

### 1-(3-Methoxypropionyl)-4-(4-methoxybenzenesulfonyl)-2,3,4,5-tetrahydro-1H-[1,4]benzodiazepine-3-carboxylic acid

### Reference Example 150

### 4-(4-Methoxybenzenesulfonyl)-1-(3-thienylcarbonyl)-2,3,4,5-tetrahydro-1H-[1,4]benzodiazepine-3-carboxylic acid

### Reference Example 151

### 4-(3-Furanylcarbonyl)-4-(4-methoxybenzenesulfonyl)-2,3,4,5-tetrahydro-1H-[1,4]benzodiazepine-3-carboxylic acid

### Reference Example 152

### 1-(trans-Crotonyl)-4-(4-methoxybenzenesulfonyl)-2,3,4,5-tetrahydro-1H-[1,4]benzodiazepine-3-carboxylic acid

### Reference Example 153

### 1-(Methacryloyl)-4-(4-methoxybenzenesulfonyl)-2,3,4,5-tetrahydro-1H-[1,4]benzodiazepine-3-carboxylic acid

### Reference Example 154

### 1-(Pyrrolidinoacetyl)-4-(4-methoxybenzenesulfonyl)-2,3,4,5-tetrahydro-1H-[1,4]benzodiazepine-3-carboxylic acid

### Reference Example 155

### 1-(Acetylaminoacetyl)-4-(4-methoxybenzenesulfonyl)-2,3,4,5-tetrahydro-1H-[1,4]benzodiazepine-3-carboxylic acid

### Reference Example 156

### 1-(Cyclopropylcarbonyl)-4-(4-methoxybenzenesulfonyl)-2,3,4,5-tetrahydro-1H-[1,4]benzodiazepine-3-carboxylic acid

white crystals, m.p. 131-135°C. Anal. for C₂₁H₂₂N₂O₆S:

| | | | |
|---|---|---|---|
| Calc'd | C,58.6; | H,5.2; | N,6.5; |
| Found | C,58.1; | H,5.5; | N,5.8; |

Mass spectrum (ES) 431.5 (M+H).

### Reference Example 157

### 1-(Cyclobutylcarbonyl)-4-(4-methoxybenzenesulfonyl)-2,3,4,5-tetrahydro-1H-[1,4]benzodiazepine-3-carboxylic acid

### Reference Example 158

### 1-(Cyclohexylcarbonyl)-4-(4-methoxybenzenesulfonyl)-2,3,4,5-tetrahydro-1H-[1,4]benzodiazepine-3-carboxylic acid

white solid. Anal. for C₂₄H₂₈N₂O₆S:

| | | | |
|---|---|---|---|
| Calc'd | C,61.0; | H,6.0; | N,5.9; |
| Found | C,57.0; | H,5.7; | N,5.4; |

Mass spectrum (ES) 471.5 (M-H).

### Reference Example 159

### (D,L)N-(4-Methoxybenzenesulfonyl)-O-(2-tetrahydropyranyl)serine, Methyl ester

A mixture of 1.44 g (5 mmol) of N-(4-methoxybenzenesulfonyl)serine, methyl ester; 1.05 g (12.5 mmol) of 3,4-dihydro-2H-pyran and 9.5 mg of 4-methylbenzene-sulfonic acid monohydrate in 5 ml of tetrahydrofuran was refluxed overnight and the mixture was concentrated to dryness under vaccum. The residue was extracted with CH₂Cl₂ and the extract washed with 2 N NaHCO₃, brine and dried with Na₂SO₄. The solution was filtered through a thin pad of hydrous magnesium silicate and the filter pad washed with CH₂Cl₂. The filtrate was concentrated to dryness and the residue (2.3 g) was extracted with three 50 ml portions of hot hexane to give 1.92g of product as a yellow oil; Mass spectrum (ES) 374.4 (MH⁺).

### Reference Example 160

### Methyl 3-Hydroxy-2-{[4-methoxybenzenesulfonyl]-[2-(4-morpholinocarbonylamino)benzyl]amino}propionate

To a mixture of 1.0 g (2.54 mmol) of methyl 2-[(2-aminobenzyl)-(4-methoxybenzenesulfonyl)amino]-3-hydroxypropionate in 8 ml of pyridine chilled at 0° to -10°C was added 740 µL (6.34 mmol) of morpholinocarbonyl chloride. The mixture was kept at 0° to 5°C overnight. The mixture was concentrated under vacuum and diluted with ethyl acetate. The solution was washed with H₂O, 2 N citric acid, and brine and dried with Na₂SO₄. The solvent was removed under vacuum to give 1.61 g of solid (yellow-orange foam). The solid was chromatographed on thick layer silica gel plates with hexane-ethyl acetate (1:3) as solvent to give 0.86 g of solid. Anal. for C₂₃H₂₉N₃O₈S:

| | | | |
|---|---|---|---|
| Calc'd | C,54.4; | H,5.8; | N,8.3; |
| Found | C,53.9; | H,5.7; | N,8.1; |

Mass spectrum (ES) 508.4 (M+H).

### Reference Example 161

### Methyl 2-{(4-Methoxybenzenesulfonyl)-[2-(4-morpholinocarbonylamino)benzyl]amino}acrylate

To a solution of 0.70 g (1.38 mmol) of methyl 3-hydroxy-2-{[4-methoxybenzenesulfonyl]-[2-(4-morpholinocarbonylamino)benzyl]amino}propionate and 769 µL (5.54 mmol) of triethylamine in 8 ml of CH₂Cl₂, cooled to 0°C, was added 0.386 g (2.03 mmol) of 4-methylbenzenesulfonyl chloride. The mixture was stirred at room temperature for 2 hours, diluted with water and extracted with CH₂Cl₂. The extract was washed with 2 N citric acid, brine and dried with Na₂SO₄. The solvent was removed to give 0.67 g of a yellow oil. Anal. for C₂₃H₂₇N₃O₇S:

| | | | | |
|---|---|---|---|---|
| Calc'd | C,56.4; | H,5.6; | N,8.6; | S,6.6; |
| Found | C,56.1; | H,5.8; | N,8.3; | S,6.6. |

### Reference Example 162

### Methyl 4-(4-Methoxybenzenesulfonyl)-1-(4-morpholinocarbonyl)-2,3,4,5-tetrahydro-1H-[1,4]-benzodiazepine-3-carboxylate

A mixture of 0.50 g (1.02 mmol) of methyl 2-{(4-methoxybenzenesulfonyl)-[2-(4-morpholinocarbonyl-amino)benzyl]amino}acrylate and 0.111 g (1.32 mmol) of anhydrous NaHCO₃ in 5 ml of anhydrous methanol was stirred at room temperature for 16 hours. An additional 55 mg of NaHCO₃ was added and the mixture stirred at room temperature for 2 hours. The solvent was removed under vacuum and the residue diluted with H₂O and extracted with ethyl acetate. The extract was washed with brine and dried with Na₂SO₄. The solvent was removed and the residue triturated with hexane-ethyl acetate to give 0.36 g of a yellow solid; Anal. calc'd for C₂₃H₂₇N₃O₇S: C,56.4; H,5.6; N,8.6; S,6.6. Found: C,56.5; H,5.7; N,8.4; S,6.7; Mass spectrum (ES) 490.3 (M+H).

### Reference Example 163

### 4-(4-Methoxybenzenesulfonyl)-1-(4-morpholinocarbonyl)-2,3,4,5-tetrahydro-1H-[1,4]benzodiazepine-3-carboxylic Acid

A mixture of 0.36 g (0.735 mmol) of methyl 4-(4-methoxybenzenesulfonyl)-1-(4-morpholinocarbonyl)-2,3,4,5-tetrahydro-1 H-[1,4]benzodiazepine-3-carboxylate and 1 ml (0.95 mmol) of 1 N NaOH in 5 ml of tetrahydrofuran was stirred at room temperature for 1 hour. The mixture was concentrated under vacuum and the acidified with 1 N HCl and cooled. The mixture was filtered and the solid washed with water to give 0.26 g of white solid. Anal. for C₂₂H₂₅N₃O₇S:

| | | | |
|---|---|---|---|
| Calc'd | C,55.6; | H,5.3; | N,8.8; |
| Found | C,53.5; | H,5.6; | N,8.3; |

Mass spectrum (ES) 474.3 (M-H).

### Reference Example 164

### Methyl 3-[(2-Tetrahydropyranyl)oxy]-2-[(4-methoxybenzenesulfonyl)-(2-nitro-4-chlorobenzyl)amino]propionate

To a mixture of 1.67 g (4.4 mmol) of (D,L) N-(4-methoxybenzenesulfonyl)-O-(2-tetrahydropyranyl) serine, methyl ester, 0.825 g (4.4 mol) of 4-chloro-2-nitrobenzyl alcohol and 1.16 g (4.4 mmol) of triphenylphosphine in 4.5 ml of tetrahydrofuran was added dropwise a solution of 0.766 g (4.4 mmol) of diethyl azodicarboxylate in 1 ml of tetrahydrofuran. The mixture was stirred at room temperature overnight and the solvent removed under vacuum. The residue was triturated with diethyl ether, filtered and the filtrate passed through a thin pad of hydrous magnesium silicate. The pad was washed with ethyl acetate and the total filtrate concentrated to dryness under vacuum to give 4.54 g of solid. The solid was chromatographed on silica gel with hexane-ethyl acetate (55:45) as solvent. The fractions containing product were combined and the solvent removed to give 0.55 g of oily solid; Mass spectrum (ES) 543.1 (M+H).

### Reference Example 165

### Methyl 2-{[2-(4-Pyridinylmethyleneamino)benzyl]-[4-methoxybenzenesulfonyl]amino}-3-hydroxypropionate

A mixture of 0.50 g (1.268 mmol) of methyl 2-[(2-aminobenzyl)-(4-methoxybenzenesulfonyl)amino]-3-hydroxypropionate and 1.268 mmol of 4-pyridinecarboxaldehyde in 7 ml of anhydrous ethanol was refluxed for 1.5 hours and the mixture concentrated under vacuum to dryness. To the residue was added H₂O and ethyl acetate. The ethyl acetate layer was separated and concentrated to dryness under vacuum. The solid was purified by thick layer chromatography on silica gel with hexane-ethyl acetate as solvent to give 0.40 g of solid product (plus a small amount of starting material). Anal. for C₂₄H₂₅N₃O₆S:

| | | | |
|---|---|---|---|
| Calc'd | C,59.6; | H,5.2; | N,8.7; |
| Found | C,57.6; | H,5.7; | N,7.4; |

Mass spectrum (ES) 484 (M+H)-product; 395.1 (M+H)-starting material.

### Reference Example 166

### Methyl 1-(Cyclohexylcarbonyl)-4-(4-methoxybenzenesulfonyl)-2,3,4,5-tetrahydro-1H-[1,4]benzodiazepine-3-carboxylate

To a solution of 0.80 g (1.64 mmol) of methyl 2-{[2-(cyclohexylcarbonyl)-aminobenzyl]-(4-methoxybenzenesulfonyl)amino}acrylate in 10 ml of methanol was added 0.207 g (2.46 mmol) of anhydrous sodium bicarbonate. The mixture was stirred for 2 days and then an additional 0.207 g of NaHCO₃ added. The mixture was stirred overnight and the solvent removed under vacuum. To the residue was added H₂O and ethyl acetate and the organic layer separated. The ethyl acetate extract was washed with brine, dried with Na₂SO₄ and the solvent removed under vacuum to give 0.83 g of the product as a yellow oil. Anal. for C₂₅H₃₀N₂O₆S:

| | | | |
|---|---|---|---|
| Calc'd | C,61.7; | H,6.2; | N,5.8; |
| Found | C,61.0; | H,6.4; | N,5.3; |

Mass spectrum (ES) 487.0 (M+H).

### Reference Example 167

### Methyl 3-Hydroxy-2-[(4-methoxybenzenesulfonyl)-(4-chloro-2-nitrobenzyl)amino]propionate

To a solution of 0.289 g (1 mmol) of methyl 3-hydroxy-2-(4-methoxybenzenesulfonylamino)propionate in 4 ml of N,N-dimethylformamide cooled in an ice bath was added 40 mg of NaH (60% in oil) (1 mmol). After the gas evolution ceased, 0.165 g (1.1 mmol) of sodium iodide was added, followed by the addition of 0.226 g (1.1 mmol) of 4-chloro-2-nitrobenzyl chloride in I ml of dimethylformamide. The solution became purple and was stirred at room temperature over the weekend. The solvent was removed under vacuum and the residue extracted with CH₂Cl₂. The extract was washed with H₂O, brine and dried with Na₂SO₄. The solvent was removed to give 0.53 g of solid which was chromatographed on thick layer silica gel plates with hexane-ethyl acetate (2:1) as solvent to give 0.143 g (31 %) of product, as crystals, m.p. 112°-114°C. Anal. for C₁₈H₁₉ClN₂O₈S:

| | | | |
|---|---|---|---|
| Calc'd | C,47.2; | H,4.2; | N,6.1; |
| Found | C,47.0; | H,4.1; | N,6.0; |

Mass spectrum (ES) 459.2 (M+H).

### Reference Example 168

### Methyl 3-Hydroxy-2-[(4-methoxybenzenesulfonyl)-(4-chloro-2-aminobenzyl)amino]propionate

A mixture of 0.454 g (1 mmol) of methyl 3-hydroxy-2-[(4-methoxy-benzenesulfonyl)-(4-chloro-2-nitrobenzyl)amino]propionate and 0.451 g (2 mmol) of SnCl₂•2H₂O in 12 ml of methanol was refluxed for 2 hours. An additional 0.451 g (2 mmol) of SnCl₂•2H₂O was added and the mixture refluxed for 2 hours. The solvent was removed and ethyl acetate added. The mixture was neutralized with 1 N NaHCO₃ and then stirred for 1 hour and filtered. The ethyl acetate layer was separated and washed with H₂O, brine and dried with Na₂SO₄. The solvent was removed to give 0.42 g of solid which was chromatographed on thick layer silica gel plates with hexane-ethyl acetate (45:55) as solvent to give 60 mg of product (R_{F} 0.66) as a glass, m.p. 99°-112°C. Anal. for C₁₈H₂₁ClN₂O₆S:

| | | | |
|---|---|---|---|
| Calc'd | C,50.4; | H,4.9; | N,6.5; |
| Found | C,49.7; | H,4.9; | N,6.4; |

Mass spectrum (ES) 429.1 (M+H).

### Reference Example 169

### Methyl 3-Hydroxy-2-[(4-methoxybenzenesulfonyl)-(4-chloro-2-aminobenzyl)amino]propionate

To a solution of 0.458 g (1 mmol) of methyl 3-hydroxy-2-[(4-methoxybenzenesulfonyl)-(4-chloro-2-nitrobenzyl)amino]propionate in 25 ml of ethanol and 25 ml of ethyl acetate was added 0.045 g of 10% Pd/C (wet - 50% H₂O). The mixture was shaken in a Parr hydrogenator under 35 pounds per square inch of hydrogen for 3 hours. The mixture was filtered through diatomaceous earth and the filtrate was concentrated to dryness under vacuum to give 0.47 g of the product as a solid (approximately 90% pure). Thin layer chromatography on silica gel, NMR and Mass spectrum (ES) 429.1 (M+H) 395.1 (M+H) indicated approximately 10% of deschloro derivative.
A mixture of 4.74 g of methyl 3-hydroxy-2-[(4-methoxybenzenesulfonyl)-(4-chloro-2-aminobenzyl)amino} propionate, and 0.470 g of 10% Pd/C (wet-50% H₂O) in 200 ml of ethyl acetate-ethanol (1: 1) was shaken in a Parr hydrogenator under 35 psi of hydrogen for 4 hours. The mixture was filtered through diatomaceous earth and the solvent removed to give 4.5 g of solid. The solid was chromatographed by HPLC on a Waters Prep machine with a 4 x 30 cm silica gel column with a step gradient of hexane-ethyl acetate (9:1 to 6:4 to 1:1 to 0:100) to give 1.56 g of a glass, m.p. 110°-123°C. Anal. for C₁₈H₂₁ClN₂O₆S:

| | | | | |
|---|---|---|---|---|
| Calc'd | C, 50.4; | H, 4.9; | N, 6.5; | Cl, 8.3; |
| Found | C, 50.3; | H, 4.8; | N, 6.5; | Cl, 7.8. |

### Reference Example 170

### N-(4-Methoxybenzenesulfonyl)-glycine, Methyl Ester

To a mixture of 12.5 g (0.1 mol) of glycine, methyl ester hydrochloride in 120 ml of CH₂Cl₂, cooled in an ice bath was added 41.7 ml (0.3 mol) of triethylamine, followed by the dropwise addition of a solution of 20.65 g (0.1 mol) of 4-methoxybenzenesulfonyl chloride in 40 ml of CH₂Cl₂. The mixture was stirred at room temperature overnight and poured into water. The organic layer was separated and washed with 2 N citric acid, H₂O, 1 N NaHCO₃, brine and dried with Na₂SO₄. The solvent was removed under vacuum to give 24.6 g of residue which was triturated with ethyl acetate to give 19.9 g of crystals, m.p. 59°-61°C. Anal. for C₁₀H₁₃NSO₅:

| | | | |
|---|---|---|---|
| Calc'd | C,46.3; | H,5.1; | N,5.4; |
| Found | C,46.2; | H,5.0; | N,5.2. |

### Reference Example 171

### Methyl 2-[(4-methoxybenzenesulfonyl)-(2-nitrobenzyl)amino]acetate

To a stirred and cooled mixture of 1.2 g (30 mmol) of NaH (58% in oil) in 50 ml of N,N-dimethylformamide was added dropwise a solution of 7.78 g (30 mmol) of N-(4-methoxybenzenesulfonyl)glycine, methyl ester in 40 ml of N,N-dimethylformamide. After gas evolution ceased, a solution of 6.80 g (32 mmol) of 2-nitrobenzyl bromide in 40 ml of N,N-dimethylformamide was added dropwise to the mixture. The mixture was then stirred at room temperature overnight under nitrogen and the solvent removed under vacuum. The residue was extracted with CH₂Cl₂ and the extract washed with H₂O, 2 N citric acid, H₂O, 1 N NaHCO₃, brine and dried with Na₂SO₄. The solution was filtered through a thin pad of hydrous magnesium silicate and the filter pad washed with CH₂Cl₂. The filtrate was concentrated under vacuum to give 11.79 g of solid. Tnturation with ethyl acetate gave 2.64 g (22%) of crystals, m.p. 114°C-116°C. Anal. for C₁₇H₁₈N₂O₇S:

| | | | |
|---|---|---|---|
| Calc'd | C,51.8; | H,4.6; | N,7.1; |
| Found | C,51.7; | H,4.6; | N,7.1. |

From the mother liquors an additional 6.49 g (55%) of product as crystals was obtained by chilling at 0°C and filtering the mother liquors.

### Reference Example 172

### Methyl 2-[(2-Aminobenzyl)-(4-methoxybenzenesulfonyl)amino]acetate

(A) To a mixture of 2.15 g (5.45 mmol) of methyl-2-[(4-methoxy-benzenesulfonyl)-(2-nitrobenzyl)amino]acetate-and 1.57-g (25 mmol) of ammonium formate in 10 ml of anhydrous methanol was added 0.42 g of 10% palladium on carbon. The mixture was stirred at room temperature for 1.5 hours and then filtered through diatomaceous earth. The filtrate was concentrated to dryness under vacuum and the residue diluted with H₂O (25 ml) and extracted with CH₂Cl₂ (75 ml). The extract was washed with brine, dried with Na₂SO₄ and the solvent removed to give 0.45 g of solid. Crystallization from ethyl acetate gave 0.124 g of white crystals, m.p. 100°-102°C. Anal. for C₁₇H₂₀N₂O₅S:

| | | | |
|---|---|---|---|
| Calc'd | C,56.0; | H,5.5; | N,7.7; |
| Found | C,56.1; | H,5.6; | N,7.6. |

(B) To a solution of 4.2 g of methyl 2-[(4-methoxybenzenesulfonyl)-(2-nitrobenzyl)amino]acetate in 200 ml of ethanol-ethyl acetate (1:1) was added 0.42 g of 10% Pd on carbon (wet -50% H₂O) and the mixture shaken in a Parr hydrogenator under 35 pounds per square inch of hydrogen for 4.5 hours at room temperature. The mixture was filtered through diatomaceous earth and the filtrate concentrated to dryness under vacuum to give 4.0 g of crystals, m.p. 100°-102°C.

### Reference Example 173

### 2-[(2-Aminobenzyl)-(4-methoxybenzenesulfonyl)amino]acetic Acid

To a solution of 5.14 g (14.1 mmol) of methyl 2-[(2-aminobenzyl)-(4-methoxybenzenesulfonyl)amino] acetate in 50 ml of methanol-tetrahydrofuran (1:1) was added 2.86 ml of 10 N NaOH and the mixture refluxed for 2 hours. The solvent was removed under vacuum and the residue partitioned between water and ether. The water layer was separated and acidified with 2 N citric acid. The solid was filtered, washed with H₂O and dried in a vacuum oven at room temperature to give 4.45 g (91%) of crystals, m.p. 145°-147°C. Anal. for C₁₆H₁₈N₂O₅S:

| | | | |
|---|---|---|---|
| Calc'd | C,54.9; | H,5.2; | N,8.0; |
| Found | C,55.1; | H,5.2; | N,7.9. |

### Reference Example 174

### Methyl 4-(4-Methoxybenzenesulfonyl)-1-(phenoxyacetyl)-2,3,4,5-tetrahydro-1H-[ 1,4]benzodiazepine-3-carboxylate

To a cooled (0°C) mixture of 1.5 g (3.8 mmol) of methyl 2-[(2-aminobenzyl)-(4-methoxybenzenesulfonyl)amino]-3-hydroxypropionate and 2.7 ml (19 mmol) of triethylamine in 15 ml of CH₂Cl₂ was added 1.58 g (11.4 mol) of phenoxyacetyl chloride. The mixture was stirred at room temperature overnight and filtered. The filtrate was washed with H₂O, 2 N citric acid, and brine and dried with Na₂SO₄. The solvent was removed to give 2.4 g of crude methyl 2-{(4-methoxybenzenesulfonyl)-[2-(phenoxyacetylamino)benzyl]amino}acrylate as an oil. Anal. for C₂₆H₂₆N₂O₇S:

| | | | |
|---|---|---|---|
| Calc'd | C,61.2; | H,5.1; | N,5.5; |
| Found | C,62.6; | H,5.1; | N,4.0; |

Mass spectrum (ES) 511 (M+H).

To a 2.0 g (3.92 mmol) sample of the preceding compound in 15 ml of methanol was added 0.494 g of anhydrous NaHCO₃ and the mixture stirred for 5 hours. The mixture was concentrated under vacuum and ethyl acetate and H₂O were added to the residue. The mixture was filtered and the organic layer of the filtrate separated, washed with brine and dried with Na₂SO₄. The solvent was removed to give 0.36 g of product as off-white crystals, m.p. 151°-153°C. Anal. for C₂₆H₂₆N₂O₇S:

| | | | |
|---|---|---|---|
| Calc'd | C,61.2; | H,5.1; | N,5.5; |
| Found | C,61.1; | H,5.1; | N,5.4; |

Mass spectrum (ES) 511 (M+H).

### Reference Example 175

### 3-hydroxymethyl-4-(4-Methoxybenzenesulfonyl)-1-(3-pyridinylmethyl)-2,3,4,5-tetrahydro-1H-[1,4]benzodiazepine

A mixture of 0.100 g (0.208 mmol) of methyl 4-(4-methoxybenzenesulfonyl)-1-(3-pyridinylcarbonyl)-2,3,4,5-tetrahydro-1H-[1,4]benzodiazepine-3-carboxylate and 3 ml of borane-tetrahydroforan complex in tetrahydrofuran (1.0 M) was refluxed overnight. The solution was cooled to room temperature, diluted with methanol and the solvent removed. Methanol was added several times and, after each addition, the solvent was removed. To the residue was added 1N NaHCO₃. The mixture was stirred for 45 minutes and then extracted with ethyl acetate. The extract was concentrated and then washed with H₂O, brine and dried with Na₂SO₄. The solvent was removed under vacuum and the residue chromatographed on thick layer silica gel plates with 10% methanol in ethyl acetate as solvent to give 60 mg of solid (R_{F} 0.26). Crystallization from ethyl acetate gave 30 mg of white crystals. Anal. for C₂₃H₂₅N₃O₄S:

| | | | | |
|---|---|---|---|---|
| Calc'd | C,62.8; | H,5.7; | N,9.6; | S,7.3; |
| Found | C,61.1; | H,5.6; | N,9.2; | S,7.3; |

Mass spectrum (ES) 440.2 (M+H).

### Reference Example 176

### Methyl 4-(4·Methoxybenzenesulfonyl)-1-(2-methoxypyridinyl-3-carbonyl)-2,3,4,5-tetrahydro-1H-[1,4]benzodiazepine-3-carboxylate

To a cooled (0°C) mixture of 1.0 g (2.54 mmol) of methyl 2-[(2-aminobenzyl)-(4-methoxybenzenesulfonyl) amino]-3-hydroxypropionate and 1.8 ml (12.68 mmol) of triethylamine in 10 ml of CH₂Cl₂ was added 0.957 g (5.58 mmol) of 2-methoxypyridine-3-carbonyl chloride in 4 ml of CH₂Cl₂. The solution was stirred at room temperature overnight, diluted with H₂O and CH₂Cl₂ and the organic layer separated. The organic layer was washed with H₂O, 2 N citric acid, and brine and dried with Na₂SO₄. The solvent was removed under vacuum to give 1.2 g of solid. The solid was chromatographed on thick layer silica gel plates with ethyl acetate-hexane (3:1) as solvent to give 0.27 g of yellow foam. Anal. for C₂₅H₂₅N₃O₇S:

| | | | | |
|---|---|---|---|---|
| Calc'd | C,58.7, | H,4.93; | N,8.21; | |
| Found | C,57.8; | H,4.5; | N,8.3; | S,6.2. |

### Reference Example 177

### 5-Methyl-2-nitrobenzyl Bromide

To a cooled (ice-water bath) mixture of 30% HBr in acetic acid (3 ml) was added 2.5 g 5-methyl-2-nitrobenzyl alcohol and the chilled solution stirred for 2 hours. The mixture was poured into ice-water and extracted with diethyl ether. The extract was washed with H₂O, brine and the solvent removed under vacuum to give a mixture of product (50%) and starting material (50%).

### Reference Example 178

### Methyl 3-Hydroxy-2-[(4-methoxybenzenesulfonyl)-(5-methyl-2-nitrobenzyl)amino]propionate

A solution of 23.14 g (0.08 mol) of methyl 3-hydroxy-2-(4-methoxybenzenesulfonylamino)propionate in 120 ml of dry N,N-dimethylformamide was added dropwise to a stirred suspension of 3.2 g (0.08 mol) of sodium hydride (57% in oil) in 120 ml of N,N-dimethylformide. When gas evolution ceased, the mixture was chilled in an ice bath and a solution of 16.4 g (0.084 mol) of 5-methyl-2-nitrobenzyl chloride in 100 ml of N,N-dimethylformamide was added. To the mixture was added 12.6 g (0.084 mol) of anhydrous sodium iodide and the mixture was chilled in an ice bath and stirred for 20 minutes. The mixture was allowed to warm to room temperature and was stirred overnight. The solvent was removed under vacuum and the residue diluted with 200 ml of H₂O and extracted with 500 ml of ethyl acetate. The aqueous layer was extracted with an additional 200 ml of ethyl acetate. The combined extract was washed with H₂O, brine and dried with Na₂SO₄. The solvent was removed to give 41.18 g of crude product. The product was chromatographed on silica gel with hexane-ethyl acetate (1:1) as solvent to give 8.14 g (R_{F} 0.38) of product as a yellow semi-solid. From a small scale run (1 mmol) the product was chromatographed twice on thick silica gel plates with hexane-ethyl acetate (1:1) to give 0.12 g of a yellow semi-solid. Anal. for C₁₉H₂₂N₂SO₈:

| | | | |
|---|---|---|---|
| Calc'd | C,52.0; | H,5.1; | N,6.4; |
| Found | C,51.7; | H,5.1; | N,6.0. |

### Reference Example 179

### Methyl 3-Hydroxy-2-[(4-methoxybenzenesulfonyl)-(2-amino-5-methylbenzyl)amino]propionate

To a solution of 3.4 g of methyl 3-hydroxy-2-[(4-methoxybenzenesulfonyl)-(5-methyl-2-nitrobenzyl)-amino]propionate in 200 ml of ethanol-ethyl acetate (1:1) was added 0.34 g of 10% palladium on carbon (wet - 50% H₂O). The mixture was then shaken in a Parr hydrogenator under 35 psi of hydrogen for 2.5 hours. The mixture was filtered through diatomaceous earth and the filtrate concentrated under vacuum to give 2.86 g of a brown oil. Anal. for C₁₉H₂₄N₂O₆S:

| | | | |
|---|---|---|---|
| Calc'd | C,55.9; | H,5.9; | N,6.9; |
| Found | C,55.6; | H,5.9; | N,6.4; |

Mass spectrum (ES) 409 (M+H).

### Reference Example 180

### Methyl 3-[(2-Tetrahydropyranyl)oxy]-2-[(-4-methoxybenzenesulfonyl)-(5-methyl-2-nitrobenzyl)amino]propionate

To a mixture of 1.75 g (4.68 mmol) of (D,L)N-(4-methoxybenzenesulfonyl)-O-(2-tetrahydropyranyl) serine, methyl ester, 0.790 g (4.68 mmol) of 5-methyl-2-nitrobenzyl alcohol and 1.23 g (4.68 mmol) of triphenylphosphine in 4.5 ml of anhydrous tetrahydrofuran was added dropwise (over 15 minutes) a solution of 0.815 g (4.68 mmol) of diethyl azodicarboxylate (DEAD) in 1 ml of tetrahydrofuran. The mixture was stirred at room temperature overnight and the solvent removed under vacuum. The residue was triturated with diethyl ether and the solid filtered off. The filtrate was concentrated to dryness under vacuum to give 4.67 g of solid. The solid was chromatographed on silica gel with hexane-ethyl acetate (1:1) to give 0.56 g of product (R_{F} 0.48).

### Reference Example 181

### Methyl 1-Methoxyacetyl-4-(4-methoxybenzenesulfonyl)-7-methyl-2,3,4,5-tetrahydro-1H-[1,4]benzodiazepine-3-carboxylate

To a cooled (0°C) mixture of 1.598 g (3.91 mmol) of methyl 3-hydroxy-2-[(4-methoxybenzenesulfonyl)-(2-amino-5-methylbenzyl)amino]propionate and 1.97 g (19.5) mmol) of triethylamine in 15 ml of dichloromethane was added 0.787 ml (8.60 mmol) of methoxyacetylchloride. The mixture was stirred at room temperature overnight. The mixture was then diluted with CH₂Cl₂ and washed with H₂O, 2 N citric acid, H₂O, brine and dried with Na₂SO₄. The solution was filtered through a thin pad of hydrous magnesium silicate and the filtrate concentrated to give 1.94 g of crude methyl 2-{[2-(methoxyacetylamino)-5-methylbenzyl]-(4-methoxy-benzenesulfonyl)-amino}acrylate as a brown oil. Mass spectrum (ES) 463.4 (M+H).

To a solution of 1.62 g (3.5 mmol) of the preceding compound in 15 ml of anhydrous methanol was added 0.382 g (4.50 mmol) of anhydrous NaHCO₃ and the mixture was stirred overnight at room temperature. The solvent was removed under vacuum and the residue partitioned between 100 ml of ethyl acetate and 20 ml of water. The ethyl acetate layer was separated and washed with H₂O, brine and dried with Na₂SO₄. The solution was filtered through a thin pad of hydrous magnesium silicate and the filtrate concentrated under vacuum to give a yellow oil. Trituration with ethyl acetate-hexane gave 1.26 g (78%) of tan crystals, m. p. 122°-124°C. Anal. for C₂₂H₂₆N₂O₇S:

| | | | |
|---|---|---|---|
| Calc'd | C,57.1; | H,5.7; | N,6.1; |
| Found | C,57.4; | H,5.7; | N,6.0. |

### Reference Example 182

### Methyl 1-Benzoyl-4-(4-methoxybenzenesulfonyl)-7-methyl-2,3,4,5-tetrahydro-1H-[1,4]benzodiazeprine-3-carboxylate

To a cooled (0°C) mixture of 1.465 g (3.586 mmol) of methyl 3-hydroxy-2-[4-methoxybenzenesulfonyl)-(2-amino-5-methylbenzyl)amino]propionate and 2.49 ml (17.93 mmol) of triethylamine in 20 ml of CH₂Cl₂ was added 0.915 ml (7.89 mmol) of benzoyl chloride. The mixture was stored at room temperature overnight, diluted with CH₂Cl₂ and washed with H₂O, 2 N citric acid, H₂O, brine and dried with Na₂SO₄. The solution was filtered through a thin pad of hydrous magnesium silicate and the filtrate concentrated under vacuum to give 1.8 g of crude methyl 2-[(2-benzoylamino-5-methylbenzyl)-(4-methoxybenzenesulfonyl)amino]acrylate as a brown oil. Anal. for C₂₆H₂₆N₂O₆S:

| | | | |
|---|---|---|---|
| Calc'd | C,63.1; | H,5.3; | N,5.7; |
| Found | C,63.9; | H,5.2; | N,5.2. |

As described for Reference Example 181, 1.825 g (3.68 mmol) of the preceding compound was stirred with 0.402 g (4.78 mmol) of NaHCO₃ in 1.5 ml of methanol to give an oil. Trituration with hexane (plus several drops of ethyl acetate) gave crystals, m. p. 58°-62°C.

### Reference Example 183

### Methyl 1-(trans-Crotonyl)-4-(4-methoxybenzenesulfonyl)-7-methyl-2,3,4,5-tetrahydro-1H-[1,4]benzadiazepine-3-carboxylate

As described for Reference Examples 181 and 182, a mixture of 1.41 g (3.455 mmol) of methyl 3-hydroxy-2-[-(4-methoxybenzenesulfonyl)-(2-amino-5-methylbenzyl)amino]propionate, 1.75 g (17.3 mmol) of triethylamine and 0.809 ml of trans-crotonyl chloride in 15 ml of CH₂Cl₂ was stirred overnight to give 1.52 g of methyl 2-{[2-(trans-crotonylamino)-5-methylbenzyl]-(4-methoxybenzenesulfonyl) amino}acrylate as a brown oil; Mass spectrum (ES) 459.4 (M+H).

As described in Reference Example 181, 1.52 g (3.31 mmol) of the preceding product was stirred with 0.362 g (4.3 mmol) of NaHCO₃ in 15 ml of methanol at room temperature overnight. To the mixture was added 0.056 g of NaHCO₃ and the mixture was heated at 80°C for 3 hours and worked up as for Reference Example 181 to give a 1.05 g of a yellow glass, m. p. 75°-84°C. Mass spectrum (ES) 459.4 (M+H).

### Reference Example 184

### 1-(trans-Crotonyl)-4-(4-methoxybenzenesulfonyl)-7-methyl-2,3,4,5-tetrahydro-1H-[1,4]benzodiazepene-3-carboxylic acid

A mixture of 1.26 g (2.72 mmol) of methyl 1-(trans-crotonyl)-4-(4-methoxybenzenesulfonyl)-7-methyl-2,3,4,5-tetrahydro-1H-[ 1,4]benzodiazepine-3-carboxylate and 3.53 ml (3.53 mmol) of 1 N NaOH in 10 ml of tetrahydrofuran was stirred at room temperature for 3 hours. The solvent was removed under vacuum and the residue dissolved in H₂O and the solution extracted with ethyl acetate. The aqueous layer was acidified with 1N HCl (pH 2) and extracted with CH₂Cl₂. The CH₂Cl₂ extract was dried with Na₂SO₄ and the solvent removed to give 1.06 g (after drying under vacuum) of solid, m. p. 101°-105° C.

### Reference Example 185

### 1-(Benzoyl)-4-(4-methoxybenzenesulfonyl)-7-methyl-2,3-4,5-tetrahydro-1H[1,4]benzodiazepine-3-carboxylic acid

A mixture of 1.18g (2.38 mml) of methyl 1-(benzoyl)-4-(4-methoxybenzenesulfonyl)-7-methyl-2,3,4,5-tetrahydro-1H-[1,4]benzodiazepine-3-carboxylate and 3.09 ml (3.09 mmol) of 1N NaOH in 10 ml of tetrahydrofuran was stored at room temperature overnight and the solvent removed under vacuum. The residue was diluted with H₂O, extracted with ethyl acetate and the aqueous layer acidified with 2N citric acid. The mixture was extracted with CH₂Cl₂ and the CH₂Cl₂ extracts were washed with H₂O, brine and dried with Na₂SO₄. The solvent was removed to give 0.82g of a light yellow glass, m.p. 95°-100°C; Mass spectrum (ES) 481.4 (M+H).

### Reference Example 186

### Methyl 4-(4-Methoxybenzenesulfonyl)-1-(2-methoxyethyl)-2,3,4,5-tetrahydro-1H-[1,4]benzodiazepine-3-carboxylate

A mixture of 1.6 g (3.57 mmol) of methyl 1-(methoxyacetyl)-4-(4-methoxybenzenesulfonyl)-2,3,4,5-tetrahydro-1H-[1,4)benzodiazepine-3-carboxylate and 32 ml of borane in tetrahydrofuran (1.0 M) was refluxed under nitrogen overnight. Methanol was added and the solvent removed. To the residue was added 25 ml of CH₂Cl₂ and 25 ml of 2 N HCl and the mixture stirred at room temperature for 1 hour. The organic layer was separated and washed with H₂O and concentrated to dryness. The residue was triturated with ethyl acetate-hexane, cooled and filtered to give 1.2 g of white crystals, m.p. 86°-90°C: Mass spectrum (ES) 435.4 (M+H). Anal. for C₂₁H₂₆N₂O₆S:

| | | | |
|---|---|---|---|
| Calc'd | C,58.1; | H,6.0; | N,6.5; |
| Found | C,58.5; | H,6.0; | N,6.5. |

### Reference Example 187

### 4-(4-Methoxybenzenesulfonyl)-1-(2-methoxyethyl)-2,3,4,5-tetrahydro-1H[1,4]benzodiazepine-3-carboxylic acid

A mixture of 1.0 g (2.3 mmol) of methyl 4-(4-methoxybenzenesulfonyl)-1-(2-methoxyethyl)-2,3,4,5-tetrahydro-1H-[1,4]-benzodiazepine-3-carboxylate and 3.0 ml of 1 N NaOH in 10 ml of tetrahydrofuran was stirred at room temperature for 2 hours and the solvent removed. To the residue was added water and the mixture acidified with 1 N HCl. The mixture was extracted with ethyl acetate and the extract was washed with brine and dried with Na₂SO₄. The solvent was removed and the residue triturated with ethyl acetate-hexane, cooled and filtered to give 0.65 g of white crystals, m.p. 164°-165°C; Mass spectrum (ES) 421.4 (M+H). Anal. for C₂₀H₂₄N₂O₆S:

| | | | |
|---|---|---|---|
| Calc'd | C,57.1; | H,5.8; | N,6.7; |
| Found | C,57.3; | H,5.7; | N,6.4. |

### Reference Example 188

### Methyl 1-(Benzyl)-4-(4-methoxybenzenesulfonyl)-2,3,4,5-tetrahydro-1H-[1,4]benzodiazepine-3-carboxylate

A mixture of 0.20 g (0.416 mmol) of methyl 1-(benzoyl)-4-(4-methoxybenzenesulfonyl)-2,3,4,5-tetrahydro-1H-[1,4]benzodiazepine-3-carboxylate and 4 ml of borane in tetrahydrofuran (1.0 M) was refluxed overnight and the solvent removed. To the residue was added 5 ml of CH₂Cl₂ and 5 ml of 2N HCl and the mixture stirred for 1 hour. The organic layer was separated and concentrated to dryness. The residue was chromatographed on thick layer silica gel plates with hexane-ethyl acetate (2:1) as solvent to give 0.140 g of a colorless oil; Mass spectrum (ES) 467.5 (M+H).

### Reference Example 189

### 4-(4-Methoxybenzenesulfonyl)-1-[4-(trifluoromethoxy)benzoyl]-8-chloro-2,3,4,5-tetrahydro-1H[1,4]benzodiazepine-3-carboxylic acid

As described for Reference Example 18, 1.46 g (3.40 mmol) of methyl 2-[(2-amino-4-chlorobenzyl)-(4-methoxybenzenesulfonyl)amino]-3-hydroxypropionate was reacted with 4-(trifluoromethoxy)benzoyl chloride to give 2.59 g of methyl 2-{2-(4-(trifluoromethoxy) benzoyl]amino-4-chlorobenzyl]amino}acrylate as a yellow oil; Mass spectrum (ES) 599.3 (M+H). The preceding compound was stirred with 0.445 g (5.29 mmol) of anhydrous NaHCO₃ in 15 ml of methanol at room temperature for 16 hours and then was heated at 80°C for 2 hours. The solvent was removed and the residue extracted with ethyl acetate. The extract was washed with H₂O, brine, and dried (Na₂SO₄). The solvent was removed and the residue crystallized from ethyl acetate-hexane to give methyl 4-(4-methoxybenzenesulfonyl)-1-[4-(trifluoromethoxy)benzoyl }-8-chloro-2,3,4,5-tetrahydro-1 H-[ 1,4]benzodiazepine-3-carboxylate as yellow crystals, m.p. 149°-151°C. Anal. for C₂₆H₂₂ClF₃O₇S:

| | | | | | |
|---|---|---|---|---|---|
| Calc'd | C,52.1; | H,3.7; | N,4.7; | Cl,6.0; | F,9.5; |
| Found | C,51.8; | H,3.6; | N,4.7; | Cl,5.9; | F,9.4. |

1.58g (2.64 mmol) of the preceding compound was stirred with 3.43 ml of 1N NaOH in 10 ml of tetrahydrofuran at room temperature for 2 hours and worked up as for Reference Example 104 to give 1.52 g of product. Crystallization from ethyl acetate-hexane gave 1.2 g of white crystals, m.p. 184°-186°C.

### Reference Example 190

### Methyl 4-(4-Methoxybenzenesulfonyl)-1-(4-morpholinoacetyl)-2,3,4,5-tetrahydro-1H[1,4] benzodiazepine-3-carboxylate

A mixture of 0.10 g (0.22 mmol) of methyl 1-(chloroacetyl)-4-(4-methoxybenzenesulfonyl)-2,3,4,5-tetrahydro-1H-[1,4]benzodiazepine-3-carboxylate, 21.2 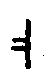 1 of morpholine and 125.4 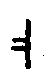 1 of N,N-diisopropylethylamine in 3 ml of CH₂Cl₂ was stirred overnight at room temperature. An additional 2.2 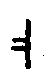 1 of morpholine was added and the solution stirred for 2 days at room temperature. The mixture was diluted with CH₂Cl₂ and washed with H₂O, brine and dried with Na₂SO₄. The solvent was removed to give the product as a solid, Mass spectrum (ES) 504.3 (M+H). Anal. for C₂₄H₂₉N₃O₇S:

| | | | |
|---|---|---|---|
| Calc'd | C,57.2; | H,5.8; | N,8.3; |
| Found | C,56.5; | H,5.6; | N,8.1. |

### Reference Example 191

### Methyl 4-(4-Methoxybenzenesulfonyl)-1-[2-(1-pyrazolyl)phenylcarbonyl]-7-methyl-2,3,4,5-tetrahydro-1H-[1,4]benzodiazepine-3-carboxylate

As described for the general reaction of ethyl 2-fluorobenzoate with amines set forth in Tetrahedron, 53, 7557-7576 (1997), ethyl 2-fluorobenzoate was reacted with pyrazole by refluxing N, N-dimethylformamide to give ethyl 2-(1-pyrazolyl)-benzoate, as a thick yellow oil. Anal. Calc'd: for C₁₂H₁₂N₂O₂: C, 66.7; H, 5.6; N 13.0: Found: C, 66.5: H, 5.4: N, 12.9; Mass spectrum (ES) 217.2 (M+H). A sample (7.02g) of this compound and 8.42 ml of 5N NaOH in 40 ml of ethanol-tetrahydrofuran (2:1) was refluxed for 2 hrs and the solvent removed.The residue was made acidic (pH6) with 2N citric acid and the precipated solid was filtered to obtain 3.7g of product. The pH of the filtrate was adjusted to 4.5 and extracted with ethyl acetate. The extract was concentrated to dryness to give 1.5g of product. The two crops were combined to give 5.2g of 2-(1-pyrazolyl)benzoic acid, mp 140-142°C. To the preceding compound (2.07 g) in 5 ml CH₂Cl₂ (chilled in an ice bath )was added 11.1 ml of a 2 Molar solution of oxalyl chloride in CH₂Cl₂ and 0.085 ml of N,N-dimethylformamide. The mixture was allowed to warm to room temperature and stirred for 4 hours. The solvent was removed and 25 ml of toluene added (twice) and removed under vacuum to give 2-(1-pyrazolyl)benzoyl chloride as a yellow solid.

A 2.3 g sample of the preceding compound was reacted with 1.5g of the compound of Reference Example 179 in 15 ml of CH₂Cl₂ and 5.12 ml of triethylamine in the manner described for Reference Example 181 to give methyl 2-[(4-methoxybenzenesulfonyl)-{2-[2-(1-pyrazolyl)phenylcarbonyl]amino-5-methylbenzyl}amino]acrylate. This compound was cyclized with NaHCO₃ in methanol in the manner described in Reference Example 181 to give methyl 4-(4-methoxybenzenesulfonyl)-1-[2-(1-pyrazolyl)phenylcarbonyl]-7-methyl-2,3,4,5-tetrahydro-1H-[1,4]benzodiazepine-3-carboxylate, m.p. 240-242° C.

A 1.16 g sample of the preceding compound was hydrolysed with 2.69 ml of 1N NaOH in 10 ml of tetrahydrofuran in the manner described for Reference Example 104 to give 0.71 g of 4-(4-methoxybenzenesulfonyl)-1-[2-(1-pyrazolyl)-phenyl-carbonyl)-7-methyl-2,3,4,5-tetrahydro-1 H[1,4]benzodiazepine-3-carboxylic acid, mp 149-151 °C.

### Reference Example 192

### Methyl 4-(4-Methoxybenzenesulfonyl)-1-[2-(4-marpholino)phenylcarbonyl }-8-chloro-2,3,4,5-tetrahydro-1H-[1,4]benzodiazepine-3-carboxylate

Ethyl 2-morpholinobenzoate prepared in the manner described in Tetrahedron, 53:7557, (1997) was refluxed with 10 N NaOH in tetrahydrofuran-ethanol (8:2) for 1.5 hrs to give 2-morpholinobenzoic acid, mp 15b-157°C. A 1.8 g sample of this compound in 5 ml of CH₂Cl₂ (chilled) was added a solution of 7.9 nil of oxalyl chloride in CH₂Cl₂ (2M) followed by the addition of 0.058 ml of N,N-dimethylformamide. The solution was stirred at room temperature for 6 hrs and the solvent removed. Toluene was added (2 times) and removed to give 2-(4-morpholino)-benzoyl chloride as a yellow solid.

In the manner described in Reference Examples 181 and 189, the preceding 2-(4-morpholino)benzoyl chloride was reacted with methyl 2-[(2-amino-4-chlorobenzyl)-(4-methoxybenzenesulfonyl)amino]-3-hydroxypropionate and the product was stirred with NaHCO₃ in methanol to give methyl 4-(4-methoxybenzenesulfonyl)-1-[2-(4-morpholino)phenylcarbonyl]-8-chloro-2,3,4,5-tetrahydro-1 H-[ 1,4]benzodiazepine-3-carboxylate, as a white solid having a mp 100-105°C.

To 0.90g of this compound in 10 ml of tetrahydrofuran was added 1.95 ml of 1 N NaOH and the solution was stirred at room temperature overnight. Acidification with 2N citric acid gave 0.82 g of solid, mp 136-143°C. [ compound, 4-(4-methoxybenzenesulfonyl)-1-[2-(4-morpholino)phenylcarbonyl]-8-chloro-2,3,4,5-tetrahydro-1H-[1,4]benzodiazepine-3-carboxylic acid ].

### Reference Example 193

### Methyl 1-(4-Ethoxybenzoyl)-4-(4-methoxybenzenesulfonyl)-2,3,4,5-tetrahydro-1H-[1,4]benzodiazepine-3-carboxylate

A mixture of 0.270 g of methyl 4-(4-methoxybenzenesulfonyl)-2,3,4,5-tetrahydro-1H-[1,4] benzodiazepine-3-carboxylate of Reference Example 12, 0.291 g of 4-ethoxybenzoyl chloride and 500 µl of triethylamine in 5 ml of CH₂Cl₂ was stirred at room temperature overnight. The mixture was diluted with CH₂Cl₂ and H₂O and the CH₂Cl₂ layer was separated and concentrated to dryness. The residue was triturated with ethyl acetate to give 0.276g of methyl 1-(4-ethoxybenzoyl)-4-(4-methoxybenzenesulfonyl)-2,3,4,5tetrahydro-1H-[1,4]benzodiazepine-3-carboxylate as white crystals, mp 187-190°C.

A 0.47 g sample of this compound was hydrolyzed with 1.2 ml of 1N NaOH in 4 ml of tetrahydrofuran. Dilution with H₂O and acidification with 1N HCl gave 0.40 g of the acid as a white solid, mp 144-152°C.

### Reference Example 194

### Methyl 4-(4-Methoxybenzenesulfonyl)-1-[2-chloro-4-(3-methyl-1-pyrazolyl)-phenylcarbonyl}-2,3,4,5-tetrahydro-1H-[1,4]benzodiazepine-3-carboxylate

As described in Example 65, methyl 4-(4-methoxybenzenesulfonyl)-2,3,4,5-tetrahydro-1H-[1,4]benzodiazepine-3-carboxylate was reacted with 4-(3-methyl-1-pyrazolyl)-2-chlorobenzoyl chloride to give methyl 4-(4-methoxybenzenesulfonyl)-1-[2-chloro-4-(3-methyl-1-pyrazolyl)phenylcarbonyl]-2,3,4,5-tetrahydro-1H-[1,4]-benzodiazepine-3-carboxylate as a white solid. Anal. for C₂₉H₂₇ClN₄O₆S:

| | | | |
|---|---|---|---|
| Calc'd | C, 58.3; | H, 4.6; | N, 9.4. |
| Found | C,58.2; | H, 4.9; | N, 8.9. |

This compound was hydrolysed with 1N NaOH in tetrahydrofuran as described in Reference Example 185 to give the benzodiazepine-3-carboxylic acid derivative as a white solid.

### Reference Example 195

### 1-Benzyl-4-(4-methoxybenzenesulfonyl)-2,3,4,5-tetrahydro-1H-[1,4]benzodiazepine-3-carboxylic acid

A mixture of 1.7 g of the compound of Reference Example 45 and 25 ml of borane in tetrahydrofuran (1.0 Molar) was refluxed under nitrogen overnight. To the solution was added 5 ml of CH₃OH, CH₂Cl₂ (40 ml) and 30 ml of 2N HCl and the mixture stirred at room temperature for 1.5 hr. The organic layer was separated, washed with brine, dried with Na₂SO₄ and the solvent removed. The residue was crystallized from ethanol-hexane to give 1.15g of methyl 1-benzyl-4-(4-methoxybenzenesulfonyl)-2,3,4,5-tetrahydro-1H-(1,4)benzodiazepine-3-carboxylate as white crystals, mp 120-122°C. A sample (1.0 g) of this compound was hydrolysed with 2.8 ml of 1 N NaOH in 7 ml of tetrahydrofuran as described in Reference Example 104 to give 0.64 g of the 2,3,4,5-tetrahydro-1H-[1,4]benzodiazepine-3-carboxylic acid derivative as white crystals, mp 183-185°C.

### Reference Example 196

### Methyl 1-(2,4-Dimethoxybenzoyl)-4-(4-methoxybenzenesulfonyl)-2,3,4,5-tetrahydro-1H-[ 1,4]benzodiazepine-3-carboxylate

To a cooled (0°C) solution of 1.0 g (2.66 mmol) of 4-(4-methoxybenzenesulfonyl)-2,3,4,5-tetrahydro-1H-[1,4]benzodiazepine-3-carboxylate from Reference Example 12 and 1.85 ml (13.3 mmol) of triethylamine in 8 ml of CH₂Cl₂ was added 1.17 g (6.65 mmol) of 2,4-dimethoxybenzoyl chloride. The mixture was stirred at room temperature overnight, diluted with CH₂Cl₂ and washed with 2 N citric acid. The organic layer was washed with H₂O, 1 N Na₂CO₃, brine and dried over Na₂SO₄. The solvent was removed and the residue was chromatographed on thick layer silica gel plates with ethyl acetate-hexane (1:1) as an eluent to give 1.0 g of methyl 1-(2,4-dimethoxybenzoyl)-4-(4-methoxybenzenesulfonyl)-2,3,4,5-tetrahydro-1 H-[1,4]-benzodiazepine-3-carboxylate as a white foam. Anal. for C₂₇H₂₈H₂O₈S:

| | | | |
|---|---|---|---|
| Calc'd | C,60.0; | H,5.2; | N,5.2; |
| Found | C,60.0; | H,5.2; | N,5.1; |

Mass Spectrum (ES): 541.0 (M+H).

A 0.80 g (1.48 mmol) sample of the preceding compound and 1.92 ml (1.92 mmol) of 1N NaOH in 5 ml of tetrahydrofuran was stirred at room temperature for 1.5 hours. The solvent was removed and the residue diluted with water. The solution was acidified with 1N HCl, chilled and filtered to give 0.70 g of 1-(2,4-dimethoxybenzoyl)-4-(4-methoxybenzenesulfonyl)-2,3,4,5-tetrahydro-1H-[1,4]benzodiazepine-3-carboxylic acid as a white solid. Anal. for C₂₆H₂₆N₂O₈S:

| | | | |
|---|---|---|---|
| Calc'd | C,59.3; | H,5.0; | N,5.3; |
| Found | C,56.1; | H,4.8; | N,5.0; |

Mass Spectrum (ES): 527.0 (M+H).

### Reference Example 197

### Methyl 4-(4-Methoxybenzenesulfonyl)-1-[2-(4-methylpiperazin-1-yl)acetyl]-2,3,4,5-tetrahydro-1H-[1,4]benzodiazepine-3-carboxylate

To a mixture of 2.5 g (6.64 mmol) of methyl 4-(4-methoxybenzenesulfonyl)-2,3,4,5-tetrahydro-1H-[1,4]benzodiazepine-3-carboxylate (Reference Example 12) and 4.63 ml (33.2 mmol) of triethylamine in 40 ml of CH₂Cl₂ cooled to 0°C was added to 1.65 g (14.63 mmol) of chloroacetyl chlonde. The solution was stirred at room temperature for 2 days, chilled to 0°C and 926 µl of triethylamine and 750 mg of chloroacetyl chloride were added thereto. The mixture was stirred at room temperature overnight, diluted with CH₂Cl₂ and H₂O. The insoluble solid was filtered off. The organic layer of the filtrate was separated, washed with brine, dried with Na₂SO₄ and filtered through diatomaceous earth. The solvent was removed and the residue triturated with ethyl acetate and a trace of ethanol. Chilling and filtering gave 0.75 g of methyl 1-(chloroacetyl)-4-(4-methoxybenzenesulfonyl)-2,3,4,5-tetrahydro-1 H-[ 1,4]benzo-diazepine-3-carboxylate (Reference Example 91). Anal. for C₂₀H₂₁ClN₂O₆S:

| | | | |
|---|---|---|---|
| Calc'd | C,53.0; | H,4.7; | N,6.2; |
| Found | C,51.6; | H,4.6; | N,5.7; |

Mass Spectrum (ES): 453.0 (M+H).

To a solution of 1.4 g (3.09 mmol) of the preceding compound in 12 ml of CH₂Cl₂ cooled to 0°C was added 1.2 ml (6.79 mmol) of N,N-diisopropylethylamine followed by the addition of 753.2 µl (6.79 mmol) of 1-methylpiperazine. The mixture was stirred at room temperature overnight, diluted with CH₂Cl₂, and washed with 2 N citric acid, H₂O, 1 M NaHCO₃, brine and dried (Na₂SO₄). The citric acid wash was made basic with saturated NaHCO₃ and then extracted with CH₂Cl₂. The extract was dried over Na₂SO₄ and the solvent removed under vacuum to give 1.10 g of methyl 4-(4-methoxybenzenesulfonyl)-1-[2-(4-methylpiperazin-1-yl)acetyl]-2,3,4,5-tetrahydro-1H-[1,4]benzodiazepine-3-carboxylate as a white glass.

A mixture of 1.0 g (1.94 mmol) of the preceding compound and 2.3 ml (2.3 mmol) of 1 N KOH in 5 ml of methanol was stirred at room temperature for 2 hours. The solvent was removed under vacuum. To the residue was added toluene (2 times) and the solvent removed under vacuum after each addition. The solid was dried at 65°C under vacuum for 6 hours to give 1.1 g of potassium 4-(4-methoxybenzenesulfonyl)-1-[2-(4-methylpiperazin-1-yl)acetyl]-2,3,4,5-tetrahydro-1H-[ 1,4]benzodiazepine-3-carboxylate as a white solid.

### Reference Example 198

### Methyl 1-Acetyl-4-(4-hydroxybenzenesulfonyl)-2,3,4,5-tetrahydro-1H-[1,4] benzodiazepine-3 -carboxylate

To a cooled (0°) solution of 2.0g (4.78 mmol) of methyl I-acetyl-4-(4-methoxybenzenesulfonyl)-2,3,4,5-tetrahydro-1H-[1,4]benzodiazepine-3-carboxylate in 14 ml of CH₂Cl₂ was added dropwise 143.3ml (14.3mmol) of a 1.0 molar solution of BBr₃ in CH₂ Cl₂. The mixture was stirred at room temperature for 1.5 hours. Ice and H₂O were added to the reaction mixture and the insolubles filtered off. The filtrate was diluted with CH₂Cl₂ and H₂O and the CH₂Cl₂ layer separated, washed with brine and dried (Na₂ SO₄). The solvent was removed under vacuum to give 1.5 g of a white foam. The solid was chromatographed on silica gel with hexane-ethyl acetate (1:1) as solvent to give a foam which was dried under vacuum to give 0.52 g of product as a white foam; Anal. Calc'd for C₁₉H₂₀N₂O₆S: C, 56 4:H, 5.0; N, 6.9 Found: C 55.1; H, 4.7: N, 6.5.

### Reference Example 199

### Methyl 4-(4-Hydroxybenzenesulfonyl)-1-(2-thienylcarbonyl)-2,3,4,5-tetrahydro-1H-[1,4]benzodiazepine-3-carboxylate

To a solution of 4.0 g (8.22 mmol) of methyl 4-(4 methoxybenzenesulfonyl)-1-(2-thienylcarbonyl)-2,3,4,5-tetrahydro-1H-[1,4]benzodiazepine-3-carboxylate in 17 ml of CH₂Cl₂ chilled to 0°C, was added slowly 16.4 ml (16.44 mmol) of 1.0 molar solution of boron tribromide in CH₂Cl₂. The mixture was stirred at room temperature overnight and diluted with CH₂Cl₂. The mixture was filtered and the solid washed with CH₂Cl₂, and H₂O. The filtrate was diluted with H₂O and the organic layer separated. The solvent was removed under vacuum and the solid chromatographed on silica gel with hexane-ethyl acetate (1:1) as solvent to give 0.80 g of off white foam; Mass Spectrum (ES) 473.5 (M+H); Anal. Calc'd for C₂₂H₂₀ N₂O₆ S₂: C, 55.9; H, 4.3: N, 5.9. Found: C, 54.5; H, 4.4; N, 5.5.

### Reference Example 200

### Methyl 1-Benzoyl-4-(4-hydroxybenzenesulfonyl)-2,3,4,5-tetrahydro-1H-[1,4] benzodiazepine-3-carboxylate

To a solution of 9.8 g (20.39 mmol) of methyl 1-benzoyl-4-(4-methoxybenzenesulfonyl)-2,3,4,5-tetraydro-1H-[1,4]benzodiazepine in 50 ml of CH₂Cl₂ cooled to 0°, was added slowly 40.8 ml (40.8 mmol) of a 1.0 molar solution of BBr₃ in CH₂Cl₂ The mixture was stirred under nitrogen at room temperature overnight. Ice and H₂O were added and the mixture diluted with CH₂Cl_{2.} The organic layer was separated and the aqueous layer extracted with ethyl acetate. The combined organic extracts (CH₂Cl₂+ethyl acetate) were concentrated under vacuum and the residue dissolved in ethyl acetate. The solution was washed with H₂O, brine and dried (Na₂SO₄). The solution was filtered through a thin pad of hydrous magnesium silicate and the filtrate concentrated to dryness. The residue was chromatographed on silica gel with hexane-ethyl acetate as solvent to give 8 g of product as an off-white foam; Mass Spectrum (ES) 467 (M+H); Anal Calc'd for C₂₄H₂₂N₂O₆S: C, 61.8; H, 4.8; N, 6.0. Found: C, 61.3; H, 4.6; N, 5.8.

Utilizing the method described in Reference Examples 198-200, the following methyl-1-substituted-4-hydroxybenzenesulfonyl)-2,3,4,5-tetrahydro-1H-[1,4] benzodiazepine-3-carboxylates can be prepared.

### Reference Example 201

### Methyl 4-(4-Hydroxybenzenesulfonyl)-1-(4-methylphenylsulfonyl)-2,3,4,5-tetrahydro-1H-[1,4]benzodiazepine-3-carboxylate.

### Reference Example 202

### Methyl 1-Methanesulfonyl-4-(4-hydroxybenzenesulfonyl)-2,3,4,5-tetrahydro-1H-[1,4]benzodiazepinef-3-carboxylate

### Reference Example 203

### Methyl 4-(4-Hydroxybenzenesulfonyl)-1-(3-pyridinylcarbonyl) 2,3,4,5-tetrahydro-1H-[1,4]benzodiazepine-3-carboxylate

### Reference Example 204

### Methyl 4-(4-Hydroxybenzenesulfonyl)-1-(4-pyridinylcarbonyl-2,3,4,5-tetrahydro-1H-[1,4]benzodiazepine-3-carboxylate

### Reference Example 205

### Methyl 1-(4-Biphenylcarbonyl)-4-(4-hydroxybenzenesulfonyl)-2,3,4,5-tetrahydro-1H-[1,4]benzodiazepine-3-carboxylate

### Reference Example 206

### Methyl 4-(4-Hydroxybenzenesulfonyl)-1-(propane-1-sulfonyl)-2,3,4,5-tetrahydro-1H-[1,4]benzodiazepine-3-carboxylate

### Reference Example 207

### Methyl 1-([1,1'-Biphenyl]-2-carbonyl)-4-(4-hydroxybenzene-sulfonyl)-2,3,4,5-tetrahydro-1H-[1,4]benzodiazepine-3-carboxylate

### Reference Example 208

### Methyl 1-(3-Fluorobenzoyl)-4-(4-hydroxybenzenesulfonyl)-2,3,4,5-tetrahydro-1H-[1,4]benzodiazepine-3-carboxylate

### Reference Example 209

### Methyl 4-(4-Hydroxybenzenesulfonyl)-1-(2-methyl-5-fluorobenzoyl)-2,3,4,5-tetrahydro-1H-[1,4]benzodiazepine-3-carboxylate

### Reference Example 210

### Methyl 4-(4-Hydroxybenzenesulfonyl)-1-(2-methyl-3-fluorobenzoyl)-2,3,4,5-tetrahydro-1 H-[1,4]benzodiazepine-3-carboxylate

### Reference Example 211

### Methyl 4-(4-Hydroxybenzenesulfonyl)-1-(3-phenylpropionyl)-2,3,4,5-tetrahydro-1 H-[1,4]benzodiazepine-3-carboxylate

### Reference Example 212

### Methyl 4-(4-Hydroxybenzenesulfonyl)-1-(2-trifluoromethyl-benzoyl)-2,3,4,5-tetrahydro-1H-[1,4]benzodiazepine-3-carboxylate

### Reference Example 213

### Methyl 1-(2-Chloro-6-trifluoromethylbenzoyl)-4-(4-hydroxybenzenesulfonyl)-2,3,4,5-tetrahydro-1H-[1,4]benzodiazepine-3-carboxylate

### Reference Example 214

### Methyl 1-(4-Fluoro-2-trifluoromethylbenzoyl)-4-(4-hydroxybenzenesulfonyl)-2,3,4,5-tetrahydro-1H-[1,4]benzodiazepine-3-carboxylate

### Reference Example 215

### Methyl 1-(2-Fluoro-6-trifluoromethybenzoyl)-4-(4-hydroxybenzenesulfonyl)-2,3,4,5-tetrahydro-1H-[1,4]benzodiazepine-3-carboxylate

### Reference Example 216

### Methyl 4-(4-Hydroxybenzenesulfonyl)-1-(2 methylbenzoyl)-2,3,4,5-tetrahydro-1H-[1,4]benzodiazepine-3-carboxylate

### Reference Example 217

### Methyl 4-(4-Hydroxybenzenesulfonyl)-1-(2-methyl-6-chlorobenzoyl)-2,3,4,5-tetrahydro-1H-[1,4]benzodiazepine-3-carboxylate

### Reference Example 218

### Methyl 1-(2,4-Dimethylbenzoyl)-4-(4-hydroxybenzenesulfonyl)-2,3,4,5-tetrahydro-1H-[1,4]benzodiazepine-3-carboxylate

### Reference Example 219

### Methyl 1-(2,5-Dimethylbenzoyl)-4-(4-hydroxybenzenesulfonyl)-2,3,4,5-tetrahydro-1H-[1,4]benzodiazepine-3-carboxylate

### Reference Example 220

### Methyl 1-(2-Chloro-4-fluorobenzoyl)-4-(4-hydroxybenzenesulfonyl)-2,3,4,5-tetrahydro-1H-[1, 4]benzodiazepine-3-carboxylate

### Reference Example 221

### Methyl 1-(2-Chlorobenzoyl)-4-(4-hydroxybenzenesulfonyl)-2,3,4,5-tetrahydro-1H-[1,4]benzodiazepine-3-carboxylate

### Reference Example 222

### Methyl 1-(2-Fluorobenzoyl)-4-(4-hydroxybenzenesulfonyl)-2,3,4,5-tetrahydro-1H-[1,4]benzodiazepine-3-carboxylate

### Reference Example 223

### Methyl 1-(2-Chloro-6-fluorobenzoyl)-4-(4-hydroxybenzene-sulfonyl)-2,3,4,5-tetrahydro-1H-[1,4]benzodiazepine-3-carboxylate

### Reference Example 224

### Methyl 1-(2,3-Difluorobenzoyl)-4-(4-hydroxybenzenesulfonyl)-2,3,4,5-tetrahydro-1H-[1,4] benzodiazepine-3-carboxylate

### Reference Example 225

### Methyl 1-(2,4-Dichlorobenzoyl)-4-(4-hydroxybenzenesulfonyl)-2,3,4,5-tetrahydro-1H-[1,4]benzodiazepine-3-carboxylate

### Reference Example 226

### Methyl 1-(2,3-Dichlorobenzoyl)-4-(4-hydroxybenzenesulfonyl)-2,3,4,5-tetrahydro-1H-[1,4]benzodiazepine-3-carboxylate

### Reference Example 227

### Methyl-1-(2,5-Dichlorobenzoyl)-4-(4-hydroxybenzenesulfonyl)-2,3,4,5-tetrahydro-1H-[1,4]benzodiazepine-3-carboxylate

### Reference Example 228

### Methyl 4-(4-Hydroxybenzenesulfonyl)-1-(2-methylthiobenzoyl)-2,3,4,5-tetrahydro-1H-[1,4]benzodiazepine-3-carboxylate

### Reference Example 229

### Methyl 4-(4-Hydroxybenzenesulfonyl)-1-(3-methyl-2-thienylcarbonyl)-2,3,4,5-tetrahydro-1H-[1,4]benzodiazepine-3-carboxylate

### Reference Example 230

### Methyl 4-(4-Hydroxybenzenesulfonyl)-1-(4-methyl-2-thienylcarbonyl)-2,3,4,5-tetrahydro-1H-[1,4]benzodiazepine-3-carboxylate

### Reference Example 231

### Methyl 1-(3-Chloro-2-thienylcarbonyl)-4-( hydroxybenzenesulfonyl)-2,3,4,5-tetrahydro-1H-[1,4]benzodiazepine-3-carboxylate

### Reference Example 232

### Methyl 1-(2-Furanylcarbonyl)-4-(4-hydroxybenzenesulfonyl)-2,3,4,5-tetrahydro-1H-[1,4]benzodiazepine-3-carboxylate

### Reference Example 233

### Methyl 4-(4-Hydroxybenzenesulfonyl)-1-(3-methyl-2-furanylcarbonyl)-2,3,4,5-tetrahydro-1H-[-1,4]benzodiazepine-3-carboxylate

### Reference Example 234

### Methyl 4-(4-Hydroxybenzenesulfonyl)-1-(4-methyl-2-furanylcarbonyl)-2,3,4,5-tetrahydro-1H-[1,4]benzodiazepine-3-carboxylate

### Reference Example 235

### Methyl 1-(5-Chloro-2-furanylcarbonyl)-4-(4-hydroxybenzenesulfonyl)-2,3,4,5-tetrahydro-1H-[1,4]benzodiazepine-3-carboxylate

### Reference Example 236

### Methyl 1-(5-Chloro-2-thienylcarbonyl)-4-(4-hydroxybenzenesulfonyl)-2,3,4,5-tetrahydro-1H-[1,4]benzodeiazepine-3-carboxylate

### Reference Example 237

### Methyl 1-Propionyl-4-(4-hydroxybenzenesulfonyl)-2,3,4,5-tetrahydro-1H-[1,4]benzodeiazepine-3-carboxylate

### Reference Example 238

### Methyl 1-Hexanoyl-4-(4-hydroxybenzenesulfonyl)-2,3,4,5-tetrahydro-1H-[1,4]benzodiazepine-3-carboxylate

### Reference Example 239

### Methyl 4-(4-Hydroxybenzenesulfonyl)-1-(3-thienylcarbonyl)-2,3,4,5-tetrahydro-1H-[1,4]benzodiazepine-3-carboxylate

### Reference Example 240

### Methyl 1-(3-Furanylcarbonyl)-4-(4-hydroxybenzenesulfonyl)-2,3,4,5-tetrahydro-1H-[1,4]benzodiazepine-3-carboxylate

### Reference Example 241

### Methyl 1-(Acetylaminoacetyl)-4-(4-hydroxybenzenesulfonyl)-2,3,4,5-tetrahydro-1H-[1,4]benzodiazepine-3-carboxylate

### Reference Example 242

### Methyl 1-(N,N Dimethylaminoacetyl)-4-(4-hydroxybenzenesulfonyl)-2,3,4,5-tetrahydro-1H-[1,4]benzodiazepine-3-carboxylate

### Reference Example 243

### Methyl 1-(Cyclopropylcarbonyl)-4-(4-hydroxybenzenesulfonyl)-2,3,4,5-tetrahydro-1H-[1,4]benzodiazepine

### Reference Example 244

### Methyl 4-(4-Hydroxybenzenesulfonyl)-1-(trifluoroacetyl)-2,3,4,5-tetrahydro-1H-[1,4]benzodiazepine

### Reference Example 245

### Methyl 1-Acetyl-4-(4-but-2-ynyloxybenzenesulfonyl)-2,3,4,5-tetrahydro-1H-[1,4]benzenediazepine-3-carboxylate

To a stirred solution of 3.73 g (14.22 mmol) of triphenylphosphine in 80 ml of toluene-tetrahydrofuran (3:1) was added 1.06 ml (14.22 mmol) of 2-butyn-1-ol and 5.0 g (12.36 mmol) of methyl 1-acetyl-4-(4-hydroxybenzenesulfonyl)-2,3,4,5-tetrahydro-1H-[1,4]benzodiazepine-3-carboxylate. To this solution under nitrogen was added slowly dropwise 2.24 ml (14.22 mmol) of diethyl azodicarboxylate. The mixture was stirred at room temperature overnight and concentrated to dryness under vacuum. To the residue was added ethyl acetate and H₂O and the solid filtered off. The filtrate was concentrated under vacuum and extracted with CH₂Cl_{2.} The extract was washed with brine, dried (Na₂SO₄) and concentrated to dryness under vacuum. The residue was triturated with ethy acetate-hexane to give 6.5 of solid. This solid was chromatographed on silica gel with ethyl acetate-hexane(1:1) as solvent to give 3.9 g of white solid; Mass Spectrum (ES) 4.57.5 (M+H); Anal. Calc'd for C₂₃H₂₄N₂O₆S: C, 60.5; H, 5.3; N, 6.1. Found: C, 60.2; H, 5.2; N, 6.2.

### Reference Example 246

### Methyl 1-Benzoyl-4-(4-but-2-ynyloxybenzenesulfonyl)-2,3,4,5-tetrahydro-1H-[1,4]benzodiazepine-3-carboxytate

To a stirred solution of 1.26 g (4.82 mmol) of triphenylphosphine in 15 ml of toluene-tetrahydrofuran (4:1) under nitrogen was added 360 uL (4.82 mmol) of 2-butyn-1-ol and 1.5 g of (3.22 mmol)of methyl 1-benzoyl-4-(4-hydroxybenzenesulfonyl)-2,3,4,5-tetrahydro-1H-[1,4]benzodiazepine-3-carboxylate. To the stirred mixture was added slowly 759 uL of diethyl azodicarboxylate and the reddish clear solution stirred overnight at room temperature. The solvent was removed under vacuum and CH₂Cl₂ added. The CH₂Cl₂ was washed with H₂O and brine, dried (Na₂SO₄) and the solvent removed. The residue was chromatographed on silica gel with hexane-ethyl acetate (1:1) to give 1.65 g of white solid; Mass Spectrum (ES) 519 (M+H); Anal Calc'd for C₂₈H₂₆N₂O₆S: C, 64.9; H, 5:1; N, 5.4. Found: C,60.5; H, 5.2; N,6.9.

### Reference Example 247

### Methyl 4-(4-But-2-ynylbenzenesulfonyl)-1-(2-thienylcarbonyl)-2,3,4,5-tetrahydro-1H-[1,4]benzodiazepine-3-carboxylate

To a stirred mixture of 0.475g (1.81mmol) of triphenylphosphine, 134.8 uL (1.84 mmol) of 2-butyn-1-ol and 0.74 g of methyl 4-(4-hydroxysbenzenesulfonyl)-1-(2-thienylcarbonyl)-2,3,4,5-tetrahydro-1H[1,4]benzodiazepine-3-carnboxylate in 7 ml of toluene and 2 ml of tetrahydrofuran was added slowly 285 uL (1.81 mmol) of diethyl azodicarboxylate. The mixture was stirred overnight at room temperature and to the mixture was added 0.475 g of triphenylphosphine, 125 uL of 2-butyn-1-ol and 0.285 of diethyl azodicarboxylate. The mixture was stirred for 2.5 hrs. at room temperature and the solvent removed. To the residue was added CH₂Cl₂ and the mixture washed with H₂O and brine. The CH₂Cl₂ layer was dried (Na₂SO₄) and the solvent removed to give 2.0 g of a yellow oil. Chromatography on silica gel with hexane-ethyl acetate (1.1) gave 1.0 g of off-white foam; Mass Spectrum (ES) 525.6 (M+H); Anal. Calc'd for C₂₆H₂₄N₂O₆S₂: C,59.5; H, 4.6; N, 5.3. Found: C, 56.1, H, 4.9; N, 7.3)

Utilizing the method described in Reference Examples 245-247, the following methyl 1-substituted-4-(4-but-2-ynyloxybenzenesulfonyl)-2,3,4,5-tetrahydro-1H-[1,4]benzodiazepine-3-carboxylate and methyl 1-substituted-4-(4-[4-substituted-but-2-ynyloxy]benzenesulfonyl)-2,3,4,5-tetrahydro-1H-[1,4]benzodiazepine-3-carboxylates can be prepared.

### Reference Example 248

### Methyl 1-Butoxyacetyl-4-(4-but-2-ynyloxybenzenesulfonyl)-2,3,4,5-tetrahydro-1H-[1,4]benzodiazepine-3-carboxylate

### Reference Example 249

### Methyl 4-(4-But-2-ynyloxybenzenesulfonyl)-1-(4-methylphenylsulfonyl)-2,3,4,5-tetrahydro-1H-[1,4]benzodiazepine-3-carboxylate

### Reference Example 250

### Methyl 1-Methansulfonyl-4-(4-but-2-ynyloxy benzenesulfonyl)-2,3,4,5-tetrahydro-1H-[1,4]benzodiazepine-3-carboxylate

### Reference Example 251

### Methyl 1-Benzoyl-4-(4-[4-methoxybut-2-ynyloxy]benzenesulfonyl)-2,3,4,5-tetrahydro-1H-[1,4]benzodiazepine-3-carboxylate

### Reference Example 252

### Methyl 1-Acetyl-4-(4-[4-methoxybut-2-ynyloxy]benzenesulfonyl)-2,3,4,5-tetrahydro-1H-[1,4]benzodiazepine-3-carboxylate

### Reference Example 253

### Methyl 4-[4-But-2-ynyloxy] benzenesulfonyl)-1-(3-pyridinylcarbonyl)-2,3,4,5-tetrahydro-1H-[1,4]benzodiazepine-3-carboxylate

### Reference Example 254

### Methyl 4-(4-Pent-2-ynyloxybenzenesulfonyl)-1-(3-pyridinylcarbonyl)-2,3,4,5-tetrahydro-1H-[ 1,4]benzodiazepine-3-carboxylate

### Reference Example 255

### Methyl 4-(4-[4-Methoxybut-2-ynyloxy]benzenesulfonyl)-1-(2-thienylcarbonyl)-2,3,4,5-tetrahydro-1H-[1,4]benzodiazepine-3-carboxylate

### Reference Example 256

### Methyl 4-(4-But-2-ynyloxybenzenesulfonyl)-1-(4-pyridinylcarbonyl)-2,3,4,5-tetrahydro-1 H-[1,4]benzodiazepine-3-carboxylate

### Reference Example 257

### Methyl 1-(4-Biphenylcarbonyl)-4-(4-but-2-ynyloxybenzenesulfonyl)-2,3,4,5-tetrahydro-1H-[1,4]benzodiazepine-3-carboxylate

### Reference Example 258

### Methyl 4-(4-[4-Methoxybut-2-ynyloxy]benzenesulfonyl)-1-(propane-1-sulfonyl)-2,3,4,5-tetrahydro-1H-[1, 4]benzodiazepine-3-carboxylate

### Reference Example 259

### Methyl 1-([1,1'-Biphenyl]-2-carbonyl)-4-(4-[4-methoxybut-2-ynyloxy] benzenesulfonyl)-2,3,4,5-tetrahydro-1H-[1,4]benzodiazepine-3-carboxylate

### Reference Example 260

### Methyl 1-(3-Fluorobenzoyl)-4-(4-pent-2-ynyloxybenzenesulfonyl)-2,3,4,5-tetrahydro-1H-[1,4]benzodiazepine-3-carboxylate

### Reference Example 261

### Methyl 4-(4-[4-Ethoxybut-2-ynyloxy]benzenesulfonyl)-1-(2 methyl-3-fluorobenzoyl)-2,3,4,5-tetrahydro-1H-[1,4]benzodiazepine-3-carboxylate

### Reference Example 262

### Methyl 4-(4-[4-Dimethylaminobut-2-ynyloxy]benzenesulfonyl)-1-(2 methyl-3-fluorobenzoyl)-2,3,4,5-tetrahydro-1H-[1,4]benzodiazepine-3-carboxylate

### Reference Example 263

### Methyl 4-(4-But-2-ynyloxybenzenesuflony)-1-(2-triflouromethylbenzoyl)-2,3,4,5-tetrahydro-1H-[1,4]benzodiazepine-3-carboxylate

### Reference Example 264

### Methyl 1-(2-Chloro-6-trifluormethylbenzoyl)-4-(4-pent-2-ynyloxybenzenesulfonyl)-2,3,4,5-tetrahydro-1H-[1,4]benzodiazepine-3-carboxylate

### Reference Example 265

### Methyl 1-(4-Fluoro-2-trifluoromethylbenzoyl)-4-(4-[4-methoxybut-2-ynyloxy]-benzenesulfonyl)-2,3,4,5-tetrahydro-1H-[1,4]benzodiazepine-3-carboxylate

### Reference Example 266

### Methyl 1-(2-Fluoro-6-trifluormethylbenzoyl)-4-(4-pent-2 ynyloxybenzenesulfonyl)-2,3,4,5-tetrahydro-1H-[1,4]benzodiazepine-3-carboxylate

### Reference Example 267

### Methyl 4-(4-But-2-ynyloxybenzenesulfonyl)-1-(2 methyl-6-chlorobenzoyl)-2,3,4,5-tetrahydro-1 H-[1,4]benzodiazepine-3-carboxylate

### Reference Example 268

### Methyl 1-(2,4-Dimethylbenzoyl)-4-(4-[4-methoxybut-2-ynyloxy]benzenesulfonyl) -2,3,4,5-tetrahydro-1H-[1,4]benzodiazepine-3-carboxylate

### Reference Example 269

### Methyl 1-(2,5-Dimethylbenzoyl)-4-(4-[4-methoxybut-2-ynyloxy]benzenesulfonyl)-2,3,4,5-tetrahydro-1 H-[1,4] benzodiazepine-3-carboxylate

### Reference Example 270

### Methyl 1-(2-Chloro-4-flurobenzoyl)-4-(4-[4-methoxybut-2-ynyloxy]benzenesulfonyl)-2,3,4,5-tetrahydro-1H-[1,4]benzodiazepine-3-carboxylate

### Reference Example 271

### Methyl 1-(2-Chlorobenzoyl)-4-(4-[4-methoxybut-2-ynyloxy]benzenesulfonyl)-2,3,4,5-tetrahydro-1H-[1,4]benzodiazepine-3-carboxylate

### Reference Example 272

### Methyl 1-(2-Chlorobenzoyl)-4-(4-pent-2-ynyloxybenzenesulfonyl)-2,3,4,5-tetrahydro-1H-[1,4] benzodiazepine-3-carboxylate

### Reference Example 273

### Methyl 1-(2-Fluorobenzoyl)-4-(4-[4-methoxybut-2-ynyloxy]benzenesulfonyl)-2,3,4,5-tetrahydro-1H-[1,4]benzodiazepine-3-carboxylate

### Reference Example 274

### Methyl 1-(2-Chloro-6-fluorobenzoyl)-4-(4-[4-methoxybut-2-ynyloxy] benzenesulfonyl)-2,3,4,5-tetrahydro-1H-[1,4] benzodiazepine-3-carboxylate

### Reference Example 275

### Methyl 1-(2,3-Difluorobenzoyl)-4-(4-[4-methoxybut-2-ynyloxy]benzenesulfonyl)-2,3,4,5-tetrahydro-1H-[1,4]benzodiazepine-3-carboxylate

### Reference Example 276

### Methyl 1-(2,4-Dichlorobenzoyl)-4-(4-[4-methoxybut-2-ynyloxy]benzenesulfonyl)-2,3,4,5-tetrahydro-1H-[1,4]benzodiazepine-3-carboxylate

### Reference Example 277

### Methyl 1-(2,4-Dichlorobenzoyl)-4-(4-pent-2-ynyloxybenzenesulfonyl)-2,3,4,5-tetrahydro-1H-[1,4]benzodiazepine-3-carboxylate

### Reference Example 278

### Methyl 1-(2,3-Dichlorobenzoyl)-4-(4-[4-methoxybut-2-ynxloxy]benzenesulfonyl)-2,3,4,5-tetrahydro-1H-[1,4]benzodiazepine-3-carboxylate

### Reference Example 279

### Methyl 1-(2,5-Dichlorobenzoyl)-4-(4-[4-hydroxybut-2-ynyloxy]-benzenesulfonyl)-2,3,4,5-tetrahydro-1H-[1,4]benzodiazepine-3-carboxylate

### Reference Example 280

### Methyl 1-(Benzoyl)-4-(4-pent-2-ynyloxybenzenesulfonyl)-2,3,4,5-tetrahydro-1H-[1,4]benzodiazepine-3-carboxylate

### Reference Example 281

### Methyl 4-(4-But-2-ynyloxybenzenesulfony)-1-(2-methylthiobenzoyl)-2,3,4,5-tetrahydro-1H-[1,4]benzodiazepine-3-carboxylate

### Reference Example 282

### Methyl 4-(4-But-2-ynyloxybenzenesulfony)-1-(3-methyl-2-thienylcarbonyl)-2,3,4,5-tetrahydro-1H-[ 1,4]benzodiazepine-3-carboxylate

### Reference Example 283

### Methyl 4-(4-[4-Hydroxybut-2-ynyloxy]benzenesulfonyl)-1-(4 methyl-2-thienylcarbonyl)-2,3,4,5-tetrahydro-1H-[1,4]benzodiazepine-3-carboxylate

### Reference Example 284

### Methyl 1-(3-Chloro-2-thienylcarbonyl)-4-(4-[4-dimethylaminobut 2-ynyloxy]-benzenesulfonyl)-2,3,4,5-tetrahydro-1H-[1,4]benzodiazepine-3-carboxylate

### Reference Example 285

### Methyl 1-(2-Furanylcarbonyl)-4-(4-[4-methylaminobut-2-ynyloxy]benzenesulfonyl)-2,3,4,5-tetrahydro-1H-[1,4]benzodiazepine-3-carboxylate

### Reference Example 286

### Methyl 4-(4-But-2-ynyloxybenzenesulfonyl)-1-(3-methyl-2-furanylcarbonyl)-2,3,4,5-tetrahydro-1 H-[1,4] benzodiazepine-3-carboxylate

### Reference Example 287

### Methyl 4-(4-[4-But-2-ynyloxybenzenesulfonyl)-1-(-4-methyl-2-furanylcarbonyl)-2,3,4,5-tetrahydro-1H-[1,4]benzodiazepine-3-carboxylate

### Reference Example 288

### Methyl 1-(5-Chloro-2-furanylcarbonyl)-4-(4-[4-ethoxybut-2-ynyloxy] benzenesulfonyl)-2,3,4,5-tetrahydro-1H-[1,4]benzodiazepine-3-carboxylate

### Reference Example 289

### Methyl 1-(5-Chloro-2-thienylcarbonyl)-4-(4-[4-hydroxybut-2-ynyloxy] benzenesulfonyl)-2,3,4,5-tetrahydro-1 H-[1,4]benzodiazepine-3-carboxylate

### Reference Example 290

### Methyl 1-Propionyl-4-(4-[4-hydroxybut-2-ynyloxy]benzenesulfonyl)-2,3,4,5-tetrahydro-1H-[1,4] benzodiazepine-3-carboxylate

### Reference Example 291

### Methyl 1-Hexanoyl-4-(4-[4-methoxybut-2-ynyloxy]benzenesulfonyl)-2,3,4,5-tetrahydro-1H-[1,4]benzodiazepine-3-carboxylate

### Reference Example 292

### Methyl 4-(4-But-2-ynyloxybenzenesulfonyl)-1-(propionyl)-2,3,4,5-tetrahydro-1H-[1,4]benzodiazepine-3-carboxylate

### Reference Example 293

### Methyl 4-(4-But-2-ynyloxybenzenesulfonyl)-1-(3-thienylcarbonyl)-2,3,4,5-tetrahydro-1H-[1,4]benzodiazepine-3-carboxylate

### Reference Example 294

### Methyl 4-(4-But-2-ynyloxybenzenesulfonyl)-1-(3-furanycarbonyl)-2,3,4,5-tetrahydro-1H-[1,4]benzodiazepine-3-carboxylate

### Reference Example 295

### Methyl 1-(Ethoxyacetyl)-4-(4-[4-methoxybut-2-ynyloxy]benzenesulfonyl)-2,3,4,5-tetrahydro-1H-[1,4]benzodiazepine-3-carboxylate

### Reference Example 296

### Methyl 1-(Acetylaminoacetyl)-4-(4-but-2-ynyloxybenzenesulfonyl)-2,3,4,5-tetrahydro-1H-[1,4]benzodiazepine-3-carboxylate

### Reference Example 297

### Methyl 1-(N,N-Dimethylaminoacety)-4-(4-[4-methoxybut-2-ynyloxy] benzenesulfonyl)-2,3,4,5-tetrahydro-1H-[1,4] benzodiazepine-3-carboxylate

### Reference Example 298

### Methyl 1-(Cyclopropylcarbonyl)-4-(4-[4-methxybut-2-ynyloxy]benzenesulfonyl )-2,3,4,5-tetrahydro-1H-[1,4]benzodiazepine-3-carboxylate

### Reference Example 299

### Methyl 1-(Cyclobutylcarbonyl)-4-(4-but-2-ynyloxybenzeneulfonyl)-2,3,4,5-tetrahydro-1H-[1,4]benzodiazepine-3-carboxylate

### Reference Example 300

### Methyl 4-(4-But-2-ynyloxybenzenesulfonyl)-1-(triflouroacety)-2,3,4,5-tetrahydro-1H-[1,4]benzodiazepine-3-carboxylate

### Reference Example 301

### Sodium 4-But-2-ynyloxybenzenesulfonate

To a solution of 52.35g (0.225 mol) of 4-hydroxybenzenesulfonate sodium salt in 1L of isopropanol and 225 mL of a 1.0N solution of sodium hydroxide was added 59.96g (0.45 mol) of 1-bromo-2-butyne. The resulting mixture was heated to 70° for 15h and then the isopropanol was removed by evaporation in vacuo. The resulting white precipitate was collected by filtration, washed with isopropanol and ether and dried under vacuum to give 56.0g (100%) of 4-but-2-ynyloxybenzenesulfonic acid sodium salt as a white solid.

### Reference Example 302

### 4-But-2-ynyloxybenzenesulfonyl chloride

To a 0° solution of 43.8 mL (0.087 mol) of oxalyl chloride in 29 mL of dichloromethane was dropwise added 6.77 mL (0.087 mol) of DMF followed by 7.24g (0.029 mol) of 4-but-2-ynyloxybenzenesulfonic acid sodium salt.. The reaction mixture was stirred for 10 minutes at 0° then let warm to room temperature and stirred for 2 days. The reaction was then poured into ice and extracted with 150 mL of hexanes. The organics were washed with water and brine, dried over Na₂SO₄, filtered and concentrated in vacuo to provide 6.23g (88%) of the sulfonyl chloride as a yellow solid; m.p. 63-65°C.; Mass Spec (EI) 243.9 (M⁺).

### Reference Example 303

### But-2-ynyloxybenzene

To a solution of 6.14g (0.023 mol) of triphenylphosphine dissolved in 100 mL of benzene and 40 mL of THF was added 1.64g (0.023 mol) of 2-butyn-1-ol. After five minutes 2.00g (0.021 mol) of phenol, dissolved in 10 mL of THF, was added to the reaction followed by 3.69 mL (0.023 mol) of diethyl azodicarboxylate. The resulting reaction mixture was stirred for 18h at room temperature and then concentrated in vacuo. The residue was chromatographed on silica gel eluting with ethyl acetate/hexanes (1:10) to provide 2.18g (70%) of the desired butynyl ether as a clear liquid;Mass Spec (Electrospray) 146.0 (MH+)

### Reference Example 304

### 4-But-2-ynyloxybenzenesulfonyl chloride

To a solution of 0.146g (1.0 mmol) of but-2-ynyloxybenzene in 0.3 mL of dichloromethane in an acetone/ice bath under N₂ was dropwise added a solution of 0.073 mL (1.1 mmol) of chlorosulfonic acid in 0.3 mL of dichloromethane. After the addition was complete, the ice bath was removed and the reaction was stirred at room temperature for 2h. To the reaction was then dropwise added 0.113 mL (1.3 mmol) of oxalyl chloride, followed by 0.015 mL DMF. The reaction was heated to reflux for 2h and then diluted with hexane and poured into ice water. The organic layer was washed with brine, dried over sodium sulfate, and concentrated in vacuo to provide 0.130mg (53%) of the desired product as a light brown solid.

The present invention may be embodied in other specific forms without departing from the spirit or essential attributes thereof and, accordingly, reference should be made to the appended claims, rather than to the foregoing specification, as indicating the scope of the invention.

### Example 1

### 1-Acetyl-4-(4-but-2-ynyloxbenzenesulfonyl)-2,3,4,5-tetrahydro-1H-[1,4]benzodiazepine-3-carboxylic acid, hydroxyamide

To a mixture of 6.0 g(13.14 mmol) of methyl 1-acetyl-4-(4-but-2-ynyloxybenxene-sulfonyl)-2,3,4,5-tetrahydo-1H-[1,4]benzodiazepine-3-carboxylate in 66 ml of tetrahydrofuran was added 17.1 (17.1 mmol) of 1N KOH. The mixture was stored at room temperature for 3 hours and concentrated to dryness. Toluene was added several times and the solvent removed under vacuum after each addition. The residue was dried under vacuum at 75°C for 2 days to give 6.5 g of the potassium salt of 1-acetyl-4-(4-but-2-ynyloxybenzenesulfonyl)-2,3,4,5-tetrahydro-1 H-[ 1,4]benzodiazepine-3-carboxylic acid; Anal. Cal'd for C₂₂H₂₂N₆O₆SK: C, 55.0; H, 4; N, 5.8. Found C; 52.0, H, 4.5; N, 5.6.

The preceding potassium salt was converted to the 3-carboxylic acid, hydroxyamide in the following manner: To a stirred and chilled (0°)solution of 26.1 ml (52.36 mmol) of oxalyl (2.0M solution in CH₂Cl₂) in 80 ml of CH₂Cl₂) was added slowly 4.05 ml (52.36 mmol) of N,N-dimethylformamide. To this viscous mixture was added a solution of the preceding 3-carboxylic acid potassium salt in 30 ml of N,N-dimethylformamide and the mixture was stirred at room temperature for 1.5 hr and chilled (0°) (solution A). A solution of 11.89 ml (0.194 ml) of hydroxylamine (50% in H₂O) in 60ml of tetrahydrofuran was cooled in an ice hath (solution B). To the cold solution B was added slowly the solution A and the mixture allowed to warm to room temperature and was stirred overnight. The mixture was diluted with 200 ml of CH₂Cl₂ and washed with 100 ml of H₂O. The organic layer was separated and the aqueous layer was extracted with CH₂Cl₂. The organic layer and extract were combined, and the solvent removed under vacuum. The residue was diluted with 300 ml of ethyl acetate and the solution washed with 20 ml each of H₂O, 2N citric acid, H₂O and with 120 ml of NaHCO₃ (2 times) and 120 ml of brine. The solution was dried (Na₂SO₄) and the solvent removed under vacuum to give a foam. Crystallization from ethyl acetate gave 2.5 g of white crystals, mp 167-169°C; Anal Calc'd for C₂H₂₃N₃O₆S: C, 57.8; H, 5.1; N, 9.2. Found: C, 57.5; H, 5.2; N, 8.9.

### Example 2

### 4-(4-But-2-ynyloxybenzenesulfonyl)-1-(2-thienylcarbonyl)-2,3,4,5-tetrahydro-1H-[1,4]benzodiazepine-3-carboxylic acid, hydroxyamide

To a mixture of 0.87 g (1.66 mmol) of methyl 4-(4-but-2-ynyloxybenzenesulfonyl)-2,3,4,5-tetrahydro-1H-[1,4]benzodiazepine-3-carboxylate in 4 ml or tetrahydrofuran was added 2.2ml (2.2 mmol) of 1N KOH. The solution was stirred at room temperature for 2.5 hrs. and then concentrated to dryness. Toluene was added repeatedly and the solvent removed after each addition. The residue was dried at 75°C for 60 hrs to give 0.99 of a foam; Anal Calc'd for C₂₅H₂₂N₂O₆S₂K: C, 54.7; H, 3.9; N, 5.1. Found: C, 47.8; H, 4.4;N, 6.0.

To a chilled (0°) solution of 2.66 ml(5.3 mmol) of oxalyl chloride (2.0M solution in CH₂Cl₂) in 8 ml of CH₂Cl₂ was added slowly 412 µL of N,N-dimethylformamide. To this solution was added a solution of 0.73g (1.33 mmol) of the preceding potassium salt in 3 ml of N,N-dimethylformamide (solution A). A solution of 1.22 ml (19.95 mmol) of hydroxylamine (50% in H₂O) in 6 ml of tetrahydrofuran was chilled to 0° (solution B). The cooled solution A was added slowly to the cooled solution B and the mixture allowed to warm to room temperature and stir overnight. The mixture was diluted with CH₂Cl₂ and H₂O and the organic layer separated. The agueous layer was extracted with CH₂Cl₂ and the organic layer and extract combined and concentrated to dryness. The residue was diluted with ethyl acetate and the solution washed with 2N citric acid, H₂O, 1N NaHCO_{3,} brine and dried (Na₂SO₄). The solvent was removed to give 0.65 g of solid. The solid was chromatographed on silica gel with hexane-ethyl acetate (1:1) and then 10% CH₃OH in ethyl acetate to give 0.20 g of a white foam; Mass spectrum (ES) 526.4(M+H); Anal Calc'd for C₂₅H₂₃N₃O₂S₂:C, 57.1; H, 4.4; N, 8.0. Found: C, 56.9; H, 4.3; N,7.8.

### Example 3

### 1-Benzoyl-4-(4-but-2-ynyloxybenzenesulfonyl)-2,3,4,5-tetrahydro-1H-[1,4]-benzodiazepine-3-carboxylic acid, hydroxyamide

To a mixture of 1.5 g (2.89 mmol) of methyl 1-benzoyl-4-(4-but-2-ynyloxybenzenesulfonyl)-2,3,4,5-tetrahydro-1H-[1,4]benzodiazepine-3-carboxylate in 7 ml of tetrahydrofuran was added 3.8 ml (3.8 mmol) of 1N KOH. The solution was stirred for 2 hours and the mixture concentrated to dryness. Toluene was added repeatedly and the solvent removed after each addition to give a solid. The solid was dried at 75°C under vacuum to give 1.6 g of the potassium salt of 1-benzoyl-4-(4-but-2-ynylbenzenesulfonyl)-2,3,4,5-tetrahydro-1H-[1,4)benzodiazepine-3-carhoxylic acid as a white foam; Mass spectrum (ES) 505.2 (M+H): Anal Calc'd for C₂₇H₂₄N₂O₆SK; C, 61.9; H, 4.5; N, 5.4. Found: C, 5.22; H, 4.6; N, 5.9.
The preceding potassium salt was converted to the 3-carboxylic acid, hydroxamide in the following manner. To a stirred and chilled (0°) solution of 4.8 ml (9.6 mmol) of oxalyl chloride (2.0M solution in CH₂Cl₂) in 14 ml of CH₂Cl₂ was added slowly 370 µL of N,N-dimethylformamide. To this mixture was added a chilled solution of the preceding 3-carboxylic acid potassium salt in 5 ml of N,N dimethylformamide. The mixture was stirred at 0° for 1.5 hours (solution A). A solution of 2.2 ml (35.9 mmol) of hydroxylamine (50% in H₂O) in 10 ml of tetrahydrofuran was cooled in an ice bath (0°) (solution B). To the stirred cold solution B was added slowly the cold solution A. The mixture was allowed to warm to room temperature and stir overnight. The mixture was diluted with CH₂Cl₂ and H₂O and the organic layer separated. The organic layer was concentrated and the residue diluted with ethyl acetatee and washed with 1M NaHCO₃, H₂O, brine and dried (Na₂SO₄). The solvent was removed to give 2.0g of a solid. Chromatography on silica gel with 10% CH₃OH in ethyl acetate gave 0.96 g of solid. The solid was dissolved in hexane-ethyl acetate (1:1) and the solution filtered through diatomaceous earth and then through silica gel (500 ml wash). The product was then eluted from the silica gel with 10% CH₃OH in ethyl acetate to give a 0.593 g of white solid; Mass spectrum (ES) 520 (M+H); Anal. Calc'd for C₂₇H₂₅N₃O₆S: C, 62.4; H, 4.9; N, 8. 1. Found: C, 61.2; H, 4.9; N, 7.9.

Utilizing the method described in Examples 1-3, the following 1-substituted-4-(4-but-2-ynyloxybenzenesulfonyl)-2,3,4,5-tetrahydro-1H-[1,4]benzodiazepine-3-carboxylic acid, hydroxyamides and 1-substituted-4-(4-[4-substituted-but-2-ynyloxy]benzenesulfonyl)-2,3,4,5-tetrahydro-1H-[1,4]benzodiazepine-3-carboxylic acid, hydroxyamides can be prepared.

### Example 4

### 1-Butoxyacetyl-4-(4-but-2-ynyloxybenzenesulfonyl)-2,3,4,5-tetrahydro-1H-[1,4]benzodiazepine-3-carboxylic acid, hydroxyamide

### Example 5

### 4-(4-But-2-ynyloxybenzenesulfonyl)-1-(4-methylphenylsulfonyl)-2,3,4,5-tetrahydro-1H-[1,4]benzodiazepine-3-carboxylic acid, hydroxyamide

### Example 6

### 4-(4-But-2-ynyloxybenzenesulfonyl)-1-methanesulfonyl-2,3,4,5-tetrahydro-1H-[1,4]benzodiazepine-3-carboxylic acid, hydroxyamide

### Example 7

### 1-Benzoyl-4-(4-[4-methoxybut-2-ynyloxy]benzenesulfonyl)-2,3,4,5-tetrahydro-1H-[1,4]benzodiazepine-3-carboxylic acid, hydroxyamide

### Example 8

### 1-Acetyl-4-(4-[4-methoxybut-2-ynyloxy]benzenesulfonyl)-2,3,4,5-tetrahydro-1H-[1,4]benzodiazepine-3-carboxylic acid, hydroxyamide

### Example 9

### 4-[4-But-2-ynyloxy]benzenesulfonyl)-1-(3-pyridinylcarbonyl)-2,3,4,5-tetrahydro-1H-[1,4]benzodiazepine-3-carboxylic acid, hydroxyamide

### Example 10

### 4-(4-Pent-2-ynyloxy benzenesulfonyl)-1-(3-pyridinylcarbonyl)-2,3,4,5-tetrahydro-1H-[1,4]benzodiazepine-3-carboxylic acid, hydroxyamide

### Example 11

### 4-(4-[4-Methoxybut-2-ynyloxy]benzenesulfonyl)-1-(2-thienylcarbonyl)-2,3,4,5-tetrahydro-1H-[1,4]benzodiazepine-3-carboxylic acid, hydroxyamide

### Example 12

### 4-(4-But-2-ynyloxybenzenesulfonyl)-1-(4-pyridinylcarbonyl)-2,3,4,5-tetrahydro-1H-[1,4]benzodiazepine-3-carboxylic acid, hydroxyamide

### Example 13

### 1-(4-Biphenylcarbonyl)-4-(4-but-2-ynyloxybenzenesulfonyl)-2,3,4,5-tetrahydro-1H-[1,4]benzodiazepine-3-carboxylic acid, hydroxyamide

### Example 14

### 4-(4-[4-Methoxybut-2-ynyloxy]benzenesulfonyl)-1-(propane-1-sulfonyl)-2,3,4,5-tetrahydro-1H-[1,4]benzodiazepine-3-carboxylic acid, hydroxyamide

### Example 15

### 1-([1,1'-Biphenyl]-2-carbonyl)-4-(4-[4-methoxybut-2-ynyloxy]benzenesulfonyl)-2,3,4,5-tetrahydro-1H-[1,4]benzodiazepine-3-carboxylic acid, hydroxyamide

### Example 16

### 1-(3-Fluorobenzoyl)-4-(4-pent-2-ynyloxybenzenesulfonyl)-2,3,4,5-tetrahydro-1H-[1,4]benzodiazepine-3-carboxylic acid, hydroxyamide

### Example 17

### 4-(4-[4-Ethoxybut-2-ynyloxy]benzenesulfonyl)-1-(2 methyl-3-fluorobenzoyl)-2,3,4,5-tetrahydro-1H-[1,4]benzodiazepine-3-carboxylic acid, hydroxyamide

### Example 18

### 4-(4-[4-Dimethylaminobut-2-ynyloxy]benzenesulfonyl)-1-(2 methyl-3-fluorobenzoyl)-2,3,4,5-tetrahydro-1H-[1,4]benzodiazepine-3-carboxylic acid, hydroxyamide

### Example 19

### 4-(4-But-2-ynyloxybenzenesuflony)-1-(2-triflouromethylbenzoyl)-2,3,4,5-tetrahydro-1H-[1,4]benzodiazepine-3-carboxylic acid, hydroxyamide

### Example 20

### 1-(2-Chloro-6-trifluormethylbenzoyl)-4-(4-pent-2-ynyloxybenzenesulfonyl)-2,3,4,5-tetrahydro-1H-[1,4]benzodiazepine-3-carboxylic acid, hydroxyamide

### Example 21

### 1-(4-Fluoro-2-trifluoromethylbenzoyl)-4-(4-[4-methoxybut-2-ynyloxy]benzenesulfonyl)-2,3,4,5-tetrahydro-1H-[1,4]benzodiazepine-3-carboxylic acid, hydroxyamide

### Example 22

### 1-(2-Fluoro-6-trifluormethylbenzoyl)-4-(4-pent-2 ynyloxybenzenesulfonyl)-2,3,4,5-tetrahydro-1H-[1,4]benzodiazepine-3-carboxylic acid, hydroxyamide

### Example 23

### 4-(4-But-2-ynyloxybenzenesulfonyl)-1-(2 methyl-6-chlorobenzoyl)-2,3,4,5-tetrahydro-1H-[1,4]benzodiazepine-3-carboxylic acid, hydroxyamide

### Example 24

### 1-(2,4-Dimethylbenzoyl)-4-(4-[4-methoxybut-2-ynyloxy]benzenesulfonyl)-2,3,4,5-tetrahydro-1H-[1,4]benzodiazepine-3-carboxylic acid, hydroxyamide

### Example 25

### 1-(2,5-Dimethylbenzoyl)-4-(4-[4-methoxybut-2-ynyloxy]benzenesulfonyl)-2,3,4,5-tetrahydro-1H-[1,4]benzodiazepine-3-carboxylic acid, hydroxyamide

### Example 26

### 1-(2-Chloro-4-flurobenzoyl)-4-(4-[4-methoxybut-2-ynyloxy]benzenesulfonyl)-2,3,4,5-tetrahydro-1H-[1,4]benzodiazepine-3-carboxylic acid, hydroxyamide

### Example 27

### 1-(2-Chlorobenzoyl)-4-(4-[4-methoxybut-2-ynyloxy]benzenesulfonyl)-2,3,4,5-tetrahydro-1H-[1,4]benzodiazepine-3-carboxylic acid, hydroxyamide

### Example 28

### 1-(2-Chlorobenzoyl)-4-(-4-pent-2-ynyloxybenzenesulfonyl)-2,3,4,5-tetrahydro-1H-[1,4]benzodiazepine-3-carboxylic acid, hydroxyamide

### Example 29

### 1-(2-Fluorobenzoyl)-4-(4-[4-methoxybut-2-ynyloxy]benzenesulfonyl)-2,3,4,5-tetrahydro-1H-[1,4]benzodiazepine-3-carboxylic acid, hydroxyamide

### Example 30

### 1-(2-Chloro-6-fluorobenzoyl)-4-(4-[4-methoxybut-2-ynyloxy]benzenesulfonyl)-2,3,4,5-tetrahydro-1H-[1,4]benzodiazepine-3-carboxylic acid, hydroxyamide

### Example 31

### 1-(2,3-Difluorobenzoyl)-4-(4-[4-methoxybut-2-ynyloxy]benzenesulfonyl)-2,3,4,5-tetrahydro-1H-[1,4]benzodiazepine-3-carboxylic acid, hydroxyamide

### Example 32

### 1-(2,4-Dichlorobenzoyl)-4-(4-[4-methoxybut-2-ynyloxy]benzenesulfonyl)-2,3,4,5-tetrahydro-1H-[1,4]benzodiazepine-3-carboxylic acid, hydroxyamide

### Example 33

### 1-(2,4-Dichlorobenzoyl)-4-(4-pent-2-ynyloxybenzenesulfonyl)-2,3,4,5-tetrahydro-1H-[1,4]benzodiazepine-3-carboxylic acid, hydroxyamide

### Example 34

### 1-(2,3-Dichlorobenzoyl)-4-(4-[4-methoxybut-2-ynxloxy]benzenesulfonyl)-2,3,4,5-tetrahydro-1H-[1,4)benzodiazepine-3-carboxylic acid, hydroxyamide

### Example 35

### 1-(2,5-Dichlorobenzoyl)-4-(4-[4-hydroxybut-2-ynyloxy]benzenesulfonyl)-2,3,4,5-tetrahydro-1H-[1,4]benzodiazepine-3-carboxylic acid, hydroxyamide

### Example 36

### 1-(Benzoyl)-4-(4-pent-2-ynyloxybenzenesulfonyl)-2,3,4,5-tetrahydro-1H-[1,4]benzodiazepine-3-carboxylic acid, hydroxyamide

### Example 37

### 4-(4-But-2-ynyloxybenzenesulfony)-1-(2-methylthiobenzoyl)-2,3,4,5-tetrahydro-1H-[1,4]benzodiazepine-3-carboxylic acid, hydroxyamide

### Example 38

### 4-(4-But-2-ynyloxybenzenesulfony)-1-(3-methyl-2-thienylcarbonyl)-2,3,4,5-tetrahydro-1H-[1,4]benzodiazepine-3-carboxylic acid, hydroxyamide

### Example 39

### 4-(4-[4-Hydroxybut-2-ynyloxy]benzenesulfonyl)-1-(4 methyl-2-thienylcarbonyl)-2,3,4,5-tetrahydro-1H-[1,4]benzodiazepine-3-carboxylic acid, hydroxyamide

### Example 40

### 1-(3-Chloro-2-thienylcarbonyl)-4-(4-[4-dimethylaminobut 2-ynyloxy]benzenesulfonyl)-2,3,4,5-tetrahydro-1H-[1,4]benzodiazepine-3-carboxylic acid, hydroxyamide

### Example 41

### 1-(2-Furanylcarbonyl)-4-(4-[4-methylaminobut-2-ynyloxy]benzenesulfonyl)-2,3,4,5-tetrahydro-1H-[1,4]benzodiazepine-3-carboxylic acid, hydroxyamide

### Example 42

### 4-(4-But-2-ynyloxybenzenesulfonyl)-1-(3-methyl-2-furanylcarbonyl)-2,3,4,5-tetrahydro-1H-[1,4]benzodiazepine-3-carboxylic acid, hydroxyamide

### Example 43

### 4-(4-But-2-ynyloxybenzenesulfonyl)-1-(-4-methyl-2-furanylcarbonyl)-2,3,4,5-tetrahydro-1H-[1,4]benzodiazepine-3-carboxylic acid, hydroxyamide

### Example 44

### 1-(5-Chloro-2-furanylcarbonyl)-4-(4-[4-ethoxybut-2-ynyloxy] benzenesulfonyl)-2,3,4,5-tetrahydro-1H-[1,4]benzodiazepine-3-carboxylic acid, hydroxyamide

### Example 45

### 1-(5-Chloro-2-thienylcarbonyl)-4-(4-[4-hydroxybut-2-ynyloxy]benzenesulfonyl)-2,3,4,5-tetrahydro-1H-[1,4]benzodiazepine-3-carboxylic acid, hydroxyamide

### Example 46

### 1-Propionyl-4-(4-[4-hydroxybut-2-ynyloxy]benzenesulfonyl)-2,3,4,5-tetrahydro-1H-[1,4]benzodiazepine-3-carboxylic acid, hydroxyamide

### Example 47

### 1-Hexanoyl-4-(4-[4-methoxybut-2-ynyloxy]benzenesulfonyl)-2,3,4,5-tetrahydro-1H-[1,4]benzodiazepine-3-carboxylic acid, hydroxyamide

### Example 48

### 4-(4-But-2-ynyloxybenzenesulfonyl)-1-(propionyl)-2,3,4,5-tetrahydro-1H-[1,4]benzodiazepine-3-carboxylic acid, hydroxyamide

### Example 49

### 4-(4-But-2-ynyloxybenzenesulfonyl)-1-(3-thienylcarbonyl)-2,3,4,5-tetrahydro-1H-[1,4]benzodiazepine-3-carboxylic acid, hydroxyamide

### Example 50

### 4-(4-But-2-ynyloxybenzenesulfonyl)-1-(3-furanycarbonyl)-2,3,4,5-tetrahydro-1H-[1,4]benzodiazepine-3-carboxylic acid, hydroxyamide

### Example 51

### 1-(Ethoxyacetyl)-4-(4-[4-methoxybut-2-ynyloxy]benzenesulfonyl)-2,3,4,5-tetrahydro-1H-[1,4]benzodiazepine-3-carboxylic acid, hydroxyamide

### Example 52

### 1-(Acetylaminoacetyl)-4-(4-but-2-ynyloxybenzenesulfonyl)-2,3,4,5-tetrahydro-1H-[1,4]benzodiazepine-3-carboxylic acid, hydroxyamide

### Example 53

### 1-(N,N-Dimethylaminoacety)-4-(4-[4-methoxybut-2-ynyloxy]benzenesulfonyl)-2,3,4,5-tetrahydro-1H-[1,4]benzodiazepine-3-carboxylic acid, hydroxyamide

### Example 54

### 1-(Cyclopropylcarbonyl)-4-(4-[4-methoxybut-2-ynyloxy]benzenesulfonyl )-2,3,4,5-tetrahydro-1H-[1,4]benzodiazepine-3-carboxylic acid, hydroxyamide

### Example 55

### 1-(Cyclobutylcarbonyl)-4-(4-but-2-ynyloxybenzenesulfonyl)-2,3,4,5-tetrahydro-1H-[1,4]benzodiazepine-3-carboxylic acid, hydroxyamide

### Example 56

### 4-(4-But-2-ynyloxybenzenesulfonyl)-1-(triflouroacety)-2,3,4,5-tetrahydro-1H-[1,4]benzodiazepine-3-carboxylic acid, hydroxyamide

### Example 57

### 4-(4-But-2-ynyloxybenzene-sulfonyl)-1-methoxyacetyl-2,3,4,5-tetrahydro-1H-[1,4]benzodiazepine-3-carboxylic acid, hydroxyamide

### Example 58

### 4-(4-[4-Methoxybut-2-ynyloxy]benzenesulfonyl)-1-(3-pyridinylcarbonyl)-2,3,4,5-tetrahydro-1H-[1,4]benzodiazepine-3-carboxylic acid, hydroxyamide

### Example 59

### 4-(4-[4-Hydroxybut-2-ynyloxy]benzenesulfonyl)-1-(4-pyridinylcarbonyl)-2,3,4,5-tetrahydro-1H-[1,4]benzo-diazepine-3-carboxylic acid, hydroxyamide

### Example 60

### 1-(Ethoxyacetyl)-4-(4-[4-ethoxybut-2-ynyloxy]benzenesulfonyl)-2,3,4,5-tetrahydro-1H-[1,4]benzodiazepine-3-carboxylic acid, hydroxyamide

### Example 61

### 1-(Acetylaminoacetyl)-4-(4-[4-methoxybut-2-ynyloxy]benzenesulfonyl)-2,3,4,5-tetrahydro-1H-[1,4]benzodiazepine-3-carboxylic acid, hydroxyamide

### Example 62

### 4-(4-[4-Methoxybut-2-ynyloxy]benzenesulfonyl)-1-(3-methyl-2-thienylcarbonyl)-2,3,4,5-tetrahydro-1H-[1,4]benzodiazepine-3-carboxylic acid, hydroxyamide

### Example 63

### 4-(4-But-2-ynyloxybenzene-sulfonyl)-1-(3-methoxypropionyl)-2,3,4,5-tetrahydro-1H-[1,4]benzodiazepine-3-carboxylic acid, hydroxyamide.

### Example 64

### 4-(4-But-2-ynyloxybenzene-sulfonyl)-1-(2-chlorobenzoyl)-2,3,4,5-tetrahydro-1H-[1,4]benzodiazepine-3-carboxylic acid, hydroxyamide.

### Example 65

### 4-(4-But-2-ynyloxybenzene-sulfonyl)-1-(2-fluorobenzoyl)-2,3,4,5-tetrahydro-1H-[1,4]benzodiazepine-3-carboxylic acid, hydroxyamide.

### Example 66

### 4-(4-But-2-ynyloxybenzene-sulfonyl)-1-(phenoxyacetyl)-2,3,4,5-tetrahydro-1H-[1,4]benzodiazepine-3-carboxylic acid, hydroxyamide.

### Example 67

### 4-(4-But-2-ynyloxybenzene-sulfonyl)-1-[2-(1-pyrazolyl)phenylcarbonyl]-7-methyl-2,3,4,5-tetrahydro-1H-[1,4]-benzodiazepene-3-carboxylic acid, hydroxyamide

### Example 68

### 4-(4-But-2-ynyloxybenzene-sulfonyl)-1-(5-chloro-2-thienylcarbonyl)-2,3,4,5-tetrahydro-1H-[1,4]benzodiazepine-3-carboxylic acid, hydroxyamide

### Example 69

### 4-(4-But-2-ynyloxybenzene-sulfonyl)-1-(5-chloro-2-furanylcarbonyl)-2,3,4,5-tetrahydro-1H-[1,4]benzodiazepine-3-carboxylic acid, hydroxyamide

### Example 70

### 4-(4-[4-Methoxybut-2-ynyloxy]-benzenesulfonyl)-1-propionyl-2,3,4,5-tetrahydro-1H-[1,4]benzodiazepine-3-carboxylic acid, hydroxyamide

### Example 71

### 4-(4-[4-Methoxybut-2-ynyloxy]benzenesulfonyl)-1-(3-thienylcarbonyl)-2,3,4,5-tetrahydro-1H-[1,4]benzo-diazepine-3-carboxylic acid, hydroxyamide

### Example 72

### 1-(Aminoacetyl)-4-(4-but-2-ynyloxybenzenesulfonyl)-2,3,4,5-tetrahydro-1H-[1,4]benzodiazepine-3-carboxylic acid, hydroxyamide

### Example 73

### 4-(4-But-2-ynyloxy-benzenesulfonyl)-1-(N,N-Dimethylaminoacetyl)-2,3,4,5-tetrahydro-1H-[1,4]benzodiazepine-3-carboxylic acid, hydroxyamide

### Example 74

### 4-(4-But-2-ynyloxybenzenesulfonyl)-1-(cycloyhexylcarbonyl)-2,3,4,5-tetrahydro-1H-[1,4]-benzodiazepine-3-carboxylic acid, hydroxyamide.

### Example 75

### 1-Methoxyacetyl-4-(4-[4-methoxybut-2-ynyloxy]benzenesulfonyl)-7-methyl-2,3,4,5-tetrahydro-1H-[1,4]-benzodiazepine-3-carboxylic acid, hydroxyamide

### Example 76

### 1-Benzoyl-4-(4-but-2-ynyloxybenzenesulfonyl)-7-methyl-2,3,4,5-tetrahydro-1H-[1,4]-benzodiazepine-3-carboxylic acid, hydroxyamide

### Example 77

### 1-(Benzoyl)-4-(4-but-2-ynyloxybenzenesulfonyl)-8-chloro-2,3,4,5-tetrahydro-1H-[1,4]benzodiazepine-3-carboxylic acid, hydroxyamide

### Example 78

### 4-(4-But-2-ynyloxybenzenesulfonyl)-1-(2 -furanylcarbonyl)-2,3,4,5-tetrahydro-1H-[1,4]benzodiazepine-3-carboxylic acid hydroxyamide

To a solution of 3.0g (6.38 mmol) of methyl 1-(2 furanylcarbonyl)-4-(4-methoxybenzenesulfonyl)-2,3,4,5-tetrahydro-1 H-[1,4]benzodiazepine-3-carboxylate in., 15 ml of CH₂Cl₂ (cooled to 0°C) was added dropwise 12.8ml (2.8 mmol) of BBr₃ in CH₂Cl₂ (1.0 M in CH₂Cl₂). The mixture was stirred at room temperature for 3 days ,diluted with CH₂Cl₂ and then ice was added. The organic layer was separated, washed with H₂O, brine and dried ((Na₂SO₄). The solvent was removed and the residue chromatographed on silica gel (flash column) with ethyl acetate-hexane (1:1) as solvent. The fractions containing product were combined, the solvent removed and the residue triturated with ethyl acetate. Chilling and filtering gave 0.72g of methyl 1-(2-furanylcarbonyl)-4-hydroxybenzenesulfonyl)-2,3,4,5-tetrahydro-1H-[1,4]benzodiazepine-3-carboxylate as a white solid, mp 204-206°C; Anal Cal'd for C₂₂H₂₀N₂O₇S:
C, 57.9; H, 4.2; N, 6.1. Found: C,57.2; H,4.3; N, 6.0.
To 1.26g (4.82 mmol) of triphenylphosphine in 10 ml of toluene and 2.5 ml of tetrahydrofuran was added 360 uL (4.82 mmol) of 2-butyn-1-ol and 1.48 g (3.2 mmol)of methyl 1-(2-furanylcarbonyl)-4-(4-hydroxybenzenesulfonyl)-1H-[1,4]-benzodiazepine-3-carboxylate. Under nitrogen was added 760 µL, (4.8 mmol) of diethyl azodicarboxylate and the mixture stirred for 2 days at room temperature. The solvent was removed under vacuum and the residue chromatographed on silica gel with ethyl acetate-hexane(1:1) as solvent. The fractions containing product were combined and the solvent removed to give a solid. Trituration with ethyl acetate followed by chilling and filtering gave 2.2g of white solid. The solid was recrystallized from ethyl acetate to give 1.53g of methyl 1-(2-furanylcarbonyl)-4-(4-but-2-ynyloxybenzenesulfonyl)-2,3,4,5-tetrahydro-1H-[1,4]benzodiazepine-3-carboxylate as a solid, mp 119°-120°C; Mass Spectrum (ES) 509.5(M+H).
To 1.8g (3.54 mmol) of the preceding compound in 10 ml of tetrahydrofuran was added 4.6 ml (4.6 mmol) of 1N KOH. The mixture was stirred at room temperature for 2.5 hr and diluted with H₂O and ethyl acetate. The aqueous layer was separated and acidified with 1N HCl. The mixture was extracted with ethyl acetate, the extract washed with brine and dried (Na₂SO₄). The solvent was removed to give 1.05g of 1-(2-furanylcarbony)-4-(4-but-2-ynyloxybenzenesulfonyl)-2,3,4,5-tetradydro-1H-[1,4] benzodiazepine-3-carboxylic acid as a white foam; Mass Spectrum (ES) 495.5(M+H); Anal Calc'd for C₂₅H₂₂N₂O₇S: C, 60.7; H, 4.5; N, 5.7. Found: C, 57.6; H, 4.8; N, 6.6.

To a solution of 3.4 ml (6.8 mmol) of oxalyl chloride (2.0M solution in CH₂Cl₂) in 8 ml of CH₂Cl₂ (0°C) was added 527 µL of N,N-dimethylformamide. To the solution was added a solution of 0.84g (1.7 mmol) of the preceding acid in 3 ml of N,N-dimethylformamide. The mixture was stirred at room temperature under nitrogen for 1.5 hr. (Solution A).

In a separate flask, 1.56 ml (25.5 mmol) of hydroxylamine (50% in H₂O) was diluted with 6 ml of tetrahydrofuran and the solution chilled to 0° (Solution B). The Solution A was added slowly to the Solution B and the mixture stirred overnight at room temperature.The mixture was diluted with CH₂Cl₂ and the solution washed with H₂O, 2N citric acid, 1M NaHCO₃ and concentrated to dryness. The solid residue was chromatographed on silica gel (flash column) with ethyl acetate-hexane (1:1) as solvent to remove impurities. The product was eluted with 10% methonal in ethyl acetate. The combined product fractions were concentrated and dilluted with ethyl acetate and the solution dried (Na₂SO₄). The solvent was removed to give a solid which was dried 20 hr at 80°C under vacuum to give 0.64g of the product hydroxamic acid.

### Example 79

### 1-Cyclopropylcarbonyl-4-(4-but-2-ynyloxybenzenesulfonyl)-2,3,4,5-tetrahydro-1H-[1,4]benzodiazepine-3-carboxylic acid, hydroxyamide

To a solution of 4..44g(10 mmol) of methyl 1-cyclopropylcarbonyl-4-(4-methoxybenzenefulfonyl)-2,3,4-tetrahydro-1H-[1,4]benzodiaxepine-3-carboxylate in 25 ml of CH₂Cl₂ chilled to 0° C was added dropwise 22 ml (22 mmol) of BBr₃ in CH₂Cl₂ (1.0 molar solution). The mature was stirred overnight, cooled and diluted with ice and H₂O. Dichloromethane was added and the organic layer separated and washed with H₂O, brine and dried (Na₂SO₄). The solvent was removed under vacuum to give a solid which was chromatographed on silica gel with the solvent ethyl acetate-hexane (1:1) to give 1.0 g of methyl 1-cyclopropylcarbonyl-4-(4-hydroxybenzene-sulfonyl)-2,3,4,5-tetrahydro-1H-[1,4] benzodiazepine-3-carboxylate as a foam.

As described for Example 57, 0.45g (1.09mmol) of the preceding compound and 123 µL (1.64 mmol) of 2-butyn-1-ol were coupled with 0.430g (1.64 mmol) of tripbenylphosphine and 2.59 µL (1.64 mol) of diethyl azodicarboxylate in 3.5 ml of toluene and 1 ml of tetrahydrofuran as solvent. The product was purified by chromatography on silica gel with ethyl acetate-hexane (1:15) as solvent to give 0.60g of methyl 4-(4-but-2-ynyloxybenzenesulfonyl)-1-(cyclopropylcarbonyl)-2,.3,4,5-tetrahydro-1H-[14]benzodiazepine-3-carboxylate as a solid. A solution of the preceding compound (0.57g; 1.18 mmol) in a mixture of 1.5 ml (1.53 mol) of 1N KOH and 3 ml of tetrahydrofuran was stirred 3 hours; concentrated and extracted with ethyl acetate. The aqueous residue was acidified with 1N HCl and extracted with ethyl acetate. The extract was washed with brine, dried (Na₂SO₄) and the solvent removed to give 0.23g of 4-(4-but-2-ynyloxybenzenesulfonyl)-1-(cyclopropylcarbonyl)-2,3,4,5-tetrahydro-1H-[1,4]benzodiazepine-3-carboxylic acid as an off-white solid.

As described for Example 57, 0.20g (0.427 mmol) of the preceding compound was reacted with 855 µL (1.7 mmol) of N,N-dimethylformanide (DMF) in 3 ml of CH₂Cl₂ and 1 ml of DMF followed by reaction with 393µL (.6.41 mmol) of hydroxylamine (50% in H₂O) in 2 ml of tetrahydrofuran to the product was dried at 80° C overnight to give 0.188g of a white foam; Mass Spectrum (ES) 484.5(M+H); Anal Calc'd for C₂₄H₂₅N₃O₆S: C, 59.6; H, 5.2; N, 8.7. Found: C, 56.2; H, 5.1; N, 8.6.

### Example 80

### (5-Fluoro-2-nitrophenyl) methanol

To 5-fluoro-2-nitrobenzoic acid (0.5 g, 2.7 mmol) was added a solution of borane-tetrahydrofuran complex (5 mL, 1.0 M, 5 mmol) and the resulting solution was heated to 70°C for 3 hours. The reaction was then cooled to room temperature and methanol was added. The mixture was concentrated and methanol was added two additional times to provide 0.48 g (100%) of a white solid. mp 82-86°C. Anal Calc'd for C₇H₆N₃O₃ F: C, 49.13; H, 3.52; N, 8.18. Found: C, 48.76; H, 3.56; N, 7.88.

### Example 81

### 2-(Bromomethyl)-4-fluoro-1-nitrobenzene

To a solution of (5-Fluoro-2-nitrophenyl) methanol (0.3 g, 1.75 mmol) in methylene chloride (5 mL0 was added triphenylphosphine (0.52 g, 2.01 mmol) and carbon tetrabromide (0.66 g, 2.01 mmol). After 3 hours, the reaction was concentrated and chromatographed using 3:1 hexane:ethyl acetate as eluant to provide 0.38 g (93%) of the desired product as white crystals. mp 38-41°C. Anal Calc'd for C₇H₅N₃O₂FBr: C, 35.93; H, 2.15; N, 5.99. Found: C, 35.97; H, 2.12; N, 5.91.

### Example 82

### Methyl 2-({[4-(2-butynyloxy)phenyl]sulfonyl}amino)-3-hydroxypropanoate

Using the procedure of reference example 170, glycine methyl ester hydrochloride was converted to the corresponding sulfonamide using 4-But-2-ynyloxybenzenesulfonyl chloride to provide methyl 2-({[4-(2-butynyloxy)phenyl]-sulfonyl}amino)-3-hydroxypropanoate.

### Example 83

### Methyl 2-{(5-fluoro-2-nitrobenzyl)[(4-methoxyphenyl)sulfonyl]amino}-3-hydroxypropanoate

To a solution was of 2-(bromomethyl)-4-fluoro-1-nitrobenzene (1.3g, 5.56 mmol) at 0 °C was added tetrabutylammonium iodide (2.05 g, 5.56 mmol). The solution was stirred at 0 °C for 1.5 hours. In a separate flask, methyl 2-({[4-(2-butynyloxy)phenyl]sulfonyl}amino)-3-hydroxypropanoate (1.73 g, 5.05 mmol) was dissolved in dimethylformamide and cooled to 0 °C. Sodium hydride (0.21g, 5.56 mmol, 60% dispersion in oil) was added and the reaction was allowed to stir at 0 °C for 0.5 hours at which time the solution of the bromide was added. The reaction was stirred overnight and then quenched with water. The mixture was extracted twice with ethyl acetate, washed with brine, dried over Na₂SO₄, concentrated in vacuo and chromatographed using 1.5 : 1 hexane:ethyl acetate as eluant to provide 1.88 g (77%) of the desired product as a white solid. mp 83-88 °C. Anal Calc'd for C₂₁H₂₁N₂O₈FS:
C, 52.50; H, 4.41; N, 5.83. Found: C, 52.44; H, 4.76; N, 5.56.

### Example 84

### Methyl 2-((2-amino-5-fluorobenzyl){[4-(2-butynyloxy)phenyl]sutfonyl}amino)-3-hydroxypropanoate

Methyl 2-{(5-fluoro-2-nitrobenzyl)[(4-methoxyphenyl)sulfonyl]amino}-3-hydroxypropanoate (1.0 g, 2.08 mmol) was dissolved in ethanol (18 mL). To this was added tin chloride dihydrate (2.35 g, 10.4 mmol) and the reaction was heated to 70 °C for 2 hours. The reaction was then cooled to room temperature and ice water followed by NaHCO₃ was added to bring the solution to pH 8. Ethyl acetate was added and the suspension was filtered through celite. The organic layer was separated and washed with brine, dried over Na₂SO₄ concentrated in vacuo and chromatographed using 1 : 1 hexane : ethyl acetate as eluant to provide 0.55 g (58%) of the desired product as a yellow oil.

### Example 85

### Methyl 1-acetyl-4-{[4-(2-butynyloxy)phenyl]sulfonyl}-7-fluoro-2,3,4,5-tetrahydro-1H-1,4-benzodiazepine-3-carboxylate

Methyl-2-((2-amino-5-fluorobenzyl) {[4-(2-butynyloxy)phenyl]-sulfonyl}-amino)-3-hydroxypropanoate (0.48 g, 1.07 mmol) was converted to the desired product 0.41 g, (95%) using acetyl chloride as the acylating agent by following the procedure outlined in reference example 181. Anal Calc'd for C₂₃H₂₃N₂O₆FS: C, 58.22; H, 4.89; N, 5.90. Found: C, 57.58; H, 4.95; N, 5.60.

### Example 86

### 1-Acetyl-4-{[4-(2-butynyloxy)phenyl]sulfonyl}-7-fluoro-2,3,4,5-tetrahydro-1H-1,4-benzodiazepine-3-carboxylic acid

Methyl 1-acetyl-4-{[4-(2-butynyloxy)phenyl]sulfonyl}-7-fluoro-2,3,4,5-tetra-hydro-1H-1,4-henzepine-3-carhoxylate was hydrolized to the carboxylic acid utilizing the procedure from reference example 185 to provide the desired acid as an off white solid.. Anal Calc'd for C₂₂H₂₁N₂O₆FS: C, 57.38; H, 4.6; N, 6.08. Found: C, 56.93; H, 4.71; N, 5.67.

### Example 87

### 1-Acetyl-4-{[4-(2-butynyloxy)phenyl]sulfonyl}-7-fluoro-N-hydroxy-2,3,4,5-tetrahydro-1H-1,4-benzodiazepine-3-carboxamide

Using the procedure of example 3, 1-acetyl-4-{[4-(2-butynyloxy)phenyl]-sulfonyl}-7-fluoro-2,3,4,5-tetrahydro-1H-1,4-benzodiazepine-3-carboxylic acid was converted to the hydroxamic acid 45 mg (15%). Mass Spectrum (ES) 476.2 (M+H).

## Claims

1. A compound of Formula 1: 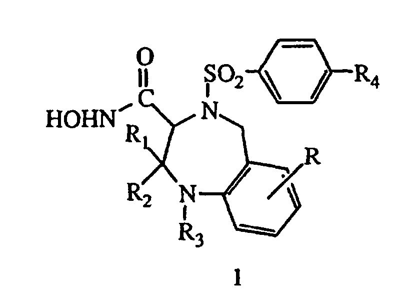 wherein
R is selected from hydrogen, (C₁-C₃) alkyl,-CN,-OR',-SR',-CF₃, -OCF₃, Cl, F, NH₂, NH(C₁-C₃)alkyl,-N(R')CO(C₁-C₃)alkyl,-N(R')(R'), NO₂, -CONH₂,-SO₂NH₂,-SO₂N(R')(R'),-N(R')COCH₂O-(C₁-C₃)alkyl, wherein R' is (C₁-C₃) alkyl or hydrogen;
R₄ is (C₁-C₆) alkyl-O- containing one triple bond, 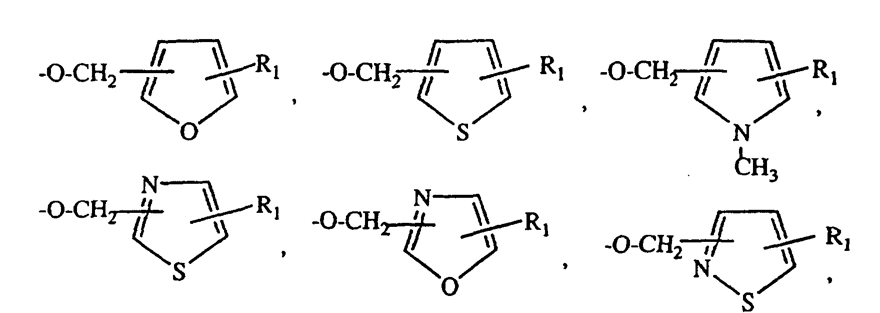 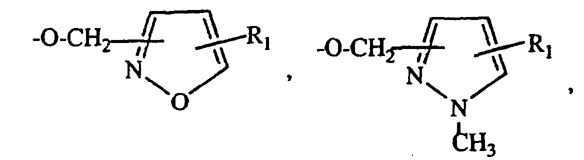 -O-CH₂- C≡C-R"
wherein R" is hydrogen, -CH₂OH, (C₁-C₆)alkyl, (C₁-C₆)alkyl-O-CH₂-, (C₁-C₆)alkyl-S-CH₂-, (C₁-C₆)alkyl-NH-CH₂-, [(C₁-C₃)alkyl]₂-NCH₂-, (C₁-C₆)cycloalkyl-O-CH₂-, [(C₁-C₃)alkyl]₂-N-(CH₂)₂₋₄NHCH₂-, [(C₁-C₃) alkyl]₂-N-(CH₂)₂₋₄N(CH₃)CH₂-, 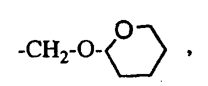 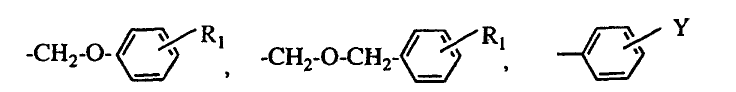 R₁ and R₂ are each, independently, hydrogen or CH₃;
R₃ is (C₁-C₈)alkyl, NH₂CH₂CO-, (C₁-C₆)alkylNHCH₂CO-, HO(CH₂)ₘCO-, HCO-, Aryl(CH₂)ₙCO-, Heteroaryl(CH₂)ₙCO-, (C₁-C₃)alkyl-O-(CH₂)ₙCO-, (C₁-C₃)alkylCO-, (C₁-C₃)alkylCO-NHCH₂CO-, (C₃-C₇)cycloalkylCO-, (C₁-C₃)alkylSO₂-, Aryl(CH₂)ₙSO₂-, Heteroaryl(CH₂)ₙSO₂-, (C₁-C₃)alkyl-O-(CH₂)ₘ SO₂-, (C₁-C₃)alkyl-O-(CH₂)ₘ, (C₁-C₃)alkyl-O-(C₁-C₃)alkyl-O-(C₁-C₃)alkyl, HO-(C₁-C₃)alkyl-O-(C₁-C₃)alkyl, Aryl-O-CH₂CO-, Heteroaryl-O-CH₂CO-, ArylCH=CHCO-, HeteroarylCH=CHCO-, (C₁-C₃)alkylCH=CHCO-, 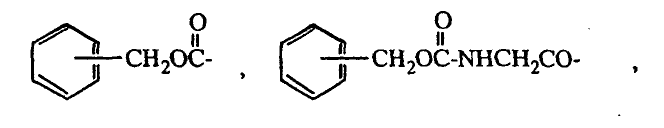 Aryl(C₁-C₃)alkyl, Heteroaryl(C₁-C₃)alkyl, ArylCH=CHCH₂-,
HeteroarylCH=CHCH₂-, (C₁-C₆)alkylCH=CHCH₂-, 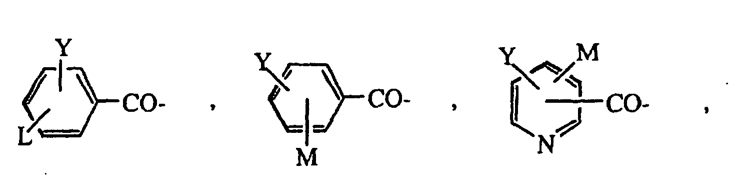 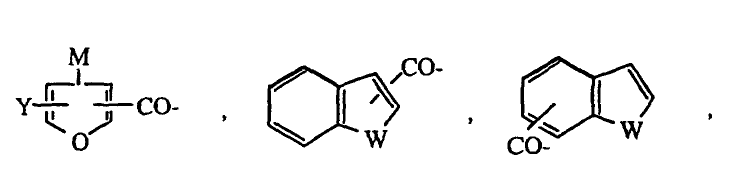 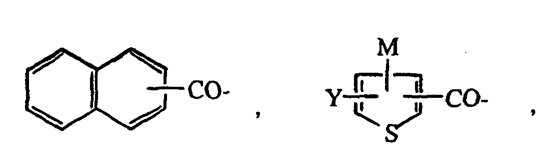 R'OCH₂ CH(OR')CO-, (R'OCH₂)₂C(R')CO-, 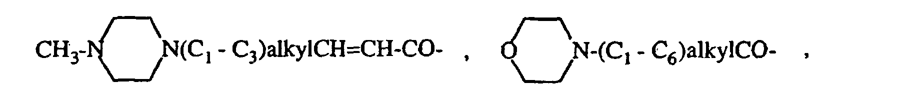 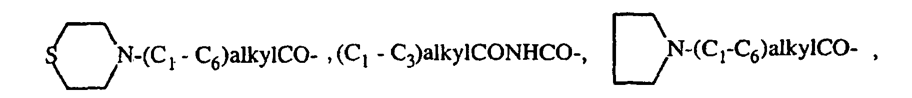 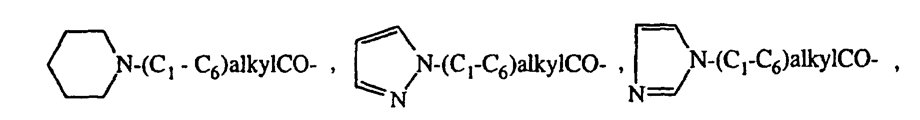 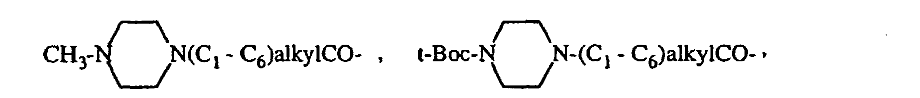 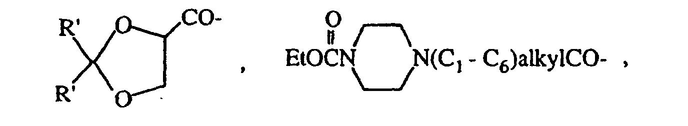 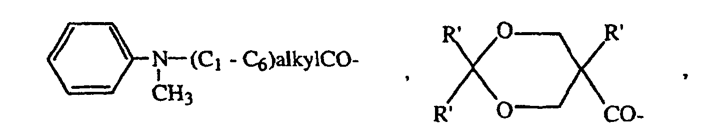 [(C₁-C₆)alkyl]₂-N-(C₁-C₆)alkyl CO-, or (C₁-C₆)alkyl-NH-(C₁-C₆)alkylCO-;
wherein
m = 1 to 3; n = 0 to 3
Aryl is 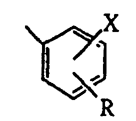 and
Heteroaryl is 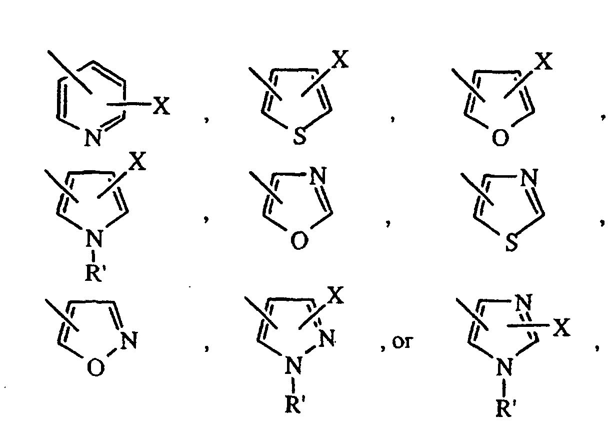 wherein X is hydrogen, halogen, (C₁-C₃) alkyl or-OCH_{3,} and R and R' are as defined above;
L is hydrogen, (C₁-C₃)alkyl,-CN,-OR',-SR',-CF₃,-OCF₃, Cl, F, NH₂,-NH-(C₁-C₃)alkyl,-N(R')CO(C₁-C₃)alkyl, N(R')(R'),-NO₂,-CONH₂,-SO₂NH₂, -SO₂N(R')(R'),-N(R')COCH₂O-(C₁-C₃)alkyl, 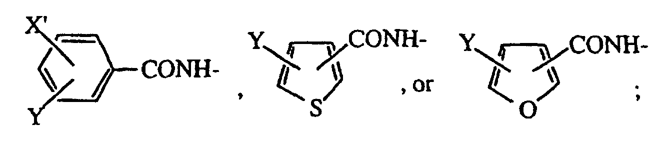 M is 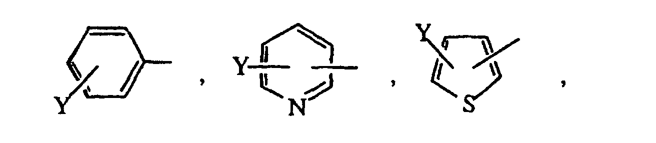 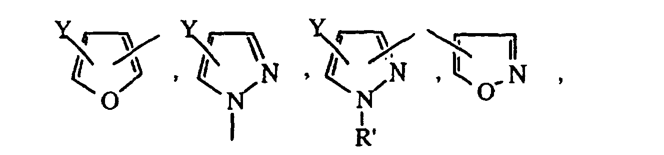 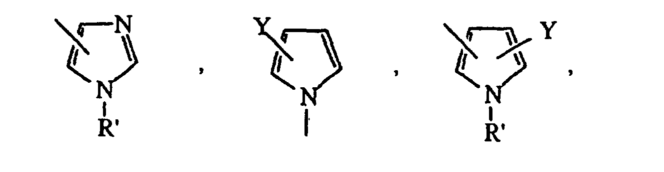 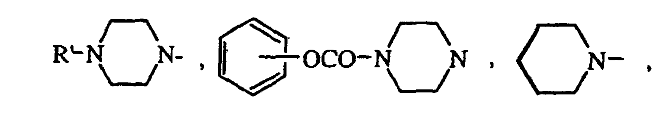 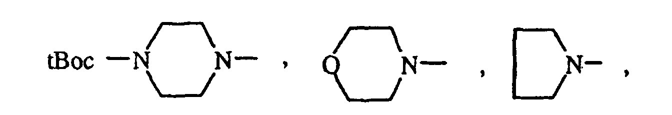 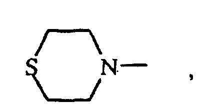 or N(R')(R') where R' is as defined above;
W is O, S, NH or N(C₁-C₃)alkyl;
Y is hydrogen, F, Cl, CF₃ or OCH₃; and X' is halogen, hydrogen, (C₁-C₃)alkyl, O-(C₁-C₃)alkyl, or-CH₂OH; and pharmaceutically acceptable salts thereof.

2. A compound according to claim 1, wherein:
R is hydrogen, (C₁-C₃) alkyl,-CN,-OR',-SR',-CF_{3,} -OCF₃, Cl, F, NH₂, NH(C₁-C₃)alkyl,-N(R')CO(C₁-C₃)alkyl,-N(R')(R'), NO₂, -CONH₂,-SO₂NH₂,-SO₂N(R')(R'), or -N(R')COCH₂O-(C₁-C₃)alkyl, wherein R' is (C₁-C₃) alkyl or hydrogen;
R₄ is (C₁-C₆) alkyl-O- containing one triple bond, 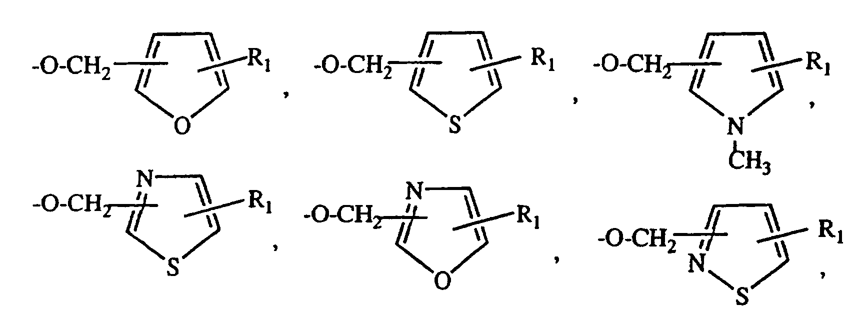 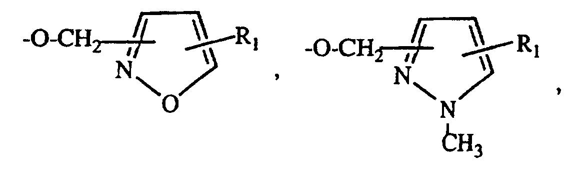 -O-CH₂-C ≡ C-R"
wherein R" is hydrogen,-CH₂OH, (C₁-C₆)alkyl, (C₁-C₆)alkyl-O-CH₂-, (C₁-C₆)alkyl-S-CH₂-, (C₁-C₆)alkyl-NH-CH₂-, [(C₁-C₃)alkyl]₂-NCH₂-, (C₁-C₆)cycloalkyl-O-CH₂-, [(C₁C₃)alkyl]₂-N-(CH₂)₂₋₄NHCH₂-, [(C₁-C₃) alkyl]₂-N-(CH₂)₂₋₄N(CH₃)CH₂-, 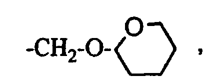 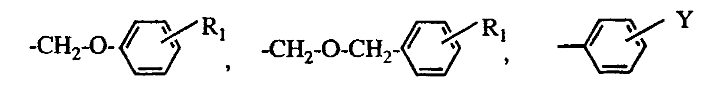 R₁ and R₂ are each, independently, hydrogen or CH₃;
R₃ is (C₁-C₈)alkyl, NH₂CH₂CO-, (C₁-C₆)alkylNHCH₂CO-, HO(CH₂)ₘCO-, HCO-, Aryl(CH₂)ₙCO-, Heteroaryl(CH₂)ₙCO-, (C₁-C₃)alkyl-O-(CH₂)ₙCO-, (C₁-C₃)alkylCO-, (C₁-C₃)alkylCO-NHCH₂CO-, (C₃-C₇)cycloalkylCO-, Aryl-O-CH₂CO-, HeteroarylOCH₂CO-, 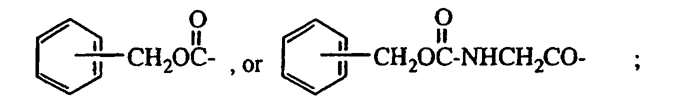 wherein
m = 1 to 3; n = 0 to 3;
Aryl is 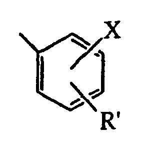 and
Heteroaryl is 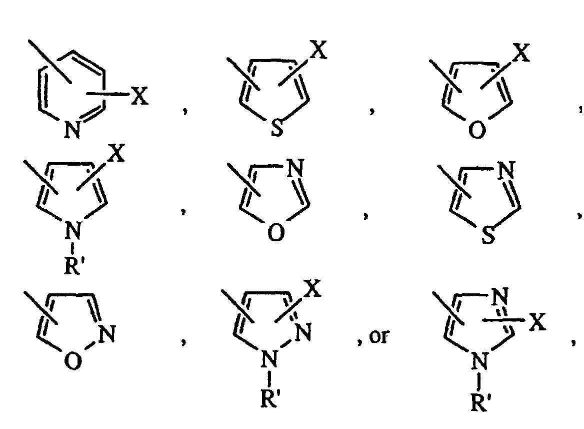 wherein X is hydrogen, halogen, (C₁-C₃) alkyl or-OCH₃ wherein R and R' are as defined above; and pharmaceutically acceptable salts thereof.

3. A compound according to claim 1, wherein
R is hydrogen, (C₁-C₃) alkyl,-CN,-OR',-SR',-CF_{3,} -OCF₃, Cl, F, NH₂, NH(C₁-C₃)alkyl,-N(R')CO(C₁-C₃)alkyl,-N(R')(R'), NO₂, -CONH₂,-SO₂NH₂,-SO₂N(R')(R'),-N(R')COCH₂O-(C₁-C₃)alkyl, wherein R' is (C₁-C₃) alkyl or hydrogen;
R₄ is (C₁-C₆) alkyl-O- containing one triple bond, 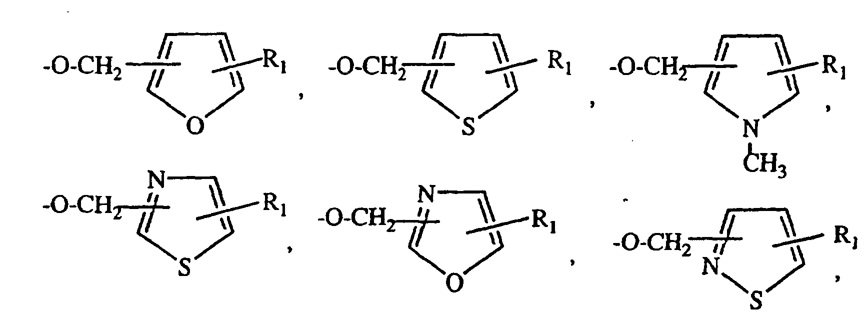 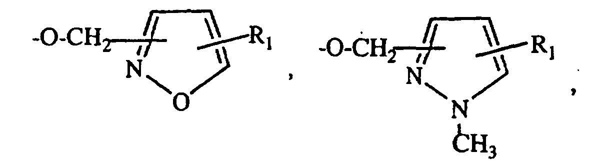 -O-CH₂-C≡C-R"
wherein R" is hydrogen, -CH₂OH, (C₁-C₆)alkyl, (C₁-C₆)alkyl-O-CH₂-, (C₁-C₆)alkyl-S-CH₂-, (C₁-C₆)alkyl-NH-CH₂-, [(C₁-C₃)alkyl]₂-NCH₂-, (C₁-C₆)cycloalkyl-O-CH₂-, [(C₁-C₃)alkyl]₂-N-(CH₂)₂₋₄NHCH₂-, [(C₁-C₃) alkyl]₂-N-(CH₂)₂₋₄N(CH₃)CH₂-, 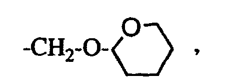 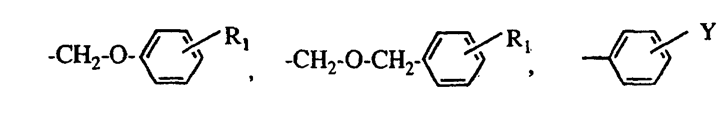 R₁ and R₂ are each, independently, hydrogen or CH₃;
R₃ is 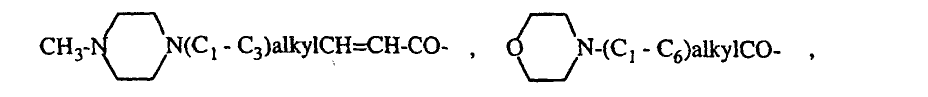 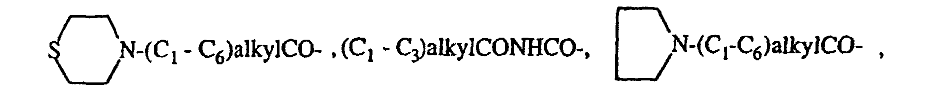 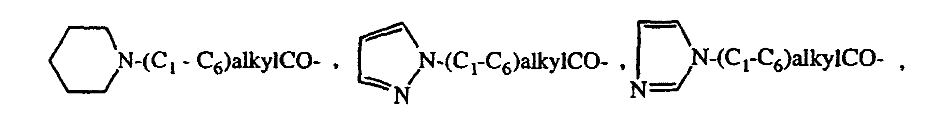 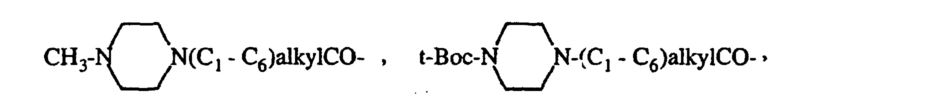 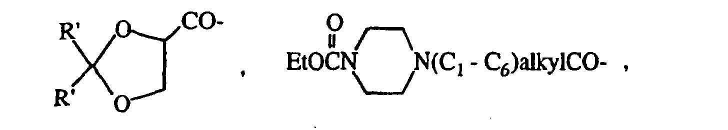 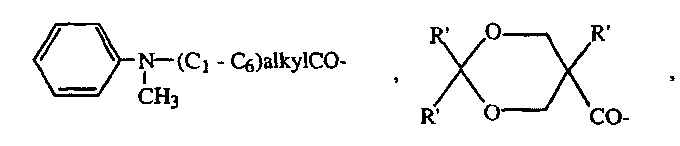 [(C₁-C₆)alkyl]₂-N-(C₁-C₆)alkyl CO-, or (C₁-C₆)alkyl-NH-(C₁-C₆)alkylCO-,
where R' is as defined above;
and pharmaceutically acceptable salts thereof.

4. A compound according to claim 1, wherein
R is hydrogen, (C₁-C₃) alkyl,-CN,-OR',-SR',-CF_{3,} -OCF₃, Cl, F, NH₂, NH(C₁-C₃)alkyl,-N(R')CO(C₁-C₃)alkyl,-N(R')(R'), NO₂, -CONH₂,-SO₂NH₂,-SO₂N(R')(R'),-N(R')COCH₂O-(C₁-C₃)alkyl, wherein R' is (C₁-C₃) alkyl or hydrogen;
R₄ is (C₁-C₆) alkyl-O- containing one triple bond, 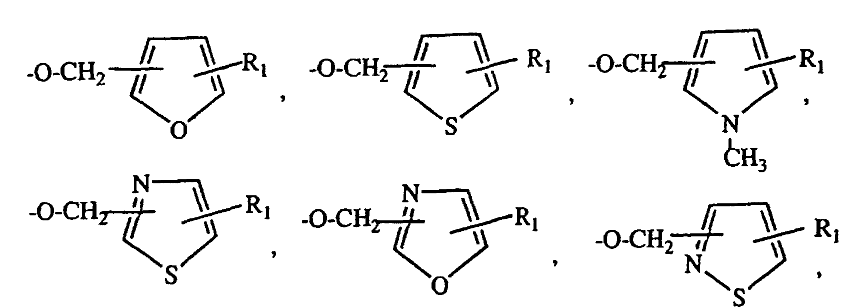 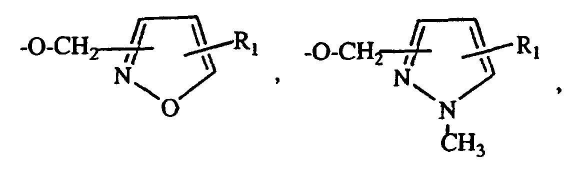 -O-CH₂-C≡C-R"
wherein R" is hydrogen, -CH₂OH, (C₁-C₆)alkyl, (C₁-C₆)alkyl-O-CH₂-, (C₁-C₆)alkyl-S-CH₂-, (C₁-C₆)alkyl-NH-CH₂-, [(C₁-C₃)alkyl]₂-NCH₂-, (C₁-C₆)cycloalkyl-O-CH₂-, [(C₁-C₃) alkyl]₂-N-(CH₂)₂₋₄NHCH₂-, [(C₁-C₃)alkyl]₂N-(CH₂)₂₋₄N(CH₃)CH₂-, 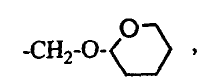 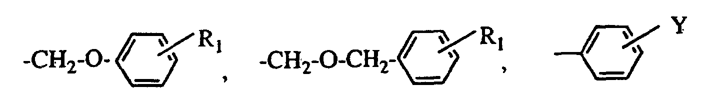 R₁ and R₂ are each, independently, hydrogen or CH₃;
R₃ is (C₁-C₃)alkylSO₂-, Aryl(CH₂)ₙSO₂-, Heteroaryl(CH₂)ₙSO₂-, or (C₁-C₃)alkyl-O-(CH₂)ₘ-SO₂,
wherein
m = 1 to 3; n = 0 to 3;
Aryl is 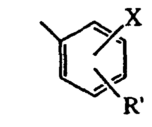 and
Heteroaryl is 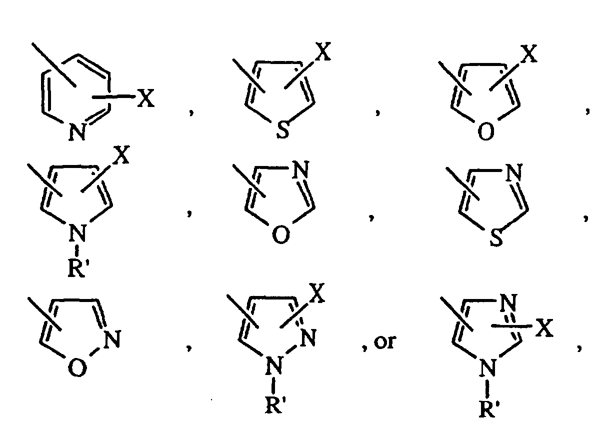 wherein X is hydrogen, halogen, (C₁-C₃) alkyl or-OCH₃ and R and R' are as defined above; and pharmaceutically acceptable salts thereof.

5. A compound according to claim 1, wherein
R is hydrogen, (C₁-C₃) alkyl,-CN,-OR',-SR',-CF_{3,}-OCF₃, Cl, F, NH₂, NH(C₁-C₃)alkyl,-N(R')CO(C₁-C₃)alkyl,-N(R')(R'), NO₂, -CONH₂,-SO₂NH₂,-SO₂N(R')(R'), or-N(R')COCH₂O-(C₁-C₃)alkyl, wherein R' is (C₁-C₃) alkyl or hydrogen;
R₄ is (C₁-C₆) alkyl-O- containing one triple bond,
-O-CH₂-C ≡ C-R"
wherein R" is hydrogen, -CH₂OH, (C₁-C₆)alkyl, (C₁-C₆)alkyl-O-CH₂-, (C₁-C₆)alkyl-S-CH₂-, (C₁-C₆)alkyl-NH-CH₂-, [(C₁-C₃)alkyl]₂-NCH₂-, (C₁-C₆)cycloalkyl-O-CH₂-, [(C₁-C₃) alkyl]₂-N-(CH₂)₂₋₄NHCH₂-, [(C₁-C₃) alkyl]₂-N-(CH₂)₂₋₄N(CH₃)CH₂-, 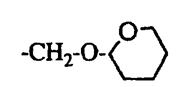 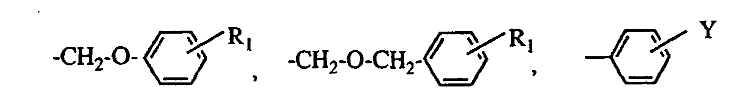 R₁ and R₂ are each, independently, hydrogen or CH₃;
R₃ is HCO-, (C₁-C₃)alkylCO-, Aryl(C₁-C₃)alkylCO-, Heteroaryl(C₁-C₃)alkylCO-, (C₁-C₃)alkyl-O-(CH₂)ₙCO-, HO(CH₂)ₘCO-, (C₁-C₇)cycloalkylCO-,
wherein
Aryl is 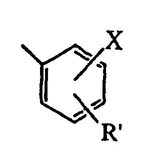 and
Heteroaryl is 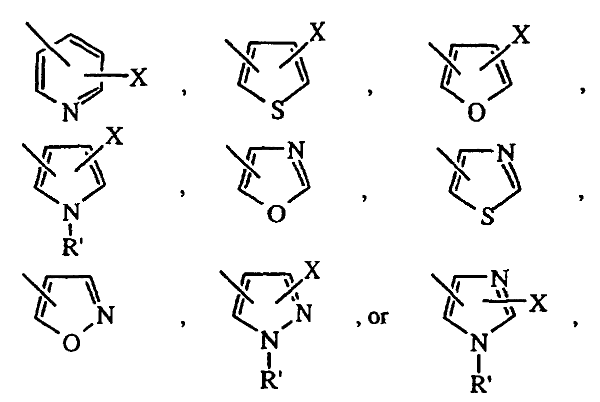 wherein X is hydrogen, halogen, (C₁-C₃) alkyl or-OCH₃ and R and R' are is as defined above;
and pharmaceutically acceptable salts thereof.

6. A compound according to claim 1, wherein
R is hydrogen, (C₁-C₃) alkyl,-CN,-OR',-SR',-CF_{3,}-OCF₃, Cl, F, NH₂, NH(C₁-C₃)alkyl,-N(R')CO(C₁-C₃)alkyl,-N(R')(R'), NO₂, -CONH₂,-SO₂NH₂,-SO₂N(R')(R'), or-N(R')COCH₂O-(C₁-C₃)alkyl, wherein R' is (C₁-C₃) alkyl or hydrogen;
R₄ is (C₁-C₆) alkyl-O- containing one triple bond,
-O-CH₂-C ≡ C-R"
wherein R" is hydrogen, -CH₂OH, (C₁-C₆)alkyl, (C₁-C₆)alkyl-O-CH₂-, (C₁-C₆)alkyl-S-CH₂-, (C₁-C₆)alkyl-NH-CH₂-, [(C₁-C₃)alkyl]₂-NCH₂-, (C₁-C₆)cycloalkyl-O-CH₂-, [(C₁-C₃)alkyl]₂-N-(CH₂)₂₋₄NHCH₂-, [(C₁-C₃) alkyl]₂-N-(CH₂)₂₋₄N(CH₃)CH₂-, 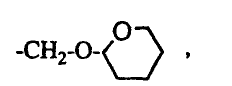 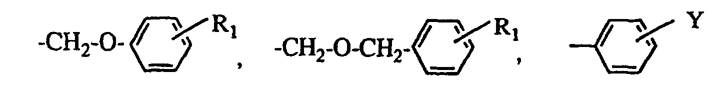 R₁ and R₂ are each, independently, hydrogen or CH₃;
R₃ is 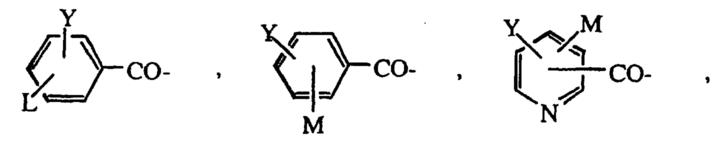 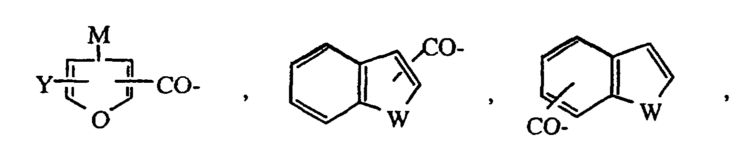 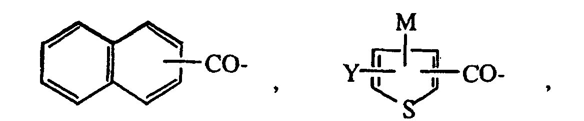 wherein
m= 1 to 3; n = 0 to 3;
L is hydrogen, (C₁-C₃)alkyl,-CN,-OR',-SR',-CF₃,-OCF₃, Cl, F, NH₂, -NH-(C₁-C₃)alkyl,-N(R')CO(C₁-C₃)alkyl, N(R')(R'),-NO₂,-CONH₂,-SO₂NH₂, -SO₂N(R')(R'),-N(R')COCH₂O-(C₁-C₃)alkyl, 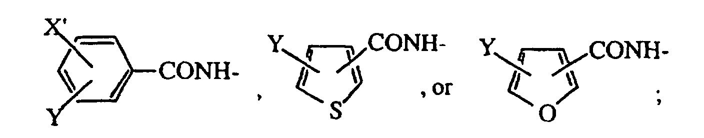 M is 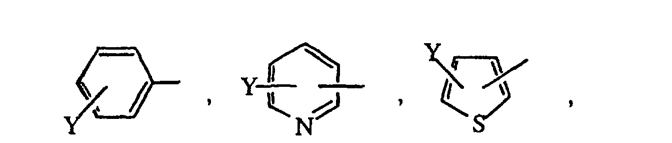 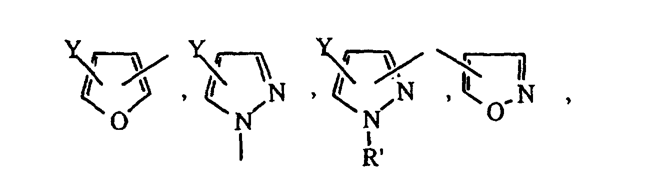 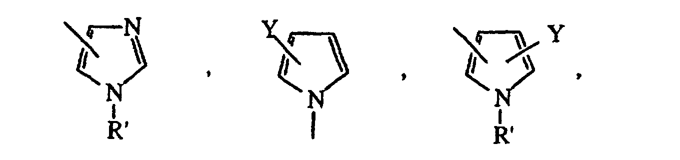 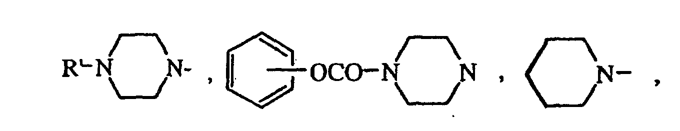 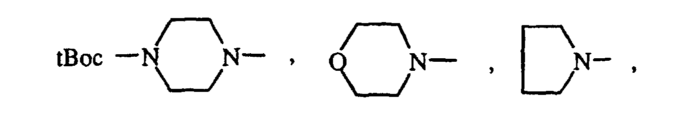 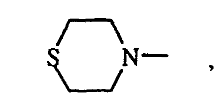 or N(R')(R') where R' is as defined above;
W is O, S, NH or N(C₁-C₃)alkyl;
Y is hydrogen, F, Cl, CF₃ or OCH₃; and X' is halogen, hydrogen, (C₁-C₃)alkyl, O-(C₁-C₃)alkyl, or-CH₂OH; and pharmaceutically acceptable salts thereof.

7. The compound according to claim 1 which is one of the following:
1-Acetyl-4-(4-but-2-ynyloxy-benzenesulfonyl)-2,3,4,5-tetrahydro-1H-[1,4]benzodiazepine-3-carboxylic acid, hydroxyamide;
4-(4-But-2-ynyloxy-benzene-sulfonyl)-1-(2-thienylcarbonyl)-2,3,4,5-tetrahydro-1H-[1,4]benzodiazepine-3-carboxylic acid, hydroxyamide;
1-Benzoyl-4-(4-but-2-ynyloxybenzenesulfonyl)-2,3,4,5-tetrahydro-1H-[1,4]benzodiazepine-3-carboxylic acid, hydroxyamide;
4-(4-But-2-ynyloxybenzene-sulfonyl)-1-(2-furanylcarbonyl)-2,3,4,5-tetrahydro-1H-[1,4]benzodiazepine-3-carboxylic acid, hydroxyamide;
4-(4-But-2-ynyloxybenzene-sulfonyl)-1-(methanesulfonyl)-2,3,4,5-tetrahydro-1H-[1,4]benzodiazepine-3-carboxylic acid, hydroxyamide;
4-(4-But-2-ynyloxybenzene-sulfonyl)-1-methoxyacetyl-2,3,4,5-tetrahydro-1H-[1,4]benzodiazepine-3-carboxylic acid, hydroxyamide;
4-(4-But-2-ynyloxybenzene-sulfonyl)-1-(3-pyridinylcarbonyl)-2,3,4,5-tetrahydro-1H-[1,4]benzodiazepine-3-carboxylic acid, hydroxyamide;
4-(4-But-2-ynyloxybenzene-sulfonyl)-1-(4-pyridinylcarbonyl)-2,3,4,5-tetrahydro-1H-[1,4]benzodiazepine-3-carboxylic acid, hydroxyamide;
1-Benzoyl-4-(4-[4-methoxybut-2-ynyloxy]benzenesulfonyl)-2,3,4,5-tetrahydro-1H-[1,4]benzodiazepine-3-carboxylic acid, hydroxyamide;
4-(4-[4-Methoxybut-2-ynyloxy]benzenesulfonyl)-1-(3-pyridinylcarbonyl)-2,3,4,5-tetrahydro-1H-[1,4]benzodiazepine-3-carboxylic acid, hydroxyamide;
4-(4-Pent-2-ynyloxy-benzene-sulfonyl)-1-(3-pyridinylcarbonyl)-2,3,4,5-tetrahydro-1H-[1,4]benzodiazepine-3-carboxylic acid, hydroxyamide;
4-(4-[4-Hydroxybut-2-ynyloxy]benzenesulfonyl)-1-(4-pyridinylcarbonyl)-2,3,4,5-tetrahydro-1H-[1,4]benzodiazepine-3-carboxylic acid, hydroxyamide;
4-(4-[4-Methoxybut-2-ynyloxy]-benzenesulfonyl)-1-(2-thienylcarbonyl)-2,3,4,5tetrahydro-1H-[1,4]benzodiazepine-3-carboxylic acid, hydroxyamide;
1-(Benzoyl)-4-(4-pent-2-ynyloxy-benzenesulfonyl)-2,3,4,5-tetrahydro-1H-[1,4]benzodiazepine-3-carboxylic acid, hydroxyamide;
1-Propionyl-4-(4-[4-hydroxybut-2-ynyloxy]benzenesulfonyl)-2,3,4,5-tetrahydro-1H-[1,4]benzodiazepine-3-carboxylic acid, hydroxyamide;
1-(N,N-Dimethylaminoacetyl)-4-(4-[4-methoxybut-2-ynyloxy]benzenesulfonyl)-2,3,4,5-tetrahydro-1H-[1,4]benzodiazepine-3-carboxylic acid, hydroxyamide;
1-(Acetylaminoacetyl)-4-(4-but-2-ynyloxybenzenesulfonyl)-2,3,4,5-tetrahydro-1H-[1,4]benzodiazepine-3-carboxylic acid, hydroxyamide;
1-(Ethoxyacetyl)-4-(4-[4-methoxybut-2-ynyloxy]benzenesulfonyl)-2,3,4,5-tetrahydro-1H-[1,4]benzodiazepine-3-carboxylic acid, hydroxyamide;
4-(4-But-2-ynyloxybenzenesulfonyl)-1-(3-thienylcarbonyl)-2,3,4,5-tetrahydro-1H-[1,4]benzodiazepine-3-carboxylic acid, hydroxyamide;
1-(Ethoxyacetyl)-4-(4-[4-ethoxybut-2-ynyloxy]benzenesulfonyl)-2,3,4,5-tetrahydro-1 H-[ 1,4]benzodiazepine-3-carboxylic acid, hydroxyamide;
1-(Acetylaminoacetyl)-4-(4-[4-methoxybut-2-ynyloxy]benzenesulfonyl)-2,3,4,5-tetrahydro-1H-[1,4]benzodiazepine-3-carboxylic acid, hydroxyamide;
1-(Cyclopropylcarbonyl)-4-(4-[4-methxybut-2-ynyloxy]benzenesulfonyl )-2,3,4,5-tetrahydro-1H-[1,4]benzodiazepine-3-carboxylic acid, hydroxyamide;
1-(Cyclobutylcarbonyl)-4-(4-but-2-ynyloxybenzeneulfonyl)-2,3,4,5-tetrahydro-1H-[1,4]benzodiazepine-3-carboxylic acid, hydroxyamide;
4-(4-But-2-ynyloxybenzene-sulfonyl)-1-(propionyl)-2,3,4,5-tetrahydro-1H-[1,4]benzodiazepine-3-carboxylic acid, hydroxyamide;
4-(4-[4-Methoxybut-2-ynyloxy]benzenesulfonyl)-1-(3-methyl-2-thienylcarbonyl)-2,3,4,5-tetrahydro-1H-[1,4]benzodiazepine-3-carboxylic acid, hydroxyamide;
4-(4-But-2-ynyloxybenzene-sulfonyl)-1-(3-methoxypropionyl)-2,3,4,5-tetrahydro-1H-[1,4]benzodiazepine-3-carboxyhc acid, hydroxyamide;
4-(4-But-2-ynyloxybenzene-sulfonyl)-1-(2-chlorobenzoyl)-2,3,4,5-tetrahydro-1H-[1,4]benzodiazepine-3-carboxylic acid, hydroxyamide;
4-(4-But-2-ynyloxybenzene-sulfonyl)-1-(2-fluorobenzoyl)-2,3,4,5-tetrahydro-1H-[1,4]berrzodiazepine-3-carboxylic acid, hydroxyamide;
4-(4-But-2-ynyloxybenzene-sulfonyl)-1-(4-methyl-2-furanylcarbonyl)-2,3,4,5-tetrahydro-1H-[1,4]benzodiazepine-3-carboxylic acid, hydroxyamide;
4-(4-But-2-ynyloxybenzene-sulfonyl)-1-(3-furanycarbonyl)-2,3,4,5-tetrahydro-1H-[1,4]benzodiazepine-3-carboxylic acid, hydroxyamide;
4-(4-But-2-ynyloxybenzene-sulfonyl)-1-(phenoxyacetyl)-2,3,4,5-tetrahydro-1H-[1,4]benzodiazepine-3-carboxylic acid, hydroxyamide;
4-(4-But-2-ynyloxybenzene-sulfonyl)-1-[2-(1-pyrazolyl)phenylcarbonyl]-7-methyl-2,3,4,5-tetrahydro-1H-[1,4]-benzodiazepene-3-carboxylic acid, hydroxyamide;
4-(4-But-2-ynyloxybenzene-sulfonyl)-1-(5-chloro-2-thienylcarbonyl)-2,3,4,5-tetrahydro-1H-[1,4]benzodiazepine-3-carboxylic acid, hydroxyamide;
4-(4-But-2-ynyloxybenzene-sulfonyl)-1-(5-chloro-2-furanylcarbonyl)-2,3,4,5-tetrahydro-1H-[1,4]benzodiazepine-3-carboxylic acid, hydroxyamide;
4-(4-[4-Methoxybut-2-ynyloxy]-benzenesulfonyl)-1-propionyl-2,3,4,5-tetrahydro-1H-[1,4]benzodiazepine-3-carboxylic acid, hydroxyamide;
4-(4-[4-Methoxybut-2-ynyloxy]benzenesulfonyl)-1-(3-thienylcarbonyl)-2,3,4,5-tetrahydro-1H-[1,4]benzodiazepine-3-carboxylic acid, hydroxyamide;
1-(Aminoacetyl)-4-(4-but-2-ynyloxybenzenesulfonyl)-2,3,4,5-tetrahydro-1H-[1,4]benzodiazepine-3-carboxylic acid, hydroxyamide;
1-Hexanoyl-4-(4-[4-methoxybut-2-ynyloxy]benzenesulfonyl)-2,3,4,5-tetrahydro-1H-[1,4]benzodiazepine-3-carboxylic acid, hydroxyamide;
4-(4-But-2-ynyloxy-benzenesulfonyl)-1-(N,N-Dimethylaminoacetyl)-2,3,4,5-tetrahydro-1H-[1,4]benzodiazepine-3-carboxylic acid, hydroxyamide;
4-(4-But-2-ynyloxybenzene-sulfonyl)-1-(cyclopropylcarbonyl)-2,3,4,5-tetrahydro-1H-[1,4]benzodiazepine-3-carboxylic acid, hydroxyamide;
4-(4-But-2-ynyloxybenzenesulfonyl)-1-(cycloyhexylcarbonyl)-2,3,4,5-tetrahydro-1H-[1,4]-benzodiazepine-3-carboxylic acid, hydroxyamide;
1-Methoxyacetyl-4-(4-[4-methoxybut-2-ynyloxy]benzenesulfonyl)-7-methyl-2,3,4,5-tetrahydro-1H-[1,4]-benzodiazepine-3-carboxylic acid, hydroxyamide;
1-Benzoyl-4-(4-but-2-ynyloxybenzenesulfonyl)-7-methyl-2,3,4,5-tetrahydro-1H-[1,4]-benzodiazepine-3-carboxylic acid, hydroxyamide;
1-(Benzoyl)-4-(4-but-2-ynyloxybenzenesulfonyl)-8-chloro-2,3,4,5-tetrahydro-1H-[1,4]benzodiazepine-3-carboxylic acid, hydroxyamide; or
1-Acetyl-4-{[4-(2-butynyloxy)phenyl]sulfonyl}-7-fluoro-N-hydroxy-2,3,4,5-tetrahydro-1 H-[ 1,4]-benzodiazepine-3-carboxamide.

8. A process for preparing a compound as claimed in claim 1 which comprises one of the following:
a) reacting a compound of formula II: 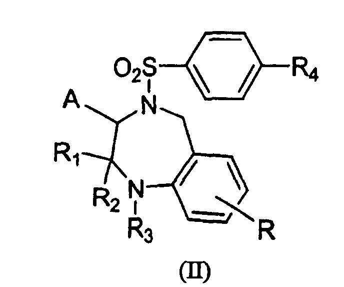 wherein R, R₁, R₂, R₃, and R₄ are defined above, and A is COOH or a reactive derivative thereof, with a compound of formula III
NH₂OH (III)
to give a corresponding compound of formula I;
b) resolving a mixture (e.g. racemate) of optically active isomers of a compound of formula I to isolate one enantiomer or diastereomer substantially free of the other enantiomer or diastereomers;
c) acidifying a basic compound of formula I with a pharmaceutically acceptable acid to give a pharmaceutically acceptable salt.

9. A pharmaceutical composition comprising a compound of Formula 1 as defined in Claim 1 or a pharmaceutically acceptable salt thereof and a pharmaceutically acceptable carrier.

10. Use of a compound of Formula 1 as claimed in Claim 1 in the preparation of a medicament for treating a disease condition mediated by matrix metalloproteinase in a mammal, said disease condition being selected from the group consisting of tumor growth, restenosis, MMP-mediated osteopenias, inflammatory diseases of the central nervous system, skin aging, osteoarthritis, rheumatoid arthritis, septic arthritis, corneal ulceration, abnormal wound healing, bone disease, proteinuria, aneurysmal aortic disease, degenerative cartilage loss, demyelinating diseases of the nervous system, cirrhosis of the liver, glomerular disease of the kidney, premature rupture of fetal membranes, inflammatory bowel disease, periodontal disease, age-related macular degeneration, diabetic retinopathy, proliferative vitreoretinopathy, retinopathy of prematurity, ocular inflammation, keratoconus, Sjogren's syndrome, myopia, ocular tumors, ocular angiogenesis/neo-vascularization and corneal graft rejection.

11. Use of a compound of formula I as claimed in Claim 1 in the preparation of a medicament for treating a patient suffering from a condition selected from the group consisting of osteoarthritis, rheumatoid arthritis, degenerative cartilage loss, and tumor growth.

## Patentansprüche

1. Verbindung der Formel 1: 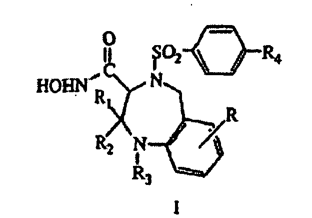 worin:
R ausgewählt wird aus Wasserstoff, (C₁-C₃)Alkyl,-CN,-OR', -SR',-CF₃,-OCF₃, Cl, F, NH₂, NH(C₁-C₃)Alkyl,-N(R')CO(C₁-C₃)Alkyl,-N(R') (R'), NO₂,-CONH₂,-SO₂NH₂,-SO₂N(R')(R'), -N(R')COCH₂O-(C₁-C₃) Alkyl, worin R' für (C₁-C₃) Alkyl oder Wasserstoff steht;
R₄ für (C₁-C₆)Alkyl-O-, welches eine Dreifachbindung enthält, 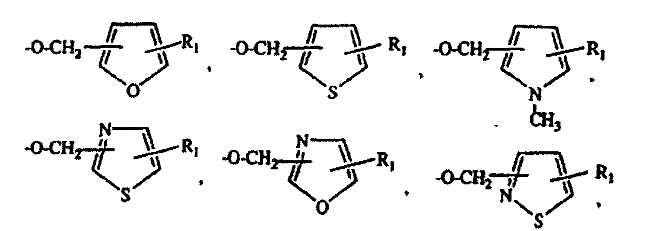 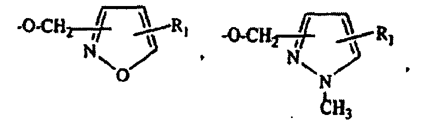 -O-CH₂-C ≡ C-R " steht, worin
R" für Wasserstoff,-CH₂OH, (C₁-C₆) Alkyl, (C₁-C₆) Alkyl-O-CH₂-, (C₁-C₆) Alkyl-S-CH₂-, (C₁-C₆) Alkyl-NH-CH₂-, [(C₁-C₃) Alkyl]₂-NCH₂-, (C₁-C₆) Cycloalkyl-O-CH₂-, [(C₁-C₃) Alkyl]₂-N-(CH₂)₂₋₄NHCH₂-, [(C₁-C₃) Alkyl]₂-N-(CH₂) ₂₋₄N (CH₃) CH₂-, 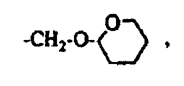 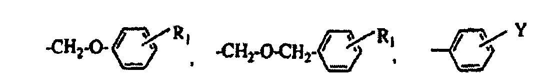 steht,
R₁ und R₂ jeweils unabhängig Wasserstoff oder CH₃ darstellen;
R₃ für (C₁-C₈)Alkyl, NH₂CH₂CO-, (C₁-C₆)AlkylNHCH₂CO-, HO (CH₂)ₘCO-, HCO-, Aryl(CH₂)ₙCO-, Heteroaryl(CH₂)ₙCO-, (C₁-C₃)Alkyl-O-(CH₂)ₙCO-, (C₁-C₃)AlkylCO-, (C₁-C₃)AlkylCO-NHCH₂CO-, (C₃-C₇) CycloalkylCO-, (C₁-C₃)AlkylSO₂-, Aryl (CH₂)ₙSO₂-, Heteroaryl (CH₂)ₙSO₂-, (C₁-C₃)Alkyl-O-(CH₂)ₘ-SO₂-, (C₁-C₃)Alkyl-O-(CH₂) ₘ, (C₁-C₃) Alkyl-O-(C₁-C₃) alkyl-O-(C₁-C₃) alkyl, HO-(C₁-C₃) Alkyl-O-(C₁-C₃)alkyl, Aryl-O-CH₂CO-, Heteroaryl-O-CH₂CO-, ArylCH=CHCO-, HeteroarylCH=CHCO-, (C₁-C₃)AlkylCH=CHCO-, 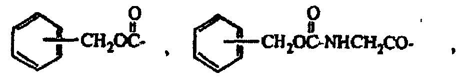 Aryl (C₁-C₃) alkyl, Heteroaryl (C₁-C₃) alkyl, Aryl CH=CHCH₂-, HeteroarylCH=CHCH₂-, (C₁-C₆)AlkylCH=CHCH₂-, 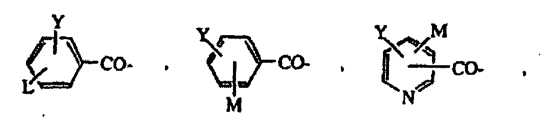 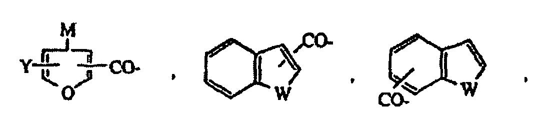 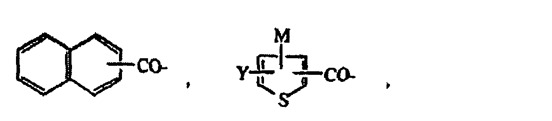 R'OCH₂ CH(OR')CO-, (R'OCH₂)₂C(R')CO-, 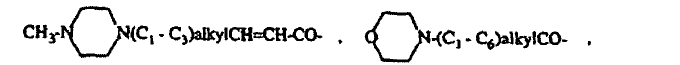 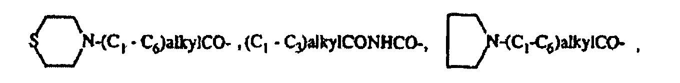 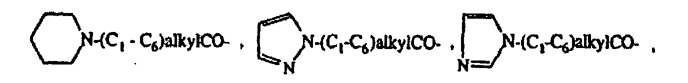 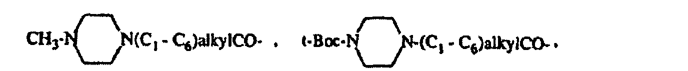 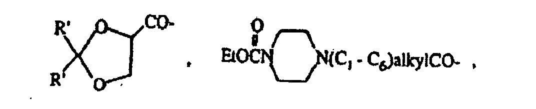 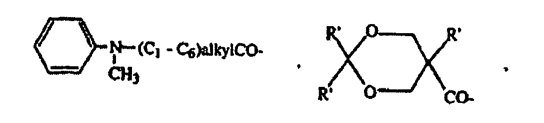 [(C₁-C₆)Alkyl]₂-N-(C₁-C₆)alkylCO- oder (C₁-C₆)Alkyl-NH-(C₁-C₆) alkylCO- steht; worin
m = 1 bis 3; n = 0 bis 3
Aryl für 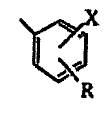 steht und
Heteroaryl für 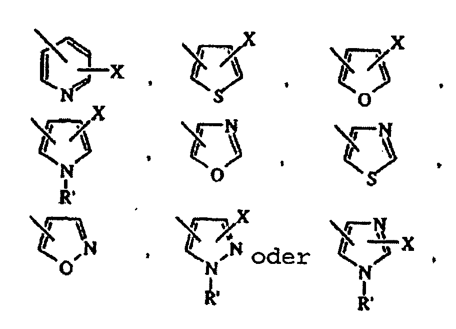 steht, worin X für Wasserstoff, Halogen, (C₁-C₃)Alkyl oder-OCH₃ steht und R und R' wie oben definiert sind;
L für Wasserstoff, (C₁-C₃)Alkyl,-CN,-OR',-SR',-CF₃, -OCF₃, Cl, F, NH₂,-NH-(C₁-C₃)Alkyl,-N(R')CO(C₁-C₃)Alkyl, N(R')(R'),-NO₂,-CONH₂, -SO₂NH₂,-SO₂N(R') (R'),-N(R')COCH₂O-(C₁-C₃)Alkyl, 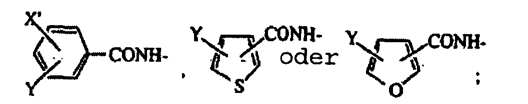 steht;
M für 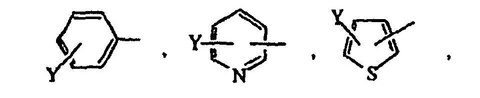 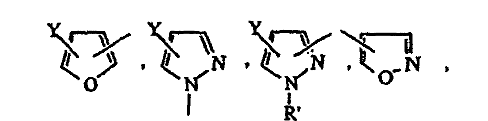 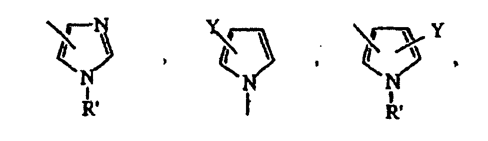 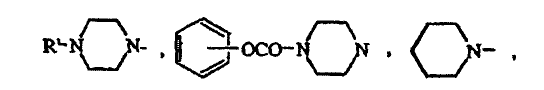 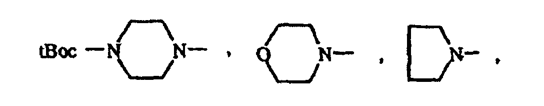 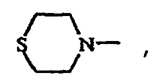 oder N(R')(R') steht, worin R' wie oben definiert ist;
W für O, S, NH oder N(C₁-C₃)Alkyl steht;
Y für Wasserstoff, F, Cl, CF₃ oder OCH₃ steht; und X' für Halogen, Wasserstoff, (C₁-C₃)Alkyl, O-(C₁-C₃)Alkyl oder -CH₂OH steht; und pharmazeutisch annehmbare Salze davon.

2. Verbindung gemäß Anspruch 1, worin:
R für Wasserstoff, (C₁-C₃)Alkyl,-CN,-OR',-SR',-CF₃, -OCF₃, Cl, F, NH₂, NH(C₁-C₃)Alkyl,-N(R')CO(C₁-C₃)Alkyl, -N(R')(R'), NO₂,-CONH₂,-SO₂NH₂,-SO₂N(R')(R') oder-N(R')COCH₂O-(C₁-C₃)Alkyl steht, worin R' für (C₁-C₃)Alkyl oder Wasserstoff steht;
R₄ für (C₁-C₆)Alkyl-O-, welches eine Dreifachbindung enthält, 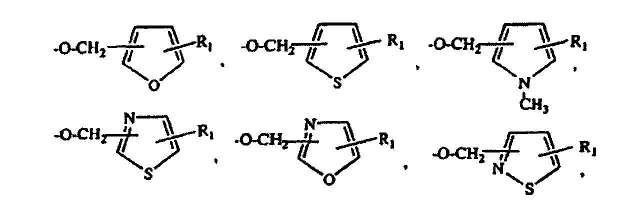 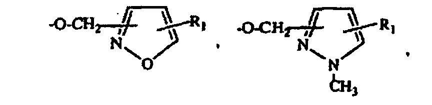 -O-CH₂-C ≡ C-R" steht, worin
R" für Wasserstoff,-CH₂OH, (C₁-C₆)Alkyl, (C₁-C₆)Alkyl-O-CH₂-, (C₁-C₆)Alkyl-S-CH₂-, (C₁-C₆)Alkyl-NH-CH₂-, [(C₁-C₃)Alkyl]₂-NCH₂-, (C₁-C₆) Cycloalkyl-O-CH₂-, [(C₁-C₃)Alkyl]₂-N-(CH₂)₂₋₄NHCH₂-, [ (C₁-C₃)Alkyl] ₂-N-(CH₂)₂₋₄N(CH₃) CH₂-, 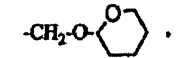 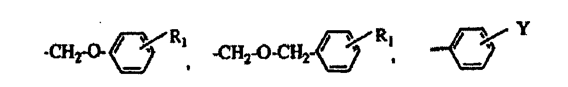 steht,
R₁ und R₂ jeweils unabhängig Wasserstoff oder CH₃ darstellen;
R₃ für (C₁-C₈)Alkyl, NH₂CH₂CO-, (C₁-C₆)AlkylNHCH₂CO-, HO (CH₂)ₘCO-, HCO-, Aryl (CH₂) ₙCO-, Heteroaryl (CH₂) ₙCO-, (C₁-C₃) Alkyl-O-(CH₂) ₙCO-, (C₁-C₃) AlkylCO-, (C₁-C₃) AlkylCO-NHCH₂CO-, (C₃-C₇)CycloalkylCO-, Aryl-O-CH₂CO-, Heteroaryl-O-CH₂CO-, 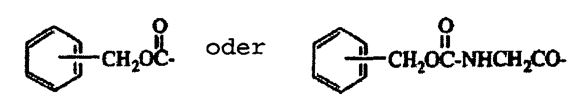 steht, worin
m = 1 bis 3; n = 0 bis 3
Aryl für 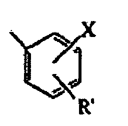 steht und
Heteroaryl für 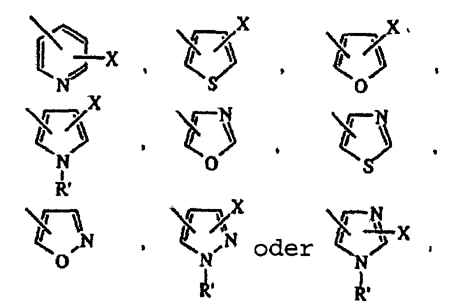 steht, worin X für Wasserstoff, Halogen, (C₁-C₃)Alkyl oder -OCH₃ steht und R und R' wie oben definiert sind; und pharmazeutisch annehmbare Salze davon.

3. Verbindung gemäß Anspruch 1, worin
R für Wasserstoff, (C₁-C₃)Alkyl,-CN,-OR',-SR',-CF₃, -OCF₃, Cl, F, NH₂, NH (C₁-C₃) Alkyl,-N (R') CO (C₁-C₃) Alkyl, -N (R') (R'), NO₂,-CONH₂,-SO₂NH₂,-SO₂N (R') (R'),-N (R') COCH₂O-(C₁-C₃) Alkyl steht, worin R' für (C₁-C₃) Alkyl oder Wasserstoff steht;
R₄ für (C₁-C₆)Alkyl-O-, welches eine Dreifachbindung enthält, 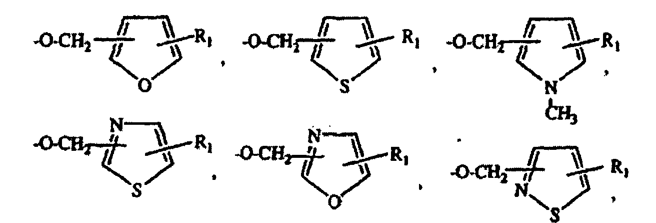 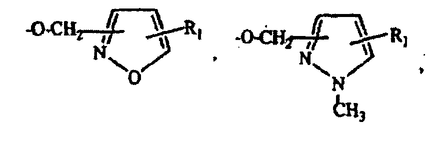 -O-CH₂-C ≡ C-R" steht, worin
R" für Wasserstoff,-CH₂OH, (C₁-C₆)Alkyl, (C₁-C₆)Alkyl-O-CH₂-, (C₁-C₆) Alkyl-S-CH₂-, (C₁-C₆)Alkyl-NH-CH₂-, [(C₁-C₃) Alkyl] ₂-NCH₂-, (C₁-C₆) Cycloalkyl-O-CH₂-, [ (C₁-C₃) Alkyl] ₂-N-(CH₂) ₂₋₄NHCH₂-, [(C₁-C₃)Alkyl]₂-N-(CH₂)₂₋₄N(CH₃)CH₂-, 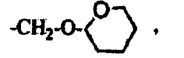 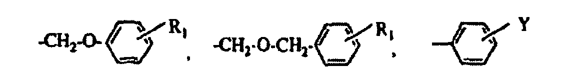 steht,
R₁ und R₂ jeweils unabhängig Wasserstoff oder CH₃ darstellen;
R₃ für 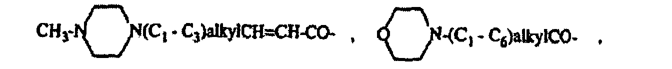 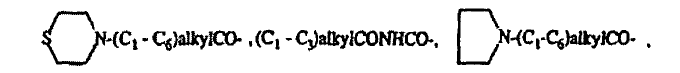 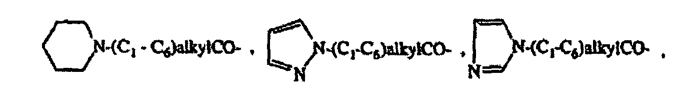 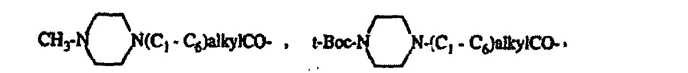 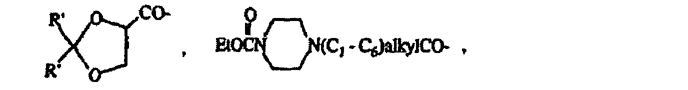 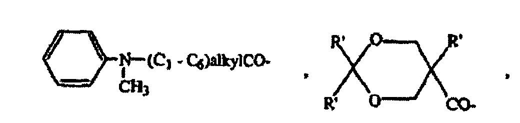 [(C₁-C₆)Alkyl]₂-N-(C₁-C₆)alkylCO- oder (C₁-C₆)Alkyl-NH-(C₁-C₆)AlkylCO- steht, worin R' wie oben definiert ist; und pharmazeutisch annehmbare Salze davon.

4. Verbindung gemäß Anspruch 1, worin
R für Wasserstoff, (C₁-C₃)Alkyl,-CN,-OR',-SR',-CF₃, -OCF₃, Cl, F, NH₂, NH(C₁-C₃)Alkyl,-N(R')CO(C₁-C₃)Alkyl, -N(R')(R'), NO₂,-CONH₂,-SO₂NH₂,-SO₂N(R')(R'),-N(R')COCH₂O-(C₁-C₃)Alkyl steht, worin R' für (C₁-C₃)Alkyl oder Wasserstoff steht;
R₄ für (C₁-C₆)Alkyl-O-, welches eine Dreifachbindung enthält 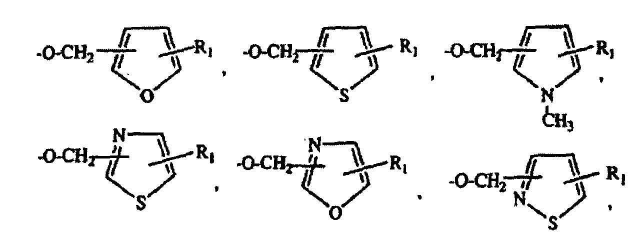 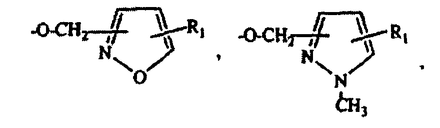 -O-CH₂-C ≡ C-R" steht, worin
R" für wasserstoff,-CH₂OH, (C₁-C₆)Alkyl, (C₁-C₆)Alkyl-O-CH₂-, (C₁-C₆)Alkyl-S-CH₂-, (C₁-C₆)Alkyl-NH-CH₂-, [(C₁-C₃)Alkyl]₂-NCH₂-, (C₁-C₆)Cycloalkyl-O-CH₂-, [(C₁-C₃) Alkyl]₂-N-(CH₂)₂₋₄NHCH₂-, [(C₁-C₃)Alkyl]₂-N-(CH₂)₂₋₄N(CH₃)CH₂-, 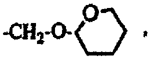 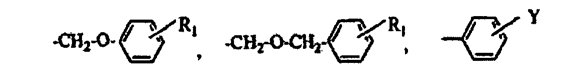 steht,
R₁ und R₂ jeweils unabhängig Wasserstoff oder CH₃ darstellen;
R₃ für (C₁-C₃)AlkylSO₂-, Aryl(CH₂)ₙSO₂-, Heteroaryl(CH₂)ₙSO₂oder (C₁-C₃) Alkyl-O-(CH₂)ₘ-SO₂ steht, worin
m = 1 bis 3; n = 0 bis 3;
Aryl für 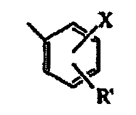 steht und
Heteroaryl für 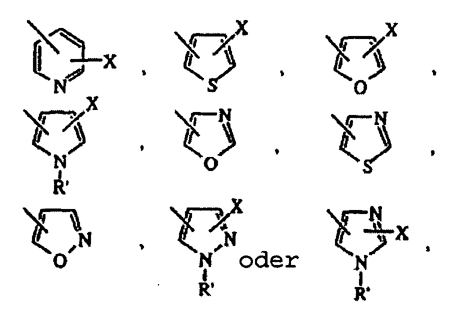 steht, worin X für Wasserstoff, Halogen, (C₁-C₃)Alkyl oder-OCH₃ steht und R und R' wie oben definiert sind; und pharmazeutisch annehmbare Salze davon.

5. Verbindung gemäß Anspruch 1, worin
R für Wasserstoff, (C₁-C₃)Alkyl,-CN,-OR',-SR',-CF₃, -OCF₃, Cl, F, NH₂, NH(C₁-C₃)Alkyl,-N(R') CO (C₁-C₃) Alkyl, -N (R') (R'), NO₂,-CONH₂,-SO₂NH₂,-SO₂N (R') (R') oder -N (R') COCH₂O-(C₁-C₃)Alkyl steht, worin R' für (C₁-C₃)Alkyl oder Wasserstoff steht;
R₄ für (C₁-C₆)Alkyl-O-, welches eine Dreifachbindung enthält,
-O-CH₂-C≡C-R" steht, worin
R" für Wasserstoff,-CH₂OH, (C₁-C₆)Alkyl, (C₁-C₆)Alkyl-O-CH₂-, (C₁-C₆)Alkyl-S-CH₂-, (C₁-C₆)Alkyl-NH-CH₂-, [(C₁-C₃)Alkyl]₂-NCH₂-, (C₁-C₆)Cycloalkyl-O-CH₂-, [(C₁-C₃)Alkyl]₂-N-(CH₂) ₂₋₄NHCH₂-, [(C₁-C₃)Alkyl]₂-N-(CH₂)₂₋₄N(CH₃)CH₂-, 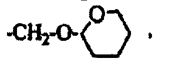 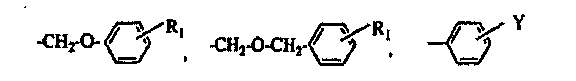 steht,
R₁ und R₂ jeweils unabhängig Wasserstoff oder CH₃ darstellen;
R₃ für HCO-, (C₁-C₃)AlkylCO-, Aryl (C₁-C₃) alkylCO-, Heteroaryl (C₁-C₃) alkylCO-, (C₁-C₃) Alkyl-O-(CH₂)ₙCO-, HO(CH₂)ₘCO-, (C₁-C₇) CycloalkylCO-steht, worin
Aryl für 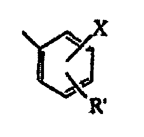 steht und
Heteroaryl für 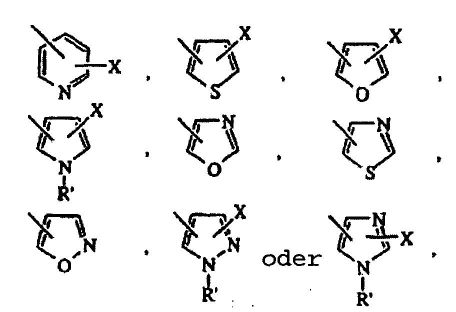 steht, worin X für Wasserstoff, Halogen, (C₁-C₃)Alkyl oder -OCH₃ steht und R und R' wie oben definiert sind; und pharmazeutisch annehmbare Salze davon.

6. Verbindung gemäß Anspruch 1, worin:
R für Wasserstoff, (C₁-C₃)Alkyl,-CN,-OR', -SR',-CF₃, -OCF₃, Cl, F, NH₂, NH(C₁-C₃)Alkyl,-N(R')CO(C₁-C₃)Alkyl, -N(R')(R'), NO₂,-CONH₂,-SO₂NH₂,-SO₂N(R')(R') oder -N (R') COCH₂O-(C₁-C₃)Alkyl steht, worin R' für (C₁-C₃)Alkyl oder Wasserstoff steht;
R₄ für (C₁-C₆)Alkyl-O-, welches eine Dreifachbindung enthält,
-O-CH₂-C≡C-R" steht, worin
R" für Wasserstoff,-CH₂OH, (C₁-C₆)Alkyl, (C₁-C₆)Alkyl-O-CH₂-, (C₁-C₆)Alkyl-S-CH₂-, (C₁-C₆)Alkyl-NH-CH₂-, [(C₁-C₃)Alkyl]₂-NCH₂-, (C₁-C₆) Cycloalkyl-O-CH₂-, [(C₁-C₃) Alkyl] ₂-N-(CH₂) ₂₋₄NHCH₂-, [(C₁-C₃) Alkyl]₂-N-(CH₂) ₂₋₄N (CH₃) CH₂-, 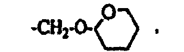 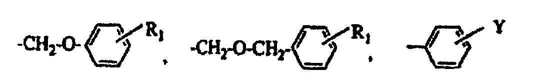 steht,
R₁ und R₂ jeweils unabhängig Wasserstoff oder CH₃ darstellen;
R₃ für 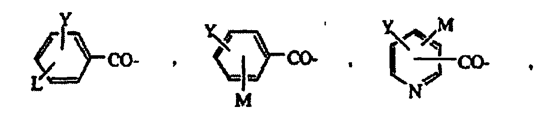 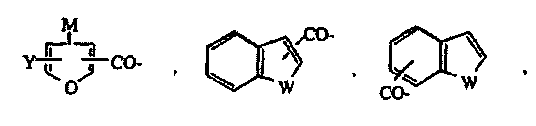 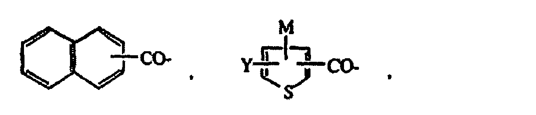 steht, worin
m = 1 bis 3; n = 0 bis 3;
L für Wasserstoff, (C₁-C₃)Alkyl,-CN,-OR',-SR',-CF₃, -OCF₃, Cl, F, NH₂, -NH-(C₁-C₃)Alkyl, -N(R')CO(C₁-C₃)Alkyl, N(R') (R'), -NO₂, -CONH₂, -SO₂NH₂, -SO₂N (R') (R'), -N(R') COCH₂O-(C₁-C₃) Alkyl, 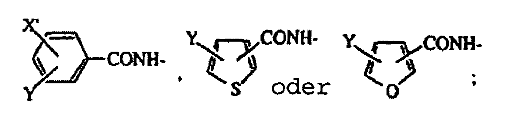 steht;
M für 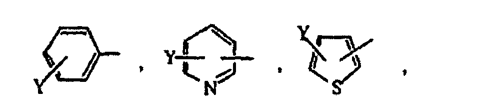 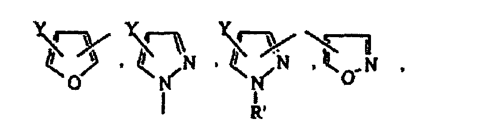 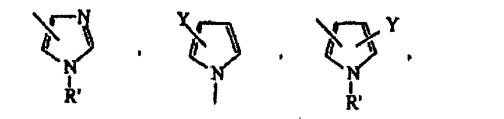 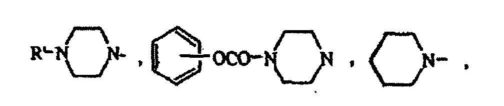 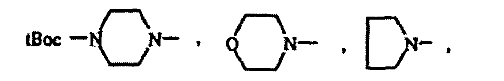 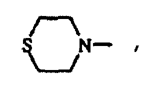 oder N(R')(R') steht, worin R' wie oben definiert ist
W für O, S, NH oder N(C₁-C₃)Alkyl steht;
Y für Wasserstoff, F, Cl, CF₃ oder OCH₃ steht; und X' für Halogen, Wasserstoff, (C₁-C₃)Alkyl, O-(C₁-C₃)Alkyl oder-CH₂OH steht; und pharmazeutisch annehmbare Salze davon.

7. Verbindung gemäß Anspruch 1, welche eine der Folgenden ist:
1-Acetyl-4-(4-but-2-inyloxy-benzolsulfonyl)-2,3,4,5-tetrahydro-1H-[1,4]benzodiazepin-3-carbonsäure, Hydroxyamid;
4-(4-But-2-inyloxy-benzol-sulfonyl)-1-(2-thienylcarbonyl)-2,3,4,5-tetrahydro-1H-[1,4]benzodiazepin-3-carbonsäure, Hydroxyamid;
1-Benzoyl-4-(4-but-2-inyloxybenzolsulfonyl)-2,3,4,5-tetrahydro-1H-[1,4]benzodiazepin-3-carbonsäure, Hydroxyamid;
4-(4-But-2-inyloxybenzol-sulfonyl)-1-(2-furanylcarbonyl)-2,3,4,5-tetrahydro-1H-[1,4]benzodiazepin-3-carbonsäure, Hydroxyamid;
4-(4-But-2-inyloxybenzol-sulfonyl)-1-(methansulfonyl)-2,3,4,5-tetrahydro-1H-[1,4]benzodiazepin-3-carbonsäure, Hydroxyamid;
4-(4-But-2-inyloxybenzol-sulfonyl)-1-methoxyacetyl-2,3,4,5-tetrahydro-1H-[1,4]benzodiazepin-3-carbonsäure, Hydroxyamid;
4-(4-But-2-inyloxybenzol-sulfonyl)-1-(3-pyridinylcarbonyl)-2,3,4,5-tetrahydro-1H-[1,4]benzodiazepin-3-carbonsäure, Hydroxyamid;
4-(4-But-2-inyloxybenzol-sulfonyl)-1-(4-pyridinylcarbonyl)-2,3,4,5-tetrahydro-1H-[1,4]benzodiazepin-3-carbonsäure, Hydroxyamid;
1-Benzoyl-4-(4-[4-methoxybut-2-inyloxy]benzolsulfonyl)-2,3,4,5-tetrahydro-1H-[1,4]benzodiazepin-3-carbonsäure, Hydroxyamid;
4-(4-[4-Methoxybut-2-inyloxy]benzolsulfonyl)-1-(3-pyridinylcarbonyl)-2,3,4,5-tetrahydro-1H-[1,4]benzodiazepin-3-carbonsäure, Hydroxyamid;
4-(4-Pent-2-inyloxy-benzol-sulfonyl)-1-(3-pyridinylcarbonyl)-2,3,4,5-tetrahydro-1H-[1,4]benzodiazepin-3-carbonsäure, Hydroxyamid;
4-(4-[4-Hydroxybut-2-inyloxy]benzolsulfonyl)-1-(4-pyridinylcarbonyl)-2,3,4,5-tetrahydro-1H-[l,4]benzodiazepin-3-carbonsäure, Hydroxyamid;
4-(4-[4-Methoxybut-2-inyloxy]-benzolsulfonyl)-1-(2-thienylcarbonyl)-2,3,4,5-tetrahydro-1H-[1,4]benzodiazepin-3-carbonsäure, Hydroxyamid;
1-(Benzoyl)-4-(4-pent-2-inyloxy-benzolsulfonyl)-2,3,4,5-tetrahydro-1H-[1,4]benzodiazepin-3-carbonsäure, Hydroxyamid;
1-Propionyl-4-(4-[4-hydroxybut-2-inyloxy]benzolsulfonyl)-2,3,4,5-tetrahydro-1H-[1,4]benzodiazepin-3-carbonsäure, Hydroxyamid;
1-(N,N-Dimethylaminoacetyl)-4-(4-[4-methoxybut-2-inyloxy]-benzolsulfonyl)-2,3,4,5-tetrahydro-1H-[1,4]benzodiazepin-3-carbonsäure, Hydroxyamid;
1-(Acetylaminoacetyl)-4-(4-but-2-inyloxybenzolsulfonyl)-2,3,4,5-tetrahydro-1H-[1,4]benzodiazepin-3-carbonsäure, Hydroxyamid;
1-(Ethoxyacetyl)-4-(4-[4-methoxybut-2-inyloxy]benzolsulfonyl)-2,3,4,5-tetrahydro-1H-[1,4]benzodiazepin-3-carbonsäure, Hydroxyamid;
4-(4-But-2-inyloxybenzolsulfonyl)-1-(3-thienylcarbonyl)-2,3,4,5-tetrahydro-1H-[1,4]benzodiazepin-3-carbonsäure, Hydroxyamid;
1-(Ethoxyacetyl)-4-(4-[4-ethoxybut-2-inyloxy] benzolsulfonyl)-2,3,4,5-tetrahydro-1H-[1,4]benzodiazepin-3-carbonsäure, Hydroxyamid;
1-(Acetylaminoacetyl)-4-(4-[4-methoxybut-2-inyloxy]benzolsulfonyl)-2,3,4,5-tetrahydro-1H-[1,4]benzodiazepin-3-carbonsäure, Hydroxyamid;
1-(Cyclopropylcarbonyl)-4-(4-[4-methoxybut-2-inyloxy]benzolsulfonyl)-2,3,4,5-tetrahydro-1H-[1,4]benzodiazepin-3-carbonsäure, Hydroxyamid;
1-(Cyclobutylcarbonyl)-4-(4-but-2-inyloxybenzolulfonyl)-2,3,4,5-tetrahydro-1H-[1,4]benzodiazepin-3-carbonsäure, Hydroxyamid;
4-(4-But-2-inyloxybenzol-sulfonyl)-1-(propionyl)-2,3,4,5-tetrahydro-1H-[1,4]benzodiazepin-3-carbonsäure, Hydroxyamid;
4-(4-[4-Methoxybut-2-inyloxy]benzolsulfonyl)-1-(3-methyl-2-thienylcarbonyl)-2,3,4,5-tetrahydro-1H-[1,4]benzodiazepin-3-carbonsäure, Hydroxyamid;
4-(4-But-2-inyloxybenzol-sulfonyl)-1-(3-methoxypropionyl)-2,3,4,5-tetrahydro-1H-[1,4]benzodiazepin-3-carbonsäure, Hydroxyamid; .
4-(4-But-2-inyloxybenzol-sulfonyl)-1-(2-chlorbenzoyl)-2,3,4,5-tetrahydro-1H-[1,4]benzodiazepin-3-carbonsäure, Hydroxyamid;
4-(4-But-2-inyloxybenzol-sulfonyl)-1-(2-fluorbenzoyl)-2,3,4,5-tetrahydro-1H-[1,4]benzodiazepin-3-carbonsäure, Hydroxyamid;
4-(4-But-2-inyloxybenzol-sulfonyl)-1-(4-methyl-2-furanylcarbonyl)-2,3,4,5-tetrahydro-1H-[1,4]benzodiazepin-3-carbonsäure, Hydroxyamid;
4-(4-But-2-inyloxybenzol-sulfonyl)-1-(3-furanylcarbonyl)-2,3,4,5-tetrahydro-1H-[1,4]benzodiazepin-3-carbonsäure, Hydroxyamid;
4-(4-But-2-inyloxybenzol-sulfonyl)-1-(phenoxyacetyl)-2,3,4,5-tetrahydro-1H-[1,4]benzodiazepin-3-carbonsäure, Hydroxyamid;
4-(4-But-2-inyloxybenzol-sulfonyl)-1-[2-(1-pyrazolyl)phenylcarbonyl]-7-methyl-2,3,4,5-tetrahydro-1H-[1,4]-benzodiazepin-3-carbonsäure, Hydroxyamid;
4-(4-But-2-inyloxybenzol-sulfonyl)-1-(5-chlor-2-thienylcarbonyl)-2,3,4,5-tetrahydro-1H-[1,4]benzodiazepin-3-carbonsäure, Hydroxyamid;
4-(4-But-2-inyloxybenzol-sulfonyl)-1-(5-chlor-2-furanylcarbonyl)-2,3,4,5-tetrahydro-1H-[1,4]benzodiazepin-3-carbonsäure, Hydroxyamid;
4-(4-[4-Methoxybut-2-inyloxy]-benzolsulfonyl)-1-propionyl-2,3,4,5-tetrahydro-1H-[1,4]benzodiazepin-3-carbonsäure, Hydroxyamid;
4-(4-[4-Methoxybut-2-inyloxy]benzolsulfonyl)-1-(3-thienylcarbonyl)-2,3,4,5-tetrahydro-1H-[1,4]benzodiazepin-3-carbonsäure, Hydroxyamid;
1-(Aminoacetyl)-4-(4-but-2-inyloxybenzolsulfonyl)-2,3,4,5-tetrahydro-1H-[1,4]benzodiazepin-3-carbonsäure, Hydroxyamid;
1-Hexanoyl-4-(4-[4-methoxybut-2-inyloxy]benzolsulfonyl)-2,3,4,5-tetrahydro-1H-[1,4]benzodiazepin-3-carbonsäure, Hydroxyamid;
4-(4-But-2-inyloxy-benzolsulfonyl)-1-(N,N-dimethylaminoacetyl)-2,3,4,5-tetrahydro-1H-[1,4]benzodiazepin-3-carbonsäure, Hydroxyamid;
4-(4-But-2-inyloxybenzol-sulfonyl)-1-(cyclopropylcarbonyl)-2,3,4,5-tetrahydro-1H-[1,4]benzodiazepin-3-carbonsäure, Hydroxyamid;
4-(4-But-2-inyloxybenzolsulfonyl)-1-(cycloyhexylcarbonyl)-2,3,4,5-tetrahydro-1H-[1,4]-benzodiazepin-3-carbonsäure, Hydroxyamid;
1-Methoxyacetyl-4-(4-[4-methoxybut-2-inyloxy]benzolsulfonyl)-7-methyl-2,3,4,5-tetrahydro-1H-[1,4]-benzodiazepin-3-carbonsäure, Hydroxyamid;
1-Benzoyl-4-(4-but-2-inyloxybenzolsulfonyl)-7-methyl-2,3,4,5-tetrahydro-1H-[1,4]-benzodiazepin-3-carbonsäure, Hydroxyamid;
1-(Benzoyl)-4-(4-but-2-inyloxybenzolsulfonyl)-8-chlor-2,3,4,5-tetrahydro-2H-[1,4]benzodiazepin-3-carbonsäure, Hydroxyamid; oder
1-Acetyl-4-{[4-(2-butinyloxy)phenyl]sulfonyl}-7-fluor-N-hydroxy-2,3,4,5-tetrahydro-1H-[1,4]-benzodiazepin-3-carboxamid.

8. Verfahren zum Herstellen einer Verbindung wie in Anspruch 1 beansprucht, welches eines der Folgenden umfasst:
a) Umsetzen einer Verbindung der Formel II: 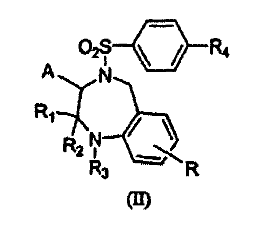 worin R, R₁, R₂, R₃ und R₄ wie oben definiert sind und A für COOH steht oder ein reaktives Derivat davon, mit einer Verbindung der Formel III
**NH**_{**2**}**OH (III)**
um eine entsprechende Verbindung der Formel I zu ergeben;
b) Trennen eines Gemisches (z.B. Racemat) aus optisch aktiven Isomeren einer Verbindung der Formel I, um ein Enantiomer oder Diastereomer im Wesentlichen frei vom anderen Enantiomer oder Diastereomer zu isolieren;
c) Säuern einer basischen Verbindung der Formel I mit einer pharmazeutisch annehmbaren Säure, um ein pharmazeutisch annehmbares Salz zu ergeben.

9. Pharmazeutische Zusammensetzung, welche eine Verbindung der Formel 1, wie in Anspruch 1 definiert, oder ein pharmazeutisch annehmbares Salz davon und einen pharmazeutisch annehmbaren Träger umfasst.

10. Verwendung einer Verbindung der Formel 1, wie in Anspruch 1 beansprucht, bei der Herstellung eines Medikaments zum Behandeln eines Krankheitszustandes, welcher durch Matrix-Metalloproteinase vermittelt wird, in einem Säuger, wobei besagter Zustand ausgewählt wird aus der Gruppe bestehend aus Tumorwachstum, Restenose, MMP-vermittelte Osteopenie, Entzündungserkrankungen des zentralen Nervensystems, Hautalterung, Osteoarthritis, Rheumatoidarthritis, septischer Arthritis, Kornea-Geschwürbildung, abnormer Wundheilung, Knochenerkrankung, Proteinurie, Aneurysma-Aorta-Erkrankung, degenerativem Knorpelverlust, Demyelinisierungserkrankung des Nervensystems, Leberzirrhose, glomerularer Erkrankung der Niere, vorzeitigem Riss fötaler Membranen, entzündlicher Darmerkrankung, Periodontalerkrankung, altersbezogener Makuladegeneration, diabetischer Retinopathie, proliferativer Vitreoretinopathie, Frühgeborenenretinopathie, okularer Entzündung, Keratoconus, Sjogren-Syndrom, Myopie, okularen Tumoren, okularer Angiogenese/Neo-Vaskularisation und kornealer Transplantatabstoßung.

11. Verwendung einer Verbindung der Formel I, wie in Anspruch 1 beansprucht, bei der Herstellung eines Medikaments zum Behandeln eines Patienten, der an einem Zustand leidet, ausgewählt aus der Gruppe bestehend aus Osteoarthritis, Rheumatoidarthritis, degenerativem Knorpelverlust und Tumorwachstum.

## Revendications

1. Composé de formule I : 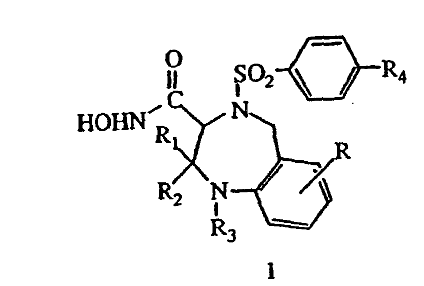 dans laquelle
R est sélectionné parmi hydrogène, alkyle en C₁-C₃, -CN, -OR', -SR', -CF₃, -OCF₃, Cl, F, NH₂, NH (alkyle en C₁-C₃), -N(R')CO(alkyle en C₁-C₃), -N(R') (R'), NO₂, -CONH₂, -SO₂NH₂, -SO₂N (R' ) (R'), -N(R') COCH₂O(alkyle en C₁-C₃), R' étant un alkyle en C₁-C₃ ou un hydrogène;
R₄ est un (alkyle en C₁-C₆)-O- contenant une triple liaison, 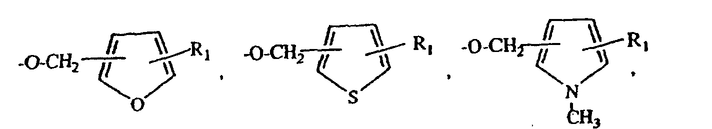 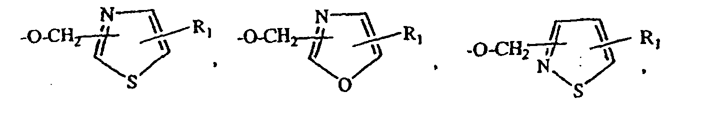 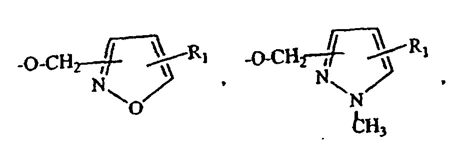 -O-CH₂-C≡C-R"
R" étant un hydrogène, -CH₂OH, alkyle en C₁-C₆, (alkyle. en C₁-C₆)-O-CH₂-, (alkyle en C₁-C₆)-S-CH₂-, (alkyle en C₁-C₆)-NH-CH₂-, [(alkyle en C₁-C₃)]₂-NCH₂-, (cycloalkyle en C₁-C₆)-O-CH₂-, [(alkyle en C₁-C₃) ] ₂-N-(CH₂) ₂₋₄NHCH₂-, [(alkyle en C₁-C₃) ] ₂-N-(CH₂) ₂₋₄N(CH₃)CH₂-, 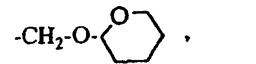 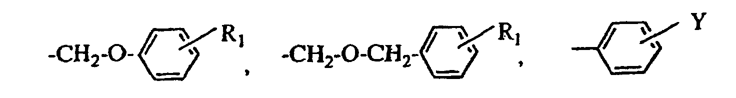
R₁ et R₂ sont chacun, indépendamment, hydrogène ou CH₃;
R₃ est alkyle en C₁-C₈, NH₂CH₂CO-, (alkyle en C₁-C₆) NHCH₂CO-, HO(CH₂)ₘCO-, HCO-, aryl (CH₂)ₙ CO-, hétéroaryl(CH₂)ₙCO-, (alkyle en C₁-C₃)-O-(CH₂)ₙCO-, (alkyle en C₁-C₃)CO-, (alkyle en C₁-C₃)CO-NHCH₂CO-, (cycloalkyle en C₃-C₇)CO-, (alkyle en C₁-C₃)SO₂-, aryl (CH₂) ₙSO₂-, hétéroaryl (CH₂) ₙSO₂-, (alkyle en C₁-C₃)-O-(CH₂)ₘ-SO₂-, (alkyle en C₁-C₃)-O-(CH₂)ₘ, (alkyle en C₁-C₃)-O-(alkyle en C₁-C₃)-O-(alkyle en C₁-C₃), HO-(alkyle en C₁-C₃)-O-(alkyle en C₁-C₃), aryl-O-CH₂CO-, hétéroaryl-O-CH₂CO-, arylCH=CHCO-, hétéroarylCH=CHCO-, (alkyle en C₁-C₃) CH=CHCO-, 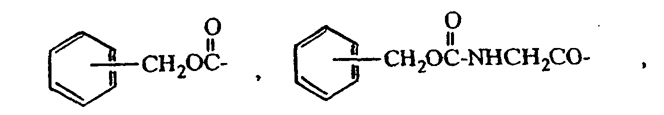 aryl (alkyle en C₁-C₃), hétéroaryl (alkyle en C₁-C₃), arylCH=CHCH₂-, hétéroarylCH=CHCH₂-, (alkyle en C₁-C₆) CH=CHCH₂-, 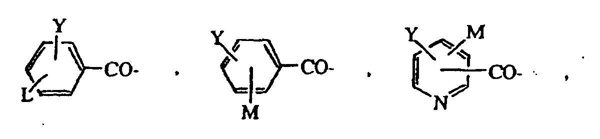 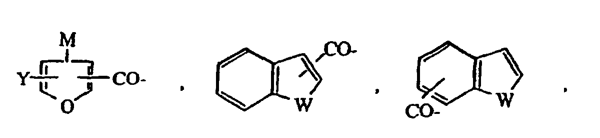 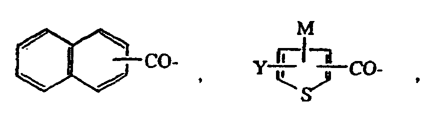 R' OCH₂CH ( OR' ) CO-, (R'OCH₂) ₂C (R') CO-, 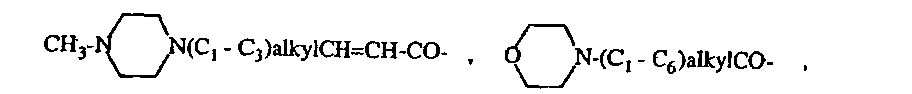 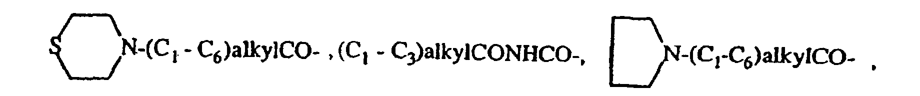 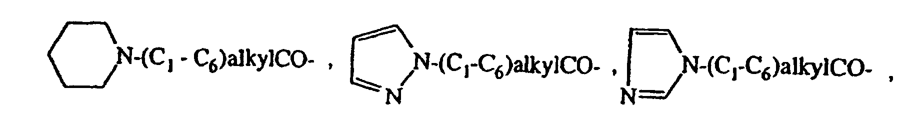 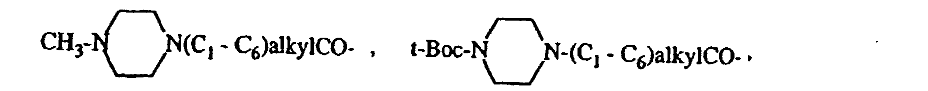 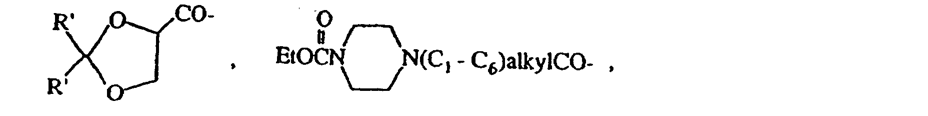 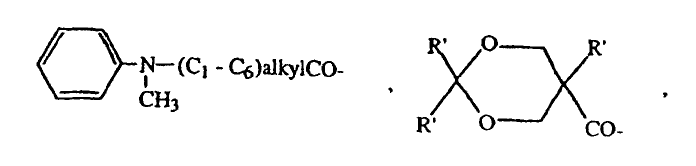 [(alkyle en C₁-C₆) ]₂-N-(alkyle en C₁-C₆) CO-, ou (alkyle en C₁-C₆)-NH-(alkyle en C₁-C₆) CO-,
dans lesquels
m = 1 à 3; n = 0 à 3;
aryle est 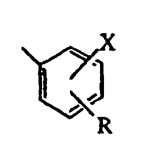 et
hétéroaryle est 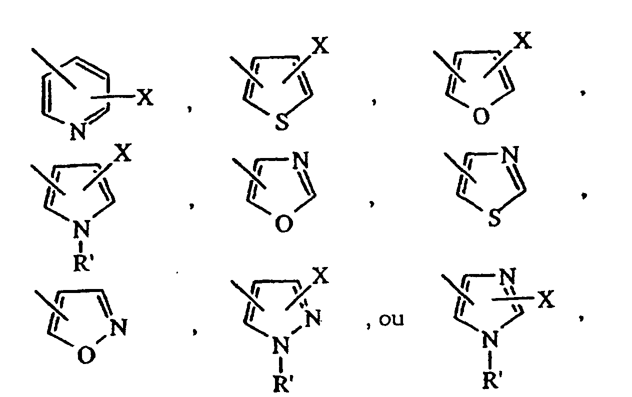
X étant hydrogène, halogène, alkyle en C₁-C₃ ou -OCH₃, et R et R' sont comme défini ci-dessus;
L est hydrogène, alkyle en C₁-C₃, -CN, -OR', -SR', -CF₃, -OCF₃, Cl, F, NH₂, -NH-(alkyle en C₁-C₃), -N(R' ) CO (alkyle en C₁-C₃), N(R') (R' ), -NO₂, -CONH₂, -SO₂NH₂, -SO₂N (R' ) (R' ), -N(R' ) COCH₂O(alkyle en C₁-C₃), 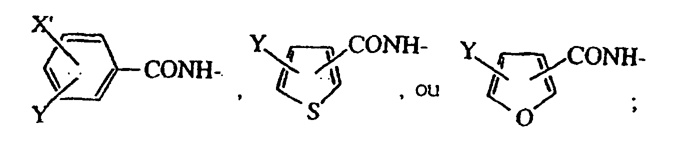
M est 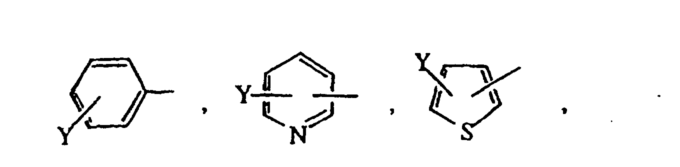 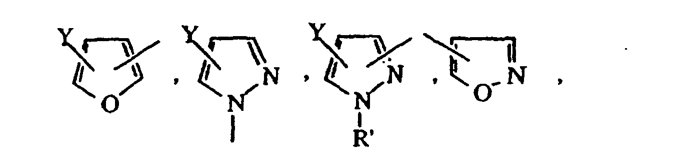 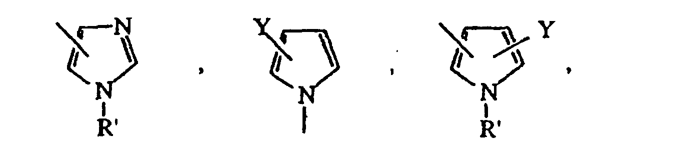 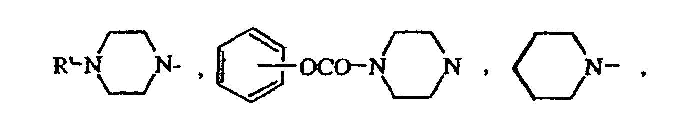 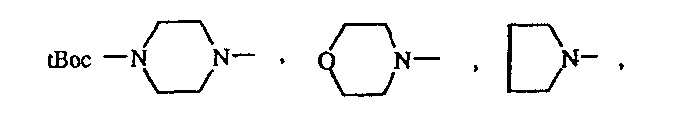 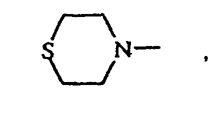
ou N(R')(R') dans lequel R' est comme défini ci-dessus;
W est O, S, NH ou N (alkyle en C₁-C₃) ;
Y est hydrogène, F, Cl, CF₃ ou OCH₃, et X' est halogène, hydrogène, alkyle en C₁-C₃, O-alkyle en C₁-C₃, ou -CH₂OH, et leurs sels pharmaceutiquement acceptables.

2. Composé suivant la revendication 1, dans lequel:
R est hydrogène, alkyle en C₁-C₃, -CN, -OR', -SR', -CF₃, -OCF₃, Cl, F, NH₂, NH(alkyle en C₁-C₃), -N(R')CO(alkyle en C₁-C₃), -N(R')(R'), NO₂, -CONH₂, -SO₂NH₂, -SO₂N(R')(R'), -N(R') COCH₂O-(alkyle en C₁-C₃), R' étant un alkyle en C₁-C₃ ou un hydrogène;
R₄ est un (alkyle en C₁-C₆)-O-contenant une triple liaison, 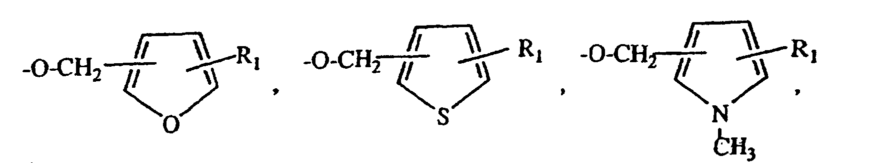 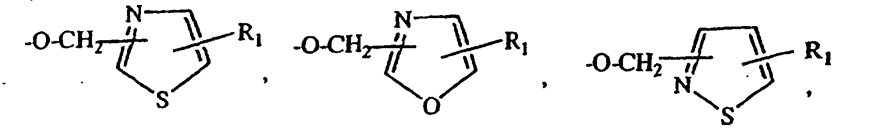 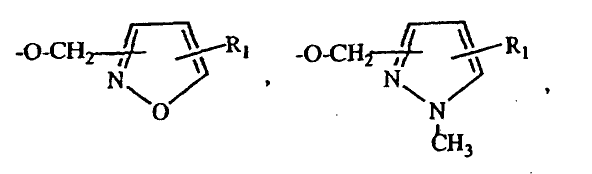 -O-CH₂-C ≡ C-R"
R" étant un hydrogène, -CH₂OH, alkyle en C₁-C₆, (alkyle en C₁-C₆)-O-CH₂-, (alkyle en C₁-C₆)-S-CH₂-, (alkyle en C₁-C₆)-NH-CH₂-, [(alkyle en C₁-C₃)]₂-NCH₂-, (cycloalkyle en C₁-C₆)-O-CH₂-, [(alkyle en C₁-C₃) ]₂-N-(CH₂) ₂₋₄NHCH₂-, [(alkyle en C₁-C₃) ]₂-N-(CH₂)₂₋₄N(CH₃)CH₂-, 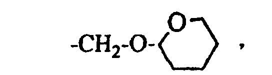 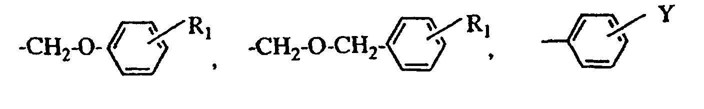
R₁ et R₂ sont chacun, indépendamment, hydrogène ou CH₃;
R₃ est alkyle en C₁-C₈, NH₂CH₂CO-, (alkyle en C₁-C₆) NHCH₂CO-, HO (CH₂) ₘCO-, HCO-, aryl (CH₂) ₙCO-, hétéroaryl (CH₂) ₙCO-, (alkyle en C₁-C₃)-O-(CH₂) ₙCO-, (alkyle en C₁-C₃) CO-, (alkyle en C₁-C₃) CO-NHCH₂CO-, (cycloalkyle en C₃-C₇) CO-, aryl-O-CH₂CO-, hétéroaryl-O-CH₂CO-, 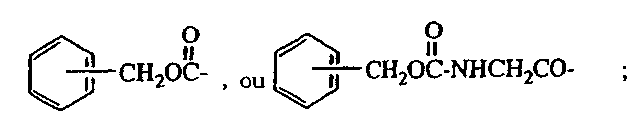
dans lesquels
m = 1 à 3; n = 0 à 3;
aryle est 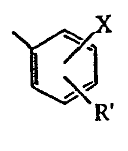 et
hétéroaryle est 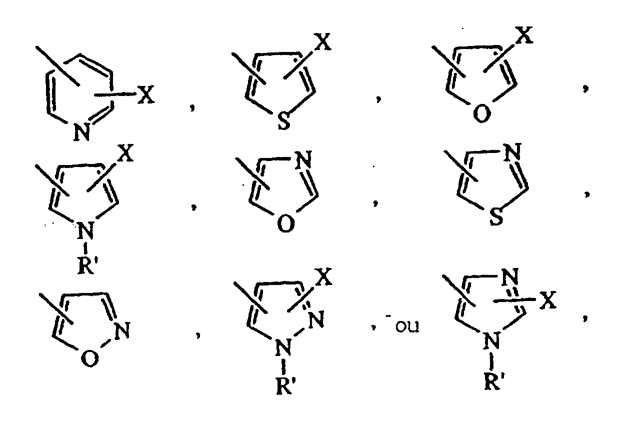
X étant hydrogène, halogène, alkyle en C₁-C₃ ou-OCH₃, et
R et R' sont comme défini ci-dessus, et leurs sels pharmaceutiquement acceptables.

3. Composé suivant la revendication 1, dans lequel
R est hydrogène, alkyle en C₁-C₃,-CN,-OR',-SR',-CF₃, -OCF₃, Cl, F, NH₂, NH(alkyle en C₁-C₃),-N (R') CO (alkyle en C₁-C₃),-N (R') (R'), NO₂,-CONH₂,-SO₂NH₂,-SO₂N (R') (R'), -N (R') COCH₂O-(alkyle en C₁-C₃), R' étant un alkyle en C₁-C₃ ou un hydrogène;
R₄ est un (alkyle en C₁-C₆)-O- contenant une triple liaison, 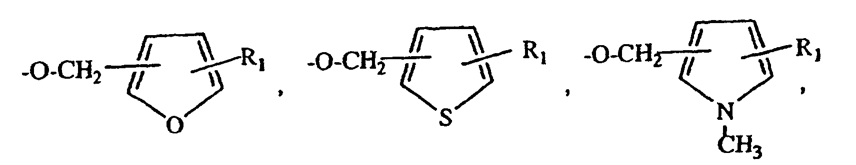 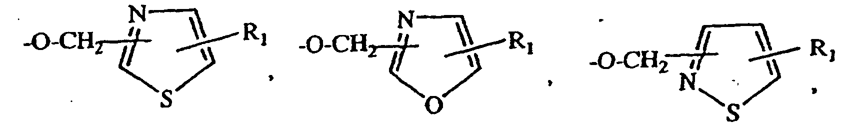 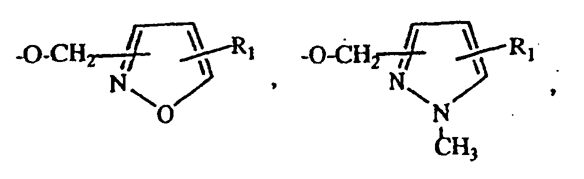 -O-CH₂-C ≡ C-R"
R" étant un hydrogène,-CH₂OH, alkyle en C₁-C₆, (alkyle en C₁-C₆)-O-CH₂-, (alkyle en C₁-C₆)-S-CH₂-, (alkyle en C₁-C₆)-NH-CH₂-, [(alkyle en C₁-C₃)]₂-NCH₂-, (cycloalkyle en C₁-C₆)-O-CH₂-, [(alkyle en C₁-C₃ ) ] ₂-N-(CH₂) ₂₋₄NHCH₂-, [ (alkyle en C₁-C₃)]₂-N-(CH₂)₂₋₄N(CH₃)CH₂-, 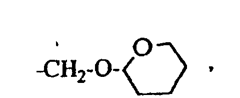 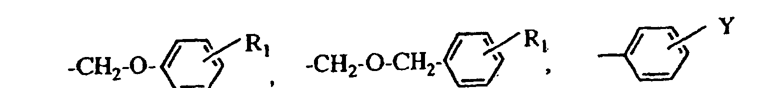
R₁ et R₂ sont chacun, indépendamment, hydrogène ou CH₃;
R₃ est 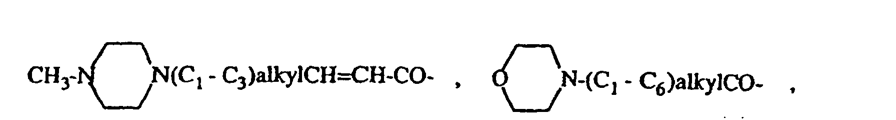 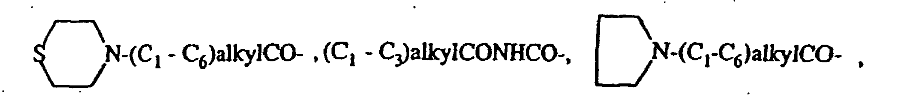 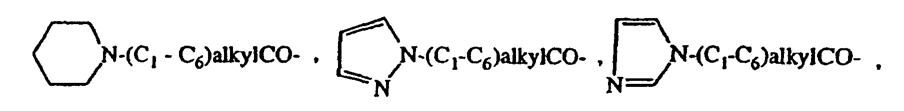 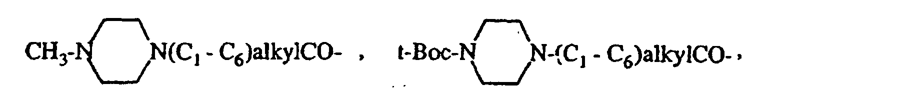 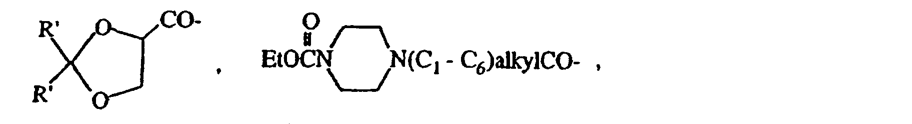 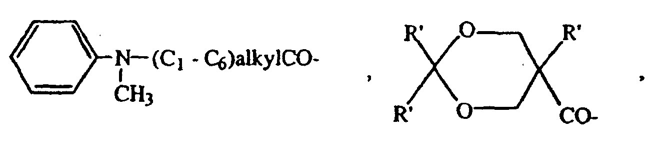 [(alkyle en C₁-C₆)]₂-N-(alkyle en C₁-C₆) CO-, ou (alkyle en C₁-C₆)-NH-(alkyle en C₁-C₆) CO-,
R' étant comme défini ci-dessus,
et leurs sels pharmaceutiquement acceptables.

4. Composé suivant la revendication 1, dans lequel:
R est hydrogène, alkyle en C₁-C₃,-CN,-OR',-SR',-CF₃, -OCF₃, Cl, F, NH₂, NH(alkyle en C₁-C₃), -N(R')CO(alkyle en C₁-C₃), -N (R' ) (R' ), NO₂,-CONH₂,-SO₂NH₂,-SO₂N (R' ) (R' ), -N(R')COCH₂O-(alkyle en C₁-C₃), R' étant un alkyle en C₁-C₃ ou un hydrogène;
R₄ est un (alkyle en C₁-C₆)-O- contenant une triple liaison, 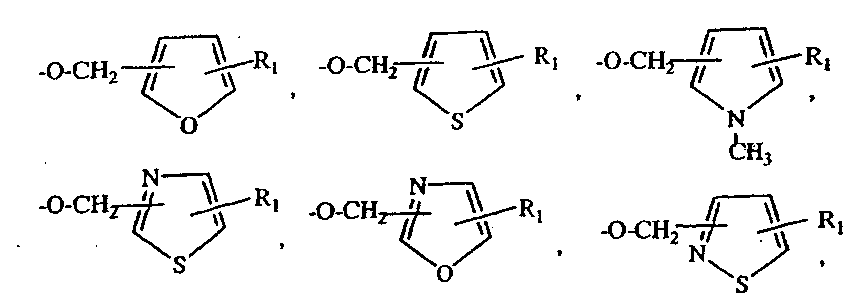 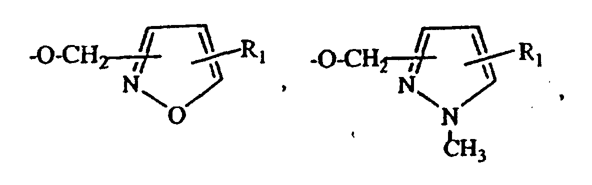 -O-CH₂-C ≡ C-R"
R" étant hydrogène,-CH₂OH, alkyle en C₁-C₆, (alkyle en C₁-C₆)-O-CH₂-, (alkyle en C₁-C₆)-S-CH₂-, (alkyle en C₁-C₆)-NH-CH₂-, [(alkyle en C₁-C₃)]₂-NCH₂-, (cycloalkyle en C₁-C₆)-O-CH₂-, [(alkyle en C₁-C₃)]₂-N-(CH₂)₂₋₄NHCH₂-, [(alkyle en C₁-C₃)]₂-N-(CH₂) ₂₋₄N(CH₃) CH₂-, 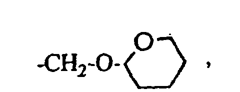 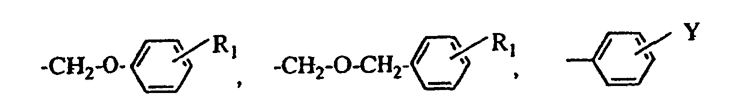
R₁ et R₂ sont chacun, indépendamment, hydrogène ou CH₃;
R₃ est (alkyle en C₁-C₃)-SO₂-, aryl (CH₂)ₙ SO₂-, hétéroaryl (CH₂)ₙ SO₂-, ou (alkyle en C₁-C₃)-O-(CH₂) ₘ-SO₂-,
dans lesquels
m = 1 à 3; n = 0 à 3;
aryle est 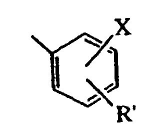 et
hétéroaryle est 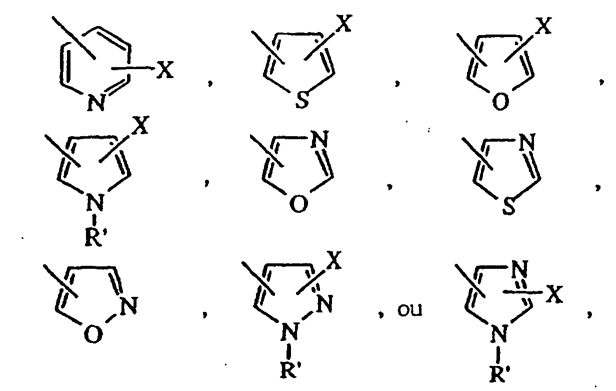
dans lesquels X est hydrogène, halogène, alkyle en C₁-C₃ ou-OCH₃, et R et R' sont comme défini ci-dessus, et leurs sels pharmaceutiquement acceptables.

5. Composé suivant la revendication 1, dans lequel:
R est hydrogène, alkyle en C₁-C₃,-CN,-OR',-SR',-CF₃, -OCF₃, Cl, F, NH₂, NH(alkyle en C₁-C₃),-N(R')CO(alkyle en C₁-C₃),-N(R')(R'), NO₂,-CONH₂,-SO₂NH₂,-SO₂N(R')(R'), -N(R')COCH₂O-(alkyle en C₁-C₃), R' étant un alkyle en C₁-C₃ ou un hydrogène;
R₄ est un (alkyle en C₁-C₆)-O-contenant une triple liaison,
-O-CH₂-C≡C-R"
R" étant hydrogène,-CH₂OH, alkyle en C₁-C₆, (alkyle en C₁-C₆)-O-CH₂-, (alkyle en C₁-C₆)-S-CH₂-, (alkyle en C₁-C₆)-NH-CH₂-, [(alkyle en C₁-C₃)]₂-NCH₂-, (cycloalkyle en C₁-C₆)-O-CH₂-, [(alkyle en C₁-C₃)]₂-N-(CH₂)₂₋₄NHCH₂-, [(alkyle en C₁-C₃)]₂-N-(CH₂)₂₋₄N(CH₃)CH₂-, 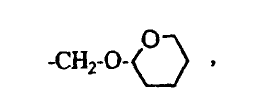 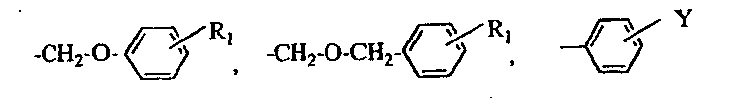
R₁ et R₂ sont chacun, indépendamment, hydrogène ou CH₃;
R₃ est HCO-, (alkyle en C₁-C₆)-CO-, aryl (alkyle en C₁-C₃) CO-, hétéroaryl (alkyle en C₁-C₃) CO-, (alkyle en C₁-C₃)-O-(CH₂)ₙCO-, HO(CH₂)ₘCO-, (cycloalkyle en C₁-C₇)-CO-,
dans lesquels
aryle est 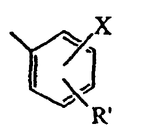 et
hétéroaryle est 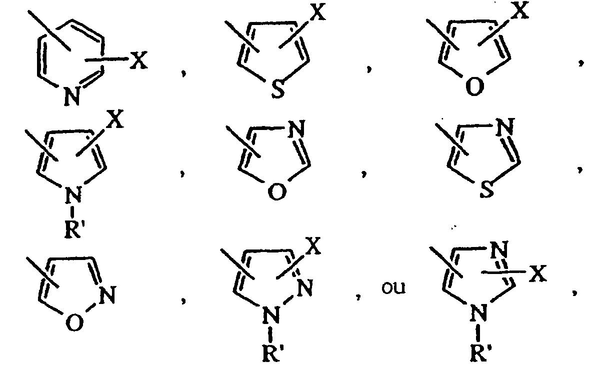
dans lesquels X est hydrogène, halogène, alkyle en C₁-C₃ ou -OCH₃, et R et R' sont comme défini ci-dessus, et leurs sels pharmaceutiquement acceptables.

6. Composé suivant la revendication 1, dans lequel:
R est hydrogène, alkyle en C₁-C₃,-CN,-OR',-SR',-CF₃, -OCF₃, Cl, F, NH₂, NH(alkyle en C₁-C₃), -N(R')CO(alkyle en C₁-C₃), -N(R') (R'), NO₂,-CONH₂,-SO₂NH₂,-SO₂N (R' ) (R'), -N(R')COCH₂O-(alkyle en C₁-C₃), R' étant un alkyle en C₁-C₃ ou un hydrogène;
R₄ est un (alkyle en C₁-C₆)-O- contenant une triple liaison,
-O-CH₂-C≡C-R"
R" étant hydrogène,-CH₂OH, alkyle en C₁-C₆, (alkyle en C₁-C₆)-O-CH₂-, (alkyle en C₁-C₆)-S-CH₂-, (alkyle en C₁-C₆)-NH-CH₂-, [(alkyle en C₁-C₃)]₂-NCH₂-, (cycloalkyle en C₁-C₆)-O-CH₂-, [(alkyle en C₁-C₃)]₂-N-(CH₂)₂₋₄NHCH₂-, [(alkyle en C₁-C₃) ]₂-N-(CH₂)₂₋₄N (CH₃) CH₂-, 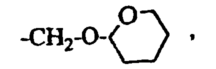 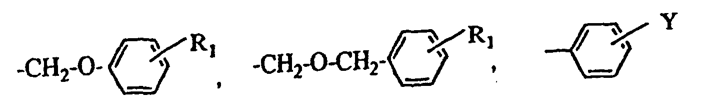
R₁ et R₂ sont chacun, indépendamment, hydrogène ou CH₃;
R₃ est 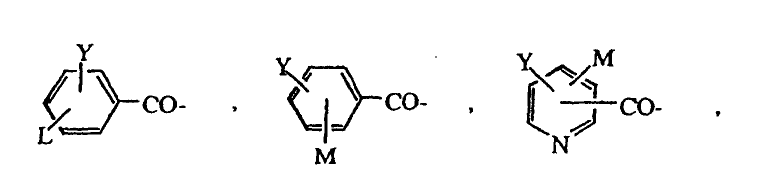 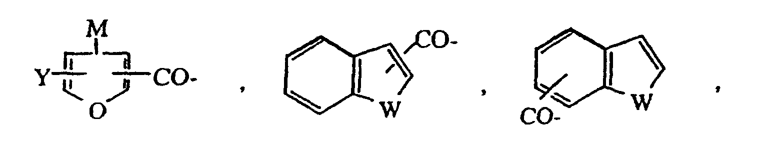 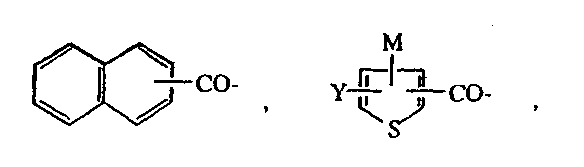
dans lesquels
m = 1 à 3; n = 0 à 3;
L est hydrogène, alkyle en C₁-C₃,-CN,-OR',-SR',-CF₃, -OCF₃, Cl, F, NH₂,-NH-(alkyle en C₁-C₃), -N(R')CO (alkyle en C₁-C₃), N (R' ) (R' ), -NO₂,-CONH₂,-SO₂NH₂,-SO₂N (R' ) (R' ), -N(R')OCH₂O-(alkyle en C₁-C₃), 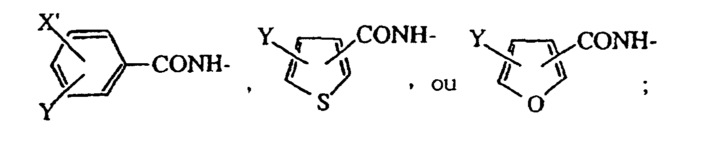
M est 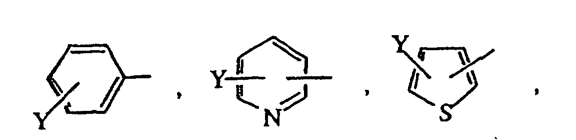 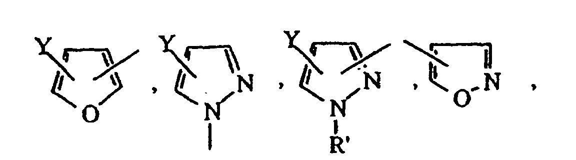 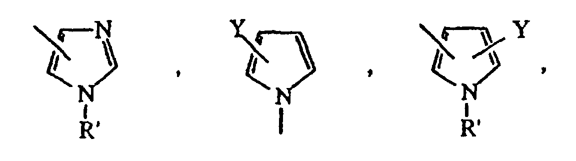 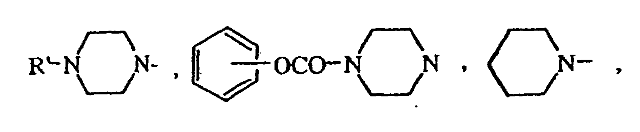 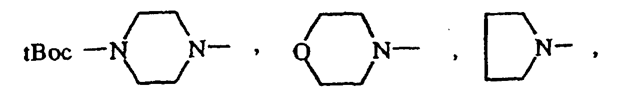 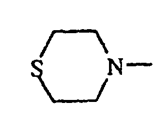
ou N(R')(R') dans lequel R' est comme défini ci-dessus;
W est O, S, NH ou N (alkyle en C₁-C₃) ;
Y est hydrogène, F, Cl, CF₃ ou OCH₃, et X' est halogène, hydrogène, alkyle en C₁-C₃, O-alkyle en C₁-C₃, ou-CH₂OH, et leurs sels pharmaceutiquement acceptables.

7. Composé suivant la revendication 1, qui est l'un des suivants:
l'hydroxamide de l'acide 1-acétyl-4-(4-but-2-ynyloxybenzènesulfonyl)-2,3,4,5-tétrahydro-1H-[1,4]benzodiazépine-3-carboxylique;
l'hydroxamide de l'acide 4-(4-but-2-ynyloxybenzènesulfonyl)-1-(2-thiénylcarbonyl)-2,3,4,5-tétrahydro-1H-[1,4]benzodiazépine-3-carboxylique;
l'hydroxamide de l'acide 1-benzoyl-4-(4-but-2-ynyloxybenzènesulfonyl)-2,3,4,5-tétrahydro-1H-[1,4]benzodiazépine-3-carboxylique;
l'hydroxamide de l'acide 4-(4-but-2-ynyloxybenzènesulfonyl)-1-(2-furannylcarbonyl)-2,3,4,5-tétrahydro-1H-[1,4]benzodiazépine-3-carboxylique;
l'hydroxamide de l'acide 4-(4-but-2-ynyloxybenzènesulfonyl)-1-(méthanesulfonyl)-2,3,4,5-tétrahydro-2H-[1,4]benzodiazépine-3-carboxylique;
l'hydroxamide de l'acide 4-(4-but-2-ynyloxybenzènesulfonyl)-1-méthoxyacétyl-2,3,4,5-tétrahydro-1H-[1,4]benzodiazépine-3-carboxylique;
l'hydroxamide de l'acide 4-(4-but-2-ynyloxybenzènesulfonyl)-1-(3-pyridinylcarbonyl)-2,3,4,5-tétrahydro-1H-[1,4]benzodiazépine-3-carboxylique;
l'hydroxamide de l'acide 4-(4-but-2-ynyloxybenzènesulfonyl)-1-(4-pyridinylcarbonyl)-2,3,4,5-tétrahydro-1H-[1,4]benzodiazépine-3-carboxylique;
l'hydroxamide de l'acide 1-benzoyl-4-[4-(4-méthoxybut-2-ynyloxy)benzènesulfonyl]-2,3,4,5-tétrahydro-1H-[1,4]benzodiazépine-3-carboxylique;
l'hydroxamide de l'acide 4-[4-(4-méthoxybut-2-ynyloxy)benzènesulfonyl]-1-(3-pyridinylcarbonyl)-2,3,4,5-tétrahydro-1H-[1,9]benzodiazépine-3-carboxylique;
l'hydroxamide de l'acide 4-(4-pent-2-ynyloxybenzènesulfonyl)-1-(3-pyridinylcarbonyl)-2,3,4,5-tétrahydro-1H-[1,4]benzodiazépine-3-carboxylique;
l'hydroxamide de l'acide 4-[4-(4-hydroxybut-2-ynyloxy)benzènesulfonyl]-1-(4-pyridinylcarbonyl)-2,3,4,5-tétrahydro-1H-[1,4]benzodiazépine-3-carboxylique;
l'hydroxamide de l'acide 4-[4-(4-méthoxybut-2-ynyloxy)benzènesulfonyl]-1-(2-thiénylcarbonyl)-2,3,4,5-tétrahydro-1H-[1,4]benzodiazépine-3-carboxylique;
l'hydroxamide de l'acide 1-(benzoyl)-4-(4-pent-2-ynyloxy-benzènesulfonyl)-2,3,4,5-tétrahydro-1H-[1,4]benzodiazépine-3-carboxylique;
l'hydroxamide de l'acide 1-propionyl-4-[4-(4-hydroxybut-2-ynyloxy)benzènesulfonyl]-2,3,4,5-tétrahydro-1H-[1,4]benzodiazépine-3-carboxylique;
l'hydroxamide de l'acide 1-(N,N-diméthylaminoacétyl)-4-[4-(4-méthoxybut-2-ynyloxy)benzènesulfonyl]-2,3,4,5-tétrahydro-1H-[1,4]benzodiazépine-3-carboxylique;
l'hydroxamide de l'acide 1-(acétylaminoacétyl)-4-(4-but-2-ynyloxybenzènesulfonyl)-2,3,4,5-tétrahydro-1H-[1,4]benzodiazépine-3-carboxylique;
l'hydroxamide de l'acide 1-(éthoxyacétyl)-4-[4-(4-méthoxybut-2-ynyloxy)benzènesulfonyl]-2,3,4,5-tétrahydro-1H-[1,4]benzodiazépine-3-carboxylique;
l'hydroxamide de l'acide 4-(4-but-2-ynyloxybenzènesulfonyl)-1-(3-thiénylcarbonyl)-2,3,4,5-tétrahydro-1H-[1,4]benzodiazépine-3-carboxylique;
l'hydroxamide de l'acide 1-(éthoxyacétyl)-4-[4-(4-éthoxybut-2-ynyloxy)benzènesulfonyl]-2,3,4,5-tétrahydro-1H-[1,4]benzodiazépine-3-carboxylique;
l'hydroxamide de l'acide 1-(acétylaminoacétyl)-4-[4-(4-méthoxybut-2-ynyloxy)benzènesulfonyl]-2,3,4,5-tétrahydro-1H-[1,4]benzodiazépine-3-carboxylique;
l'hydroxamide de l'acide 1-(cyclopropylcarbonyl)-4-[4-(4-méthoxybut-2-ynyloxy)benzènesulfonyl]-2,3,4,5-tétrahydro-1H-[1,4]benzodiazépine-3-carboxylique;
l'hydroxamide de l'acide 1-(cyclobutylcarbonyl)-4-(4-but-2-ynyloxybenzènesulfonyl)-2,3,4,5-tétrahydro-1H-[1,4]benzodiazépine-3-carboxylique;
l'hydroxamide de l'acide 4-(4-but-2-ynyloxybenzènesulfonyl)-1-(propionyl)-2,3,4,5-tétrahydro-1H-[1,4]benzodiazépine-3-carboxylique;
l'hydroxamide de l'acide 4-[4-(4-méthoxybut-2-ynyloxy)benzènesulfonyl]-1-(3-méthyl-2-thiénylcarbonyl)-2,3,4,5-tétrahydro-1H-[1,4]benzodiazépine-3-carboxylique;
l'hydroxamide de l'acide 4-(4-but-2-ynyloxybenzènesulfonyl)-1-(3-méthoxypropionyl)-2,3,4,5-tétrahydro-1H-[1,4]benzodiazépine-3-carboxylique;
l'hydroxamide de l'acide 4-(4-but-2-ynyloxybenzènesulfonyl)-1-(2-chlorobenzoyl)-2,3,4,5-tétrahydro-1H-[1,4]benzodiazépine-3-carboxylique;
l'hydroxamide de l'acide 4-(4-but-2-ynyloxybenzènesulfonyl)-1-(2-fluorobenzoyl)-2,3,4,5-tétrahydro-1H-[1,4]benzodiazépine-3-carboxylique;
l'hydroxamide de l'acide 4-(4-but-2-ynyloxybenzènesulfonyl)-1-(4-méthyl-2-furannylcarbonyl)-2,3,4,5-tétrahydro-1H-[1,4]benzodiazépine-3-carboxylique;
l'hydroxamide de l'acide 4-(4-but-2-ynyloxybenzènesulfonyl)-1-(3-furannylcarbonyl)-2,3,4,5-tétrahydro-1H-[1,4]benzodiazépine-3-carboxylique;
l'hydroxamide de l'acide 4-(4-but-2-ynyloxybenzènesulfonyl)-1-(phénoxyacétyl)-2,3,4,5-tétrahydro-1H-[1,4]benzodiazépine-3-carboxylique;
l'hydroxamide de l'acide 4-(4-but-2-ynyloxybenzènesulfonyl)-1-[2-(1-pyrazolyl)phénylcarbonyl]-7-méthyl-2,3,4,5-tétrahydro-1H-[1,4]benzodiazépine-3-carboxylique;
l'hydroxamide de l'acide 4-(4-but-2-ynyloxybenzènesulfonyl)-1-(5-chloro-2-thiénylcarbonyl)-2,3,4,5-tétrahydro-1H-[1,4]benzodiazépine-3-carboxylique;
l'hydroxamide de l'acide 4-(4-but-2-ynyloxybenzènesulfonyl)-1-(5-chloro-2-furannylcarbonyl)-2,3,4,5-tétrahydro-1H-[1,4]benzodiazépine-3-carboxylique;
l'hydroxamide de l'acide 4-[4-(4-méthoxybut-2-ynyloxy)benzènesulfonyl]-2-propionyl-2,3,4,5-tétrahydro-1H-[1,4]benzodiazépine-3-carboxylique;
l'hydroxamide de l'acide 4-[4-(4-méthoxybut-2-ynyloxy)benzènesulfonyl]-1-(3-thiénylcarbonyl)-2,3,4,5-tétrahydro-1H-[1,4]benzodiazépine-3-carboxylique;
l'hydroxamide de l'acide 1-(aminoacétyl)-4-(4-but-2-ynyloxybenzènesulfonyl)-2,3,4,5-tétrahydro-1H-[1,4]benzodiazépine-3-carboxylique;
l'hydroxamide de l'acide 1-hexanoyl-4-[4-(4-méthoxybut-2-ynyloxy)benzènesulfonyl]-2,3,4,5-tétrahydro-1H-[1,4]benzodiazépine-3-carboxylique;
l'hydroxamide de l'acide 4-(4-but-2-ynyloxybenzènesulfonyl)-1-(N,N-diméthylaminoacétyl)-2,3,4,5-tétrahydro-1H-[1,4]benzodiazépine-3-carboxylique;
l'hydroxamide de l'acide 4-(4-but-2-ynyloxybenzènesulfonyl)-1-(cyclopropylcarbonyl)-2,3,4,5-tétrahydro-1H-[1,4]benzodiazépine-3-carboxylique;
l'hydroxamide de l'acide 4-(4-but-2-ynyloxybenzènesulfonyl)-1-(cyclohexylcarbonyl)-2,3,4,5-tétrahydro-1H-[1,4]benzodiazépine-3-carboxylique;
l'hydroxamide de l'acide 1-méthoxyacétyl-4-[4-(4-méthoxybut-2-ynyloxy)benzènesulfonyl]-7-méthyl-2,3,4,5-tétrahydro-1H-[1,4]benzodiazépine-3-carboxylique;
l'hydroxamide de l'acide 1-benzoyl-4-(4-but-2-ynyloxybenzènesulfonyl)-7-méthyl-2,3,4,5-tétrahydro-1H-[1,4]benzodiazépine-3-carboxylique;
l'hydroxamide de l'acide 1-(benzoyl)-4-(4-but-2-ynyloxybenzènesulfonyl)-8-chloro-2,3,4,5-tétrahydro-1H-[1,4]benzodiazépine-3-carboxylique, ou
le 1-acétyl-4-{[4-(2-butynyloxy)phényl]sulfonyl}-7-fluoro-N-hydroxy-2,3,4,5-tétrahydro-1H-[1,4]benzodiazépine-3-carboxamide.

8. Procédé de préparation d'un composé suivant la revendication 1, qui comprend l'une des suivantes:
a) réaction d'un composé de formule II: 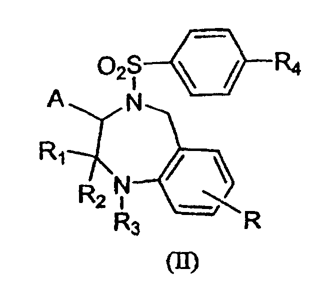 dans laquelle R, R₁, R₂, R₃ et R₄ sont comme défini ci-dessus, et A est COOH ou un dérivé réactif de celui-ci, avec un composé de formule III
NH₂OH (III)
pour donner un composé correspondant de formule I;
b) résolution d'un mélange (par exemple un racémate) d'isomères optiquement actifs d'un composé de formule I pour isoler un énantiomère ou diastéréoisomère pratiquement débarrassé de l'autre énantiomère ou des autres diastéréoisomères;
c) acidification d'un composé basique de formule I avec un acide pharmaceutiquement acceptable pour donner un sel pharmaceutiquement acceptable.

9. Composition pharmaceutique comprenant un composé de formule I suivant la revendication 1 ou un sel pharmaceutiquement acceptable de celui-ci et un vecteur pharmaceutiquement acceptable.

10. Utilisation d'un composé de formule I suivant la revendication 1 dans la préparation d'un médicament destiné à traiter un état pathologique médié par une métalloprotéinase de matrice chez un mammifère, ledit état pathologique étant sélectionné dans le groupe composé de la croissance tumorale, de la resténose, des ostéopénies médiées par les MMP, des maladies inflammatoires du système nerveux central, du vieillissement de la peau, de l'ostéoarthrite, de la polyarthrite rhumatoïde, de l'arthrite septique, de l'ulcération de la cornée, de la cicatrisation anormale, des maladies osseuses, de la protéinurie, de la maladie aortique aneurysmique, de la perte dégénérative de cartilage, des maladies de démyélinisation du système nerveux, de la cirrhose hépatique, de la maladie glomérulaire du rein, de la rupture prématurée des membranes foetales, de la maladie inflammatoire des intestins, des maladies périodontiques, de la dégénérescence maculaire liée au vieillissement, de la rétinopathie diabétique, de la vitrorétinopathie proliférative, de la rétinopathie du prématuré, des inflammations oculaires, du kératocône, du syndrome de Sjögren, de la myopie, des tumeurs oculaires, de l'angiogenèse/néovascularisation oculaire et du rejet des greffes cornéennes.

11. Utilisation d'un composé de formule I suivant la revendication 1 dans la préparation d'un médicament destiné à traiter un patient souffrant d'une affection sélectionnée dans le groupe composé de l'ostéoarthrite, de la polyarthrite rhumatoïde, de la perte dégénérative de cartilage et de la croissance tumorale.
